(19) Europäisches Patentamt / European Patent Office / Office européen des brevets

(11) **EP 3 695 783 A1**

(12) **EUROPEAN PATENT APPLICATION**

(43) Date of publication:
**19.08.2020 Bulletin 2020/34**

(51) Int Cl.:
*A61B 5/11* *(2006.01)*　　　*G01S 7/41* *(2006.01)*
*G01S 13/56* *(2006.01)*　　*G06K 9/00* *(2006.01)*
*H04L 29/08* *(2006.01)*　　*G01S 13/66* *(2006.01)*
*G01S 13/88* *(2006.01)*　　*G08B 13/181* *(2006.01)*

(21) Application number: **20157771.5**

(22) Date of filing: **17.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.02.2019 US 201962806688 P**
　　　　　　**15.02.2019 US 201962806694 P**
　　　　　　**12.05.2019 US 201962846688 P**
　　　　　　**12.07.2019 US 201962873781 P**
　　　　　　**15.09.2019 US 201962900565 P**
　　　　　　**18.09.2019 US 201962902357 P**
　　　　　　**18.12.2019 US 201962950093 P**
　　　　　　**13.02.2020 US 202016790610**
　　　　　　**13.02.2020 US 202016790627**

(71) Applicant: **Origin Wireless, Inc.**
**Greenbelt, MD 20770 (US)**

(72) Inventors:
 • **WU, Chenshu**
  **Greenbelt, MD 20770 (US)**
 • **ZHANG, Feng**
  **Greenbelt, MD 20770 (US)**

 • **WANG, Beibei**
  **Clarksville, MD 21029 (US)**
 • **HU, Yuqian**
  **College Park, MD 20740 (US)**
 • **LIU, K. J. Ray**
  **Potomac, MD 20854 (US)**
 • **AU, Oscar Chi-Lim**
  **San Jose, CA 95136 (US)**
 • **ZENG, Xiaolu**
  **Greenbelt, MD 20770 (US)**
 • **LAI, Hung-Quoc Duc**
  **Parkville, MD 21234 (US)**
 • **CLAFFEY, David N**
  **Somerville, MA 02143 (US)**
 • **LEE, Jeng-Feng**
  **Cambridge, MA 02139 (US)**
 • **MAI, Chao-Lun**
  **Cambridge, MA 02139 (US)**
 • **WU, Zhung-Han**
  **College Park, MD 20740 (US)**
 • **BUGOS, Dan**
  **Washington, DC 20010 (US)**
 • **SHIH, Chun-Chia Jack**
  **Hanover, MD 21076 (US)**

(74) Representative: **Boult Wade Tennant LLP**
**Salisbury Square House**
**8 Salisbury Square**
**London EC4Y 8AP (GB)**

(54) **METHOD, APPARATUS, AND SYSTEM FOR WIRELESS GAIT RECOGNITION**

(57)　Methods, apparatus and systems for wireless gait recognition are described. In one example, a described system comprises: a transmitter, a receiver, and a processor. The transmitter is configured for transmitting a first wireless signal towards an object in a venue through a wireless multipath channel of the venue. The receiver is configured for: receiving a second wireless signal through the wireless multipath channel between the transmitter and the receiver. The second wireless signal differs from the first wireless signal due to the wireless multipath channel which is impacted by a rhythmic motion of the object. The processor is configured for: obtaining a time series of channel information (CI) of the wireless multipath channel based on the second wireless signal, monitoring the rhythmic motion of the object based on the time series of CI (TSCI), and triggering a response action based on a result of the monitoring.

EP 3 695 783 A1

FIG. 1B

**Description**

**CROSS-REFERENCE TO RELATED APPLICATIONS**

The present application hereby incorporates by reference the entirety of the disclosures of, and claims priority to, each of the following cases:

(a) U.S. Provisional Patent application 62/806,688, entitled "METHOD, APPARATUS, AND SYSTEM FOR WIRELESS GAIT RECOGNITION", filed on February 15, 2019,
(b) U.S. Provisional Patent application 62/806,694, entitled "METHOD, APPARATUS, AND SYSTEM FOR OUTDOOR TARGET TRACKING", filed on February 15, 2019,
(c) U.S. Provisional Patent application 62/846,688, entitled "Method, Apparatus, and System for Processing and Presenting Life Log based on a Wireless Signal", filed on May 12, 2019,
(d) U.S. Provisional Patent application 62/873,781, entitled "METHOD, APPARATUS, AND SYSTEM FOR IMPROVING TOPOLOGY OF WIRELESS SENSING SYSTEMS", filed on July 12, 2019,
(e) U.S. Provisional Patent application 62/900,565, entitled "QUALIFIED WIRELESS SENSING SYSTEM", filed on September 15, 2019,
(f) U.S. Provisional Patent application 62/902,357, entitled "METHOD, APPARATUS, AND SYSTEM FOR AUTOMATIC AND OPTIMIZED DEVICE-TO-CLOUD CONNECTION FOR WIRELESS SENSING", filed on September 18, 2019,
(g) U.S. Provisional Patent application 62/950,093, entitled "METHOD, APPARATUS, AND SYSTEM FOR TARGET POSITIONING", filed on December 18, 2019,
(h) U.S. Patent application 16/790,610, entitled "METHOD, APPARATUS, AND SYSTEM FOR WIRELESS GAIT RECOGNITION", filed on February 13, 2020,
(i) U.S. Patent application 16/790,627, entitled "METHOD, APPARATUS, AND SYSTEM FOR OUTDOOR TARGET TRACKING", filed on February 13, 2020.

**TECHNICAL FIELD**

The present teaching generally relates to gait recognition. More specifically, the present teaching relates to: identifying an object or an individual in an indoor environment by recognizing its rhythmic motion, e.g. a gait, based on wireless channel information; and localizing and tracking targets outdoor, based on wireless channel information obtained from a massive multiple-input multiple-output (MIMO) system.

**BACKGROUND**

Gait, an individual's way of walking, is increasingly perceived as not only an essential vital sign but also an effective biometric marker. On one hand, gait, in particular the walking speed, is considered as a valid and sensitive measure appropriate for monitoring and assessing functional decline and general health, leading to its designation as the sixth vital sign. Gait reflects both functional and physiological changes, and has been shown to be indicative and predictive of a number of health status, including mobility disability, response to rehabilitation, falls, and cognitive decline, etc. Progression of gait is related to clinically meaningful changes in life quality and health condition. Therefore, continuous monitoring of gait at home, rather than occasionally in-hospital clinical testing, is of great interest for individual's healthcare.
On the other hand, gait provides distinctive biometric features of an individual, underlying a promising way of human identification. As a complex functional activity, many factors influence one's gait, rendering it as a unique behavioral trait. Research has shown that gait recognition could be even more reliable than face recognition because there are tens of identifying characteristics entangled in gait, making it extremely difficult, if possible, to impersonate someone else's walking patterns. Compared with other human recognition systems, gait recognition is particularly attractive since it can operate remotely, passively, and non-intrusively, without any active cooperation of individuals. Gait refers to the way or manner of walking (e.g. of a person with two feet, or an animal with two, four or multiple feet). Walking is a simple yet finely choreographed function, harmonizing many muscles over a complex bone and joint structure to deliver biomechanical locomotion. A person's gait cycle comprises two phases: the stance and swing phases, and further seven stages. The stance phase starts with the initial heel contact of one foot and ends when the same foot's toe leaves off the ground. The swing phase follows immediately with the action of the leg swinging forward and lasts until next heel contact.
For a 2-legged animals (e.g. human, or bird), the two feet may move with alternate motions. When the left foot touches the ground, the right foot may be swinging forward, and vice versa. For 4-legged animals (e.g. horse, cat, dog), in one gait, the front two legs (and/or the back two legs) may have alternate motions (e.g. when a horse trots). In another gait, the front two legs (and/or the back two legs) may touch the ground contemporaneously, or swing forward contemporaneously (e.g. when a horse gallops). For multi-legged creatures (e.g. caterpillar), the gait patterns may be even more complicated. For example, successive pairs of legs may move in coordinated manner (e.g. in wavefronts). The two legs in each pair may have varying degree of phase difference/lag. Gait may serve as a vital sign as well as a biometric cue. Gait has been shown to reflect health and functional status and can be indicative and predictive of a number of health status including mobility disability, response to rehabilitation, falls, and cognitive decline, etc. Progression of gait is related to clinically meaningful changes in life quality and health condition. Therefore, continuous monitoring of gait at home, rather than occasional in-hospital clinical testing, is of great interest for the well-being and health care of individuals, especially for care-givers (e.g. spouse, children, relatives, friends, long term care provider). Gait speed, also often termed walking speed, is the most important information being measured and concerned for healthcare. It has been recommended as a pragmatic and essential clinical indicator of well-being.

There are some existing non-wireless gait measurement and recognition systems based on cameras, floor sensors, and/or wearable sensors (e.g. accelerometer) to capture gait-related information. There are some disadvantages of the existing non-wireless systems. First, typically in these systems, the target subjects (e.g. the person) must cooperate, e.g. walk in a particular direction, in a designated path, or in a certain manner. Second, only certain restricted areas can be monitored by the existing non-wireless systems, e.g. an instrumented walkway with floor sensors installed in/under a floor mat, or an area covered by an installed cameras. The restricted area is very small and must be within a short distance in line-of-sight (LOS) of the instruments. Such systems are thus not suitable for unrestricted areas in daily usage (e.g. the person's home, or a mall, or a station, or an elderly care facility). They are not convenient and/or comfortable enough for ubiquitous applications in smart homes and smart buildings. Third, the equipment may be too expensive and the installation may be too labor intensive if the areas are to be instrumented for gait monitoring using the existing systems. Fourth, camera systems cause privacy problems to users. Fifth, floor sensors require significant installation effort and hardware cost. Sixth, wearables are useless if the person forgets or avoids wearing them to be monitored.

There are some existing wireless gait monitoring systems based on Doppler effect and radar. Disadvantages of existing wireless/RF based systems include: (a) some system needs specialized hardware which are expensive and hard to maintain; (b) some system requires very large bandwidth; (c) some requires special phased antennas; (d) Doppler-based systems reflect only partial speed projected on a specific direction rather than the entire speed; (e) Doppler-based system can only operate in a narrow LOS area (typically within 4 to 5 meters); (f) some systems measure features that are only remotely related to gait; (g) the features may be location/venue dependent, i.e. they may work in one venue, but not in another venue, thus requiring retraining for every different location.

Target localization and tracking including indoor and outdoor have been the interest of corresponding researchers over several decades because of their crucial roles in modern navigation and search-rescue system. In general, accurate indoor localization system can greatly improve people's life such as navigating a passenger to the airport gate, helping customers to find their favorite items in a big mall. Moreover, it can also guide the programmed robots to move heavy objects to our desirable destination, which can not only emancipate people from the boring and time wasting work but also greatly improve the efficiency of modern automatic production line. On the other hand, a reliable outdoor location system such as the most famous global positioning system (GPS) has been widely used in civil, military and commercial applications all over the world. However, GPS requires an unobstructed line-of-sight (LOS) path to at least four GPS satellites to compute the location of corresponding targets. GPS resource which can be utilized by civil and commercial services are strictly limited. Thus, it only provides 10 meters accuracy in daily activities although centimeter.

Generally, outdoor localization method use direction-of-arrival (DOA) to measure the bearing while time-of-arrival (TOA) to compute the range of the target with respect to the receiver. Evidently, they require accurate time measurements which are very sensitive to the distortion and noise in practice. Moreover, outdoor localization performance based on DOA and AOA is also strongly limited by its angular resolution which is related to the aperture, dimension and elements of the antenna deployed. Existing methods computed the source location from the data directly. To get a more accurate result, they often call for data association or center fusion process, which turns to be an NP-hard problem usually. Finding the optimal solution for such a kind of questions is either computation prohibited or requiring special devices.

Recently, a fifth regeneration (5G) technology called massive MIMO is introduced to mainly focus on communication related problems such as spectral efficiency, resource allocation, communication complexity, inner user interference, channel capacity and estimation. However, related work about how to use massive MIMO to develop an efficient way for outdoor target localization and tracking is still open. Navigation systems have been widely used in modern applications, among which GPS is the most popular one. However, GPS cannot work well in non-line-of-sight (NLOS) situation because of its requirement of an unobstructed line-of-sight (LOS) to four or more GPS satellites. As a result, inertial navigation system (INS) has been regarded as a supplement of GPS because it is a self-contained navigation technique. In an INS, moving speed and direction estimations are necessary to dead reckon the position of a moving object. As a result, how to estimate the moving speed and direction of a target has also been studied.

Accelerometer, gyroscope and magnetometer are the three most commonly used sensors in an INS. In general, INS adopts certain data fusion methods to jointly use the information extracted from different sensors to estimate the moving speed and direction of the target. They can be accurate when the target is relatively stable. However, they suffer from the unavoidable mechanical resistance or magnetic interference, which causes accumulative errors from the truth, especially over a long time.

Vision/image based method aided by camera devices is another kind of popular ways to detect the moving speed and direction for metro vehicles. For example, the continuous image sequences of the road surface texture are analyzed to get the vehicle speed and direction estimations. To solve the high frame rate requirement, two parallel vehicle-borne devices are adopted to take the images simultaneously. Then, the vehicle speed and moving direction are extracted by image matching and parameter calibration schemes. Although those vision based methods can achieve good accuracy after rounds of improvement, their requirements of sufficient image resolution and computational power becomes a bottleneck in real-time applications.

In addition, a fifth regeneration (5G) network will be deployed with ultra-wide bandwidths at Giga-Hertz frequency and large antenna arrays to offer Giga-Hertz data rates. But no effective positioning method has been disclosed for 5G networks. Thus, existing systems and methods for target positioning and tracking are not entirely satisfactory, and it is desirable for supplementary technologies that can enable high accuracy outdoor localization.

**SUMMARY**

The present teaching generally relates to a system for monitoring rhythmic motions like a gait. In one embodiment, the present teaching relates to a system for monitoring gait based on wireless signals and channel information of a wireless multipath channel that is impacted by the gait motion. The system may also recognize the gait, to identify and verify an individual accordingly. In another embodiment, the present teaching discloses localizing and tracking a target outdoor, based on wireless channel information obtained from a massive multiple-input multiple-output (MIMO) system.

In one embodiment, a system for rhythmic motion monitoring is described. The system comprises: a transmitter, a receiver, and a processor. The transmitter is configured for transmitting a first wireless signal towards an object in a venue through a wireless multipath channel of the venue. The receiver is configured for: receiving a second wireless signal through the wireless multipath channel between the transmitter and the receiver. The second wireless signal differs from the first wireless signal due to the wireless multipath channel which is impacted by a rhythmic motion of the object. The processor is configured for: obtaining a time series of channel information (CI) of the wireless multipath channel based on the second wireless signal, monitoring the rhythmic motion of the object based on the time series of CI (TSCI), and triggering a response action based on a result of the monitoring. According to various embodiments, the processor may be physically coupled to at least one of the transmitter and the receiver.

In another embodiment, a described apparatus for rhythmic motion monitoring is in a venue where a transmitter and a receiver are located. The described apparatus comprises: a processor and at least one of the transmitter and the receiver. The transmitter is configured for transmitting a first wireless signal through a wireless multipath channel of the venue. The receiver is configured for receiving a second wireless signal through the wireless multipath channel. The second wireless signal differs from the first wireless signal due to the wireless multipath channel which is impacted by a rhythmic motion of an object in the venue. The processor is configured for: obtaining a time series of channel information (CI) of the wireless multipath channel based on the second wireless signal, monitoring the rhythmic motion of the object based on the time series of CI (TSCI), and triggering a response action based on a result of the monitoring.

In one embodiment, the apparatus includes the receiver but not the transmitter. The receiver receives the second wireless signal and extracts the CI, e.g. a channel state information (CSI), for performing the rhythmic motion monitoring. In another embodiment, the apparatus includes the transmitter but not the receiver. The CSI is extracted by the receiver and obtained by the processor for rhythmic motion monitoring. In still another embodiment, the apparatus includes the transmitter but not the receiver. The CSI is extracted at the receiver that sends the CSI to the transmitter. The rhythmic motion monitoring is performed at the transmitter.

In a different embodiment, a method, implemented by a processor, a memory communicatively coupled with the processor, and a set of instructions stored in the memory to be executed by the processor, is described. The method comprises: obtaining a time series of channel information (CI) of a wireless multipath channel of a venue. A transmitter transmits a first wireless signal towards an object in a venue through the wireless multipath channel of the venue. A receiver receives a second wireless signal through the wireless multipath channel and computes the time series of CI (TSCI) of the wireless multipath channel based on the second wireless signal. The second wireless signal differs from the first wireless signal due to the wireless multipath channel which is impacted by a rhythmic motion of the object in the venue. The method further comprises: monitoring the rhythmic motion of the object based on the TSCI; and triggering a response action based on a result of the monitoring.

In one embodiment, a tracking system is described. The tracking system comprises: a transmitter, a receiver, and a processor. The transmitter is configured for transmitting a first wireless signal through a wireless multipath channel. The receiver is configured for receiving a second wireless signal through the wireless multipath channel between the transmitter and the receiver. One of the transmitter and the receiver is a located device at a known location. The other of the transmitter and the receiver is a moving device. The second wireless signal differs from the first wireless signal due to the wireless multipath channel which is impacted by a movement of the moving device. At least one of the transmitter and the receiver comprises a number of antennas. The number is larger than a threshold. The processor is configured for: obtaining a plurality of time series of channel information (CI) of the wireless multipath channel based on the second wireless signal, computing a spatial-temporal information (STI) of the moving device based on at least one of: the plurality of time series of CI (TSCI) and a past STI, and tracking the moving device based on the STI. According to various embodiments, the processor may be physically coupled to at least one of the transmitter and the receiver.

In one embodiment, an additional transmitter may be installed at a reset location (e.g. an entrance, or at the top of a door frame) where it repeatedly broadcast an additional wireless signals into a particular area (e.g. the door opening). The additional wireless signals serve like a beacon from a lighthouse. Any receiver receiving the beacon signal knows where it is.

A location is near the target area and adaptively determined based on at least one of: a height of the moving device and a likelihood of the height. The additional transmitter may be at the top of a door frame aiming the beacon signal downward. The coverage area is basically a beam or a cone, which has a relatively large uncertainty of the location of the receiver. If the height of the receiver (moving device) is known, the location of the moving device can be refined. In other words, the coverage area can be narrowed based on the height.

In one embodiment, the additional transmitter is stationary. In another embodiment, the additional transmitter (and/or the transmitter) is not stationary and does not use directional antenna. Instead, the additional transmitter (and/or the transmitter) may move around and use an omni-antenna. The additional transmitter (and/or the transmitter) has a way to obtain its instantaneous location (e.g. based on GPS, Bluetooth). When the receiver receives the beacon signal from the additional transmitter (and/or the transmitter), it can obtain a "reset" location based on the instantaneous location of the additional transmitter (and/or the transmitter). There may be a server (location database) that keep track of the instantaneous location of the additional transmitter (and/or the transmitter) and share the location with the moving device.

In one embodiment, the object may have a complex motion (e.g. both leg motion and hand motion). The wireless signal may capture the leg motions of a person and the additional wireless signal may capture the hand motions of the person.

In one embodiment, only one wearable receiver receiving sounding signals from two different transmitters. As the multipaths are different, the two received wireless (sounding) signal may be dominated by different motions of the person. If one transmitter is at a lower position, the wireless signal may capture predominantly the foot/leg motion. Perhaps the second transmitter is at a high position such that the second wireless signal may capture predominant the hand motion.

In another embodiment, an apparatus for object tracking is disclosed. The described apparatus comprises: a processor and at least one of the transmitter and the receiver. The transmitter is configured for transmitting a first wireless signal through a wireless multipath channel. The receiver is configured for receiving a second wireless signal through the wireless multipath channel. One of the transmitter and the receiver is a located device. The other of the transmitter and the receiver is a moving device moving with an object. The second wireless signal differs from the first wireless signal due to the wireless multipath channel which is impacted by a movement of the object. At least one of the transmitter and the receiver comprises at least 16 antennas. The processor is configured for: obtaining a plurality of time series of channel information (CI) of the wireless multipath channel based on the second wireless signal, computing an intermediate quantity (IQ) of a current movement of the moving device based on a set of similarity scores associated with many pairs of CI of the plurality of time series of CI (TSCI), each pair comprising two temporally adjacent CI of a TSCI of the plurality of TSCI, computing a spatial-temporal information (STI) of the current movement of the moving device based on at least one of: the IQ, the plurality of TSCI, a time quantity associated with the current movement, a past IQ, and a past STI, and tracking, based on the STI, at least one of: the moving device and the object.

In one embodiment, the apparatus includes the receiver but not the transmitter. The receiver receives the second wireless signal and extracts the CI, e.g. a channel state information (CSI), for performing the object tracking. In another embodiment, the apparatus includes the transmitter but not the receiver. The CSI is extracted by the receiver and obtained by the processor for object tracking. In still another embodiment, the apparatus includes the transmitter but not the receiver. The CSI is extracted at the receiver that sends the CSI to the transmitter. The object tracking is performed at the transmitter.

In a different embodiment, a method is described. The method comprises: obtaining a plurality of time series of channel information (CI) of a wireless multipath channel, using a processor, a memory communicatively coupled with the processor, and a set of instructions stored in the memory to be executed by the processor. The plurality of time series of CI (TSCI) are extracted from a wireless signal transmitted between a transmitter and a receiver through the wireless multipath channel. Each of the plurality of TSCI is associated with a pair of a transmit antenna on the transmitter and a receive antenna on the receiver. One of the transmitter and the receiver is a located device at a known location. The other of the transmitter and the receiver is a moving device moving with an object. The wireless multipath channel is impacted by a movement of the object. At least one of the transmitter and the receiver comprises more than 16 antennas. The method further comprises: computing a spatial-temporal information (STI) of the moving device based on at least one of: the plurality of TSCI and a past STI, and tracking the object based on the STI.

Other concepts relate to software for implementing the present teaching on wireless rhythmic motion monitoring and object tracking in a rich-scattering environment. Additional novel features will be set forth in part in the description which follows, and in part will become apparent to those skilled in the art upon examination of the following and the accompanying drawings or may be learned by production or operation of the examples. The novel features of the present teachings may be realized and attained by practice or use of various aspects of the methodologies, instrumentalities and combinations set forth in the detailed examples discussed below.

## BRIEF DESCRIPTION OF DRAWINGS

The methods, systems, and/or devices described herein are further described in terms of exemplary embodiments. These exemplary embodiments are described in detail with reference to the drawings. These embodiments are non-limiting exemplary embodiments, in which like reference numerals represent similar structures throughout the several views of the drawings.

FIG. 1A illustrates an exemplary multipath model in rich-scattering indoor environments, where an object (e.g., a human body) is simplified as a single reflector producing only one major reflection path.

FIG. 1B illustrates another exemplary multipath model in rich-scattering indoor environments, where an object scatters the signal and produce multiple paths, according to some embodiments of the present teaching.

FIG. 2A illustrates an example of a combined speed signal, according to some embodiments of the present teaching.

FIG. 2B illustrates an example of a difference of a combined speed signal, according to some embodiments of the present teaching.

FIG. 2C illustrates an exemplary matrix of combined speed signals, according to some embodiments of the present teaching.

FIG. 2D illustrates an exemplary matrix of differences of combined speed signals, according to some embodiments of the present teaching.

FIG. 3 illustrates an exemplary performance of speed estimation, according to some embodiments of the present teaching.

FIG. 4 illustrates a performance comparison of exemplary speed estimation methods, according to some embodiments of the present teaching.

FIG. 5 illustrates an exemplary performance of a periodic autocorrelation function (ACF) of a walking speed, according to some embodiments of the present teaching.

FIG. 6 illustrates an exemplary performance of a gait cycle estimation, according to some embodiments of the present teaching.

FIG. 7 illustrates an exemplary performance of an extracted stable walking period, according to some embodiments of the present teaching.

FIG. 8 illustrates an exemplary performance of a continuous monitoring of walking parameters over time, according to some embodiments of the present teaching.

FIG. 9 illustrates an exemplary performance of a progression of harmonic ratios of a short walking trace, according to some embodiments of the present teaching.

FIG. 10 illustrates exemplary relations among stride, cycle time, and speed, according to some embodiments of the present teaching.

FIG. 11A illustrates exemplary features of speed deviation, according to some embodiments of the present teaching.

FIG. 11B illustrates exemplary histograms of speed deviation, according to some embodiments of the present teaching.

FIG. 12 illustrates exemplary recurrent plots for different users, according to some embodiments of the present teaching.

FIG. 13 illustrates exemplary scaled ACF features, according to some embodiments of the present teaching.

FIG. 14 illustrates an exemplary feature correlation matrix, according to some embodiments of the present teaching.

FIG. 15 illustrates an exemplary method for gait recognition, according to some embodiments of the present teaching.

FIG. 16 illustrates an exemplary performance of RR vs. number of users, according to some embodiments of the present teaching.

FIG. 17 illustrates an exemplary set-up for a base station with massive MIMO antennas, according to some embodiments of the present teaching.

FIG. 18A illustrates an exemplary signal propagation geometry between $r_0$ and $r_s$ in a 3-D model, according to some embodiments of the present teaching.

FIG. 18B illustrates an exemplary signal propagation geometry between $r_0$ and $r_s$ in a 2-D model, according to some embodiments of the present teaching.

FIG. 19A illustrates an exemplary ACF distribution around an intended position (x=0, y=0) for a speed and position estimation, according to some embodiments of the present teaching.

FIG. 19B illustrates an exemplary ACF distribution along the cross beam direction position (x=0) for a speed and position estimation, according to some embodiments of the present teaching.

FIG. 19C illustrates an exemplary ACF distribution (x=0, y≥0) and corresponding peak definition for a speed and position estimation, according to some embodiments of the present teaching.

FIG. 20 illustrates an exemplary signal propagation geometry when $r_0$ moves, according to some embodiments of the present teaching.

FIG. 21 illustrates an exemplary curve fitting by local regression, according to some embodiments of the present teaching.

FIG. 22 illustrates an exemplary speed and direction estimation based on a scenario with two base stations, according to some embodiments of the present teaching.

FIG. 23 illustrates an exemplary angle ambiguity caused by a complementary angle, according to some embodiments of the present teaching.

FIG. 24 illustrates an exemplary speed estimation based on a scenario with three base stations, according to some embodiments of the present teaching.

FIG. 25 illustrates two adjacent positions $r_{s_1}$ and $r_{s_2}$, according to some embodiments of the present teaching.

FIG. 26A shows a geometrical illustration of opposite vertical angle ambiguity for two stations, according to some embodiments of the present teaching.

FIG. 26B shows a geometrical illustration of opposite vertical angle ambiguity for three stations, according to some embodiments of the present teaching.

FIG. 27 shows a geometrical illustration of two adjacent positions $r_{s_1}$ and $r_{s_2}$, according to some embodiments of the present teaching.

FIG. 28 shows another geometrical illustration of opposite vertical angle ambiguity, according to some embodiments of the present teaching.

FIG. 29A illustrates speed estimation error versus antenna number, according to some embodiments of the present teaching.

FIG. 29B illustrates position estimation error versus antenna number, according to some embodiments of the present teaching.

FIG. 29C illustrates normalized position estimation error versus antenna number, according to some embodiments of the present teaching.

FIG. 30 illustrates an exemplary method for direction updating, according to some embodiments of the present teaching.

FIG. 31 illustrates a convex hull formed by AoA measurements, according to some embodiments of the present teaching.

FIG. 32 illustrates a method of target positioning by RSS-based ranging, according to some embodiments of the present teaching.

FIG. 33 illustrates a flow chart of an exemplary method for wireless object tracking, according to some embodiments of the present teaching.

FIG. 34 shows an exemplary network topology of four devices, according to some embodiments of the present teaching.

FIG. 35 shows an exemplary network topology of three devices, according to some embodiments of the present teaching.

FIG. 36 shows exemplary flow chart and components of a Master Origin device, according to some embodiments of the present teaching.

FIG. 37 shows an exemplary network topology of six devices, according to some embodiments of the present teaching.

FIG. 38 shows an exemplary network topology of nine devices in the local area network, according to some embodiments of the present teaching.

FIG. 39 shows exemplary performances of motion detections based on passive infrared (PIR) sensing and WiFi sensing, according to some embodiments of the present teaching.

FIG. 40 shows an exemplary setup for breathing monitoring, according to some embodiments of the present teaching.

FIG. 41 illustrates an exemplary diagram for a system of object motion detection, according to some embodiments of the present teaching.

FIG. 42 illustrates an exemplary diagram for a system of object motion detection, according to some embodiments of the present teaching.

FIG. 43 illustrates an exemplary day view showing separate instances of sleep, according to some embodiments of the present teaching.

FIG. 44A and FIG. 44B illustrate exemplary weekly views showing a 24 hour scale, according to some embodiments of the present teaching.

FIG. 45A and FIG. 45B illustrate exemplary home views showing real-time breathing rate and movement index, according to some embodiments of the present teaching.

FIGs. 46-52 illustrate more exemplary views of lifelog display, according to some embodiments of the present teaching.

## DETAILED DESCRIPTION

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

In one embodiment, the present teaching discloses a method, apparatus, device, system, and/or software (method/apparatus/device/system/software) of a wireless monitoring system. A time series of channel information (CI) of a wireless multipath channel (channel) may be obtained (e.g. dynamically) using a processor, a memory communicatively coupled with the processor and a set of instructions stored in the memory. The time series of CI (TSCI) may be extracted from a wireless signal (signal) transmitted between a Type 1 heterogeneous wireless device (e.g. wireless transmitter, TX) and a Type 2 heterogeneous wireless device (e.g. wireless receiver, RX) in a venue through the channel. The channel may be impacted by an expression (e.g. motion, movement, expression, and/or change in position/pose/shape/expression) of an object in the venue. A characteristics and/or a spatial-temporal information (STI, e.g. motion information) of the object and/or of the motion of the object may be monitored based on the TSCI. A task may be performed based on the characteristics and/or STI. A presentation associated with the task may be generated in a user-interface (UI) on a device of a user. The TSCI may be a wireless signal stream. The TSCI or each CI may be preprocessed. A device may be a station (STA). The symbol "A/B" means "A and/or B" in the present teaching.

The expression may comprise placement, placement of moveable parts, location, position, orientation, identifiable place, region, spatial coordinate, presentation, state, static expression, size, length, width, height, angle, scale, shape, curve, surface, area, volume, pose, posture, manifestation, body language, dynamic expression, motion, motion sequence, gesture, extension, contraction, distortion, deformation, body expression (e.g. head, face, eye, mouth, tongue, hair, voice, neck, limbs, arm, hand, leg, foot, muscle, moveable parts), surface expression (e.g. shape, texture, material, color, electromagnetic (EM) characteristics, visual pattern, wetness, reflectance, translucency, flexibility), material property (e.g. living tissue, hair, fabric, metal, wood, leather, plastic, artificial material, solid, liquid, gas, temperature), movement, activity, behavior, change of expression, and/or some combination.

The wireless signal may comprise: transmitted/received signal, EM radiation, RF signal/transmission, signal in licensed/unlicensed /ISM band, bandlimited signal, baseband signal, wireless/mobile/cellular communication signal, wireless/mobile/cellular network signal, mesh signal, light signal/communication, downlink/uplink signal, unicast/multicast/broadcast signal, standard (e.g. WLAN, WWAN, WPAN, WBAN, international, national, industry, defacto, IEEE, IEEE 802, 802.11/15/16, WiFi, 802.11n/ac/ax/be, 3G/4G/LTE/5G/6G/7G/8G, 3GPP, Bluetooth, BLE, Zigbee, RFID, UWB, WiMax) compliant signal, protocol signal, standard frame, beacon/pilot/probe/enquiry/acknowledgement/handshake/synchronization signal, management/control/data frame, management/control/data signal, standardized wireless/cellular communication protocol, reference signal, source signal, motion probe/detection/sensing signal, and/or series of signals. The wireless signal may comprise a line-of-sight (LOS), and/or a non-LOS component (or path/link). Each CI may be extracted/generated/computed/sensed at a layer (e.g. PHY/MAC layer in OSI model) of Type 2 device and may be obtained by an application (e.g. software, firmware, driver, app, wireless monitoring software/system).

The wireless multipath channel may comprise: a communication channel, analog frequency channel (e.g. with analog carrier frequency near 700/800/900MHz, 1.8/1.8/2.4/3/5/6/27/60 GHz), coded channel (e.g. in CDMA), and/or channel of a wireless network/system (e.g. WLAN, WiFi, mesh, LTE, 4G/5G, Bluetooth, Zigbee, UWB, RFID, microwave). It may comprise more than one channel. The channels may be consecutive (e.g. with adjacent/overlapping bands) or non-consecutive channels (e.g. non-overlapping WiFi channels, one at 2.4GHz and one at 5GHz).

The TSCI may be extracted from the wireless signal at a layer of the Type 2 device (e.g. a layer of OSI reference model, physical layer, data link layer, logical link control layer, media access control (MAC) layer, network layer, transport layer, session layer, presentation layer, application layer, TCP/IP layer, internet layer, link layer). The TSCI may be extracted from a derived signal (e.g. baseband signal, motion detection signal, motion sensing signal) derived from the wireless signal (e.g. RF signal). It may be (wireless) measurements sensed by the communication protocol (e.g. standardized protocol) using existing mechanism (e.g. wireless/cellular communication standard/network, 3G/LTE/4G/5G/6G/7G/8G, WiFi, IEEE 802.11/15/16). The derived signal may comprise a packet with at least one of: a preamble, a header and a payload (e.g. for data/control/management in wireless links/networks). The TSCI may be extracted from a probe signal (e.g. training sequence, STF, LTF, L-STF, L-LTF, L-SIG, HE-STF, HE-LTF, HE-SIG-A, HE-SIG-B, CEF) in the packet. A motion detection/sensing signal may be recognized/identified base on the probe signal. The packet may be a standard-compliant protocol frame, management frame, control frame, data frame, sounding frame, excitation frame, illumination frame, null data frame, beacon frame, pilot frame, probe frame, request frame, response frame, association frame, reassociation frame, disassociation frame, authentication frame, action frame, report frame, poll frame, announcement frame, extension frame, enquiry frame, acknowledgement frame, RTS frame, CTS frame, QoS frame, CF-Poll frame, CF-Ack frame, block acknowledgement frame, reference frame, training frame, and/or synchronization frame.

The packet may comprise a control data and/or a motion detection probe. A data (e.g. ID/parameters/ characteristics/ settings/ control signal/command/instruction/ notification/ broadcasting-related information of the Type 1 device) may be obtained from the payload. The wireless signal may be transmitted by the Type 1 device. It may be received by the Type 2 device. A database (e.g. in local server, hub device, cloud server, storage network) may be used to store the TSCI, characteristics, STI, signatures, patterns, behaviors, trends, parameters, analytics, output responses, identification information, user information, device information, channel information, venue (e.g. map, environmental model, network, proximity devices/networks) information, task information, class/category information, presentation (e.g. UI) information, and/or other information.

The Type 1/Type 2 device may comprise at least one of: electronics, circuitry, transmitter (TX)/receiver (RX)/transceiver, RF interface, "Origin Satellite"/"Tracker Bot", unicast/multicast/broadcasting device, wireless source device, source/destination device, wireless node, hub device, target device, motion detection device, sensor device, remote/wireless sensor device, wireless communication device, wireless-enabled device, standard compliant device, and/or receiver. The Type 1 (or Type 2) device may be heterogeneous because, when there are more than one instances of Type 1 (or Type 2) device, they may have different circuitry, enclosure, structure, purpose, auxiliary functionality, chip/IC, processor, memory, software, firmware, network connectivity, antenna, brand, model, appearance, form, shape, color, material, and/or specification. The Type 1/Type 2 device may comprise: access point, router, mesh router, internet-of-things (IoT) device, wireless terminal, one or more radio/RF subsystem/wireless interface (e.g. 2.4GHz radio, 5GHz radio, front haul radio, backhaul radio), modem, RF front end, RF/radio chip or integrated circuit (IC).

At least one of: Type 1 device, Type 2 device, a link between them, the object, the characteristics, the STI, the monitoring of the motion, and the task may be associated with an identification (ID) such as UUID. The Type 1/Type 2/another device may obtain/store/retrieve/ access/preprocess/condition/process/analyze/monitor/apply the TSCI. The Type 1 and Type 2 devices may communicate network traffic in another channel (e.g. Ethernet, HDMI, USB, Bluetooth, BLE, WiFi, LTE, other network, the wireless multipath channel) in parallel to the wireless signal. The Type 2 device may passively observe/monitor/receive the wireless signal from the Type 1 device in the wireless multipath channel without establishing connection (e.g. association/authentication) with, or requesting service from, the Type 1 device.

The transmitter (i.e. Type 1 device) may function as (play role of) receiver (i.e. Type 2 device) temporarily, sporadically, continuously, repeatedly, interchangeably, alternately, simultaneously, concurrently, and/or contemporaneously; and vice versa. A device may function as Type 1 device (transmitter) and/or Type 2 device (receiver) temporarily, sporadically, continuously, repeatedly, simultaneously, concurrently, and/or contemporaneously. There may be multiple wireless nodes each being Type 1 (TX) and/or Type 2 (RX) device. A TSCI may be obtained between every two nodes when they exchange/communicate wireless signals. The characteristics and/or STI of the object may be monitored individually based on a TSCI, or jointly based on two or more (e.g. all) TSCI. The motion of the object may be monitored actively (in that Type 1 device, Type 2 device, or both, are wearable of/associated with the object) and/or passively (in that both Type 1 and Type 2 devices are not wearable of/associated with the object). It may be passive because the object may not be associated with the Type 1 device and/or the Type 2 device. The object (e.g. user, an automated guided vehicle or AGV) may not need to carry/install any wearables/fixtures (i.e. the Type 1 device and the Type 2 device are not wearable/attached devices that the object needs to carry in order perform the task). It may be active because the object may be associated with either the Type 1 device and/or the Type 2 device. The object may carry (or installed) a wearable/a fixture (e.g. the Type 1 device, the Type 2 device, a device communicatively coupled with either the Type 1 device or the Type 2 device).

The presentation may be visual, audio, image, video, animation, graphical presentation, text, etc. A computation of the task may be performed by a processor (or logic unit) of the Type 1 device, a processor (or logic unit) of an IC of the Type 1 device, a processor (or logic unit) of the Type 2 device, a processor of an IC of the Type 2 device, a local server, a cloud server, a data analysis subsystem, a signal analysis subsystem, and/or another processor. The task may be performed with/without reference to a wireless fingerprint or a baseline (e.g. collected, processed, computed, transmitted and/or stored in a training phase/survey/current survey/previous survey/recent survey/initial wireless survey, a passive fingerprint), a training, a profile, a trained profile, a static profile, a survey, an initial wireless survey, an initial setup, an installation, a re-training, an updating and a reset.

The Type 1 device (TX device) may comprise at least one heterogeneous wireless transmitter. The Type 2 device (RX device) may comprise at least one heterogeneous wireless receiver. The Type 1 device and the Type 2 device may be collocated. The Type 1 device and the Type 2 device may be the same device. Any device may have a data processing unit/apparatus, a computing unit/system, a network unit/system, a processor (e.g. logic unit), a memory communicatively coupled with the processor, and a set of instructions stored in the memory to be executed by the processor. Some processors, memories and sets of instructions may be coordinated. There may be multiple Type 1 devices interacting (e.g. communicating, exchange signal/control/notification/other data) with the same Type 2 device (or multiple Type 2 devices), and/or there may be multiple Type 2 devices interacting with the same Type 1 device. The multiple Type 1 devices/Type 2 devices may be synchronized and/or asynchronous, with same/different window width/size and/or time shift, same/different synchronized start time, synchronized end time, etc. Wireless signals sent by the multiple Type 1 devices may be sporadic, temporary, continuous, repeated, synchronous, simultaneous, concurrent, and/or contemporaneous. The multiple Type 1 devices/Type 2 devices may operate independently and/or collaboratively. A Type 1 and/or Type 2 device may have/comprise/be heterogeneous hardware circuitry (e.g. a heterogeneous chip or a heterogeneous IC capable of generating/receiving the wireless signal, extracting CI from received signal, or making the CI available). They may be communicatively coupled to same or different servers (e.g. cloud server, edge server, local server, hub device).

Operation of one device may be based on operation, state, internal state, storage, processor, memory output, physical location, computing resources, network of another device. Difference devices may communicate directly, and/or via another device/server/hub device/cloud server. The devices may be associated with one or more users, with associated settings. The settings may be chosen once, pre-programmed, and/or changed (e.g. adjusted, varied, modified)/varied over time. There may be additional steps in the method. The steps and/or the additional steps of the method may be performed in the order shown or in another order. Any steps may be performed in parallel, iterated, or otherwise repeated or performed in another manner. A user may be human, adult, older adult, man, woman, juvenile, child, baby, pet, animal, creature, machine, computer module/software, etc.

In the case of one or multiple Type 1 devices interacting with one or multiple Type 2 devices, any processing (e.g. time domain, frequency domain) may be different for different devices. The processing may be based on locations, orientation, direction, roles, user-related characteristics, settings, configurations, available resources, available bandwidth, network connection, hardware, software, processor, co-processor, memory, battery life, available power, antennas, antenna types, directional/unidirectional characteristics of the antenna, power setting, and/or other parameters/characteristics of the devices.

The wireless receiver (e.g. Type 2 device) may receive the signal and/or another signal from the wireless transmitter (e.g. Type 1 device). The wireless receiver may receive another signal from another wireless transmitter (e.g. a second Type 1 device). The wireless transmitter may transmit the signal and/or another signal to another wireless receiver (e.g. a second Type 2 device). The wireless transmitter, wireless receiver, another wireless receiver and/or another wireless transmitter may be moving with the object and/or another object. The another object may be tracked.

The Type 1 and/or Type 2 device may be capable of wirelessly coupling with at least two Type 2 and/or Type 1 devices. The Type 1 device may be caused/controlled to switch/establish wireless coupling (e.g. association, authentication) from the Type 2 device to a second Type 2 device at another location in the venue. Similarly, the Type 2 device may be caused/controlled to switch/establish wireless coupling from the Type 1 device to a second Type 1 device at yet another location in the venue. The switching may be controlled by a server (or a hub device), the processor, the Type 1 device, the Type 2 device, and/or another device. The radio used before and after switching may be different. A second wireless signal (second signal) may be caused to be transmitted between the Type 1 device and the second Type 2 device (or between the Type 2 device and the second Type 1 device) through the channel. A second TSCI of the channel extracted from the second signal may be obtained. The second signal may be the first signal. The characteristics, STI and/or another quantity of the object may be monitored based on the second TSCI. The Type 1 device and the Type 2 device may be the same. The characteristics, STI and/or another quantity with different time stamps may form a waveform. The waveform may be displayed in the presentation.

The wireless signal and/or another signal may have data embedded. The wireless signal may be a series of probe signals (e.g. a repeated transmission of probe signals, a re-use of one or more probe signals). The probe signals may change/vary over time. A probe signal may be a standard compliant signal, protocol signal, standardized wireless protocol signal, control signal, data signal, wireless communication network signal, cellular network signal, WiFi signal, LTE/5G/6G/7G signal, reference signal, beacon signal, motion detection signal, and/or motion sensing signal. A probe signal may be formatted according to a wireless network standard (e.g. WiFi), a cellular network standard (e.g. LTE/5G/6G), or another standard. A probe signal may comprise a packet with a header and a payload. A probe signal may have data embedded. The payload may comprise data. A probe signal may be replaced by a data signal. The probe signal may be embedded in a data signal. The wireless receiver, wireless transmitter, another wireless receiver and/or another wireless transmitter may be associated with at least one processor, memory communicatively coupled with respective processor, and/or respective set of instructions stored in the memory which when executed cause the processor to perform any and/or all steps needed to determine the STI (e.g. motion information), initial STI, initial time, direction, instantaneous location, instantaneous angle, and/or speed, of the object. The processor, the memory and/or the set of instructions may be associated with the Type 1 device, one of the at least one Type 2 device, the object, a device associated with the object, another device associated with the venue, a cloud server, a hub device, and/or another server.

The Type 1 device may transmit the signal in a broadcasting manner to at least one Type 2 device(s) through the channel in the venue. The signal is transmitted without the Type 1 device establishing wireless connection (e.g. association, authentication) with any Type 2 device, and without any Type 2 device requesting services from the Type 1 device. The Type 1 device may transmit to a particular media access control (MAC) address common for more than one Type 2 devices. Each Type 2 device may adjust its MAC address to the particular MAC address. The particular MAC address may be associated with the venue. The association may be recorded in an association table of an Association Server (e.g. hub device). The venue may be identified by the Type 1 device, a Type 2 device and/or another device based on the particular MAC address, the series of probe signals, and/or the at least one TSCI extracted from the probe signals. For example, a Type 2 device may be moved to a new location in the venue (e.g. from another venue). The Type 1 device may be newly set up in the venue such that the Type 1 and Type 2 devices are not aware of each other. During set up, the Type 1 device may be instructed/guided/ caused/ controlled (e.g. using dummy receiver, using hardware pin setting/connection, using stored setting, using local setting, using remote setting, using downloaded setting, using hub device, or using server) to send the series of probe signals to the particular MAC address. Upon power up, the Type 2 device may scan for probe signals according to a table of MAC addresses (e.g. stored in a designated source, server, hub device, cloud server) that may be used for broadcasting at different locations (e.g. different MAC address used for different venue such as house, office, enclosure, floor, multi-storey building, store, airport, mall, stadium, hall, station, subway, lot, area, zone, region, district, city, country, continent). When the Type 2 device detects the probe signals sent to the particular MAC address, the Type 2 device can use the table to identify the venue based on the MAC address. A location of a Type 2 device in the venue may be computed based on the particular MAC address, the series of probe signals, and/or the at least one TSCI obtained by the Type 2 device from the probe signals. The computing may be performed by the Type 2 device. The particular MAC address may be changed (e.g. adjusted, varied, modified) over time. It may be changed according to a time table, rule, policy, mode, condition, situation and/or change. The particular MAC address may be selected based on availability of the MAC address, a pre-selected list, collision pattern, traffic pattern, data traffic between the Type 1 device and another device, effective bandwidth, random selection, and/or a MAC address switching plan. The particular MAC address may be the MAC address of a second wireless device (e.g. a dummy receiver, or a receiver that serves as a dummy receiver).

The Type 1 device may transmit the probe signals in a channel selected from a set of channels. At least one CI of the selected channel may be obtained by a respective Type 2 device from the probe signal transmitted in the selected channel. The selected channel may be changed (e.g. adjusted, varied, modified) over time. The change may be according to a time table, rule, policy, mode, condition, situation, and/or change. The selected channel may be selected based on availability of channels, random selection, a pre-selected list, co-channel interference, inter-channel interference, channel traffic pattern, data traffic between the Type 1 device and another device, effective bandwidth associated with channels, security criterion, channel switching plan, a criterion, a quality criterion, a signal quality condition, and/or consideration.

The particular MAC address and/or an information of the selected channel may be communicated between the Type 1 device and a server (e.g. hub device) through a network. The particular MAC address and/or the information of the selected channel may also be communicated between a Type 2 device and a server (e.g. hub device) through another network. The Type 2 device may communicate the particular MAC address and/or the information of the selected channel to another Type 2 device (e.g. via mesh network, Bluetooth, WiFi, NFC, ZigBee, etc.). The particular MAC address and/or selected channel may be chosen by a server (e.g. hub device). The particular MAC address and/or selected channel may be signaled in an announcement channel by the Type 1 device, the Type 2 device and/or a server (e.g. hub device). Before being communicated, any information may be pre-processed.

Wireless connection (e.g. association, authentication) between the Type 1 device and another wireless device may be established (e.g. using a signal handshake). The Type 1 device may send a first handshake signal (e.g. sounding frame, probe signal, request-to-send RTS) to the another device. The another device may reply by sending a second handshake signal (e.g. a command, or a clear-to-send CTS) to the Type 1 device, triggering the Type 1 device to transmit the signal (e.g. series of probe signals) in the broadcasting manner to multiple Type 2 devices without establishing connection with any Type 2 device. The second handshake signals may be a response or an acknowledge (e.g. ACK) to the first handshake signal. The second handshake signal may contain a data with information of the venue, and/or the Type 1 device. The another device may be a dummy device with a purpose (e.g. primary purpose, secondary purpose) to establish the wireless connection with the Type 1 device, to receive the first signal, and/or to send the second signal. The another device may be physically attached to the Type 1 device.

In another example, the another device may send a third handshake signal to the Type 1 device triggering the Type 1 device to broadcast the signal (e.g. series of probe signals) to multiple Type 2 devices without establishing connection (e.g. association, authentication) with any Type 2 device. The Type 1 device may reply to the third special signal by transmitting a fourth handshake signal to the another device. The another device may be used to trigger more than one Type 1 devices to broadcast. The triggering may be sequential, partially sequential, partially parallel, or fully parallel. The another device may have more than one wireless circuitries to trigger multiple transmitters in parallel. Parallel trigger may also be achieved using at least one yet another device to perform the triggering (similar to what as the another device does) in parallel to the another device. The another device may not communicate (or suspend communication) with the Type 1 device after establishing connection with the Type 1 device. Suspended communication may be resumed. The another device may enter an inactive mode, hibernation mode, sleep mode, stand-by mode, low-power mode, OFF mode and/or power-down mode, after establishing the connection with the Type 1 device. The another device may have the particular MAC address so that the Type 1 device sends the signal to the particular MAC address. The Type 1 device and/or the another device may be controlled and/or coordinated by a first processor associated with the Type 1 device, a second processor associated with the another device, a third processor associated with a designated source and/or a fourth processor associated with another device. The first and second processors may coordinate with each other.

A first series of probe signals may be transmitted by a first antenna of the Type 1 device to at least one first Type 2 device through a first channel in a first venue. A second series of probe signals may be transmitted by a second antenna of the Type 1 device to at least one second Type 2 device through a second channel in a second venue. The first series and the second series may/may not be different. The at least one first Type 2 device may/may not be different from the at least one second Type 2 device. The first and/or second series of probe signals may be broadcasted without connection (e.g. association, authentication) established between the Type 1 device and any Type 2 device. The first and second antennas may be same/different. The two venues may have different sizes, shape, multipath characteristics. The first and second venues may overlap. The respective immediate areas around the first and second antennas may overlap. The first and second channels may be same/different. For example, the first one may be WiFi while the second may be LTE. Or, both may be WiFi, but the first one may be 2.4GHz WiFi and the second may be 5GHz WiFi. Or, both may be 2.4GHz WiFi, but have different channel numbers, SSID names, and/or WiFi settings.

Each Type 2 device may obtain at least one TSCI from the respective series of probe signals, the CI being of the respective channel between the Type 2 device and the Type 1 device. Some first Type 2 device(s) and some second Type 2 device(s) may be the same. The first and second series of probe signals may be synchronous/asynchronous. A probe signal may be transmitted with data or replaced by a data signal. The first and second antennas may be the same. The first series of probe signals may be transmitted at a first rate (e.g. 30Hz). The second series of probe signals may be transmitted at a second rate (e.g. 200Hz). The first and second rates may be same/different. The first and/or second rate may be changed (e.g. adjusted, varied, modified) over time. The change may be according to a time table, rule, policy, mode, condition, situation, and/or change. Any rate may be changed (e.g. adjusted, varied, modified) over time. The first and/or second series of probe signals may be transmitted to a first MAC address and/or second MAC address respectively. The two MAC addresses may be same/different. The first series of probe signals may be transmitted in a first channel. The second series of probe signals may be transmitted in a second channel. The two channels may be same/different. The first or second MAC address, first or second channel may be changed over time. Any change may be according to a time table, rule, policy, mode, condition, situation, and/or change.

The Type 1 device and another device may be controlled and/or coordinated, physically attached, or may be of/in/of a common device. They may be controlled by/connected to a common data processor, or may be connected to a common bus interconnect/ network/ LAN/ Bluetooth network/ NFC network/ BLE network/ wired network/ wireless network/ mesh network/ mobile network/ cloud. They may share a common memory, or be associated with a common user, user device, profile, account, identity (ID), identifier, household, house, physical address, location, geographic coordinate, IP subnet, SSID, home device, office device, and/or manufacturing device. Each Type 1 device may be a signal source of a set of respective Type 2 devices (i.e. it sends a respective signal (e.g. respective series of probe signals) to the set of respective Type 2 devices). Each respective Type 2 device chooses the Type 1 device from among all Type 1 devices as its signal source. Each Type 2 device may choose asynchronously. At least one TSCI may be obtained by each respective Type 2 device from the respective series of probe signals from the Type 1 device, the CI being of the channel between the Type 2 device and the Type 1 device. The respective Type 2 device chooses the Type 1 device from among all Type 1 devices as its signal source based on identity (ID) or identifier of Type 1/Type 2 device, task to be performed, past signal source, history (e.g. of past signal source, Type 1 device, another Type 1 device, respective Type 2 receiver, and/or another Type 2 receiver), threshold for switching signal source, and/or information of a user, account, access info, parameter, characteristics, and/or signal strength (e.g. associated with the Type 1 device and/or the respective Type 2 receiver). Initially, the Type 1 device may be signal source of a set of initial respective Type 2 devices (i.e. the Type 1 device sends a respective signal (series of probe signals) to the set of initial respective Type 2 devices) at an initial time. Each initial respective Type 2 device chooses the Type 1 device from among all Type 1 devices as its signal source.

The signal source (Type 1 device) of a particular Type 2 device may be changed (e.g. adjusted, varied, modified) when (1) time interval between two adjacent probe signals (e.g. between current probe signal and immediate past probe signal, or between next probe signal and current probe signal) received from current signal source of the Type 2 device exceeds a first threshold; (2) signal strength associated with current signal source of the Type 2 device is below a second threshold; (3) a processed signal strength associated with current signal source of the Type 2 device is below a third threshold, the signal strength processed with low pass filter, band pass filter, median filter, moving average filter, weighted averaging filter, linear filter and/or non-linear filter; and/or (4) signal strength (or processed signal strength) associated with current signal source of the Type 2 device is below a fourth threshold for a significant percentage of a recent time window (e.g. 70%, 80%, 90%). The percentage may exceed a fifth threshold. The first, second, third, fourth and/or fifth thresholds may be time varying.

Condition (1) may occur when the Type 1 device and the Type 2 device become progressively far away from each other, such that some probe signal from the Type 1 device becomes too weak and is not received by the Type 2 device. Conditions (2)-(4) may occur when the two devices become far from each other such that the signal strength becomes very weak.

The signal source of the Type 2 device may not change if other Type 1 devices have signal strength weaker than a factor (e.g. 1, 1.1, 1.2, or 1.5) of the current signal source. If the signal source is changed (e.g. adjusted, varied, modified), the new signal source may take effect at a near future time (e.g. the respective next time). The new signal source may be the Type 1 device with strongest signal strength, and/or processed signal strength. The current and new signal source may be same/different.

A list of available Type 1 devices may be initialized and maintained by each Type 2 device. The list may be updated by examining signal strength and/or processed signal strength associated with the respective set of Type 1 devices. A Type 2 device may choose between a first series of probe signals from a first Type 1 device and a second series of probe signals from a second Type 1 device based on: respective probe signal rate, MAC addresses, channels, characteristics/properties/ states, task to be performed by the Type 2 device, signal strength of first and second series, and/or another consideration.

The series of probe signals may be transmitted at a regular rate (e.g. 100 Hz). The series of probe signals may be scheduled at a regular interval (e.g. 0.01s for 100 Hz), but each probe signal may experience small time perturbation, perhaps due to timing requirement, timing control, network control, handshaking, message passing, collision avoidance, carrier sensing, congestion, availability of resources, and/or another consideration. The rate may be changed (e.g. adjusted, varied, modified). The change may be according to a time table (e.g. changed once every hour), rule, policy, mode, condition and/or change (e.g. changed whenever some event occur). For example, the rate may normally be 100Hz, but changed to 1000Hz in demanding situations, and to 1Hz in low power/standby situation. The probe signals may be sent in burst.

The probe signal rate may change based on a task performed by the Type 1 device or Type 2 device (e.g. a task may need 100 Hz normally and 1000 Hz momentarily for 20 seconds). In one example, the transmitters (Type 1 devices), receivers (Type 2 device), and associated tasks may be associated adaptively (and/or dynamically) to classes (e.g. classes that are: low-priority, high-priority, emergency, critical, regular, privileged, non-subscription, subscription, paying, and/or non-paying). A rate (of a transmitter) may be adjusted for the sake of some class (e.g. high priority class). When the need of that class changes, the rate may be changed (e.g. adjusted, varied, modified). When a receiver has critically low power, the rate may be reduced to reduce power consumption of the receiver to respond to the probe signals. In one example, probe signals may be used to transfer power wirelessly to a receiver (Type 2 device), and the rate may be adjusted to control the amount of power transferred to the receiver.

The rate may be changed by (or based on): a server (e.g. hub device), the Type 1 device and/or the Type 2 device. Control signals may be communicated between them. The server may monitor, track, forecast and/or anticipate the needs of the Type 2 device and/or the tasks performed by the Type 2 device, and may control the Type 1 device to change the rate. The server may make scheduled changes to the rate according to a time table. The server may detect an emergency situation and change the rate immediately. The server may detect a developing condition and adjust the rate gradually. The characteristics and/or STI (e.g. motion information) may be monitored individually based on a TSCI associated with a particular Type 1 device and a particular Type 2 device, and/or monitored jointly based on any TSCI associated with the particular Type 1 device and any Type 2 device, and/or monitored jointly based on any TSCI associated with the particular Type 2 device and any Type 1 device, and/or monitored globally based on any TSCI associated with any Type 1 device and any Type 2 device. Any joint monitoring may be associated with: a user, user account, profile, household, map of venue, environmental model of the venue, and/or user history, etc.

A first channel between a Type 1 device and a Type 2 device may be different from a second channel between another Type 1 device and another Type 2 device. The two channels may be associated with different frequency bands, bandwidth, carrier frequency, modulation, wireless standards, coding, encryption, payload characteristics, networks, network ID, SSID, network characteristics, network settings, and/or network parameters, etc. The two channels may be associated with different kinds of wireless system (e.g. two of the following: WiFi, LTE, LTE-A, LTE-U, 2.5G, 3G, 3.5G, 4G, beyond 4G, 5G, 6G, 7G, a cellular network standard, UMTS, 3GPP, GSM, EDGE, TDMA, FDMA, CDMA, WCDMA, TD-SCDMA, 802.11 system, 802.15 system, 802.16 system, mesh network, Zigbee, NFC, WiMax, Bluetooth, BLE, RFID, UWB, microwave system, radar like system). For example, one is WiFi and the other is LTE. The two channels may be associated with similar kinds of wireless system, but in different network. For example, the first channel may be associated with a WiFi network named "Pizza and Pizza" in the 2.4GHz band with a bandwidth of 20MHz while the second may be associated with a WiFi network with SSID of "StarBud hotspot" in the 5GHz band with a bandwidth of 40MHz. The two channels may be different channels in same network (e.g. the "StarBud hotspot" network).

In one embodiment, a wireless monitoring system may comprise training a classifier of multiple events in a venue based on training TSCI associated with the multiple events. A CI or TSCI associated with an event may be considered/may comprise a wireless sample/characteristics/fingerprint associated with the event (and/or the venue, the environment, the object, the motion of the object, a state/ emotional state/ mental state/ condition/ stage/ gesture/ gait/ action/ movement/ activity/ daily activity/ history/ event of the object, etc.). For each of the multiple known events happening in the venue in a respective training (e.g. surveying, wireless survey, initial wireless survey) time period associated with the known event, a respective training wireless signal (e.g. a respective series of training probe signals) may be transmitted by an antenna of a first Type 1 heterogeneous wireless device using a processor, a memory and a set of instructions of the first Type 1 device to at least one first Type 2 heterogeneous wireless device through a wireless multipath channel in the venue in the respective training time period.

At least one respective time series of training CI (training TSCI) may be obtained asynchronously by each of the at least one first Type 2 device from the (respective) training signal. The CI may be CI of the channel between the first Type 2 device and the first Type 1 device in the training time period associated with the known event. The at least one training TSCI may be preprocessed. The training may be a wireless survey (e.g. during installation of Type 1 device and/or Type 2 device).

For a current event happening in the venue in a current time period, a current wireless signal (e.g. a series of current probe signals) may be transmitted by an antenna of a second Type 1 heterogeneous wireless device using a processor, a memory and a set of instructions of the second Type 1 device to at least one second Type 2 heterogeneous wireless device through the channel in the venue in the current time period associated with the current event. At least one time series of current CI (current TSCI) may be obtained asynchronously by each of the at least one second Type 2 device from the current signal (e.g. the series of current probe signals). The CI may be CI of the channel between the second Type 2 device and the second Type 1 device in the current time period associated with the current event. The at least one current TSCI may be preprocessed.

The classifier may be applied to classify at least one current TSCI obtained from the series of current probe signals by the at least one second Type 2 device, to classify at least one portion of a particular current TSCI, and/or to classify a combination of the at least one portion of the particular current TSCI and another portion of another TSCI. The classifier may partition TSCI (or the characteristics/STI or other analytics or output responses) into clusters and associate the clusters to specific events/ objects/ subjects/ locations/ movements/ activities. Labels/tags may be generated for the clusters. The clusters may be stored and retrieved. The classifier may be applied to associate the current TSCI (or characteristics/STI or the other analytics/output response, perhaps associated with a current event) with: a cluster, a known/specific event, a class/ category/ group/ grouping/ list/ cluster/ set of known events/subjects/locations/movements/activities, an unknown event, a class/ category/ group/ grouping/ list/ cluster/ set of unknown events/subjects/locations/movements/activities, and/or another event/subject/location/movement/activity/ class/ category/ group/ grouping/ list/ cluster/ set. Each TSCI may comprise at least one CI each associated with a respective timestamp. Two TSCI associated with two Type 2 devices may be different with different: starting time, duration, stopping time, amount of CI, sampling frequency, sampling period. Their CI may have different features. The first and second Type 1 devices may be at same location in the venue. They may be the same device. The at least one second Type 2 device (or their locations) may be a permutation of the at least one first Type 2 device (or their locations). A particular second Type 2 device and a particular first Type 2 device may be the same device. A subset of the first Type 2 device and a subset of the second Type 2 device may be the same. The at least one second Type 2 device and/or a subset of the at least one second Type 2 device may be a subset of the at least one first Type 2 device. The at least one first Type 2 device and/or a subset of the at least one first Type 2 device may be a permutation of a subset of the at least one second Type 2 device. The at least one second Type 2 device and/or a subset of the at least one second Type 2 device may be a permutation of a subset of the at least one first Type 2 device. The at least one second Type 2 device and/or a subset of the at least one second Type 2 device may be at same respective location as a subset of the at least one first Type 2 device. The at least one first Type 2 device and/or a subset of the at least one first Type 2 device may be at same respective location as a subset of the at least one second Type 2 device.

The antenna of the Type 1 device and the antenna of the second Type 1 device may be at same location in the venue. Antenna(s) of the at least one second Type 2 device and/or antenna(s) of a subset of the at least one second Type 2 device may be at same respective location as respective antenna(s) of a subset of the at least one first Type 2 device. Antenna(s) of the at least one first Type 2 device and/or antenna(s) of a subset of the at least one first Type 2 device may be at same respective location(s) as respective antenna(s) of a subset of the at least one second Type 2 device.

A first section of a first time duration of the first TSCI and a second section of a second time duration of the second section of the second TSCI may be aligned. A map between items of the first section and items of the second section may be computed. The first section may comprise a first segment (e.g. subset) of the first TSCI with a first starting /ending time, and/or another segment (e.g. subset) of a processed first TSCI. The processed first TSCI may be the first TSCI processed by a first operation. The second section may comprise a second segment (e.g. subset) of the second TSCI with a second starting time and a second ending time, and another segment (e.g. subset) of a processed second TSCI. The processed second TSCI may be the second TSCI processed by a second operation. The first operation and/or the second operation may comprise: subsampling, re-sampling, interpolation, filtering, transformation, feature extraction, pre-processing, and/or another operation.

A first item of the first section may be mapped to a second item of the second section. The first item of the first section may also be mapped to another item of the second section. Another item of the first section may also be mapped to the second item of the second section. The mapping may be one-to-one, one-to-many, many-to-one, many-to-many. At least one function of at least one of: the first item of the first section of the first TSCI, another item of the first TSCI, timestamp of the first item, time difference of the first item, time differential of the first item, neighboring timestamp of the first item, another timestamp associated with the first item, the second item of the second section of the second TSCI, another item of the second TSCI, timestamp of the second item, time difference of the second item, time differential of the second item, neighboring timestamp of the second item, and another timestamp associated with the second item, may satisfy at least one constraint.

One constraint may be that a difference between the timestamp of the first item and the timestamp of the second item may be upper-bounded by an adaptive (and/or dynamically adjusted) upper threshold and lower-bounded by an adaptive lower threshold.

The first section may be the entire first TSCI. The second section may be the entire second TSCI. The first time duration may be equal to the second time duration. A section of a time duration of a TSCI may be determined adaptively (and/or dynamically). A tentative section of the TSCI may be computed. A starting time and an ending time of a section (e.g. the tentative section, the section) may be determined. The section may be determined by removing a beginning portion and an ending portion of the tentative section. A beginning portion of a tentative section may be determined as follows. Iteratively, items of the tentative section with increasing timestamp may be considered as a current item, one item at a time.

In each iteration, at least one activity measure/index may be computed and/or considered. The at least one activity measure may be associated with at least one of: the current item associated with a current timestamp, past items of the tentative section with timestamps not larger than the current timestamp, and/or future items of the tentative section with timestamps not smaller than the current timestamp. The current item may be added to the beginning portion of the tentative section if at least one criterion (e.g. quality criterion, signal quality condition) associated with the at least one activity measure is satisfied.

The at least one criterion associated with the activity measure may comprise at least one of: (a) the activity measure is smaller than an adaptive (e.g. dynamically adjusted) upper threshold, (b) the activity measure is larger than an adaptive lower threshold, (c) the activity measure is smaller than an adaptive upper threshold consecutively for at least a predetermined amount of consecutive timestamps, (d) the activity measure is larger than an adaptive lower threshold consecutively for at least another predetermined amount of consecutive timestamps, (e) the activity measure is smaller than an adaptive upper threshold consecutively for at least a predetermined percentage of the predetermined amount of consecutive timestamps, (f) the activity measure is larger than an adaptive lower threshold consecutively for at least another predetermined percentage of the another predetermined amount of consecutive timestamps, (g) another activity measure associated with another timestamp associated with the current timestamp is smaller than another adaptive upper threshold and larger than another adaptive lower threshold, (h) at least one activity measure associated with at least one respective timestamp associated with the current timestamp is smaller than respective upper threshold and larger than respective lower threshold, (i) percentage of timestamps with associated activity measure smaller than respective upper threshold and larger than respective lower threshold in a set of timestamps associated with the current timestamp exceeds a threshold, and (j) another criterion (e.g. a quality criterion, signal quality condition).

An activity measure/index associated with an item at time T1 may comprise at least one of: (1) a first function of the item at time T1 and an item at time T1-D1, wherein D1 is a pre-determined positive quantity (e.g. a constant time offset), (2) a second function of the item at time T1 and an item at time T1+D1, (3) a third function of the item at time T1 and an item at time T2, wherein T2 is a pre-determined quantity (e.g. a fixed initial reference time; T2 may be changed (e.g. adjusted, varied, modified) over time; T2 may be updated periodically; T2 may be the beginning of a time period and T1 may be a sliding time in the time period), and (4) a fourth function of the item at time T1 and another item.

At least one of: the first function, the second function, the third function, and/or the fourth function may be a function (e.g. F(X, Y, ...)) with at least two arguments: X and Y. The two arguments may be scalars. The function (e.g. F) may be a function of at least one of: X, Y, (X-Y), (Y-X), abs(X-Y), $X^a$, $Y^b$, abs($X^a - Y^b$), $(X-Y)^a$, (X/Y), (X+a)/(Y+b), ($X^a/Y^b$), and (($X/Y)^a$-b), wherein a and b are may be some predetermined quantities. For example, the function may simply be abs(X-Y), or $(X-Y)^2$, $(X-Y)^4$. The function may be a robust function. For example, the function may be $(X-Y)^2$ when abs (X-Y) is less than a threshold T, and (X-Y)+a when abs(X-Y) is larger than T. Alternatively, the function may be a constant when abs(X-Y) is larger than T. The function may also be bounded by a slowly increasing function when abs(X-y) is larger than T, so that outliers cannot severely affect the result. Another example of the function may be (abs(X/Y)-a), where a=1. In this way, if X=Y (i.e. no change or no activity), the function will give a value of 0. If X is larger than Y, (X/Y) will be larger than 1 (assuming X and Y are positive) and the function will be positive. And if X is less than Y, (X/Y) will be smaller than 1 and the function will be negative. In another example, both arguments X and Y may be n-tuples such that X=$(x_1, x_2, ..., x_n)$ and Y=$(y_1, y_2, ..., y_n)$. The function may be a function of at least one of: $x_i$, $y_i$, $(x_i - y_i)$, $(y_i - x_i)$, abs$(x_i - y_i)$, $x_i{}^a$, $y_i{}^b$, abs$(x_i{}^a-y_i{}^b)$, $(x_i-y_i)^a$, $(x_i/y_i)$, $(x_i+a)/(y_i+b)$, $(x_i{}^a/y_i{}^b)$, and $((x_i/y_i)^a-b)$, wherein i is a component index of the n-tuple X and Y, and 1<=i<=n. E.g. component index of $x_1$ is i=1, component index of $x_2$ is i=2. The function may comprise a component-by-component summation of another function of at least one of the following: $x_i$, $y_i$, $(x_i - y_i)$, $(y_i - x_i)$, abs$(x_i - y_i)$, $x_i{}^a$, $y_i{}^b$, abs$(x_i{}^a-y_i{}^b)$, $(x_i - y_i)^a$, $(x_i/y_i)$, $(x_i+a)/(y_i+b)$, $(x_i{}^a/y_i{}^b)$, and $((x_i / y_i)^a-b)$, wherein i is the component index of the n-tuple X and Y. For example, the function may be in a form of sum$_{i=1}^n$(abs$(x_i/y_i)$-1)/n, or sum$_{i=1}^n w_i$*(abs$(x_i/y_i)$-1), where $w_i$ is some weight for component i.

The map may be computed using dynamic time warping (DTW). The DTW may comprise a constraint on at least one of: the map, the items of the first TSCI, the items of the second TSCI, the first time duration, the second time duration, the first section, and/or the second section. Suppose in the map, the $i^{th}$ domain item is mapped to the $j^{th}$ range item. The constraint may be on admissible combination of i and j (constraint on relationship between i and j). Mismatch cost between a first section of a first time duration of a first TSCI and a second section of a second time duration of a second TSCI may be computed.

The first section and the second section may be aligned such that a map comprising more than one links may be established between first items of the first TSCI and second items of the second TSCI. With each link, one of the first items with a first timestamp may be associated with one of the second items with a second timestamp. A mismatch cost between the aligned first section and the aligned second section may be computed. The mismatch cost may comprise a function of: an item-wise cost between a first item and a second item associated by a particular link of the map, and a link-wise cost associated with the particular link of the map.

The aligned first section and the aligned second section may be represented respectively as a first vector and a second vector of same vector length. The mismatch cost may comprise at least one of: an inner product, inner-product-like quantity, quantity based on correlation, correlation indicator, quantity based on covariance, discriminating score, distance, Euclidean distance, absolute distance, Lk distance (e.g. L1, L2, ...), weighted distance, distance-like quantity and/or another similarity value, between the first vector and the second vector. The mismatch cost may be normalized by the respective vector length.

A parameter derived from the mismatch cost between the first section of the first time duration of the first TSCI and the second section of the second time duration of the second TSCI may be modeled with a statistical distribution. At least one of: a scale parameter, location parameter and/or another parameter, of the statistical distribution may be estimated. The first section of the first time duration of the first TSCI may be a sliding section of the first TSCI. The second section of the second time duration of the second TSCI may be a sliding section of the second TSCI. A first sliding window may be applied to the first TSCI and a corresponding second sliding window may be applied to the second TSCI. The first sliding window of the first TSCI and the corresponding second sliding window of the second TSCI may be aligned.

Mismatch cost between the aligned first sliding window of the first TSCI and the corresponding aligned second sliding window of the second TSCI may be computed. The current event may be associated with at least one of: the known event, the unknown event and/or the another event, based on the mismatch cost.

The classifier may be applied to at least one of: each first section of the first time duration of the first TSCI, and/or each second section of the second time duration of the second TSCI, to obtain at least one tentative classification results. Each tentative classification result may be associated with a respective first section and a respective second section.

The current event may be associated with at least one of: the known event, the unknown event, a class/category/ group/ grouping/list/ set of unknown events, and/or the another event, based on the mismatch cost. The current event may be associated with at least one of: the known event, the unknown event and/or the another event, based on a largest number of tentative classification results in more than one sections of the first TSCI and corresponding more than sections of the second TSCI. For example, the current event may be associated with a particular known event if the mismatch cost points to the particular known event for N consecutive times (e.g. N=10). In another example, the current event may be associated with a particular known event if the percentage of mismatch cost within the immediate past N consecutive N pointing to the particular known event exceeds a certain threshold (e.g. >80%). In another example, the current event may be associated with a known event that achieves smallest mismatch cost for the most times within a time period. The current event may be associated with a known event that achieves smallest overall mismatch cost, which is a weighted average of at least one mismatch cost associated with the at least one first sections. The current event may be associated with a particular known event that achieves smallest of another overall cost. The current event may be associated with the "unknown event" if none of the known events achieve mismatch cost lower than a first threshold T1 in a sufficient percentage of the at least one first section. The current event may also be associated with the "unknown event" if none of the events achieve an overall mismatch cost lower than a second threshold T2. The current event may be associated with at least one of: the known event, the unknown event and/or the another event, based on the mismatch cost and additional mismatch cost associated with at least one additional section of the first TSCI and at least one additional section of the second TSCI. The known events may comprise at least one of: a door closed event, door open event, window closed event, window open event, multi-state event, on-state event, off-state event, intermediate state event, continuous state event, discrete state event, human-present event, human-absent event, sign-of-life-present event, and/or a sign-of-life-absent event.

A projection for each CI may be trained using a dimension reduction method based on the training TSCI. The dimension reduction method may comprise at least one of: principal component analysis (PCA), PCA with different kernel, independent component analysis (ICA), Fisher linear discriminant, vector quantization, supervised learning, unsupervised learning, self-organizing maps, auto-encoder, neural network, deep neural network, and/or another method. The projection may be applied to at least one of: the training TSCI associated with the at least one event, and/or the current TSCI, for the classifier. The classifier of the at least one event may be trained based on the projection and the training TSCI associated with the at least one event. The at least one current TSCI may be classified/categorized based on the projection and the current TSCI. The projection may be re-trained using at least one of: the dimension reduction method, and another dimension reduction method, based on at least one of: the training TSCI, at least one current TSCI before retraining the projection, and/or additional training TSCI. The another dimension reduction method may comprise at least one of: principal component analysis (PCA), PCA with different kernels, independent component analysis (ICA), Fisher linear discriminant, vector quantization, supervised learning, unsupervised learning, self-organizing maps, auto-encoder, neural network, deep neural network, and/or yet another method. The classifier of the at least one event may be re-trained based on at least one of: the re-trained projection, the training TSCI associated with the at least one events, and/or at least one current TSCI. The at least one current TSCI may be classified based on: the re-trained projection, the re-trained classifier, and/or the current TSCI.

Each CI may comprise a vector of complex values. Each complex value may be preprocessed to give the magnitude of the complex value. Each CI may be preprocessed to give a vector of non-negative real numbers comprising the magnitude of corresponding complex values. Each training TSCI may be weighted in the training of the projection. The projection may comprise more than one projected components. The projection may comprise at least one most significant projected component. The projection may comprise at least one projected component that may be beneficial for the classifier.

The channel information (CI) may be associated with/may comprise signal strength, signal amplitude, signal phase, spectral power measurement, modem parameters (e.g. used in relation to modulation/ demodulation in digital communication systems such as WiFi, 4G/LTE), dynamic beamforming information, transfer function components, radio state (e.g. used in digital communication systems to decode digital data, baseband processing state, RF processing state, etc.), measurable variables, sensed data, coarse-grained/ fine-grained information of a layer (e.g. physical layer, data link layer, MAC layer, etc.), digital setting, gain setting, RF filter setting, RF front end switch setting, DC offset setting, DC correction setting, IQ compensation setting, effect(s) on the wireless signal by the environment (e.g. venue) during propagation, transformation of an input signal (the wireless signal transmitted by the Type 1 device) to an output signal (the wireless signal received by the Type 2 device), a stable behavior of the environment, a state profile, wireless channel measurements, received signal strength indicator (RSSI), channel state information (CSI), channel impulse response (CIR), channel frequency response (CFR), characteristics of frequency components (e.g. subcarriers) in a bandwidth, channel characteristics, channel filter response, timestamp, auxiliary information, data, meta data, user data, account data, access data, security data, session data, status data, supervisory data, household data, identity (ID), identifier, device data, network data, neighborhood data, environment data, real-time data, sensor data, stored data, encrypted data, compressed data, protected data, and/or another channel information. Each CI may be associated with a time stamp, and/or an arrival time. A CSI can be used to equalize/undo/ minimize/reduce the multipath channel effect (of the transmission channel) to demodulate a signal similar to the one transmitted by the transmitter through the multipath channel. The CI may be associated with information associated with a frequency band, frequency signature, frequency phase, frequency amplitude, frequency trend, frequency characteristics, frequency-like characteristics, time domain element, frequency domain element, time-frequency domain element, orthogonal decomposition characteristics, and/or non-orthogonal decomposition characteristics of the signal through the channel. The TSCI may be a stream of wireless signals (e.g. CI).

The CI may be preprocessed, processed, postprocessed, stored (e.g. in local memory, portable/mobile memory, removable memory, storage network, cloud memory, in a volatile manner, in a non-volatile manner), retrieved, transmitted and/or received. One or more modem parameters and/or radio state parameters may be held constant. The modem parameters may be applied to a radio subsystem. The modem parameters may represent a radio state. A motion detection signal (e.g. baseband signal, and/or packet decoded/demodulated from the baseband signal, etc.) may be obtained by processing (e.g. down-converting) the first wireless signal (e.g. RF/WiFi/LTE/5G signal) by the radio subsystem using the radio state represented by the stored modem parameters. The modem parameters/radio state may be updated (e.g. using previous modem parameters or previous radio state). Both the previous and updated modem parameters/radio states may be applied in the radio subsystem in the digital communication system. Both the previous and updated modem parameters/radio states may be compared/ analyzed/ processed/ monitored in the task.

The channel information may also be modem parameters (e.g. stored or freshly computed) used to process the wireless signal. The wireless signal may comprise a plurality of probe signals. The same modem parameters may be used to process more than one probe signals. The same modem parameters may also be used to process more than one wireless signals. The modem parameters may comprise parameters that indicate settings or an overall configuration for the operation of a radio subsystem or a baseband subsystem of a wireless sensor device (or both). The modem parameters may include one or more of: a gain setting, an RF filter setting, an RF front end switch setting, a DC offset setting, or an IQ compensation setting for a radio subsystem, or a digital DC correction setting, a digital gain setting, and/or a digital filtering setting (e.g. for a baseband subsystem). The CI may also be associated with information associated with a time period, time signature, timestamp, time amplitude, time phase, time trend, and/or time characteristics of the signal. The CI may be associated with information associated with a time-frequency partition, signature, amplitude, phase, trend, and/or characteristics of the signal. The CI may be associated with a decomposition of the signal. The CI may be associated with information associated with a direction, angle of arrival (AoA), angle of a directional antenna, and/or a phase of the signal through the channel. The CI may be associated with attenuation patterns of the signal through the channel. Each CI may be associated with a Type 1 device and a Type 2 device. Each CI may be associated with an antenna of the Type 1 device and an antenna of the Type 2 device.

The CI may be obtained from a communication hardware (e.g. of Type 2 device, or Type 1 device) that is capable of providing the CI. The communication hardware may be a WiFi-capable chip/IC (integrated circuit), chip compliant with a 802.11 or 802.16 or another wireless/radio standard, next generation WiFi-capable chip, LTE-capable chip, 5G-capable chip, 6G/7G/8G-capable chip, Bluetooth-enabled chip, NFC (near field communication)-enabled chip, BLE (Bluetooth low power)-enabled chip, UWB chip, another communication chip (e.g. Zigbee, WiMax, mesh network), etc. The communication hardware computes the CI and stores the CI in a buffer memory and make the CI available for extraction. The CI may comprise data and/or at least one matrices related to channel state information (CSI). The at least one matrices may be used for channel equalization, and/or beam forming, etc. The channel may be associated with a venue. The attenuation may be due to signal propagation in the venue, signal propagating/reflection/ refraction/ diffraction through/at/around air (e.g. air of venue), refraction medium/reflection surface such as wall, doors, furniture, obstacles and/or barriers, etc. The attenuation may be due to reflection at surfaces and obstacles (e.g. reflection surface, obstacle) such as floor, ceiling, furniture, fixtures, objects, people, pets, etc. Each CI may be associated with a timestamp. Each CI may comprise N1 components (e.g. N1 frequency domain components in CFR, N1 time domain components in CIR, or N1 decomposition components). Each component may be associated with a component index. Each component may be a real, imaginary, or complex quantity, magnitude, phase, flag, and/or set. Each CI may comprise a vector or matrix of complex numbers, a set of mixed quantities, and/or a multi-dimensional collection of at least one complex numbers.

Components of a TSCI associated with a particular component index may form a respective component time series associated with the respective index. A TSCI may be divided into N1 component time series. Each respective component time series is associated with a respective component index. The characteristics/STI of the motion of the object may be monitored based on the component time series. In one example, one or more ranges of CI components (e.g. one range being from component 11 to component 23, a second range being from component 44 to component 50, and a third range having only one component) may be selected based on some criteria/cost function/signal quality metric (e.g. based on signal-to-noise ratio, and/or interference level) for further processing.

A component-wise characteristic of a component-feature time series of a TSCI may be computed. The component-wise characteristics may be a scalar (e.g. energy) or a function with a domain and a range (e.g. an autocorrelation function, transform, inverse transform). The characteristics/STI of the motion of the object may be monitored based on the component-wise characteristics. A total characteristics (e.g. aggregate characteristics) of the TSCI may be computed based on the component-wise characteristics of each component time series of the TSCI. The total characteristics may be a weighted average of the component-wise characteristics. The characteristics/STI of the motion of the object may be monitored based on the total characteristics. An aggregate quantity may be a weighted average of individual quantities.

The Type 1 device and Type 2 device may support WiFi, WiMax, 3G/beyond 3G, 4G/beyond 4G, LTE, LTE-A, 5G, 6G, 7G, Bluetooth, NFC, BLE, Zigbee, UWB, UMTS, 3GPP, GSM, EDGE, TDMA, FDMA, CDMA, WCDMA, TD-SCDMA, mesh network, proprietary wireless system, IEEE 802.11 standard, 802.15 standard, 802.16 standard, 3GPP standard, and/or another wireless system.

A common wireless system and/or a common wireless channel may be shared by the Type 1 transceiver and/or the at least one Type 2 transceiver. The at least one Type 2 transceiver may transmit respective signal contemporaneously (or: asynchronously, synchronously, sporadically, continuously, repeatedly, concurrently, simultaneously and/or temporarily) using the common wireless system and/or the common wireless channel. The Type 1 transceiver may transmit a signal to the at least one Type 2 transceiver using the common wireless system and/or the common wireless channel.

Each Type 1 device and Type 2 device may have at least one transmitting/receiving antenna. Each CI may be associated with one of the transmitting antenna of the Type 1 device and one of the receiving antenna of the Type 2 device. Each pair of a transmitting antenna and a receiving antenna may be associated with a link, a path, a communication path, signal hardware path, etc. For example, if the Type 1 device has M (e.g. 3) transmitting antennas, and the Type 2 device has N (e.g. 2) receiving antennas, there may be MxN (e.g. 3x2=6) links or paths. Each link or path may be associated with a TSCI.

The at least one TSCI may correspond to various antenna pairs between the Type 1 device and the Type 2 device. The Type 1 device may have at least one antenna. The Type 2 device may also have at least one antenna. Each TSCI may be associated with an antenna of the Type 1 device and an antenna of the Type 2 device. Averaging or weighted averaging over antenna links may be performed. The averaging or weighted averaging may be over the at least one TSCI. The averaging may optionally be performed on a subset of the at least one TSCI corresponding to a subset of the antenna pairs.

Timestamps of CI of a portion of a TSCI may be irregular and may be corrected so that corrected timestamps of time-corrected CI may be uniformly spaced in time. In the case of multiple Type 1 devices and/or multiple Type 2 devices, the corrected timestamp may be with respect to the same or different clock. An original timestamp associated with each of the CI may be determined. The original timestamp may not be uniformly spaced in time. Original timestamps of all CI of the particular portion of the particular TSCI in the current sliding time window may be corrected so that corrected timestamps of time-corrected CI may be uniformly spaced in time.

The characteristics and/or STI (e.g. motion information) may comprise: location, location coordinate, change in location, position (e.g. initial position, new position), position on map, height, horizontal location, vertical location, distance, displacement, speed, acceleration, rotational speed, rotational acceleration, direction, angle of motion, azimuth, direction of motion, rotation, path, deformation, transformation, shrinking, expanding, gait, gait cycle, head motion, repeated motion, periodic motion, pseudo-periodic motion, impulsive motion, sudden motion, fall-down motion, transient motion, behavior, transient behavior, period of motion, frequency of motion, time trend, temporal profile, temporal characteristics, occurrence, change, temporal change, change of CI, change in frequency, change in timing, change of gait cycle, timing, starting time, initiating time, ending time, duration, history of motion, motion type, motion classification, frequency, frequency spectrum, frequency characteristics, presence, absence, proximity, approaching, receding, identity/identifier of the object, composition of the object, head motion rate, head motion direction, mouth-related rate, eye-related rate, breathing rate, heart rate, tidal volume, depth of breath, inhale time, exhale time, inhale time to exhale time ratio, airflow rate, heart heat-to-beat interval, heart rate variability, hand motion rate, hand motion direction, leg motion, body motion, walking rate, hand motion rate, positional characteristics, characteristics associated with movement (e.g. change in position/location) of the object, tool motion, machine motion, complex motion, and/or combination of multiple motions, event, signal statistics, signal dynamics, anomaly, motion statistics, motion parameter, indication of motion detection, motion magnitude, motion phase, similarity score, distance score, Euclidean distance, weighted distance, $L\_1$ norm, $L\_2$ norm, $L\_k$ norm for $k>2$, statistical distance, correlation, correlation indicator, auto-correlation, covariance, auto-covariance, cross-covariance, inner product, outer product, motion signal transformation, motion feature, presence of motion, absence of motion, motion localization, motion identification, motion recognition, presence of object, absence of object, entrance of object, exit of object, a change of object, motion cycle, motion count, gait cycle, motion rhythm, deformation motion, gesture, handwriting, head motion, mouth motion, heart motion ,internal organ motion, motion trend, size, length, area, volume, capacity, shape, form, tag, starting/initiating location, ending location, starting/initiating quantity, ending quantity, event, fall-down event, security event, accident event, home event, office event, factory event, warehouse event, manufacturing event, assembly line event, maintenance event, car-related event, navigation event, tracking event, door event, door-open event, door-close event, window event, window-open event, window-close event, repeatable event, one-time event, consumed quantity, unconsumed quantity, state, physical state, health state, well-being state, emotional state, mental state, another event, analytics, output responses, and/or another information. The characteristics and/or STI may be computed/monitored based on a feature computed from a CI or a TSCI (e.g. feature computation/extraction). A static segment or profile (and/or a dynamic segment/profile) may be identified /computed/analyzed/ monitored/ extracted/ obtained/ marked/ presented/ indicated/ highlighted/ stored/ communicated based on an analysis of the feature. The analysis may comprise a motion detection/movement assessment/presence detection. Computational workload may be shared among the Type 1 device, the Type 2 device and another processor.

The Type 1 device and/or Type 2 device may be a local device. The local device may be: a smart phone, smart device, TV, sound bar, set-top box, access point, router, repeater, wireless signal repeater/extender, remote control, speaker, fan, refrigerator, microwave, oven, coffee machine, hot water pot, utensil, table, chair, light, lamp, door lock, camera, microphone, motion sensor, security device, fire hydrant, garage door, switch, power adapter, computer, dongle, computer peripheral, electronic pad, sofa, tile, accessory, home device, vehicle device, office device, building device, manufacturing device, watch, glasses, clock, television, oven, air-conditioner, accessory, utility, appliance, smart machine, smart vehicle, internet-of-thing (IoT) device, internet-enabled device, computer, portable computer, tablet, smart house, smart office, smart building, smart parking lot, smart system, and/or another device.

Each Type 1 device may be associated with a respective identifier (e.g. ID). Each Type 2 device may also be associated with a respective identify (ID). The ID may comprise: numeral, combination of text and numbers, name, password, account, account ID, web link, web address, index to some information, and/or another ID. The ID may be assigned. The ID may be assigned by hardware (e.g. hardwired, via dongle and/or other hardware), software and/or firmware. The ID may be stored (e.g. in database, in memory, in server (e.g. hub device), in the cloud, stored locally, stored remotely, stored permanently, stored temporarily) and may be retrieved. The ID may be associated with at least one record, account, user, household, address, phone number, social security number, customer number, another ID, another identifier, timestamp, and/or collection of data. The ID and/or part of the ID of a Type 1 device may be made available to a Type 2 device. The ID may be used for registration, initialization, communication, identification, verification, detection, recognition, authentication, access control, cloud access, networking, social networking, logging, recording, cataloging, classification, tagging, association, pairing, transaction, electronic transaction, and/or intellectual property control, by the Type 1 device and/or the Type 2 device.

The object may be person, user, subject, passenger, child, older person, baby, sleeping baby, baby in vehicle, patient, worker, high-value worker, expert, specialist, waiter, customer in mall, traveler in airport/ train station/ bus terminal/ shipping terminals, staff/worker/customer service personnel in factory/ mall/ supermarket/ office/ workplace, serviceman in sewage/air ventilation system/lift well, lifts in lift wells, elevator, inmate, people to be tracked/ monitored, animal, plant, living object, pet, dog, cat, smart phone, phone accessory, computer, tablet, portable computer, dongle, computing accessory, networked devices, WiFi devices, IoT devices, smart watch, smart glasses, smart devices, speaker, keys, smart key, wallet, purse, handbag, backpack, goods, cargo, luggage, equipment, motor, machine, air conditioner, fan, air conditioning equipment, light fixture, moveable light, television, camera, audio and/or video equipment, stationary, surveillance equipment, parts, signage, tool, cart, ticket, parking ticket, toll ticket, airplane ticket, credit card, plastic card, access card, food packaging, utensil, table, chair, cleaning equipment/tool, vehicle, car, cars in parking facilities, merchandise in warehouse/ store/ supermarket/ distribution center, boat, bicycle, airplane, drone, remote control car/plane/ boat, robot, manufacturing device, assembly line, material/unfinished part/robot/wagon/ transports on factory floor, object to be tracked in airport/shopping mart/supermarket, non-object, absence of an object, presence of an object, object with form, object with changing form, object with no form, mass of fluid, mass of liquid, mass of gas/smoke, fire, flame, electromagnetic (EM) source, EM medium, and/or another object. The object itself may be communicatively coupled with some network, such as WiFi, MiFi, 3G/ 4G/ LTE/ 5G/ 6G/ 7G, Bluetooth, NFC, BLE, WiMax, Zigbee, UMTS, 3GPP, GSM, EDGE, TDMA, FDMA, CDMA, WCDMA, TD-SCDMA, mesh network, adhoc network, and/or other network. The object itself may be bulky with AC power supply, but is moved during installation, cleaning, maintenance, renovation, etc. It may also be installed in moveable platform such as lift, pad, movable, platform, elevator, conveyor belt, robot, drone, forklift, car, boat, vehicle, etc. The object may have multiple parts, each part with different movement (e.g. change in position/location). For example, the object may be a person walking forward. While walking, his left hand and right hand may move in different direction, with different instantaneous speed, acceleration, motion, etc.

The wireless transmitter (e.g. Type 1 device), the wireless receiver (e.g. Type 2 device), another wireless transmitter and/or another wireless receiver may move with the object and/or another object (e.g. in prior movement, current movement and/or future movement. They may be communicatively coupled to one or more nearby device. They may transmit TSCI and/or information associated with the TSCI to the nearby device, and/or each other. They may be with the nearby device. The wireless transmitter and/or the wireless receiver may be part of a small (e.g. coin-size, cigarette box size, or even smaller), light-weight portable device. The portable device may be wirelessly coupled with a nearby device.

The nearby device may be smart phone, iPhone, Android phone, smart device, smart appliance, smart vehicle, smart gadget, smart TV, smart refrigerator, smart speaker, smart watch, smart glasses, smart pad, iPad, computer, wearable computer, notebook computer, gateway. The nearby device may be connected to a cloud server, local server (e.g. hub device) and/or other server via internet, wired internet connection and/or wireless internet connection. The nearby device may be portable. The portable device, the nearby device, a local server (e.g. hub device) and/or a cloud server may share the computation and/or storage for a task (e.g. obtain TSCI, determine characteristics/STI of the object associated with the movement (e.g. change in position/location) of the object, computation of time series of power (e.g. signal strength) information, determining/computing the particular function, searching for local extremum, classification, identifying particular value of time offset, de-noising, processing, simplification, cleaning, wireless smart sensing task, extract CI from signal, switching, segmentation, estimate trajectory/path/track, process the map, processing trajectory/path/track based on environment models/constraints/limitations, correction, corrective adjustment, adjustment, map-based (or model-based) correction, detecting error, checking for boundary hitting, thresholding) and information (e.g. TSCI). The nearby device may/ may not move with the object. The nearby device may be portable/ not portable/ moveable/ non-moveable. The nearby device may use battery power, solar power, AC power and/or other power source. The nearby device may have replaceable/non-replaceable battery, and/or rechargeable/non-rechargeable battery. The nearby device may be similar to the object. The nearby device may have identical (and/or similar) hardware and/or software to the object. The nearby device may be a smart device, network enabled device, device with connection to WiFi/ 3G/ 4G/ 5G/ 6G/ Zigbee/ Bluetooth/ NFC/ UMTS/ 3GPP/ GSM/ EDGE/ TDMA/ FDMA/ CDMA/ WCDMA/ TD-SCDMA/ adhoc network/ other network, smart speaker, smart watch, smart clock, smart appliance, smart machine, smart equipment, smart tool, smart vehicle, internet-of-thing (IoT) device, internet-enabled device, computer, portable computer, tablet, and another device. The nearby device and/or at least one processor associated with the wireless receiver, the wireless transmitter, the another wireless receiver, the another wireless transmitter and/or a cloud server (in the cloud) may determine the initial STI of the object. Two or more of them may determine the initial spatial-temporal info jointly. Two or more of them may share intermediate information in the determination of the initial STI (e.g. initial position).

In one example, the wireless transmitter (e.g. Type 1 device, or Tracker Bot) may move with the object. The wireless transmitter may send the signal to the wireless receiver (e.g. Type 2 device, or Origin Register) or determining the initial STI (e.g. initial position) of the object. The wireless transmitter may also send the signal and/or another signal to another wireless receiver (e.g. another Type 2 device, or another Origin Register) for the monitoring of the motion (spatial-temporal info) of the object. The wireless receiver may also receive the signal and/or another signal from the wireless transmitter and/or the another wireless transmitter for monitoring the motion of the object. The location of the wireless receiver and/or the another wireless receiver may be known. In another example, the wireless receiver (e.g. Type 2 device, or Tracker Bot) may move with the object. The wireless receiver may receive the signal transmitted from the wireless transmitter (e.g. Type 1 device, or Origin Register) for determining the initial spatial-temporal info (e.g. initial position) of the object. The wireless receiver may also receive the signal and/or another signal from another wireless transmitter (e.g. another Type 1 device, or another Origin Register) for the monitoring of the current motion (e.g. spatial-temporal info) of the object. The wireless transmitter may also transmit the signal and/or another signal to the wireless receiver and/or the another wireless receiver (e.g. another Type 2 device, or another Tracker Bot) for monitoring the motion of the object. The location of the wireless transmitter and/or the another wireless transmitter may be known.

The venue may be a space such as a sensing area, room, house, office, property, workplace, hallway, walkway, lift, lift well, escalator, elevator, sewage system, air ventilations system, staircase, gathering area, duct, air duct, pipe, tube, enclosed space, enclosed structure, semi-enclosed structure, enclosed area, area with at least one wall, plant, machine, engine, structure with wood, structure with glass, structure with metal, structure with walls, structure with doors, structure with gaps, structure with reflection surface, structure with fluid, building, roof top, store, factory, assembly line, hotel room, museum, classroom, school, university, government building, warehouse, garage, mall, airport, train station, bus terminal, hub, transportation hub, shipping terminal, government facility, public facility, school, university, entertainment facility, recreational facility, hospital, pediatric/neonatal wards, seniors home, elderly care facility, geriatric facility, community center, stadium, playground, park, field, sports facility, swimming facility, track and/or field, basketball court, tennis court, soccer stadium, baseball stadium, gymnasium, hall, garage, shopping mart, mall, supermarket, manufacturing facility, parking facility, construction site, mining facility, transportation facility, highway, road, valley, forest, wood, terrain, landscape, den, patio, land, path, amusement park, urban area, rural area, suburban area, metropolitan area, garden, square, plaza, music hall, downtown facility, over-air facility, semi-open facility, closed area, train platform, train station, distribution center, warehouse, store, distribution center, storage facility, underground facility, space (e.g. above ground, outer-space) facility, floating facility, cavern, tunnel facility, indoor facility, open-air facility, outdoor facility with some walls/ doors/ reflective barriers, open facility, semi-open facility, car, truck, bus, van, container, ship/boat, submersible, train, tram, airplane, vehicle, mobile home, cave, tunnel, pipe, channel, metropolitan area, downtown area with relatively tall buildings, valley, well, duct, pathway, gas line, oil line, water pipe, network of interconnecting pathways/alleys/roads/tubes/cavities/ caves/pipe-like structure/air space/fluid space, human body, animal body, body cavity, organ, bone, teeth, soft tissue, hard tissue, rigid tissue, non-rigid tissue, blood/body fluid vessel, windpipe, air duct, den, etc. The venue may be indoor space, outdoor space, The venue may include both the inside and outside of the space. For example, the venue may include both the inside of a building and the outside of the building. For example, the venue can be a building that has one floor or multiple floors, and a portion of the building can be underground. The shape of the building can be, e.g., round, square, rectangular, triangle, or irregular-shaped. These are merely examples. The disclosure can be used to detect events in other types of venue or spaces.

The wireless transmitter (e.g. Type 1 device) and/or the wireless receiver (e.g. Type 2 device) may be embedded in a portable device (e.g. a module, or a device with the module) that may move with the object (e.g. in prior movement and/or current movement). The portable device may be communicatively coupled with the object using a wired connection (e.g. through USB, microUSB, Firewire, HDMI, serial port, parallel port, and other connectors) and/or a connection (e.g. Bluetooth, Bluetooth Low Energy (BLE), WiFi, LTE, NFC, ZigBee). The portable device may be a lightweight device. The portable may be powered by battery, rechargeable battery and/or AC power. The portable device may be very small (e.g. at sub-millimeter scale and/or sub-centimeter scale), and/or small (e.g. coin-size, card-size, pocket-size, or larger). The portable device may be large, sizable, and/or bulky (e.g. heavy machinery to be installed). The portable device may be a WiFi hotspot, access point, mobile WiFi (MiFi), dongle with USB/micro USB/ Firewire/ other connector, smartphone, portable computer, computer, tablet, smart device, internet-of-thing (IoT) device, WiFi-enabled device, LTE-enabled device, a smart watch, smart glass, smart mirror, smart antenna, smart battery, smart light, smart pen, smart ring, smart door, smart window, smart clock, small battery, smart wallet, smart belt, smart handbag, smart clothing/garment, smart ornament, smart packaging, smart paper/ book/ magazine/ poster/ printed matter/ signage/ display/ lighted system/ lighting system, smart key/ tool, smart bracelet/ chain/ necklace/ wearable/ accessory, smart pad/ cushion, smart tile/ block/ brick/ building material/ other material, smart garbage can/ waste container, smart food carriage/ storage, smart ball/ racket, smart chair/ sofa/ bed, smart shoe/ footwear/ carpet/ mat/ shoe rack, smart glove/ hand wear/ ring/ hand ware, smart hat/ headwear/ makeup/ sticker/ tattoo, smart mirror, smart toy, smart pill, smart utensil, smart bottle/food container, smart tool, smart device, IoT device, WiFi enabled device, network enabled device, 3G/4G/5G/6G enabled device, UMTS devices, 3GPP devices, GSM devices, EDGE devices, TDMA devices, FDMA devices, CDMA devices, WCDMA devices, TD-SCDMA devices, embeddable device, implantable device, air conditioner, refrigerator, heater, furnace, furniture, oven, cooking device, television/ set-top box (STB)/ DVD player/ audio player/ video player/ remote control, hi-fi, audio device, speaker, lamp/ light, wall, door, window, roof, roof tile/ shingle/ structure/ attic structure/ device/ feature/ installation/ fixtures, lawn mower/ garden tools/ yard tools/ mechanics tools/ garage tools/, garbage can/ container, 20-ft/40-ft container, storage container, factory/ manufacturing/ production device, repair tools, fluid container, machine, machinery to be installed, vehicle, cart, wagon, warehouse vehicle, car, bicycle, motorcycle, boat, vessel, airplane, basket/ box/ bag/ bucket/ container, smart plate/ cup/ bowl/ pot/ mat/ utensils/ kitchen tools/ kitchen devices/ kitchen accessories/ cabinets/ tables/ chairs/ tiles/ lights/ water pipes/ taps/ gas range/ oven/ dishwashing machine/ etc. The portable device may have a battery that may be replaceable, irreplaceable, rechargeable, and/or non-rechargeable. The portable device may be wirelessly charged. The portable device may be a smart payment card. The portable device may be a payment card used in parking lots, highways, entertainment parks, or other venues/facilities that need payment. The portable device may have an identity (ID)/identifier as described above.

An event may be monitored based on the TSCI. The event may be an object related event, such as fall-down of the object (e.g. an person and/or a sick person), rotation, hesitation, pause, impact (e.g. a person hitting a sandbag, door, window, bed, chair, table, desk, cabinet, box, another person, animal, bird, fly, table, chair, ball, bowling ball, tennis ball, football, soccer ball, baseball, basketball, volley ball), two-body action (e.g. a person letting go a balloon, catching a fish, molding a clay, writing a paper, person typing on a computer), car moving in a garage, person carrying a smart phone and walking around an airport/mall/government building/office/etc., autonomous moveable object/machine moving around (e.g. vacuum cleaner, utility vehicle, car, drone, self-driving car). The task or the wireless smart sensing task may comprise: object detection, presence detection, proximity detection, object recognition, activity recognition, object verification, object counting, daily activity monitoring, well-being monitoring, vital sign monitoring, health condition monitoring, baby monitoring, elderly monitoring, sleep monitoring, sleep stage monitoring, walking monitoring, exercise monitoring, tool detection, tool recognition, tool verification, patient detection, patient monitoring, patient verification, machine detection, machine recognition, machine verification, human detection, human recognition, human verification, baby detection, baby recognition, baby verification, human breathing detection, human breathing recognition, human breathing estimation, human breathing verification, human heart beat detection, human heart beat recognition, human heart beat estimation, human heart beat verification, fall-down detection, fall-down recognition, fall-down estimation, fall-down verification, emotion detection, emotion recognition, emotion estimation, emotion verification, motion detection, motion degree estimation, motion recognition, motion estimation, motion verification, periodic motion detection, periodic motion recognition, periodic motion estimation, periodic motion verification, repeated motion detection, repeated motion recognition, repeated motion estimation, repeated motion verification, stationary motion detection, stationary motion recognition, stationary motion estimation, stationary motion verification, cyclo-stationary motion detection, cyclo-stationary motion recognition, cyclo-stationary motion estimation, cyclo-stationary motion verification, transient motion detection, transient motion recognition, transient motion estimation, transient motion verification, trend detection, trend recognition, trend estimation, trend verification, breathing detection, breathing recognition, breathing estimation, breathing estimation, human biometrics detection, human biometric recognition, human biometrics estimation, human biometrics verification, environment informatics detection, environment informatics recognition, environment informatics estimation, environment informatics verification, gait detection, gait recognition, gait estimation, gait verification, gesture detection, gesture recognition, gesture estimation, gesture verification, machine learning, supervised learning, unsupervised learning, semi-supervised learning, clustering, feature extraction, featuring training, principal component analysis, eigen-decomposition, frequency decomposition, time decomposition, time-frequency decomposition, functional decomposition, other decomposition, training, discriminative training, supervised training, unsupervised training, semi-supervised training, neural network, sudden motion detection, fall-down detection, danger detection, life-threat detection, regular motion detection, stationary motion detection, cyclo-stationary motion detection, intrusion detection, suspicious motion detection, security, safety monitoring, navigation, guidance, map-based processing, map-based correction, model-based processing/correction, irregularity detection, locationing, room sensing, tracking, multiple object tracking, indoor tracking, indoor position, indoor navigation, energy management, power transfer, wireless power transfer, object counting, car tracking in parking garage, activating a device/system (e.g. security system, access system, alarm, siren, speaker, television, entertaining system, camera, heater/air-conditioning (HVAC) system, ventilation system, lighting system, gaming system, coffee machine, cooking device, cleaning device, housekeeping device), geometry estimation, augmented reality, wireless communication, data communication, signal broadcasting, networking, coordination, administration, encryption, protection, cloud computing, other processing and/or other task. The task may be performed by the Type 1 device, the Type 2 device, another Type 1 device, another Type 2 device, a nearby device, a local server (e.g. hub device), edge server, a cloud server, and/or another device. The task may be based on TSCI between any pair of Type 1 device and Type 2 device. A Type 2 device may be a Type 1 device, and vice versa. A Type 2 device may play/perform the role (e.g. functionality) of Type 1 device temporarily, continuously, sporadically, simultaneously, and/or contemporaneously, and vice versa. A first part of the task may comprise at least one of: preprocessing, processing, signal conditioning, signal processing, post-processing, processing sporadically/ continuously/ simultaneously/ contemporaneously/ dynamically/ adaptive/ on-demand/ as-needed, calibrating, denoising, feature extraction, coding, encryption, transformation, mapping, motion detection, motion estimation, motion change detection, motion pattern detection, motion pattern estimation, motion pattern recognition, vital sign detection, vital sign estimation, vital sign recognition, periodic motion detection, periodic motion estimation, repeated motion detection/estimation, breathing rate detection, breathing rate estimation, breathing pattern detection, breathing pattern estimation, breathing pattern recognition, heart beat detection, heart beat estimation, heart pattern detection, heart pattern estimation, heart pattern recognition, gesture detection, gesture estimation, gesture recognition, speed detection, speed estimation, object locationing, object tracking, navigation, acceleration estimation, acceleration detection, fall-down detection, change detection, intruder (and/or illegal action) detection, baby detection, baby monitoring, patient monitoring, object recognition, wireless power transfer, and/or wireless charging.

A second part of the task may comprise at least one of: a smart home task, smart office task, smart building task, smart factory task (e.g. manufacturing using a machine or an assembly line), smart internet-of-thing (IoT) task, smart system task, smart home operation, smart office operation, smart building operation, smart manufacturing operation (e.g. moving supplies/parts/raw material to a machine/an assembly line), IoT operation, smart system operation, turning on a light, turning off the light, controlling the light in at least one of: a room, region, and/or the venue, playing a sound clip, playing the sound clip in at least one of: the room, the region, and/or the venue, playing the sound clip of at least one of: a welcome, greeting, farewell, first message, and/or a second message associated with the first part of the task, turning on an appliance, turning off the appliance, controlling the appliance in at least one of: the room, the region, and/or the venue, turning on an electrical system, turning off the electrical system, controlling the electrical system in at least one of: the room, the region, and/or the venue, turning on a security system, turning off the security system, controlling the security system in at least one of: the room, the region, and/or the venue, turning on a mechanical system, turning off a mechanical system, controlling the mechanical system in at least one of: the room, the region, and/or the venue, and/or controlling at least one of: an air conditioning system, heating system, ventilation system, lighting system, heating device, stove, entertainment system, door, fence, window, garage, computer system, networked device, networked system, home appliance, office equipment, lighting device, robot (e.g. robotic arm), smart vehicle, smart machine, assembly line, smart device, internet-of-thing (IoT) device, smart home device, and/or a smart office device.

The task may include: detect a user returning home, detect a user leaving home, detect a user moving from one room to another, detect/control/lock/unlock/open/ close/partially open a window/door/garage door/blind/curtain/panel/solar panel/sun shade, detect a pet, detect/monitor a user doing something (e.g. sleeping on sofa, sleeping in bedroom, running on treadmill, cooking, sitting on sofa, watching TV, eating in kitchen, eating in dining room, going upstairs/downstairs, going outside/coming back, in the rest room), monitor/detect location of a user/pet, do something (e.g. send a message, notify/report to someone) automatically upon detection, do something for the user automatically upon detecting the user, turn on/off/dim a light, turn on/off music/radio/home entertainment system, turn on/off/adjust/control TV/HiFi/set-top-box (STB)/ home entertainment system/smart speaker/smart device, turn on/off/adjust air conditioning system, turn on/off/adjust ventilation system, turn on/off/adjust heating system, adjust/control curtains/light shades, turn on/off/wake a computer, turn on/off/pre-heat/control coffee machine/hot water pot, turn on/off/control/preheat cooker/oven/microwave oven/another cooking device, check/adjust temperature, check weather forecast, check telephone message box, check mail, do a system check, control/adjust a system, check/control/arm/disarm security system/baby monitor, check/control refrigerator, give a report (e.g. through a speaker such as Google home, Amazon Echo, on a display/screen, via a webpage/email/messaging system/notification system).

For example, when a user arrives home in his car, the task may be to, automatically, detect the user or his car approaching, open the garage door upon detection, turn on the driveway/garage light as the user approaches the garage, turn on air conditioner/ heater/ fan, etc. As the user enters the house, the task may be to, automatically, turn on the entrance light, turn off driveway/garage light, play a greeting message to welcome the user, turn on the music, turn on the radio and tuning to the user's favorite radio news channel, open the curtain/blind, monitor the user's mood, adjust the lighting and sound environment according to the user's mood or the current/imminent event (e.g. do romantic lighting and music because the user is scheduled to eat dinner with girlfriend in 1 hour) on the user's daily calendar, warm the food in microwave that the user prepared in the morning, do a diagnostic check of all systems in the house, check weather forecast for tomorrow's work, check news of interest to the user, check user's calendar and to-do list and play reminder, check telephone answer system/messaging system/email and give a verbal report using dialog system/speech synthesis, remind (e.g. using audible tool such as speakers/HiFi/ speech synthesis/sound/voice/ music/song/sound field/ background sound field/ dialog system, using visual tool such as TV/entertainment system/ computer/ notebook/smart pad/display/light/color/ brightness/patterns/symbols, using haptic tool/virtual reality tool/gesture/ tool, using a smart device/appliance/material/furniture/ fixture, using web tool/server/ hub device/cloud server/fog server/edge server/home network/mesh network, using messaging tool/notification tool/communication tool/scheduling tool/email, using user interface/GUI, using scent/smell/ fragrance/taste, using neural tool/nervous system tool, using a combination) the user of his mother's birthday and to call her, prepare a report, and give the report (e.g. using a tool for reminding as discussed above). The task may turn on the air conditioner/heater/ventilation system in advance, or adjust temperature setting of smart thermostat in advance, etc. As the user moves from the entrance to the living room, the task may be to turn on the living room light, open the living room curtain, open the window, turn off the entrance light behind the user, turn on the TV and set-top box, set TV to the user's favorite channel, adjust an appliance according to the user's preference and conditions/states (e.g. adjust lighting and choose/play music to build a romantic atmosphere), etc.

Another example may be: When the user wakes up in the morning, the task may be to detect the user moving around in the bedroom, open the blind/curtain, open the window, turn off the alarm clock, adjust indoor temperature from night-time temperature profile to day-time temperature profile, turn on the bedroom light, turn on the restroom light as the user approaches the restroom, check radio or streaming channel and play morning news, turn on the coffee machine and preheat the water, turn off security system, etc. When the user walks from bedroom to kitchen, the task may be to turn on the kitchen and hallway lights, turn off the bedroom and restroom lights, move the music/message/reminder from the bedroom to the kitchen, turn on the kitchen TV, change TV to morning news channel, lower the kitchen blind and open the kitchen window to bring in fresh air, unlock backdoor for the user to check the backyard, adjust temperature setting for the kitchen, etc. Another example may be: When the user leaves home for work, the task may be to detect the user leaving, play a farewell and/or have-a-good-day message, open/close garage door, turn on/off garage light and driveway light, turn off/dim lights to save energy (just in case the user forgets), close/lock all windows/doors (just in case the user forgets), turn off appliance (especially stove, oven, microwave oven), turn on/arm the home security system to guard the home against any intruder, adjust air conditioning/heating/ventilation systems to "away-from-home" profile to save energy, send alerts/reports/updates to the user's smart phone, etc.

A motion may comprise at least one of: a no-motion, resting motion, non-moving motion, movement, change in position/location, deterministic motion, transient motion, fall-down motion, repeating motion, periodic motion, pseudo-periodic motion, periodic/repeated motion associated with breathing, periodic/repeated motion associated with heartbeat, periodic/repeated motion associated with living object, periodic/repeated motion associated with machine, periodic/repeated motion associated with man-made object, periodic/repeated motion associated with nature, complex motion with transient element and periodic element, repetitive motion, non-deterministic motion, probabilistic motion, chaotic motion, random motion, complex motion with non-deterministic element and deterministic element, stationary random motion, pseudo-stationary random motion, cyclo-stationary random motion, non-stationary random motion, stationary random motion with periodic autocorrelation function (ACF), random motion with periodic ACF for period of time, random motion that is pseudo-stationary for a period of time, random motion of which an instantaneous ACF has a pseudo-periodic/repeating element for a period of time, machine motion, mechanical motion, vehicle motion, drone motion, air-related motion, wind-related motion, weather-related motion, water-related motion, fluid-related motion, ground-related motion, change in electro-magnetic characteristics, sub-surface motion, seismic motion, plant motion, animal motion, human motion, normal motion, abnormal motion, dangerous motion, warning motion, suspicious motion, rain, fire, flood, tsunami, explosion, collision, imminent collision, human body motion, head motion, facial motion, eye motion, mouth motion, tongue motion, neck motion, finger motion, hand motion, arm motion, shoulder motion, body motion, chest motion, abdominal motion, hip motion, leg motion, foot motion, body joint motion, knee motion, elbow motion, upper body motion, lower body motion, skin motion, below-skin motion, subcutaneous tissue motion, blood vessel motion, intravenous motion, organ motion, heart motion, lung motion, stomach motion, intestine motion, bowel motion, eating motion, breathing motion, facial expression, eye expression, mouth expression, talking motion, singing motion, eating motion, gesture, hand gesture, arm gesture, keystroke, typing stroke, user-interface gesture, man-machine interaction, gait, dancing movement, coordinated movement, and/or coordinated body movement.

The heterogeneous IC of the Type 1 device and/or any Type 2 receiver may comprise low-noise amplifier (LNA), power amplifier, transmit-receive switch, media access controller, baseband radio, 2.4 GHz radio, 3.65 GHz radio, 4.9 GHz radio, 5 GHz radio, 5.9GHz radio, below 6GHz radio, below 60 GHz radio and/or another radio. The heterogeneous IC may comprise a processor, a memory communicatively coupled with the processor, and a set of instructions stored in the memory to be executed by the processor. The IC and/or any processor may comprise at least one of: general purpose processor, special purpose processor, microprocessor, multi-processor, multi-core processor, parallel processor, CISC processor, RISC processor, microcontroller, central processing unit (CPU), graphical processor unit (GPU), digital signal processor (DSP), application specific integrated circuit (ASIC), field programmable gate array (FPGA), embedded processor (e.g. ARM), logic circuit, other programmable logic device, discrete logic, and/or a combination. The heterogeneous IC may support broadband network, wireless network, mobile network, mesh network, cellular network, wireless local area network (WLAN), wide area network (WAN), and metropolitan area network (MAN), WLAN standard, WiFi, LTE, LTE-A, LTE-U, 802.11 standard, 802.11a, 802.11b, 802.11g, 802.11n, 802.11ac, 802.11ad, 802.11af, 802,11ah, 802.11ax, 802.11ay, mesh network standard, 802.15 standard, 802.16 standard, cellular network standard, 3G, 3.5G, 4G, beyond 4G, 4.5G, 5G, 6G, 7G, 8G, 9G, UMTS, 3GPP, GSM, EDGE, TDMA, FDMA, CDMA, WCDMA, TD-SCDMA, Bluetooth, Bluetooth Low-Energy (BLE), NFC, Zigbee, WiMax, and/or another wireless network protocol.

The processor may comprise general purpose processor, special purpose processor, microprocessor, microcontroller, embedded processor, digital signal processor, central processing unit (CPU), graphical processing unit (GPU), multi-processor, multi-core processor, and/or processor with graphics capability, and/or a combination. The memory may be volatile, non-volatile, random access memory (RAM), Read Only Memory (ROM), Electrically Programmable ROM (EPROM), Electrically Erasable Programmable ROM (EEPROM), hard disk, flash memory, CD-ROM, DVD-ROM, magnetic storage, optical storage, organic storage, storage system, storage network, network storage, cloud storage, edge storage, local storage, external storage, internal storage, or other form of non-transitory storage medium known in the art. The set of instructions (machine executable code) corresponding to the method steps may be embodied directly in hardware, in software, in firmware, or in combinations thereof. The set of instructions may be embedded, pre-loaded, loaded upon boot up, loaded on the fly, loaded on demand, pre-installed, installed, and/or downloaded.

The presentation may be a presentation in an audio-visual way (e.g. using combination of visual, graphics, text, symbols, color, shades, video, animation, sound, speech, audio, etc.), graphical way (e.g. using GUI, animation, video), textual way (e.g. webpage with text, message, animated text), symbolic way (e.g. emoticon, signs, hand gesture), or mechanical way (e.g. vibration, actuator movement, haptics, etc.).

Computational workload associated with the method is shared among the processor, the Type 1 heterogeneous wireless device, the Type 2 heterogeneous wireless device, a local server (e.g. hub device), a cloud server, and another processor.

An operation, pre-processing, processing and/or postprocessing may be applied to data (e.g. TSCI, autocorrelation, features of TSCI). An operation may be preprocessing, processing and/or postprocessing. The preprocessing, processing and/or postprocessing may be an operation. An operation may comprise preprocessing, processing, post-processing, scaling, computing a confidence factor, computing a line-of-sight (LOS) quantity, computing a non-LOS (NLOS) quantity, a quantity comprising LOS and NLOS, computing a single link (e.g. path, communication path, link between a transmitting antenna and a receiving antenna) quantity, computing a quantity comprising multiple links, computing a function of the operands, filtering, linear filtering, nonlinear filtering, folding, grouping, energy computation, lowpass filtering, bandpass filtering, highpass filtering, median filtering, rank filtering, quartile filtering, percentile filtering, mode filtering, finite impulse response (FIR) filtering, infinite impulse response (IIR) filtering, moving average (MA) filtering, autoregressive (AR) filtering, autoregressive moving averaging (ARMA) filtering, selective filtering, adaptive filtering, interpolation, decimation, subsampling, upsampling, resampling, time correction, time base correction, phase correction, magnitude correction, phase cleaning, magnitude cleaning, matched filtering, enhancement, restoration, denoising, smoothing, signal conditioning, enhancement, restoration, spectral analysis, linear transform, nonlinear transform, inverse transform, frequency transform, inverse frequency transform, Fourier transform (FT), discrete time FT (DTFT), discrete FT (DFT), fast FT (FFT), wavelet transform, Laplace transform, Hilbert transform, Hadamard transform, trigonometric transform, sine transform, cosine transform, DCT, power-of-2 transform, sparse transform, graph-based transform, graph signal processing, fast transform, a transform combined with zero padding, cyclic padding, padding, zero padding, feature extraction, decomposition, projection, orthogonal projection, non-orthogonal projection, over-complete projection, eigen-decomposition, singular value decomposition (SVD), principle component analysis (PCA), independent component analysis (ICA), grouping, sorting, thresholding, soft thresholding, hard thresholding, clipping, soft clipping, first derivative, second order derivative, high order derivative, convolution, multiplication, division, addition, subtraction, integration, maximization, minimization, least mean square error, recursive least square, constrained least square, batch least square, least absolute error, least mean square deviation, least absolute deviation, local maximization, local minimization, optimization of a cost function, neural network, recognition, labeling, training, clustering, machine learning, supervised learning, unsupervised learning, semi-supervised learning, comparison with another TSCI, similarity score computation, quantization, vector quantization, matching pursuit, compression, encryption, coding, storing, transmitting, normalization, temporal normalization, frequency domain normalization, classification, clustering, labeling, tagging, learning, detection, estimation, learning network, mapping, remapping, expansion, storing, retrieving, transmitting, receiving, representing, merging, combining, splitting, tracking, monitoring, matched filtering, Kalman filtering, particle filter, intrapolation, extrapolation, histogram estimation, importance sampling, Monte Carlo sampling, compressive sensing, representing, merging, combining, splitting, scrambling, error protection, forward error correction, doing nothing, time varying processing, conditioning averaging, weighted averaging, arithmetic mean, geometric mean, harmonic mean, averaging over selected frequency, averaging over antenna links, logical operation, permutation, combination, sorting, AND, OR, XOR, union, intersection, vector addition, vector subtraction, vector multiplication, vector division, inverse, norm, distance, and/or another operation. The operation may be the preprocessing, processing, and/or post-processing. Operations may be applied jointly on multiple time series or functions.

The function (e.g. function of operands) may comprise: scalar function, vector function, discrete function, continuous function, polynomial function, characteristics, feature, magnitude, phase, exponential function, logarithmic function, trigonometric function, transcendental function, logical function, linear function, algebraic function, nonlinear function, piecewise linear function, real function, complex function, vector-valued function, inverse function, derivative of function, integration of function, circular function, function of another function, one-to-one function, one-to-many function, many-to-one function, many-to-many function, zero crossing, absolute function, indicator function, mean, mode, median, range, statistics, histogram, variance, standard deviation, measure of variation, spread, dispersion, deviation, divergence, range, interquartile range, total variation, absolute deviation, total deviation, arithmetic mean, geometric mean, harmonic mean, trimmed mean, percentile, square, cube, root, power, sine, cosine, tangent, cotangent, secant, cosecant, elliptical function, parabolic function, hyperbolic function, game function, zeta function, absolute value, thresholding, limiting function, floor function, rounding function, sign function, quantization, piecewise constant function, composite function, function of function, time function processed with an operation (e.g. filtering), probabilistic function, stochastic function, random function, ergodic function, stationary function, deterministic function, periodic function, repeated function, transformation, frequency transform, inverse frequency transform, discrete time transform, Laplace transform, Hilbert transform, sine transform, cosine transform, triangular transform, wavelet transform, integer transform, power-of-2 transform, sparse transform, projection, decomposition, principle component analysis (PCA), independent component analysis (ICA), neural network, feature extraction, moving function, function of moving window of neighboring items of time series, filtering function, convolution, mean function, histogram, variance/standard deviation function, statistical function, short-time transform, discrete transform, discrete Fourier transform, discrete cosine transform, discrete sine transform, Hadamard transform, eigen-decomposition, eigenvalue, singular value decomposition (SVD), singular value, orthogonal decomposition, matching pursuit, sparse transform, sparse approximation, any decomposition, graph-based processing, graph-based transform, graph signal processing, classification, identifying a class/group/category, labeling, learning, machine learning, detection, estimation, feature extraction, learning network, feature extraction, denoising, signal enhancement, coding, encryption, mapping, remapping, vector quantization, lowpass filtering, highpass filtering, bandpass filtering, matched filtering, Kalman filtering, preprocessing, postprocessing, particle filter, FIR filtering, IIR filtering, autoregressive (AR) filtering, adaptive filtering, first order derivative, high order derivative, integration, zero crossing, smoothing, median filtering, mode filtering, sampling, random sampling, resampling function, downsampling, down-converting, upsampling, up-converting, interpolation, extrapolation, importance sampling, Monte Carlo sampling, compressive sensing, statistics, short term statistics, long term statistics, autocorrelation function, cross correlation, moment generating function, time averaging, weighted averaging, special function, Bessel function, error function, complementary error function, Beta function, Gamma function, integral function, Gaussian function, Poisson function, etc. Machine learning, training, discriminative training, deep learning, neural network, continuous time processing, distributed computing, distributed storage, acceleration using GPU/DSP/coprocessor/multicore/multiprocessing may be applied to a step (or each step) of this disclosure.

A frequency transform may include Fourier transform, Laplace transform, Hadamard transform, Hilbert transform, sine transform, cosine transform, triangular transform, wavelet transform, integer transform, power-of-2 transform, combined zero padding and transform, Fourier transform with zero padding, and/or another transform. Fast versions and/or approximated versions of the transform may be performed. The transform may be performed using floating point, and/or fixed point arithmetic.

An inverse frequency transform may include inverse Fourier transform, inverse Laplace transform, inverse Hadamard transform, inverse Hilbert transform, inverse sine transform, inverse cosine transform, inverse triangular transform, inverse wavelet transform, inverse integer transform, inverse power-of-2 transform, combined zero padding and transform, inverse Fourier transform with zero padding, and/or another transform. Fast versions and/or approximated versions of the transform may be performed. The transform may be performed using floating point, and/or fixed point arithmetic.

A quantity/feature from a TSCI may be computed. The quantity may comprise statistic of at least one of: motion, location, map coordinate, height, speed, acceleration, movement angle, rotation, size, volume, time trend, pattern, one-time pattern, repeating pattern, evolving pattern, time pattern, mutually excluding patterns, related/correlated patterns, cause-and-effect, correlation, short-term/long-term correlation, tendency, inclination, statistics, typical behavior, atypical behavior, time trend, time profile, periodic motion, repeated motion, repetition, tendency, change, abrupt change, gradual change, frequency, transient, breathing, gait, action, event, suspicious event, dangerous event, alarming event, warning, belief, proximity, collision, power, signal, signal power, signal strength, signal intensity, received signal strength indicator (RSSI), signal amplitude, signal phase, signal frequency component, signal frequency band component, channel state information (CSI), map, time, frequency, time-frequency, decomposition, orthogonal decomposition, non-orthogonal decomposition, tracking, breathing, heart beat, statistical parameters, cardiopulmonary statistics/analytics (e.g. output responses), daily activity statistics/analytics, chronic disease statistics/analytics, medical statistics/analytics, an early (or instantaneous or contemporaneous or delayed) indication/suggestion/sign/indicator/verifier/ detection/symptom of a disease/condition/ situation, biometric, baby, patient, machine, device, temperature, vehicle, parking lot, venue, lift, elevator, spatial, road, fluid flow, home, room, office, house, building, warehouse, storage, system, ventilation, fan, pipe, duct, people, human, car, boat, truck, airplane, drone, downtown, crowd, impulsive event, cyclo-stationary, environment, vibration, material, surface, 3-dimensional, 2-dimensional, local, global, presence, and/or another measurable quantity/variable.

Sliding time window may have time varying window width. It may be smaller at the beginning to enable fast acquisition and may increase over time to a steady-state size. The steady-state size may be related to the frequency, repeated motion, transient motion, and/or STI to be monitored. Even in steady state, the window size may be adaptively (and/or dynamically) changed (e.g. adjusted, varied, modified) based on battery life, power consumption, available computing power, change in amount of targets, the nature of motion to be monitored, etc.

The time shift between two sliding time windows at adjacent time instance may be constant/variable/locally adaptive/dynamically adjusted over time. When shorter time shift is used, the update of any monitoring may be more frequent which may be used for fast changing situations, object motions, and/or objects. Longer time shift may be used for slower situations, object motions, and/or objects. The window width/size and/or time shift may be changed (e.g. adjusted, varied, modified) upon a user request/choice. The time shift may be changed automatically (e.g. as controlled by processor/ computer/server/hub device/cloud server) and/or adaptively (and/or dynamically).

At least one characteristics (e.g. characteristic value, or characteristic point) of a function (e.g. auto-correlation function, auto-covariance function, cross-correlation function, cross-covariance function, power spectral density, time function, frequency domain function, frequency transform) may be determined (e.g. by an object tracking server, the processor, the Type 1 heterogeneous device, the Type 2 heterogeneous device, and/or another device). The at least one characteristics of the function may include: a maximum, minimum, extremum, local maximum, local minimum, local extremum, local extremum with positive time offset, first local extremum with positive time offset, n^th local extremum with positive time offset, local extremum with negative time offset, first local extremum with negative time offset, n^th local extremum with negative time offset, constrained maximum, constrained minimum, constrained extremum, significant maximum, significant minimum, significant extremum, slope, derivative, higher order derivative, maximum slope, minimum slope, local maximum slope, local maximum slope with positive time offset, local minimum slope, constrained maximum slope, constrained minimum slope, maximum higher order derivative, minimum higher order derivative, constrained higher order derivative, zero-crossing, zero crossing with positive time offset, n^th zero crossing with positive time offset, zero crossing with negative time offset, n^th zero crossing with negative time offset, constrained zero-crossing, zero-crossing of slope, zero-crossing of higher order derivative, and/or another characteristics. At least one argument of the function associated with the at least one characteristics of the function may be identified. Some quantity (e.g. spatial-temporal information of the object) may be determined based on the at least one argument of the function.

A characteristics (e.g. characteristics of motion of an object in the venue) may comprise at least one of: an instantaneous characteristics, short-term characteristics, repetitive characteristics, recurring characteristics, history, incremental characteristics, changing characteristics, deviational characteristics, phase, magnitude, degree, time characteristics, frequency characteristics, time-frequency characteristics, decomposition characteristics, orthogonal decomposition characteristics, non-orthogonal decomposition characteristics, deterministic characteristics, probabilistic characteristics, stochastic characteristics, autocorrelation function (ACF), mean, variance, standard deviation, measure of variation, spread, dispersion, deviation, divergence, range, interquartile range, total variation, absolute deviation, total deviation, statistics, duration, timing, trend, periodic characteristics, repetition characteristics, long-term characteristics, historical characteristics, average characteristics, current characteristics, past characteristics, future characteristics, predicted characteristics, location, distance, height, speed, direction, velocity, acceleration, change of the acceleration, angle, angular speed, angular velocity, angular acceleration of the object, change of the angular acceleration, orientation of the object, angular of rotation, deformation of the object, shape of the object, change of shape of the object, change of size of the object, change of structure of the object, and/or change of characteristics of the object.

At least one local maximum and at least one local minimum of the function may be identified. At least one local signal-to-noise-ratio-like (SNR-like) parameter may be computed for each pair of adjacent local maximum and local minimum. The SNR-like parameter may be a function (e.g. linear, log, exponential function, monotonic function) of a fraction of a quantity (e.g. power, magnitude) of the local maximum over the same quantity of the local minimum. It may also be the function of a difference between the quantity of the local maximum and the same quantity of the local minimum. Significant local peaks may be identified or selected. Each significant local peak may be a local maximum with SNR-like parameter greater than a threshold T1 and/or a local maximum with amplitude greater than a threshold T2. The at least one local minimum and the at least one local minimum in the frequency domain may be identified/computed using a persistence-based approach.

A set of selected significant local peaks may be selected from the set of identified significant local peaks based on a selection criterion (e.g. a quality criterion, a signal quality condition). The characteristics/STI of the object may be computed based on the set of selected significant local peaks and frequency values associated with the set of selected significant local peaks. In one example, the selection criterion may always correspond to select the strongest peaks in a range. While the strongest peaks may be selected, the unselected peaks may still be significant (rather strong).

Unselected significant peaks may be stored and/or monitored as "reserved" peaks for use in future selection in future sliding time windows. As an example, there may be a particular peak (at a particular frequency) appearing consistently over time. Initially, it may be significant but not selected (as other peaks may be stronger). But in later time, the peak may become stronger and more dominant and may be selected. When it became "selected", it may be back-traced in time and made "selected" in the earlier time when it was significant but not selected. In such case, the back-traced peak may replace a previously selected peak in an early time. The replaced peak may be the relatively weakest, or a peak that appear in isolation in time (i.e. appearing only briefly in time).

In another example, the selection criterion may not correspond to select the strongest peaks in the range. Instead, it may consider not only the "strength" of the peak, but the "trace" of the peak - peaks that may have happened in the past, especially those peaks that have been identified for a long time. For example, if a finite state machine (FSM) is used, it may select the peak(s) based on the state of the FSM. Decision thresholds may be computed adaptively (and/or dynamically) based on the state of the FSM.

A similarity score and/or component similarity score may be computed (e.g. by a server (e.g. hub device), the processor, the Type 1 device, the Type 2 device, a local server, a cloud server, and/or another device) based on a pair of temporally adjacent CI of a TSCI. The pair may come from the same sliding window or two different sliding windows. The similarity score may also be based on a pair of, temporally adjacent or not so adjacent, CI from two different TSCI. The similarity score and/or component similar score may be/comprise: time reversal resonating strength (TRRS), correlation, cross-correlation, auto-correlation, correlation indicator, covariance, cross-covariance, auto-covariance, inner product of two vectors, distance score, norm, metric, quality metric, signal quality condition, statistical characteristics, discrimination score, neural network, deep learning network, machine learning, training, discrimination, weighted averaging, preprocessing, denoising, signal conditioning, filtering, time correction, timing compensation, phase offset compensation, transformation, component-wise operation, feature extraction, finite state machine, and/or another score. The characteristics and/or STI may be determined/computed based on the similarity score.

Any threshold may be pre-determined, adaptively (and/or dynamically) determined and/or determined by a finite state machine. The adaptive determination may be based on time, space, location, antenna, path, link, state, battery life, remaining battery life, available power, available computational resources, available network bandwidth, etc.

A threshold to be applied to a test statistics to differentiate two events (or two conditions, or two situations, or two states), A and B, may be determined. Data (e.g. CI, channel state information (CSI), power parameter) may be collected under A and/or under B in a training situation. The test statistics may be computed based on the data. Distributions of the test statistics under A may be compared with distributions of the test statistics under B (reference distribution), and the threshold may be chosen according to some criteria. The criteria may comprise: maximum likelihood (ML), maximum aposterior probability (MAP), discriminative training, minimum Type 1 error for a given Type 2 error, minimum Type 2 error for a given Type 1 error, and/or other criteria (e.g. a quality criterion, signal quality condition). The threshold may be adjusted to achieve different sensitivity to the A, B and/or another event/condition/situation/state. The threshold adjustment may be automatic, semi-automatic and/or manual. The threshold adjustment may be applied once, sometimes, often, periodically, repeatedly, occasionally, sporadically, and/or on demand. The threshold adjustment may be adaptive (and/or dynamically adjusted). The threshold adjustment may depend on the object, object movement/location/ direction/action, object characteristics/ STI/size/ property/trait/habit/behavior, the venue, feature/ fixture/ furniture/barrier/ material/ machine/living thing/thing/object/boundary/ surface/ medium that is in/at/of the venue, map, constraint of the map (or environmental model), the event/state/ situation/condition, time, timing, duration, current state, past history, user, and/or a personal preference, etc.

A stopping criterion (or skipping or bypassing or blocking or pausing or passing or rejecting criterion) of an iterative algorithm may be that change of a current parameter (e.g. offset value) in the updating in an iteration is less than a threshold. The threshold may be 0.5, 1, 1.5, 2, or another number. The threshold may be adaptive (and/or dynamically adjusted). It may change as the iteration progresses. For the offset value, the adaptive threshold may be determined based on the task, particular value of the first time, the current time offset value, the regression window, the regression analysis, the regression function, the regression error, the convexity of the regression function, and/or an iteration number.

The local extremum may be determined as the corresponding extremum of the regression function in the regression window. The local extremum may be determined based on a set of time offset values in the regression window and a set of associated regression function values. Each of the set of associated regression function values associated with the set of time offset values may be within a range from the corresponding extremum of the regression function in the regression window.

The searching for a local extremum may comprise robust search, minimization, maximization, optimization, statistical optimization, dual optimization, constraint optimization, convex optimization, global optimization, local optimization an energy minimization, linear regression, quadratic regression, higher order regression, linear programming, nonlinear programming, stochastic programming, combinatorial optimization, constraint programming, constraint satisfaction, calculus of variations, optimal control, dynamic programming, mathematical programming, multi-objective optimization, multi-modal optimization, disjunctive programming, space mapping, infinite-dimensional optimization, heuristics, metaheuristics, convex programming, semidefinite programming, conic programming, cone programming, integer programming, quadratic programming, fractional programming, numerical analysis, simplex algorithm, iterative method, gradient descent, subgradient method, coordinate descent, conjugate gradient method, Newton's algorithm, sequential quadratic programming, interior point method, ellipsoid method, reduced gradient method, quasi-Newton method, simultaneous perturbation stochastic approximation, interpolation method, pattern search method, line search, non-differentiable optimization, genetic algorithm, evolutionary algorithm, dynamic relaxation, hill climbing, particle swarm optimization, gravitation search algorithm, simulated annealing, memetic algorithm, differential evolution, dynamic relaxation, stochastic tunneling, Tabu search, reactive search optimization, curve fitting, least square, simulation based optimization, variational calculus, and/or variant. The search for local extremum may be associated with an objective function, loss function, cost function, utility function, fitness function, energy function, and/or an energy function.

Regression may be performed using regression function to fit sampled data (e.g. CI, feature of CI, component of CI) or another function (e.g. autocorrelation function) in a regression window. In at least one iteration, a length of the regression window and/or a location of the regression window may change. The regression function may be linear function, quadratic function, cubic function, polynomial function, and/or another function. The regression analysis may minimize at least one of: error, aggregate error, component error, error in projection domain, error in selected axes, error in selected orthogonal axes, absolute error, square error, absolute deviation, square deviation, higher order error (e.g. third order, fourth order), robust error (e.g. square error for smaller error magnitude and absolute error for larger error magnitude, or first kind of error for smaller error magnitude and second kind of error for larger error magnitude), another error, weighted sum (or weighted mean) of absolute/square error (e.g. for wireless transmitter with multiple antennas and wireless receiver with multiple antennas, each pair of transmitter antenna and receiver antenna form a link), mean absolute error, mean square error, mean absolute deviation, and/or mean square deviation. Error associated with different links may have different weights. One possibility is that some links and/or some components with larger noise or lower signal quality metric may have smaller or bigger weight.), weighted sum of square error, weighted sum of higher order error, weighted sum of robust error, weighted sum of the another error, absolute cost, square cost, higher order cost, robust cost, another cost, weighted sum of absolute cost, weighted sum of square cost, weighted sum of higher order cost, weighted sum of robust cost, and/or weighted sum of another cost. The regression error determined may be an absolute error, square error, higher order error, robust error, yet another error, weighted sum of absolute error, weighted sum of square error, weighted sum of higher order error, weighted sum of robust error, and/or weighted sum of the yet another error.

The time offset associated with maximum regression error (or minimum regression error) of the regression function with respect to the particular function in the regression window may become the updated current time offset in the iteration.

A local extremum may be searched based on a quantity comprising a difference of two different errors (e.g. a difference between absolute error and square error). Each of the two different errors may comprise an absolute error, square error, higher order error, robust error, another error, weighted sum of absolute error, weighted sum of square error, weighted sum of higher order error, weighted sum of robust error, and/or weighted sum of the another error.

The quantity may be compared with a reference data or a reference distribution, such as an F-distribution, central F-distribution, another statistical distribution, threshold, threshold associated with probability/histogram, threshold associated with probability/histogram of finding false peak, threshold associated with the F-distribution, threshold associated the central F-distribution, and/or threshold associated with the another statistical distribution.

The regression window may be determined based on at least one of: the movement (e.g. change in position/location) of the object, quantity associated with the object, the at least one characteristics and/or STI of the object associated with the movement of the object, estimated location of the local extremum, noise characteristics, estimated noise characteristics, signal quality metric, F-distribution, central F-distribution, another statistical distribution, threshold, preset threshold, threshold associated with probability/histogram, threshold associated with desired probability, threshold associated with probability of finding false peak, threshold associated with the F-distribution, threshold associated the central F-distribution, threshold associated with the another statistical distribution, condition that quantity at the window center is largest within the regression window, condition that the quantity at the window center is largest within the regression window, condition that there is only one of the local extremum of the particular function for the particular value of the first time in the regression window, another regression window, and/or another condition.

The width of the regression window may be determined based on the particular local extremum to be searched. The local extremum may comprise first local maximum, second local maximum, higher order local maximum, first local maximum with positive time offset value, second local maximum with positive time offset value, higher local maximum with positive time offset value, first local maximum with negative time offset value, second local maximum with negative time offset value, higher local maximum with negative time offset value, first local minimum, second local minimum, higher local minimum, first local minimum with positive time offset value, second local minimum with positive time offset value, higher local minimum with positive time offset value, first local minimum with negative time offset value, second local minimum with negative time offset value, higher local minimum with negative time offset value, first local extremum, second local extremum, higher local extremum, first local extremum with positive time offset value, second local extremum with positive time offset value, higher local extremum with positive time offset value, first local extremum with negative time offset value, second local extremum with negative time offset value, and/or higher local extremum with negative time offset value.

A current parameter (e.g. time offset value) may be initialized based on a target value, target profile, trend, past trend, current trend, target speed, speed profile, target speed profile, past speed trend, the motion or movement (e.g. change in position/location) of the object, at least one characteristics and/or STI of the object associated with the movement of object, positional quantity of the object, initial speed of the object associated with the movement of the object, predefined value, initial width of the regression window, time duration, value based on carrier frequency of the signal, value based on subcarrier frequency of the signal, bandwidth of the signal, amount of antennas associated with the channel, noise characteristics, signal h metric, and/or an adaptive (and/or dynamically adjusted) value. The current time offset may be at the center, on the left side, on the right side, and/or at another fixed relative location, of the regression window.

In the presentation, information may be displayed with a map (or environmental model) of the venue. The information may comprise: location, zone, region, area, coverage area, corrected location, approximate location, location with respect to (w.r.t.) a map of the venue, location w.r.t. a segmentation of the venue, direction, path, path w.r.t. the map and/or the segmentation, trace (e.g. location within a time window such as the past 5 seconds, or past 10 seconds; the time window duration may be adjusted adaptively (and/or dynamically); the time window duration may be adaptively (and/or dynamically) adjusted w.r.t. speed, acceleration, etc.), history of a path, approximate regions/zones along a path, history/ summary of past locations, history of past locations of interest, frequently-visited areas, customer traffic, crowd distribution, crowd behavior, crowd control information, speed, acceleration, motion statistics, breathing rate, heart rate, presence/absence of motion, presence/absence of people or pets or object, presence/absence of vital sign, gesture, gesture control (control of devices using gesture), location-based gesture control, information of a location-based operation, identity (ID) or identifier of the respect object (e.g. pet, person, self-guided machine/device, vehicle, drone, car, boat, bicycle, self-guided vehicle, machine with fan, air-conditioner, TV, machine with movable part), identification of a user (e.g. person), information of the user, location/speed/acceleration/direction/motion/gesture/gesture control/ motion trace of the user, ID or identifier of the user, activity of the user, state of the user, sleeping/resting characteristics of the user, emotional state of the user, vital sign of the user, environment information of the venue, weather information of the venue, earthquake, explosion, storm, rain, fire, temperature, collision, impact, vibration, event, door-open event, door-close event, window-open event, window-close event, fall-down event, burning event, freezing event, water-related event, wind-related event, air-movement event, accident event, pseudo-periodic event (e.g. running on treadmill, jumping up and down, skipping rope, somersault, etc.), repeated event, crowd event, vehicle event, gesture of the user (e.g. hand gesture, arm gesture, foot gesture, leg gesture, body gesture, head gesture, face gesture, mouth gesture, eye gesture, etc.). The location may be 2-dimensional (e.g. with 2D coordinates), 3-dimensional (e.g. with 3D coordinates). The location may be relative (e.g. w.r.t. a map or environmental model) or relational (e.g. halfway between point A and point B, around a corner, up the stairs, on top of table, at the ceiling, on the floor, on a sofa, close to point A, a distance R from point A, within a radius of R from point A, etc.). The location may be expressed in rectangular coordinate, polar coordinate, and/or another representation.

The information (e.g. location) may be marked with at least one symbol. The symbol may be time varying. The symbol may be flashing and/or pulsating with or without changing color/intensity. The size may change over time. The orientation of the symbol may change over time. The symbol may be a number that reflects an instantaneous quantity (e.g. vital sign/ breathing rate/heart rate/gesture/state/status/action/motion of a user, temperature, network traffic, network connectivity, status of a device/machine, remaining power of a device, status of the device, etc.). The rate of change, the size, the orientation, the color, the intensity and/or the symbol may reflect the respective motion. The information may be presented visually and/or described verbally (e.g. using prerecorded voice, or voice synthesis). The information may be described in text. The information may also be presented in a mechanical way (e.g. an animated gadget, a movement of a movable part).

The user-interface (UI) device may be a smart phone (e.g. iPhone, Android phone), tablet (e.g. iPad), laptop (e.g. notebook computer), personal computer (PC), device with graphical user interface (GUI), smart speaker, device with voice/audio/speaker capability, virtual reality (VR) device, augmented reality (AR) device, smart car, display in the car, voice assistant, voice assistant in a car, etc. The map (or environmental model) may be 2-dimensional, 3-dimensional and/or higher-dimensional. (e.g. a time varying 2D/3D map/environmental model) Walls, windows, doors, entrances, exits, forbidden areas may be marked on the map or the model. The map may comprise floor plan of a facility. The map or model may have one or more layers (overlays). The map/model may be a maintenance map/model comprising water pipes, gas pipes, wiring, cabling, air ducts, crawl-space, ceiling layout, and/or underground layout. The venue may be segmented/subdivided/zoned/grouped into multiple zones/ regions/geographic regions/ sectors/ sections/ territories/ districts/ precincts/ localities/ neighborhoods/ areas/ stretches/expanse such as bedroom, living room, storage room, walkway, kitchen, dining room, foyer, garage, first floor, second floor, rest room, offices, conference room, reception area, various office areas, various warehouse regions, various facility areas, etc. The segments/regions/areas may be presented in a map/model. Different regions may be color-coded. Different regions may be presented with a characteristic (e.g. color, brightness, color intensity, texture, animation, flashing, flashing rate, etc.). Logical segmentation of the venue may be done using the at least one heterogeneous Type 2 device, or a server (e.g. hub device), or a cloud server, etc.

Here is an example of the disclosed system, apparatus, and method. Stephen and his family want to install the disclosed wireless motion detection system to detect motion in their 2000 sqft two-storey town house in Seattle, Washington. Because his house has two storeys, Stephen decided to use one Type 2 device (named A) and two Type 1 devices (named B and C) in the ground floor. His ground floor has predominantly three rooms: kitchen, dining room and living room arranged in a straight line, with the dining room in the middle. The kitchen and the living rooms are on opposite end of the house. He put the Type 2 device (A) in the dining room, and put one Type 1 device (B) in the kitchen and the other Type 1 device (C) in the living room. With this placement of the devices, he is practically partitioning the ground floor into 3 zones (dining room, living room and kitchen) using the motion detection system. When motion is detected by the AB pair and the AC pair, the system would analyze the motion information and associate the motion with one of the 3 zones.

When Stephen and his family go out on weekends (e.g. to go for a camp during a long weekend), Stephen would use a mobile phone app (e.g. Android phone app or iPhone app) to turn on the motion detection system. When the system detects motion, a warning signal is sent to Stephen (e.g. an SMS text message, an email, a push message to the mobile phone app, etc.). If Stephen pays a monthly fee (e.g. $10/month), a service company (e.g. security company) will receive the warning signal through wired network (e.g. broadband) or wireless network (e.g. home WiFi, LTE, 3G, 2.5G, etc.) and perform a security procedure for Stephen (e.g. call him to verify any problem, send someone to check on the house, contact the police on behalf of Stephen, etc.). Stephen loves his aging mother and cares about her well-being when she is alone in the house. When the mother is alone in the house while the rest of the family is out (e.g. go to work, or shopping, or go on vacation), Stephen would turn on the motion detection system using his mobile app to ensure the mother is ok. He then uses the mobile app to monitor his mother's movement in the house. When Stephen uses the mobile app to see that the mother is moving around the house among the 3 regions, according to her daily routine, Stephen knows that his mother is doing ok. Stephen is thankful that the motion detection system can help him monitor his mother's well-being while he is away from the house.

On a typical day, the mother would wake up at around 7 AM. She would cook her breakfast in the kitchen for about 20 minutes. Then she would eat the breakfast in the dining room for about 30 minutes. Then she would do her daily exercise in the living room, before sitting down on the sofa in the living room to watch her favorite TV show. The motion detection system enables Stephen to see the timing of the movement in each of the 3 regions of the house. When the motion agrees with the daily routine, Stephen knows roughly that the mother should be doing fine. But when the motion pattern appears abnormal (e.g. there is no motion until 10 AM, or she stayed in the kitchen for too long, or she remains motionless for too long, etc.), Stephen suspects something is wrong and would call the mother to check on her. Stephen may even get someone (e.g. a family member, a neighbor, a paid personnel, a friend, a social worker, a service provider) to check on his mother.

At some time, Stephen feels like repositioning the Type 2 device. He simply unplugs the device from the original AC power plug and plug it into another AC power plug. He is happy that the wireless motion detection system is plug-and-play and the repositioning does not affect the operation of the system. Upon powering up, it works right away. Sometime later, Stephen is convinced that our wireless motion detection system can really detect motion with very high accuracy and very low alarm, and he really can use the mobile app to monitor the motion in the ground floor. He decides to install a similar setup (i.e. one Type 2 device and two Type 1 devices) in the second floor to monitor the bedrooms in the second floor. Once again, he finds that the system set up is extremely easy as he simply needs to plug the Type 2 device and the Type 1 devices into the AC power plug in the second floor. No special installation is needed. And he can use the same mobile app to monitor motion in the ground floor and the second floor. Each Type 2 device in the ground floor/second floor can interact with all the Type 1 devices in both the ground floor and the second floor. Stephen is happy to see that, as he doubles his investment in the Type 1 and Type 2 devices, he has more than double the capability of the combined systems.

According to various embodiments, each CI (CI) may comprise at least one of: channel state information (CSI), frequency domain CSI, frequency representation of CSI, frequency domain CSI associated with at least one sub-band, time domain CSI, CSI in domain, channel response, estimated channel response, channel impulse response (CIR), channel frequency response (CFR), channel characteristics, channel filter response, CSI of the wireless multipath channel, information of the wireless multipath channel, timestamp, auxiliary information, data, meta data, user data, account data, access data, security data, session data, status data, supervisory data, household data, identity (ID), identifier, device data, network data, neighborhood data, environment data, real-time data, sensor data, stored data, encrypted data, compressed data, protected data, and/or another CI. In one embodiment, the disclosed system has hardware components (e.g. wireless transmitter/receiver with antenna, analog circuitry, power supply, processor, memory) and corresponding software components. According to various embodiments of the present teaching, the disclosed system includes Bot (referred to as a Type 1 device) and Origin (referred to as a Type 2 device) for vital sign detection and monitoring. Each device comprises a transceiver, a processor and a memory.

The disclosed system can be applied in many cases. In one example, the Type 1 device (transmitter) may be a small WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. In one example, the Type 2 (receiver) may be a WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. The Type 1 device and Type 2 devices may be placed in/near a conference room to count people. The Type 1 device and Type 2 devices may be in a well-being monitoring system for older adults to monitor their daily activities and any sign of symptoms (e.g. dementia, Alzheimer's disease). The Type 1 device and Type 2 device may be used in baby monitors to monitor the vital signs (breathing) of a living baby. The Type 1 device and Type 2 devices may be placed in bedrooms to monitor quality of sleep and any sleep apnea. The Type 1 device and Type 2 devices may be placed in cars to monitor well-being of passengers and driver, detect any sleeping of driver and detect any babies left in a car. The Type 1 device and Type 2 devices may be used in logistics to prevent human trafficking by monitoring any human hidden in trucks and containers. The Type 1 device and Type 2 devices may be deployed by emergency service at disaster area to search for trapped victims in debris. The Type 1 device and Type 2 devices may be deployed in an area to detect breathing of any intruders. There are numerous applications of wireless breathing monitoring without wearables.

Hardware modules may be constructed to contain the Type 1 transceiver and/or the Type 2 transceiver. The hardware modules may be sold to/used by variable brands to design, build and sell final commercial products. Products using the disclosed system and/or method may be home/office security products, sleep monitoring products, WiFi products, mesh products, TV, STB, entertainment system, HiFi, speaker, home appliance, lamps, stoves, oven, microwave oven, table, chair, bed, shelves, tools, utensils, torches, vacuum cleaner, smoke detector, sofa, piano, fan, door, window, door/window handle, locks, smoke detectors, car accessories, computing devices, office devices, air conditioner, heater, pipes, connectors, surveillance camera, access point, computing devices, mobile devices, LTE devices, 3G/4G/5G/6G devices, UMTS devices, 3GPP devices, GSM devices, EDGE devices, TDMA devices, FDMA devices, CDMA devices, WCDMA devices, TD-SCDMA devices, gaming devices, eyeglasses, glass panels, VR goggles, necklace, watch, waist band, belt, wallet, pen, hat, wearables, implantable device, tags, parking tickets, smart phones, etc.

The summary may comprise: analytics, output response, selected time window, subsampling, transform, and/or projection. The presenting may comprise presenting at least one of: monthly/weekly/daily view, simplified/detailed view, cross-sectional view, small/large form-factor view, color-coded view, comparative view, summary view, animation, web view, voice announcement, and another presentation related to the periodic/repetition characteristics of the repeating motion.

A Type 1/Type 2 device may be an antenna, a device with antenna, a device with a housing (e.g. for radio, antenna, data/signal processing unit, wireless IC, circuits), device that has interface to attach/connect to/link antenna, device that is interfaced to/attached to/connected to/linked to another device/system/computer/phone/network/data aggregator, device with a user interface(UI)/graphical UI/display, device with wireless transceiver, device with wireless transmitter, device with wireless receiver, internet-of-thing (IoT) device, device with wireless network, device with both wired networking and wireless networking capability, device with wireless integrated circuit (IC), Wi-Fi device, device with Wi-Fi chip (e.g. 802.11a/b/g/n/ac/ax standard compliant), Wi-Fi access point (AP), Wi-Fi client, Wi-Fi router, Wi-Fi repeater, Wi-Fi hub, Wi-Fi mesh network router/hub/AP, wireless mesh network router, adhoc network device, wireless mesh network device, mobile device (e.g. 2G/2.5G/3G/3.5G/4G/LTE/ 5G/6G/7G, UMTS, 3GPP, GSM, EDGE, TDMA, FDMA, CDMA, WCDMA, TD-SCDMA), cellular device, base station, mobile network base station, mobile network hub, mobile network compatible device, LTE device, device with LTE module, mobile module (e.g. circuit board with mobile-enabling chip (IC) such as Wi-Fi chip, LTE chip, BLE chip), Wi-Fi chip (IC), LTE chip, BLE chip, device with mobile module, smart phone, companion device (e.g. dongle, attachment, plugin) for smart phones, dedicated device, plug-in device, AC-powered device, battery-powered device, device with processor/memory/set of instructions, smart device/gadget/items: clock, stationary, pen, user-interface, paper, mat, camera, television (TV), set-top-box, microphone, speaker, refrigerator, oven, machine, phone, wallet, furniture, door, window, ceiling, floor, wall, table, chair, bed, nightstand, air-conditioner, heater, pipe, duct, cable, carpet, decoration, gadget, USB device, plug, dongle, lamp/light, tile, ornament, bottle, vehicle, car, AGV, drone, robot, laptop, tablet, computer, harddisk, network card, instrument, racket, ball, shoe, wearable, clothing, glasses, hat, necklace, food, pill, small device that moves in the body of creature (e.g. in blood vessels, in lymph fluid, digestive system), and/or another device. The Type 1 device and/or Type 2 device may be communicatively coupled with: the internet, another device with access to internet (e.g. smart phone), cloud server (e.g. hub device), edge server, local server, and/or storage. The Type 1 device and/or the Type 2 device may operate with local control, can be controlled by another device via a wired/wireless connection, can operate automatically, or can be controlled by a central system that is remote (e.g. away from home).

In one embodiment, a Type B device may be a transceiver that may perform as both Origin (a Type 2 device, a Rx device) and Bot (a Type 1 device, a Tx device), i.e., a Type B device may be both Type 1 (Tx) and Type 2 (Rx) devices (e.g. simultaneously or alternately), for example, mesh devices, a mesh router, etc. In one embodiment, a Type A device may be a transceiver that may only function as Bot (a Tx device), i.e., Type 1 device only or Tx only, e.g., simple IoT devices. It may have the capability of Origin (Type 2 device, Rx device), but somehow it is functioning only as Bot in the embodiment. All the Type A and Type B devices form a tree structure. The root may be a Type B device with network (e.g. internet) access. For example, it may be connected to broadband service through a wired connection (e.g. Ethernet, cable modem, ADSL/HDSL modem) connection or a wireless connection (e.g. LTE, 3G/4G/5G, WiFi, Bluetooth, microwave link, satellite link, etc.). In one embodiment, all the Type A devices are leaf node. Each Type B device may be the root node, non-leaf node, or leaf node.

The present teaching discloses systems for monitoring rhythmic motions like gait based on wireless signals and channel information of a wireless multipath channel that is impacted by the gait motion. The systems may also recognize the gait, to identify and verify an individual accordingly.

Gait recognition is particularly appealing for a range of ubiquitous applications that need human identification since it can be achieved at a distance without any active user cooperation. For example, a smart building would automatically open the door for an authorized user when she walks in. A smart home would personalize the temperature and ambient light for a recognized user. A smart TV therein would react with her favorite programs. Smart home devices like Google Home and Amazon Alexa could directly interact with her in a more friendly way. For all of these to function, the user needs to do nothing but walk habitually inside the space. An easy-to-deploy and convenient system, however, is demanded for continuous and passive gait recognition.

Traditional gait measurement and recognition systems usually rely on cameras, floor sensors, and/or wearables to capture gait information. The target subjects have to either walk within restricted areas (typically only an instrumented walkway) or wear body sensors (e.g., accelerometers). Therefore, they are mainly limited to research and clinical usage, and are not convenient and comfortable enough for ubiquitous applications in smart homes and smart buildings. Recently, a new type of gait recognition using wireless signals is on the horizon. Radio signals (e.g., WiFi) are everywhere and can penetrate obstacles like walls and furniture, underpinning the potential of through-the-wall gait sensing. Existing approaches, however, require subjects to walk on a predefined path in a predefined direction. Hence they are only suitable for confined areas (e.g., a 5-meter corridor-like narrow path) with a strong Line-Of-Sight (LOS) condition. Moreover, most of existing works do not physically measure gait. They merely extract RF-based features that are supposed to be representative of walking patterns, making them location and environment dependent since those RF features are usually entangled with the surrounding environments. Most importantly, none of these systems can work for Non-LOS (NLOS) scenarios, not to mention through-the-wall sensing.

A wireless/RF based system disclosed in the present teaching can achieve many benefits: e.g., (a) the disclosed system can operate in both LOS and non-line-of-sight (NLOS) conditions; (b) the disclosed system does not require the monitored person to cooperate (i.e. the person is free to walk in any manner he wants); (c) the disclosed system uses common off-the-shelf (COTS) components and thus is less expensive; (d) each device used in the disclosed system can cover a large area; (e) the disclosed system can be installed easily; (f) the disclosed system does not need a camera to intrude into privacy; (g) the disclosed system does not have a wearable device for the person to wear; and (h) the disclosed system is location independent.

One goal of the disclosed system is to obtain features computable from channel state information (CSI) that are good for recognition/verification while imposing little constraint on the person to be monitored, e.g. no wearable and no constraint on the way the person walk. In general, the disclosed system can monitor a rhythmic motion of an object based on time series of CI. For example, breathing is periodic and rhythmic. Gait is somewhat periodic, but different gait cycles can be different. A "step" phase corresponding to the left foot touching the ground can be different from the "step" phase corresponding to the right foot. The asymmetry between the left step and the right step (e.g. when one leg is wounded) is an important feature to assess a lot of health related issues. Thus, a gait motion is called rhythmic, instead of periodic, as it is indeed generated in a rhythmic manner, with underlying alternate motion of the left foot and the right foot. The disclosed systems and methods in the present teaching can be applied to any rhythmic motion, although gait is used as an example of rhythmic motion for illustration below.

The Type 1 device and/or Type 2 device (together with the wireless signal, TSCI, processor, memory, set of instructions) can be used to monitor other kinds of motion as well, such as transient motion (e.g. fall down, gesture, writing, body motion, people movement, vehicle motion, in-vehicle activities).

The Type 1 device and/or the Type 2 device may be a standalone device, or an embedded device. It may be connected to another system (for power, signaling, network access, etc.) using a connector/port such as USB (e.g. Type A/B/C/D/E, micro-USB, mini-USB, etc.), Thunderbolt, Firewire, Lightning (e.g. in Apple devices such as iPhone, iPad, AirPods, etc.), OBD (on-board-diagnostic port), cigarette lighter port (e.g. 12V), PCI (e.g. PCI, PCI Express, etc.), VGA, DVI, HDMI, parallel port, serial port, ADAT, BNC, D-SUB, F, MIDI,UHF, MCX, N, PS/2, RCA, SATA, S-video, SATA, mSATA, m.2, SMA, SMB, SMC, S/PDIF, RJ-11, RJ-45, SCSI, TNC, TS, TRS, UHF, mini-UHF, XLR, coaxial, optical, Ethernet, display port, etc.

Any device may be powered by battery (e.g. AA battery, AAA battery, coin cell battery, button cell battery, miniature battery, bank of batteries, power bank, car battery, hybrid battery, vehicle battery, container battery, non-rechargeable battery, rechargeable battery, NiCd battery, NiMH battery, Lithium ion battery, Zinc carbon battery, Zinc chloride battery, lead acid battery, alkaline battery, battery with wireless charger, smart battery, solar battery, boat battery, plane battery, other battery, temporary energy storage device, capacitor, fly wheel).

Any device may be powered by DC or direct current (e.g. from battery as described above, power generator, power convertor, solar panel, rectifier, DC-DC converter, with various voltages such as 1.2V, 1.5V, 3V, 5V, 6V, 9V, 12V, 24V, 40V, 42V, 48V, 110V, 220V, 380V, etc.) and may thus have a DC connector or a connector with at least one pin for DC power.

Any device may be powered by AC or alternating current (e.g. wall socket in a home, transformer, invertor, shorepower, with various voltages such as 100V, 110V, 120V, 100-127V, 200V, 220V, 230V, 240V, 220-240V, 100-240V, 250V, 380V, 50Hz, 60Hz, etc.) and thus may have an AC connector or a connector with at least one pin for AC power. The Type 1 device and/or the Type 2 device may be positioned (e.g. installed, placed, moved to) in the venue or outside the venue.

For example, in a vehicle (e.g. a car, truck, lorry, bus, special vehicle, tractor, digger, excavator, teleporter, bulldozer, crane, forklift, electric trolley, AGV, emergency vehicle, freight, wagon, trailer, container, boat, ferry, ship, submersible, airplane, air-ship, lift, mono-rail, train, tram, rail-vehicle, railcar, etc.), the Type 1 device and/or Type 2 device may be an embedded device embedded in the vehicle, or an add-on device (e.g. after-market device) plugged into a port in the vehicle (e.g. OBD port/socket, USB port/socket, accessory port/socket, 12V auxiliary power outlet, and/or 12V cigarette lighter port/socket).

For example, one device (e.g. Type 2 device) may be plugged into 12V cigarette lighter/accessory port or OBD port or the USB port (e.g. of a car/truck/vehicle) while the other device (e.g. Type 1 device) may be plugged into 12V cigarette lighter/accessory port or the OBD port or the USB port. The OBD port and/or USB port can provide power, signaling and/or network (of the car/truck/vehicle). The two devices may jointly monitor the passengers including children/babies in the car. They may be used to count the passengers, recognize the driver, detect presence of passenger in a particular seat/position in the vehicle.

In another example, one device may be plugged into 12V cigarette lighter/accessory port or OBD port or the USB port of a car/truck/vehicle while the other device may be plugged into 12V cigarette lighter/accessory port or OBD port or the USB port of another car/truck/vehicle.

In another example, there may be many devices of the same type A (e.g. Type 1 or Type 2) in many heterogeneous vehicles/portable devices/smart gadgets (e.g. automated guided vehicle/AGV, shopping/luggage/moving cart, parking ticket, golf cart, bicycle, smart phone, tablet, camera, recording device, smart watch, roller skate, shoes, jackets, goggle, hat, eye-wear, wearable, Segway, scooter, luggage tag, cleaning machine, vacuum cleaner, pet tag/collar/wearable/implant), each device either plugged into 12V accessory port/OBD port/USB port of a vehicle or embedded in a vehicle. There may be one or more device of the other type B (e.g. B is Type 1 if A is Type 2, or B is Type 2 if A is Type 1) installed at locations such as gas stations, street lamp post, street corners, tunnels, multi-storey parking facility, scattered locations to cover a big area such as factory/ stadium/ train station/ shopping mall. The Type A device may be located, tracked or monitored based on the TSCI.

In one embodiment, a scattering multipath model is considered to derive a statistical approach on top of the scattering model for passive speed estimation, which can capture speed when a target is up to 10 meters away from the link, and/or behind the walls. The proposed model offers new directions and opportunities for indoor wireless sensing. Moreover, a gait recognition system, called "GaitWay" is built based on walking speed alone. By extracting a range of physical plausible features, GaitWay can recognize a subject's gait independently from location, orientation, environments, and the user's apparel.

Given numerous multipaths, the disclosed system can statistically investigate the channel properties by accounting for all multipaths together based on a disclosed method. The target's moving speed can be calculated from the ACF of CSI. Built upon the statistical property of numerous multipaths, the disclosed method is independent of the environments, locations, and user orientations. The disclosed method automatically detects stable period during a user's normal activities. The disclosed system can use the ACF of the speed to measure such walking periodicity. The disclosed system can estimate the gait cycle time, and segment the speed series for every individual step. To monitor and assess a subject's gait, the disclosed system can investigate three straight-forward properties, i.e., average walking speed, gait cycle time, and stride length. In addition, the disclosed system can also adopt a measure for stability and symmetry, e.g., harmonic ratio, to evaluate gait progression. The disclosed system can leverage Support Vector Machine (SVM) to recognize different gaits based on features extracted from speed estimation, including speed, acceleration, symmetry, rhythmicity and cadence, smoothness, recurrent quantification analysis and ACF features.

A 2-legged animal (e.g. a person) would have two step segments for one gait cycle. For example, a first step segment may correspond to forward movement of left foot, and a second step segment may correspond to forward movement of right foot. A 4-legged animal (e.g. a dog) would have 4 step segments for one gait cycle. Each step segment may correspond to a movement of each foot. The step segments may have different duration.

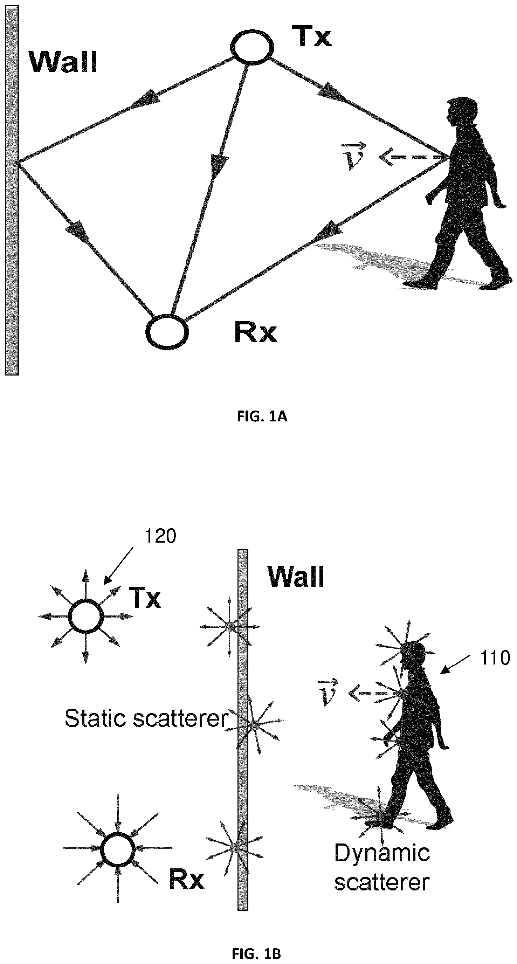

The disclosed system can investigate a distinct rich scattering multipath model to apply the disclosed method. Instead of simplifying an object (e.g., a human body) as a single reflector producing only one major reflection path in a model Doppler Frequency Shift (DFS) as shown in FIG. 1A, FIG. 1B illustrates an exemplary multipath model in rich-scattering indoor environments, where an object scatters the signal and produce multiple paths, according to some embodiments of the present teaching. As shown in FIG. 1B, the human body 110 is seen as multiple scatterers, which reflect signals in diverse directions and superimpose at the Tx 120 together with signals scattered by other objects from any directions. Given numerous multipaths, rather than geometrically analyzing a specific reflection path or assuming a dominant one with others ignored, the disclosed system can statistically investigate the channel properties by accounting for all multipaths together. It turns out that the target's moving speed can be calculated from the ACF of CSI. Built upon the statistical property of numerous multipaths, the disclosed method is independent of the environments, locations, and user orientations. In contrast to previous reflection model that fails in rich multipath environments, the proposed model works even better with more multipaths and supports through-the-wall sensing.

The disclosed system can also track speed through the walls. Consider a wireless transmission pair each equipped with omni-directional antennas. The channel frequency response (CFR), also called the channel state information (CSI), for the multipath channel at time t may be generally modeled as

$$H(t,f) = \sum_{l \in \Omega} a_l(t) exp(-j2\pi f \tau_l(t)), \tag{1}$$

where al(t) and τl(t) denote the complex amplitude and propagation delay of the 1-th multipath component (MPC), respectively, and $\Omega$ stands for the set of MPCs.

Due to the timing and frequency synchronization offsets and additive thermal noise, the real measurement of CFR H~(t,f) is expressed as

$$\tilde{H}(t,f) = exp(-j(\alpha(t) + \beta(t)f))H(t,f) + n(t,f), \tag{2}$$

where $\alpha(t)$ and $\beta(t)$ are the random initial and linear phase distortions at time t, respectively.

The radio signals are scattered by numerous scatterers, such as walls, ceilings, floors, furniture, human bodies, etc. Due to the superposition principal of EM waves, the CSI H(t,f) can be decomposed as

$$H(t,f) = \sum_{i \in \Omega_s(t)} H_i(t,f) + \sum_{j \in \Omega_d(t)} H_j(t,f) + \varepsilon(t,f), \tag{3}$$

where $\Omega_s(1)$ denotes the set of static scatterers, $\Omega_d(t)$ denotes the set of dynamic scatterers, and Hi(t,f) stands for the part contributed by the i-th scatterer. $\varepsilon(t,f)$ is the noise term, which can be approximated as additive white Gaussian noise (AWGN) with variance $\sigma^2(f)$ and is statistically independent of Hi(t,f). The intuition behind the decomposition is that each scatterer can be treated as a "virtual Tx" diffusing the received EM waves in all directions and then these EM waves add up together at the receive antenna after bouncing off the interior objects indoors. As a result, H(t,f) actually measures the sum of the electric fields of all the incoming EM waves. In practice, within a sufficiently short period, it is reasonable to assume that both the sets $\Omega_s(t)$ and $\Omega_d(t)$ change slowly in time and they can be approximated as time-invariant sets.

One can consider a 2-D scattering model, where all the scatterers are within the same horizontal plane. Due to the channel reciprocity, EM waves traveling in both directions will undergo the same physical perturbations (i.e., reflection, refraction, diffraction, etc.). Therefore, if the receiver were transmitting EM waves, the CSI "measured" at the i-th scatterer or "virtual Tx" would be identical to Hi(t,f). If the speed of the i-th scatterer is vi, then a continuous limit representation of Hi(t,f) can be expressed as

$$H_i(t,f) = \int_0^{2\pi} F_i(\theta, f) \exp(-jkv_i \cos(\theta)t) d\theta, \qquad (4)$$

where Fi(θ,f) denotes the complex channel gain of the MPC from direction θ for the i-th scatterer, and $k = \frac{2\pi}{\lambda}$ is the wave number where λ is the wavelength.

Based on the statistical theory of EM fields developed for reverberation cavities, which approximates indoor environments well, Fi(θ,f), for ∀i, can be represented as a random variable with the following properties: for ∀θ, Fi(θ,f) is a circularly-symmetric Gaussian random variable with the same variance σF2 (f); for ∀θ1/= θ2, Fi(θ1,f) and Fi(θ2,f) are statistically independent; for ∀i/= j ∈ Ωd, Fi(θ1,f) and Fj(θ2,f) are statistically independent for ∀θ1 and ∀θ2.

With the above properties, now one can investigate how the ACF of CSI relates to the speed vi. The mean of Hi(t,f) equals to zero, i.e., E[Hi(t,f)] = 0, where E[·] denotes the expectation operator. Then, the covariance of two CSIs with time lag τ can be written as

$$\text{Cov}[H_i(t,f), H_i(t+\tau, f)] = \mathbb{E}[H_i(t,f)H_i^*(t+\tau, f)] = 2\pi\sigma_{F_i}^2(f)J_0(kv_i\tau), \quad (5)$$

where J0(·) is the 0th-order Bessel function of the first kind 46]: $J_0(x) = \frac{1}{2\pi} \int_0^{2\pi} \exp(-jx\cos(\theta)) d\theta$. The ACF of Hi(t,f) with time lag τ, denoted as ρHi(τ,f), is derived as

$$\rho_{H_i}(\tau, f) = \frac{\text{Cov}[H_i(t,f), H_i(t+\tau, f)]}{\text{Cov}[H_i(t,f), H_i(t,f)]} = J_0(kv_i\tau). \qquad (6)$$

Similarly, the ACF of the CSI H(t,f) with time lag τ, denoted as ρH(τ,f), can be obtained as

$$\rho_H(\tau, f) = \frac{\sum_{i \in \Omega_d} \sigma_{F_i}^2(f) J_0(kv_i\tau) + \sigma^2(f)\delta(\tau)}{\sum_{i \in \Omega_d} \sigma_{F_i}^2(f) + \sigma^2(f)}, \qquad (7)$$

where δ(·) is the Dirac's delta function. As seen, ρH(τ,f) is a linear combination of the ACF of Hi(t,f) and the weight of each term equals to the energy scattered by that corresponding scatterer.

Consider that there is only one person moving in the monitored area. The speed of all the scatterers can be approximated to be the same, i.e., vi = v, for ∀i ∈ Ωd.

Then, ρH(τ,f) can be simplified as

$$\rho_H(\tau, f) = \frac{\sum_{i \in \Omega_d} \sigma_{F_i}^2(f) + \sigma^2(f)\delta(\tau)}{\sum_{i \in \Omega_d} \sigma_{F_i}^2(f) + \sigma^2(f)} J_0(kv\tau) \triangleq \alpha(f)J_0(kv\tau), \qquad (8)$$

where α(f) is defined as the gain of each subcarrier f. Equation (8) bridges the moving speed of human body and the second order statistics, i.e., ACF, of CSI.

In practice, the sample ACF is used instead, which is an estimate of the ACF, and one can use n(τ,f) to stand for the estimation noise of the ACF, i.e.,

$$\hat{\rho}_H(\tau, f) = \alpha(f)J_0(kv\tau) + n(\tau, f). \qquad (9)$$

Since the term J0(kvτ) in (9) is a function of moving speed v, it is called the speed signal in the following. From (9), one can derive the moving speed v from the ACF measurement $\hat{\rho}_H(\tau,f)$. In practice, however, the signal-to-noise ratio (SNR) of the speed signal on each subcarrier modulated by human movement can be very low, especially when the person being monitored is far away from the link or behind walls. As a second-order statistic, ACF circumvents the phase issue and is synchronized over all subcarriers, allowing direct combination of ACF measured on different subcarriers. In the following, a novel scheme is proposed based on Maximal Ratio Combining (MRC) that combines the speed signals measured on multiple subcarriers in an optimal way such that the SNR of the speed signal is maximized. MRC is a diversity combining method in telecommunications that optimizes SNR by combining signals received on multiple antennas and is applicable here by treating subcarriers as receiving diversity.

When $\alpha(f)$ is small, i.e., H(t,f) is dominated by the white noise, each tap of the ACF follows a zero-mean normal distribution with equal variance 1/N, i.e.,

$$n(\tau,f) \sim \mathcal{N}(0, 1/N),$$

where N is the number of samples used in the ACF estimation. Therefore, the variance of $n(\tau,f)$ in (9) is the same for different subcarriers. Considering the fact that the noise terms of different subcarriers are statistically independent, it can be shown that the MRC scheme achieves the maximum of the SNR of the speed signal J0(kv$\tau$), i.e.,

$$S(\tau) = \sum_{f \in \mathcal{F}} w^*(f)\hat{\rho}_H(\tau, f) = \left(\sum_{f \in \mathcal{F}} w^*(f)\alpha(f)\right) J_0(kv\tau) + \sum_{f \in \mathcal{F}} w^*(f)n(\tau, f), \qquad (10)$$

where S($\tau$) is called the combined speed signal, $w^*(f)$ denotes the optimal combining weight for subcarrier f, and $w^*(f)$ is linearly proportional to the gain $\alpha(f)$.

The gain $\alpha(f)$ on each subcarrier, however, is not directly available from CSI. Fortunately, since J0(kv$\tau$) is continuous at time lag 0, i.e., lim$\tau\to0$ J0(kv$\tau$) = 1, one can have $\alpha(f)$ = lim$\tau\to0$ $\rho$H($\tau$,f) according to (8). Therefore, when the channel sampling rate Fs is sufficiently high, $\alpha(f)$ can be estimated as the quantity $\hat{\rho}_H$($\tau$ = 1/Fs,f), the first tap of the ACF, and $w^*(f)$ becomes

$$\hat{w}^*(f) = \hat{\rho}_H(\tau = 1/F_s, f). \qquad (11)$$

The intuition that MRC maximizes the SNR is that, when combining all subcarriers appropriately, the "good" subcarriers will boost the signal while the "bad" subcarriers will help attenuate the noise since their noise terms are independent.

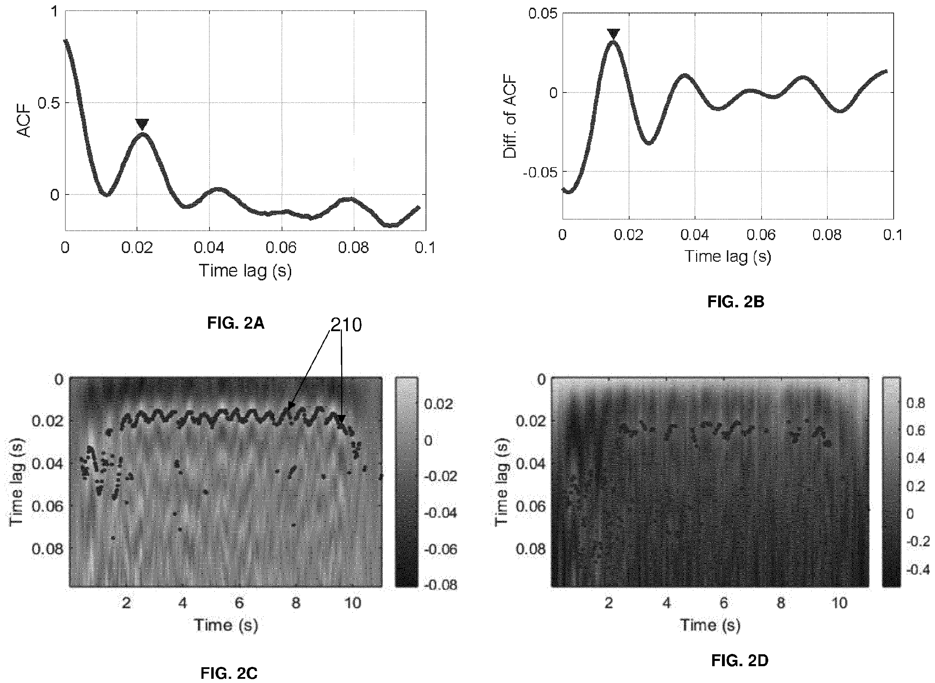

FIG. 2A shows an example of the combined speed signal, and FIG. 2C shows the matrix of the combined speed signal, where each column of the matrix corresponds to a combined speed signal. As shown in FIG. 2A, the shape of the combined speed signal resembles the Bessel function J0(x) with x = kv$\tau$, and the speed v can be extracted by matching their key characteristics, e.g., the locations of the first peak or valley. One can use the first peak in one embodiment of GaitWay, i.e., the speed is calculated as

$$\hat{v} = \frac{x_0}{k\hat{\tau}} = \frac{x_0\lambda}{2\pi\hat{\tau}}, \qquad (12)$$

where x0 is constant value corresponding to the first peak of Bessel function J0(x), and $\hat{\tau}$ is the time lag corresponding to the first peak in the combined speed signal, as marked by the dots 210 in FIG. 2C.

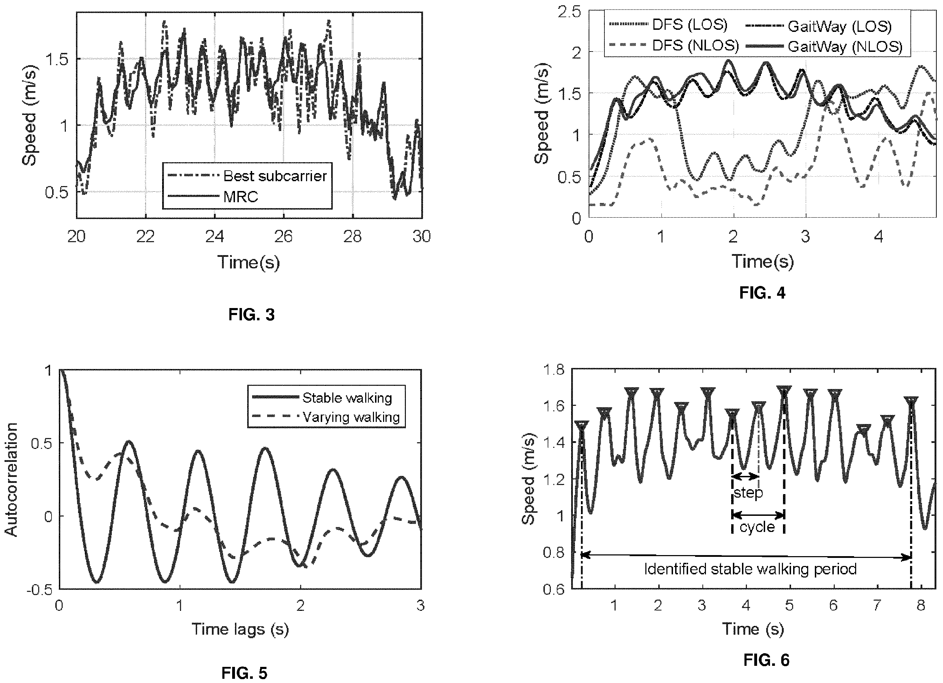

In one embodiment, two steps are further taken to enhance the speed estimation. First, to facilitate peak finding, the difference of the combined speed signal is used, as shown in FIG. 2B and FIG. 2D. In this case, x0 becomes the location corresponding to the first peak of the derivative of J0(x). Second, the disclosed system can use the phase difference between two receive antennas to eliminate errors in the raw phase. Thanks to ACF, the disclosed approach is insensitive to the initial phase offsets. FIG. 3 shows the speed estimates of a 10-second period during a user's continuous walking. FIG. 3 also shows that MRC largely enhances the speed estimation.

The disclosed system can implement the DFS-based method and compare it with the proposed approach by real measurements. Specifically, the disclosed system can set up two links (one Tx with two Rx in a line), one with LOS and the other with NLOS condition. A user is asked to walk towards the links and the two receivers can measure the CSI simultaneously. FIG. 4 depicts the speed estimated by GaitWay and DFS-based methods, respectively. As seen, GaitWay accurately captures the speed using either the LOS or NLOS link, preserving the precise in-step speed changes. The speed estimates on both links are highly consistent with each other, with only marginal differences in the absolute values. The DFS-based method, however, fails to capture the precise speeds in both LOS and NLOS scenarios.

Rather than only allowing a user to walk along a predefined straight path with an approximately constant speed and assuming all data are collected during stable walking, the disclosed system aims at acquiring gait information for free natural walking. A user may perform various activities, including walking, sitting, standing, and typing, etc. A user will also walk with different speeds, especially when one is starting to walk from standing still, making a turn, or about to stop, etc. During these periods, the walking speed does not necessarily reflect the most distinctive and stable gait characteristics. Hence, the first step for gait analysis and recognition is to identify a period of stable walking, during which a subject walks normally with habitual pace.

One can devise an algorithm that automatically detects stable period during a user's normal activities. When a user is walking smoothly, the observed speed will reach to a certain range with repetitive patterns due to the periodic step rhythms.

The disclosed system can use the ACF of the speed to measure such walking periodicity. As shown in FIG. 5, when a user is walking stably, evident peaks will be observed from the ACF of the speed. In contrast, the ACF will be more flatted out for varying walking. The disclosed system can apply a sliding window (e.g. 3 seconds) to the speed estimates and calculate the ACF for each window. The disclosed system can then employ peak detection on the ACF of the speed and examine the first peak. A period will be considered as stable walking only if a continuous series of reliable peaks are observed.

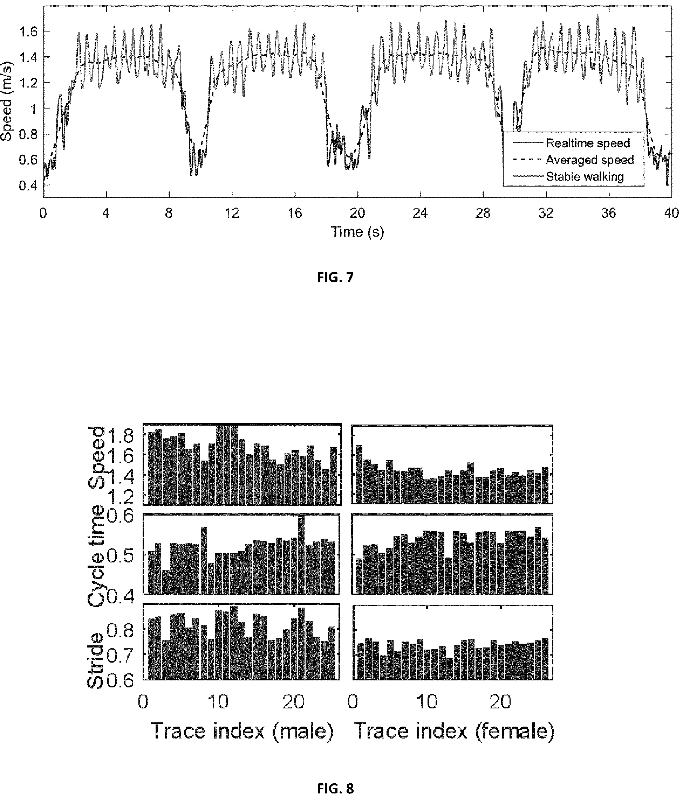

To be more robust, the disclosed system can further check the averaged center trend of the walking speed. A walking period will be used for gait analysis only when the average speed is larger than a certain value (e.g., 0.7 m/s, which is smaller than normal human walking speed ranging from 1.0 m/s to 2.0 m/s). FIG. 7 illustrates an example of the identified stable walking periods. The speed is measured when a user is walking back and forth along a 10-meter corridor for 4 times. ach stable period then becomes a gait instance with a speed series V = [v(ti),i = 1,2,···,M].

The disclosed system can also estimate a gait cycle, which is defined as the duration between two consecutive events that the same heel hits the ground during walking. The disclosed system not only estimates the gait cycle time, but also segments the speed series for every individual step. During normal human walking, a subject's speed will experience an increase followed by a decrease, resulting in a speed peak for each step. Therefore, the disclosed system can perform a simple peak detection on the speed series to identify steps, as shown in FIG. 6. To combat noises and outliers, certain constraints (including peak prominence and height) are applied for peak detection. When all steps are identified, the disclosed system can trim the walking period by removing the duration before the first peak and after the last peak. The remained trace becomes a valid gait instance for further analysis in one embodiment of GaitWay.

The disclosed system can also analyze individual gait. Various gait properties can be analyzed from the walking speed and the identified gait cycles. To monitor and assess a subject's gait, the disclosed system can investigate three properties, i.e., average walking speed, gait cycle time, and stride length. In addition, the disclosed system can also adopt a measure for stability and symmetry, i.e., harmonic ratio, to evaluate gait progression. A stride is a complete gait cycle, which includes 2 step segments for a 2-legged animal, 3 step segments for a 3-legged being, and 4 step segments for a 4-legged being.

In one embodiment, the average walking speed is simply taken as the mean value of instantaneous estimates of a user's walking speed. As shown in FIG. 8, the disclosed measurements demonstrate that different users have different habitual speeds, and a user's walking speed varies over time.

In one embodiment, the gait cycle time is computed as the mean duration of every two consecutive steps. The middle row of FIG. 8 illustrates the average cycle time of two users' walking instances measured at different locations and time. Over the 20 traces, variances of 0.7 ms and 0.6 ms are observed for the two users, respectively.

In one embodiment, the stride length estimation can be performed by the disclosed system as well. Thanks to the accurate speed estimation, one can intuitively derive the stride length by integrating the speed estimates over the time duration of each step. The bottom row of FIG. 8 depicts the estimated stride lengths for two users.

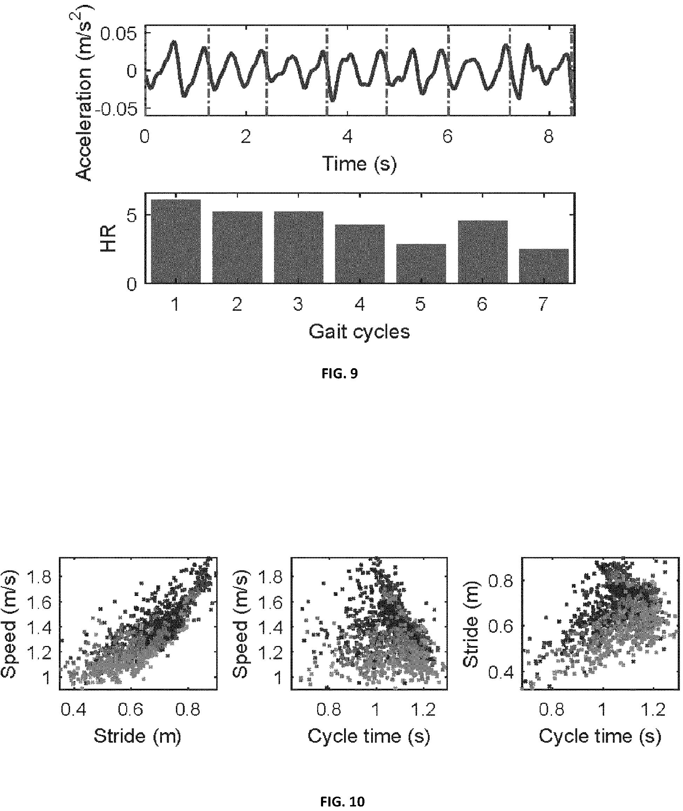

The harmonic ratio (HR) may be adopted as a quantitative measure of walking smoothness. There is an increasing interest in using HR technique to study the impacts of various pathologies as well as monitor rehabilitation. HR examines the step-to-step symmetry within a stride by quantifying the harmonic composition of the accelerations for a given stride. It first conducts Discrete Fourier Transform (DFT) on the acceleration within each stride. The HR may be defined as the ratio of the sum of the amplitudes of the even harmonics to the sum of the amplitudes of the odd harmonics. The disclosed system can use the first twenty harmonics to calculate the HRs, as justified for normal cadences for which the majority of the power occurs below 10Hz. FIG. 9 illustrates the HRs of a walking trace of 7 cycles (14 steps), demonstrating the progression of step-to-step symmetry during the walk.

The disclosed system can investigate the pair-wise relationships among stride length, stride time, and walking speed and make a remark for research related to pedestrian dead-reckoning. To this end, the disclosed system can integrate the measurements of 6 users collected under different scenarios and visualize the relationships in FIG. 10. As shown, stride length evidently changes with respect to walking speed for different users, indicated by different gray scales. Therefore, it introduces significant errors to assume a fixed stride length, and accordingly derives walking distance by multiplying it with step count, as done by many previous works on pedestrian dead-reckoning.

In addition, in the disclosed measurements, the data are collected and extracted automatically when the subject is walking around freely, without being asked to walk along a predefined path or a predefined direction, nor to walk with an intentional speed. In practice, the involved subject does not even need to be aware of the data collection. This is a critical property that underpins a continuous gait monitoring and recognition system. It significantly differs from previous works that exert parts or all of these constraints to testers in order to obtain environmentally repeatable features for human recognition, or to derive DFS.

Instead of extracting data-driven features directly from CSI measurements, which are implausible and contain environment-dependent features, the disclosed system performs human identification by extracting true gait features, which are physically plausible and environmentally irrelevant, from the speed estimates. In addition to the above-discussed parameters for gait monitoring, the disclosed system can devise a number of features to characterize various aspects of one's gait pattern.

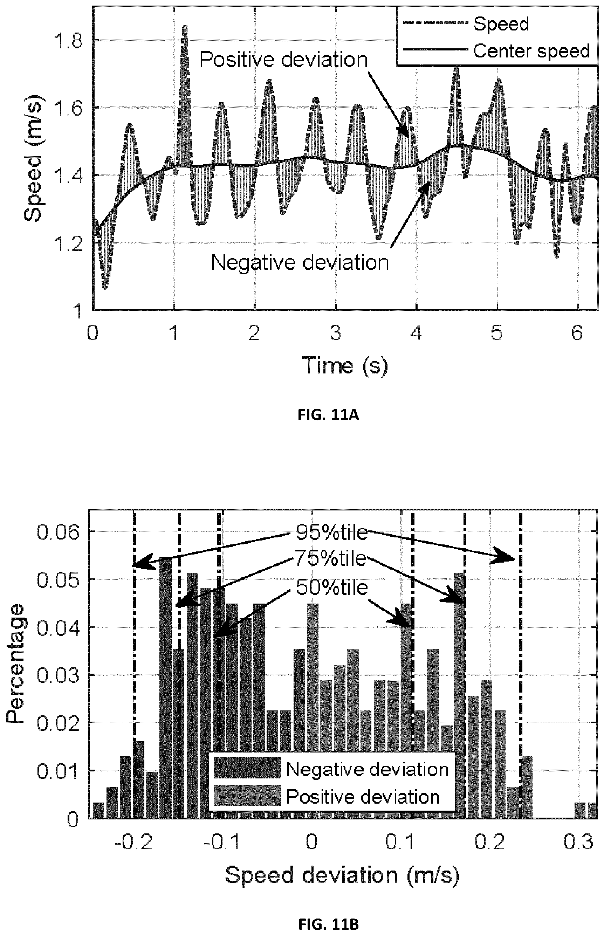

For gait speed, the disclosed system does not directly use the average walking speed as a feature since a subject's speed varies considerably over time. The disclosed system can exploit features that are independent from the mean walking speed. As shown in FIG. 11A, the disclosed system can first detrend the absolute speed by subtracting the average center speed. Then the disclosed system can calculate the different percentile values (e.g. taking 95% tile, 75% tile, and 50% tile in one embodiment of GaitWay as shown in FIG. 11B) of the speed deviations. Specifically, the disclosed system can take these percentile values of the positive deviations, negative deviations, and the absolutes of all deviations, respectively.

An acceleration may be computed as the derivatives of speed. The disclosed system can take the maximum, minimum, and variance of the acceleration. Since the walking acceleration also exhibits sinusoid-like patterns, the disclosed system can also identify the peaks and valleys of the acceleration sequence and compute the respective variances.

A level of symmetry is also a feature of a gait. The disclosed system can calculate the step time and stride lengths of left and right foot respectively and take their means and standard deviations as features. The difference of each feature between two feet is derived as a measure of gait symmetry.

Rhythmicity and cadence can be measured based on a gait. Referring back to FIG. 5, the disclosed system can calculate the ACF of the walking speed. If a user walks in a regularly rhythmic manner, the speed ACF will exhibit multiple prominent peaks and will decay slowly. Hence the ACF embodies the walking rhythmicity, or dynamic stability. The disclosed system can thus develop several features based on the ACF of the speed. In one embodiment, the disclosed system GaitWay can apply a sliding window to calculate a series of ACF for each walking instance, resulting in a speed ACF matrix. From there, the disclosed system can first identify the prominent peaks and the corresponding delays of each ACF and then extract the following single-valued features: the mean and variance of the values of the first peaks of the ACFs and the number of identified prominent peaks, the variance of peak cycles (i.e., differences of peak delays for each ACF, corresponding to the differences in consecutive step cycle time), and the ratio of ACFs in the matrix that do not observe a prominent first peak.

In one embodiment, harmonic ratio (HR) is used as a measure of gait smoothness. For every gait cycle during walking, the disclosed system can obtain one HR value. To obtain a single value feature for a walking trace, the disclosed system can take the median and variance of the HR values.

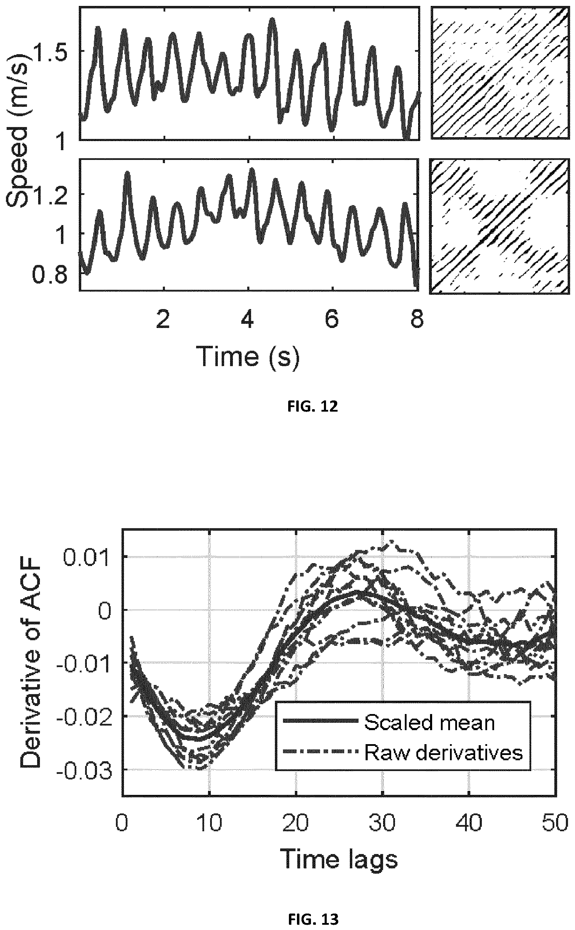

To quantify the gait variability, the disclosed system can adopt Recurrence Quantification Analysis (RQA), a method of nonlinear data analysis which quantifies the number and duration of recurrences of a dynamical system presented by its phase space trajectory. It describes the recurrence property by analyzing the recurrent plot (RP) that visualizes and reveals all the times when the phase space trajectory of the dynamical system visits roughly the same area in the phase space. Such an RP is mathematically expressed as an N*N matrix R: $R_{ij} = \Theta(\varepsilon - \|\vec{x}_i - \vec{x}_j\|)$, $\vec{x}_i \in \mathbb{R}^m$, $i,j = 1,2,\cdots,N$, where N is the number of states, $\varepsilon$ is a predefined cutoff distance, $\|\cdot\|$ is a norm and $\Theta(\cdot)$ the Heaviside function. In one embodiment of GaitWay, the state space trajectory X is constructed from the speed series $\{v_i, i = 1,2,\cdots,L\}$ with an embedding dimension of 5 and a delay of 10 samples. FIG. 12 depicts two illustrative RPs for two different users, where the upper RP presents more diagonal lines, indicating more stable and periodic gait. A number of measures can be derived by RQA based on the RP. In one embodiment of GaitWay, one can exploit the below four measures to reflect different properties of RP: (1) a recurrence rate which is the percentage of recurrence points in an RP; (2) a determinism which is the percentage of recurrence points that form diagonal lines; (3) the Shannon entropy of the probability distribution of the diagonal line lengths; and (4) the average diagonal line length. The measurements show that RQA reaches a stable value when calculated over 4 gait cycles, suggesting a shortest length for stable walking period detection. In practice, the disclosed system can relax the minimum to 3 cycles (i.e., 6 steps) to gather more available data. Walking period with less than 3 cycles may not be considered for gait recognition.

The disclosed system can also investigate the ACF of CSI (as shown in FIGs. 2A-2D) for feature extraction. Since the ACF is entwined with walking speed yet is independent of location and environment, it can serve as a signature for gait classification. Specifically, rather than using all ACFs within a walking period, one can consider the ACF corresponding to the speed peaks, as identified in FIG. 6. Note that the peak speeds could be different within a walking trace, i.e., the locations of the first peak of the ACF vary over time. Hence the disclosed system can align all the ACFs to a scale corresponding to the mean peak speed. To make it more apparent, the disclosed system can take the difference of each ACF and then average the aligned ACF differences. FIG. 13 illustrates an example of the scaled ACF differences. The disclosed system can use the first 50 taps as a feature vector in the disclosed system.

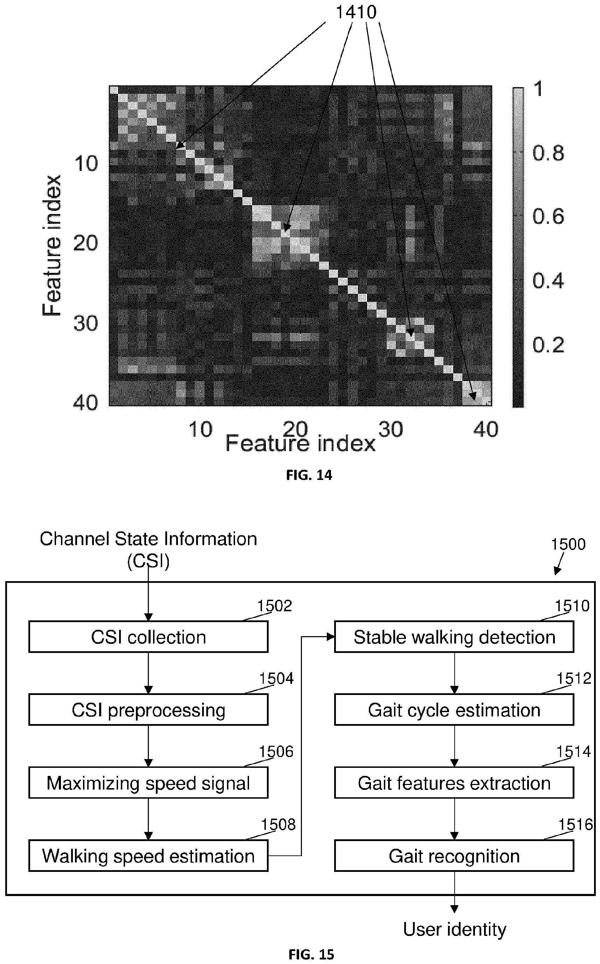

The disclosed system can fuse all the above features together, resulting in a 90-dimensional feature vector for each gait instance. The disclosed system can conduct the following two steps for potential dimension deduction. First, the disclosed system can investigate whether these features are correlated with each other or not. The disclosed system can calculate the pair-wise correlations of all the features (except for the 50-dimensional ACF features that are taken as a whole) and depict the correlation matrix as in FIG. 14. Most of the extracted features are independent from each other. Some features are more correlated than others, as indicated by four more clustered areas 1410 along the diagonal line in the correlation matrix. For example, features #2 to #8 are all related to cycle time and thus more correlated. Similarly, features #16 to #23 are different measures of the speed deviation. Features #30 to #34 are acceleration-related while features #38 to #40 are all extracted by RQA. The disclosed system can eliminate one of each pair of highly correlated features for classification. Second, the disclosed system can employ an outcome-based approach for feature selection. Specifically, the disclosed system can perform 10-fold cross validation with and without a specific feature, and keep that feature only if it improves the output classification accuracy.

Given the features extracted, the disclosed system can identify a user (from others) by the gait patterns. Following the gait recognition, one can consider two identification scenarios: single user verification that validates whether a user is the target subject or an unknown stranger, and multiple user recognition that identifies which target subject the user is among a set of candidates. The disclosed system can leverage Support Vector Machine (SVM), a classification technique, for this purpose. The disclosed system can use SVM instead of the popular deep learning techniques mainly because the primary goal here is to demonstrate the effectiveness of the speed estimates and the physical plausible features for gait recognition. In one embodiment, one can keep applying deep learning in the disclosed system.

For single user verification, the disclosed system can train a gait model for the subject by building a binary classifier, which sees the subject's gait instances as positive class and several benchmark users' as negative class. The benchmark data could be obtained from available standard public database. In one embodiment of GaitWay, they are randomly selected from the experiment participants. To authenticate the target person, the disclosed system can calculate the probability that an instance fits the target class. A testing gait instance is considered belonging to the target subject when the probability is higher than a threshold, and is otherwise rejected. In practice, the threshold can be defined as different sensitive level by the users to adapt to different authentication applications.

To recognize multiple users, the disclosed system can train a one-vs-all binary classifier for each user, with the gait instances from this user as the positive class and the instances from all other candidates as the negative class. Then given a gait instance for testing, the disclosed system can feed it into every classifier and obtain the fitness probability that the instance belongs to each class. The gait instance may be assigned to the user from whose classifier the highest fitness probability is observed. In one embodiment, the disclosed system can use a SVM tool with the Radial Basis Function (RBF) kernel. The optimal values for parameters $\gamma$ and c are selected by grid search with 10-fold cross validation. Features may be scaled to [0,1] for classification.

In one embodiment, a rhythmic motion of the object, e.g. a gait of a person, can be monitored based on a time series of intermediate quantity (IQ), which is computed by the disclosed system based on channel information of a wireless multipath channel, where the wireless multipath channel is impacted by the rhythmic motion of the object. In one embodiment, after the time series of speed (IQ) is obtained, a time series of speed is analyzed in search of a time window of stable gait motion (e.g. a rhythmic motion). When there is a stable gait, the mean (average) speed should not be too small, and there should be a strong local maximum in the autocorrelation function of the IQ. In another embodiment, a peak detection is performed to compute local maximum of speed in the time window of stable rhythmic motion of the object.

In one embodiment, a "step" segment of time window is defined as the time from a local maximum to the next local maximum. A step segment may correspond approximately to the left foot touching the ground, and the next step segment may then correspond approximately to the right foot touching the ground. It is "approximate" because the heel does not necessarily start to touch the ground or leave the ground at the time of maximum speed (IQ). Two consecutive step segments, together, can define a gait cycle or motion cycle. In another embodiment, the local maximum can be replaced by local minimum in this claim. In another embodiment, the time window of stable rhythmic motion may be adjusted e.g. by pruning/removing some IQ from both ends of the time window as the rhythmic motion at both ends may not be fully stable or in steady state. If a person starts from motionless to walk, some initial moments in the time window may have the person still in the "acceleration" mode, and some of the last moments in the time window may have the person already in "deceleration" mode. One way to identify these non-steady-state IQ is that the time difference between adjacent local maxima may be increasing, or decreasing. In steady state, the time difference should be somehow stable. There are some gait-speed related features. Gait speed (e.g. walking speed) itself is important for health-related monitoring. But gait speed may be less useful for gait recognition (i.e. recognizing a person based on his/her gait). Instead, analytics derived from gait speed such as speed deviation, peak variance, valley variance, harmonic ratio may be more important. Gait features or motion features may be related to a step segment (e.g. half of a motion cycle for a 2-legged animal, a quarter of a motion cycle for a 4-legged animal, or 1/N of a motion cycle for a N-legged animal). When a N-legged animal moves forward, each of the N legs takes turns to have maximum forward (or positive) speed. The IQ (speed) may have N local maximum in a motion cycle of the N-legged animal. A step segment may comprise a time period from a local maximum to the next local maximum, or a phase-shifted version of the time period.

For a 2-legged person, a motion cycle has two step segments. Odd step segments may correspond to left foot movement (or left step in a motion cycle) while even step segments may correspond to right foot movement (or right step in a motion cycle). As an example, each left step may correspond to the period from left foot touching the ground to right foot touching the ground. Each right step may correspond to the period from right foot touching the ground to left foot touching the ground. Alternatively, each left step may correspond to the period from left foot leaving the ground to right foot leaving the ground. Each right step may correspond to the period from right foot leaving the ground to left foot leaving the ground.

Some motion features are related to a stride or a motion cycle. For 4-legged animals, odd motion cycles may correspond to two legs and the even cycles may correspond to the other two legs. In galloping, for example, the odd cycles may correspond to the front two legs, and the even cycles may correspond to the rear two legs. In trotting, the odd cycles may correspond to front left leg and rear right leg and the even cycles may correspond to rear left leg and front right leg. Some motion features are related to N-step segments (e.g. a 3-legged device may have 3 step segments). The motion cycles may form a wavefront, with 3 phases. Phase 1 (cycles 1, 4, 7, ...) may correspond to leg 1 (or legs 2 & 3). Phase 2 (cycles 2, 5, 8, ...) may correspond to leg 2 (or legs 1 and 3). Phase 3 (cycles 3, 6, 9, ...) may correspond to leg 3 (or legs 1 and 2).

FIG. 15 illustrates an exemplary method 1500 for gait recognition, according to some embodiments of the present teaching. As shown in FIG. 15, a channel state information (CSI) of a wireless multipath channel is first collected at operation 1502. The wireless multipath channel is impacted by a gait of a moving person or animal. At operation 1504, the CSI is preprocessed. Based on the preprocessing, a speed signal is maximized, e.g. based on MRC, at operation 1506 to achieve a maximum SNR. At operation 1508, the walking speed is estimated based on the maximum SNR.

At operation 1510, a stable walking period is detected. Based on the stable walking period, a gait cycle is estimated at operation 1512. Then gait related features are extracted at operation 1514 from the gait cycle. At operation 1516, the gait is recognized based on the extracted gait features. For example, by comparing the gait features with features stored in a database, a user identity is determined for the user moving with the gait.

The disclosed system GaitWay can be implemented on commodity WiFi devices and conduct experiments in a typical building with an area of about 5, 000 ft2. In one embodiment, one can consider different settings by placing the WiFi Tx and Rx at different locations during multiple sessions of data collection. In one embodiment, the disclosed system can have 6 different settings, where the Tx and Rx are put on a stand with a height of about 1 m. The Tx and Rx are separated by 8 to 11 meters for all settings, blocked by one or multiple walls. The Tx and Rx are both commercial laptops equipped with off-the-shelf WiFi network interface card and unmodified omnidirectional chip antennas. The disclosed system can use 5.8 GHz channels (by default channel 161) with a bandwidth of 40 MHz. There are a number of WiFi devices co-existing on the same channel.

In one embodiment, the disclosed system can collected gait instances from 11 human subjects, of which 5 are female and 6 are male. During data collection, the users were walking around continuously and freely in their natural way. The user was free to walk through any area as will. Some of them read news, play mobile games, or talk on the phone while walking. The experiments were conducted on 4 different days over a six-month period. For each day, the disclosed system collected data for two sessions at different time. The disclosed system obtained 8 sessions of data in total, each under a different setting. Users wore different clothes (from summer to autumn) during different sessions of data collection. For each session, the disclosed system measured for about 10~20 minutes of walking for each subject. All human subjects involved in the data collection were approved by IRB. The data were anonymized for privacy concern. In total, the disclosed system can collect about 1030 minutes of walking data from the 11 participants, from which the disclosed system can extract around 970 minutes of walking (i.e., there are about 60 minutes during which a subject is out of effective coverage of the link and the speed is not captured). From these data, the disclosed system can extract 5,283 gait instances of effective stable walking, which occupies about 680 minutes, approximately 67% of the total walking duration. The effective percentage is limited in the data collection because users are walking freely as will with frequently stop-and-go and turning behaviors that could not serve as reliable gait measurements. In practice, the training data collection would be more efficient if the users are cooperative in gait measurements. Comparing with many previous works that require the users to repeatedly walk on a fixed pathway, the disclosed system GaitWay largely eases the task and boosts gait collection to a large scale.

One can separately study the performance of three cases: single user verification, dual user distinction, a special case of multiple user recognition, and general multiple user recognition. Following the gait recognition described above, the disclosed system can use the Receiver's Operating Curve (ROC) for the False Acceptance Rate (FAR) and False Rejection Rate (FRR), and the Equal Error Rate (EER), the point on the ROC where the FAR equals the FRR, to evaluate verification, and Recognition Rate (RR) for recognition evaluation. All the below results of the disclosed system GaitWay are obtained on a 10-fold validation basis.

To evaluate the performance of GaitWay for single user verification, the disclosed system can test each subject in the dataset by using the gait instances of all other users as negative class. The disclosed system can shuffle the training and testing data for 10 folds and depict the integrated results. In one embodiment, GaitWay achieves an EER of 12.58% when using 70% of gait instances for training. In one embodiment, the performance slightly degrades when more sessions over time are involved.

Before evaluating multiple user recognition, it is interesting to study a special scenario of two users since it is very common in practice that two persons share an office room or two residents live in one apartment. It would be particularly useful if the disclosed system can distinguish one from another. The disclosed system can conduct binary classification for every pair of subjects in the dataset. One can consider precision and recall by treating one user as the positive class and the other as the negative class for each group. In one embodiment, GaitWay yields remarkable performance, with an average precision and recall of 94.84% and 95.21% respectively for 55 pairs of users when using 70% of data for training. An accuracy of >90% can be achieved with only 20% of data for training. With the automatic data collection and gait extraction, such an amount of data can be easily gathered by a walk of about 20 minutes, making GaitWay friendly for user enrollment.

The recognition for multiple users is more difficult than verification or pair distinction. In one embodiment, the RR changes with gait variations when involving different sessions, and the performance degrades as the number of sessions increases. This is because a user's gait speed may vary considerably over time. For example, one user in the experiments had a walking speed of about 0.8 m/s in one session while about 1.4 m/s in another session. Although GaitWay circumvents the use of absolute speed, the performance may still be influenced by dramatic changes in walking speed since the disclosed system is built upon walking speed alone. Unless otherwise specified, the disclosed system GaitWay can use all 8 sessions of data in the following evaluation.

As the data are collected and extracted automatically, the duration and step amounts of each gait instance would be different. The disclosed system can thus analyze whether the lengths of walking samples will affect the recognition accuracy. The disclosed system can analyze the length distribution of all testing gait instances. A subject's gait may vary for a very long walking, while too short instances do not comprehensively embody one's gait features.

The disclosed system can analyze the impact of different sessions by examining the error source distribution over data from different sessions. In one embodiment, while most of the data sessions produce similar performance, session #3 sees the worst performance of only 42.19%. This is because the Tx-Rx link is heavily blocked by a reinforced concrete main pillar of about 1m × 1m, in addition to two walls, and thus the scattering signals can be hardly captured by the Rx. One can inspect the speed estimates from session #3, which affirm to be more noisy than other sessions.

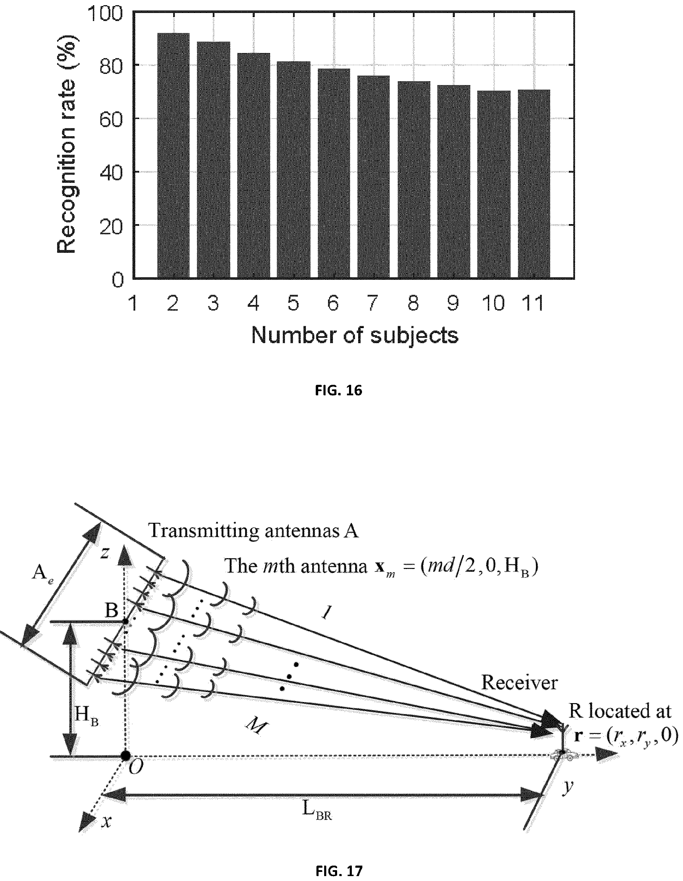

To study the impacts of subject number, one can traverse all 2036 possible combinations of the 11 subjects and integrate the results in FIG. 16. In general, the RR gradually decreases with more users being involved. The disclosed system GaitWay retains a remarkable RR of over 80% when there are 5 subjects, demonstrating promising potential for smart home where there are usually a few residents in a house. In one embodiment, subjects with lower RRs also suffer from relatively larger FAR and FRR among others, indicating that their gait patterns are less distinctive.

The disclosed system GaitWay can run in real time on personal computers. For 1 minute of data, it takes about 27 seconds (20s for speed estimation, 6s for stable period identification, and < 1s for feature extraction) in total to process. SVM costs 164s and 2s for training and testing with 1,000 instances, respectively. As training can be done offline, this cost is negligible.

Gait and rhythmic motion recognition based on the disclosed systems and methods is particularly appealing for a range of ubiquitous applications that need human identification since it can be achieved at a distance without any active user cooperation. For example, a smart building would automatically open the door for an authorized user when she walks in. A smart home would personalize the temperature and ambient light for a recognized user. A smart TV therein would react with her favorite programs. Smart home devices like Google Home and Amazon Alexa could directly interact with her in a more friendly way. For all of these to function, the user needs to do nothing but walk habitually inside the space, according to various embodiments of the present teaching.

In one embodiment, the disclosed system can monitor a rhythmic motion of an object based on channel information of a wireless multipath channel, and trigger a response action based on a result of the monitoring. According to various embodiments, the object may be a person, and the rhythmic motion being monitored may be his walking gait or hand motion or breathing. The monitoring may include an identification of the object. Upon identifying the person, the disclosed system may trigger a response action like: generating a personalized action for the person; and/or controlling a device based on a setting associated with the person. The setting may be based on the time/date of a day and month/season of the year, or a daily/weekly/monthly/yearly routine. For example, the disclosed system may send a signal to: turn on/off or adjust setting of a lamp/lighting/window/window blind/fan/TV/audio/cleaning machine, generate a greeting dialog/recommendation/suggestion/report/briefing/calendar briefing or play music/radio on a smart speaker or user interface, tune radio/TV/streaming device/media player to a channel/source, pre-heat/start a coffee machine/oven/cooking device/car, prepare car/vehicle/garage, unlock door, turn on/off alarm, start/stop some system, set air conditioner/heat to a certain temperature, turn on a computer, warm/cook some food, etc., based on a preferred setting, favorite setting, or timed setting, associated with the person.

In one example, the rhythmic motion may be a complex rhythmic motion (e.g. a person's dancing motion) formed by a number of simple, coordinated motion (e.g. the person's head motion, neck motion, left/right hand motion, left/right wrist motion, left/right finger motion, left/right arm motion, left/right shoulder motion, waist motion, hip motion, left leg motion, left foot motion, right leg motion, etc.). A stride may correspond to a period or cycle of the complex rhythmic motion. A step segment may correspond to a simple motion in the stride (a "step" within a cycle of the complex motion). A stride may be formed by combining a series of N consecutive step segments. A stride may be decomposed into N step segments. A step segment may correspond to a simple motion, or no motion. In one embodiment, the monitoring may include detecting some event/action, e.g. the person falling down, the person acting in a rush, the person is doing some activity such as dancing, playing, sitting down, standing up, hopping, jumping, the person is in a good/bad mood/certain emotional state, etc. The response may include: alarming a caregiver/caretaker/manager/police/ doctor/emergency response service or team; recording/monitoring the activity for health monitoring purpose; counting the amount of steps; determining if daily activity/exercise target is achieved; or prompting the person for a preferred action (e.g. play matching music during exercise) or environment setting. In one embodiment, the object may be a dog; and the rhythmic motion may be the gait. The response action may be to prepare dog food/drink, water, lock/unlock door, adjust temperature/window/fan, etc.

In one embodiment, a system for rhythmic motion monitoring is described. The system comprises: a transmitter, a receiver, and a processor. The transmitter is configured for transmitting a first wireless signal towards an object in a venue through a wireless multipath channel of the venue. The receiver is configured for: receiving a second wireless signal through the wireless multipath channel between the transmitter and the receiver. The second wireless signal differs from the first wireless signal due to the wireless multipath channel which is impacted by a rhythmic motion of the object. The processor is configured for: obtaining a time series of channel information (CI) of the wireless multipath channel based on the second wireless signal, monitoring the rhythmic motion of the object based on the time series of CI (TSCI), and triggering a response action based on a result of the monitoring. According to various embodiments, the processor may be physically coupled to at least one of the transmitter and the receiver.

In one embodiment, the processor is further configured for: computing a time series of intermediate quantity (IQ) based on the TSCI; and monitoring the rhythmic motion of the object based on the time series of IQ. In one embodiment, the system further comprises: an additional transmitter configured for transmitting a third wireless signal through an additional wireless multipath channel; and an additional receiver configured for receiving a fourth wireless signal through the additional wireless multipath channel, wherein the fourth wireless signal differs from the third wireless signal due to the additional wireless multipath channel which is impacted by the rhythmic motion of the object in the venue, wherein the processor is further configured for: obtaining an additional TSCI of the additional wireless multipath channel based on the fourth wireless signal; computing an additional time series of IQ based on the additional TSCI; and monitoring the rhythmic motion jointly based on the time series of IQ and the additional time series of IQ. In one embodiment, the additional transmitter is the transmitter; and the third wireless signal is the first wireless signal. In another embodiment, the additional receiver is the receiver.

In one embodiment, the processor is further configured for: computing at least one local characteristic of the IQ in the time window of the stable rhythmic motion, wherein the at least one local characteristic comprises at least one of: a local maximum, local minimum, zero crossing, local maximum of a derivation of the IQ, local minimum of the derivative, and zero crossing of the derivative; segmenting the time window into at least one step segment based on time stamps associated with the at least one local characteristic of the IQ, each step segment spanning from a time associated with a local characteristic to another time associated with a next local characteristic; and identifying at least one motion cycle, each motion cycle comprising N consecutive step segments, wherein N is a positive integer. In another embodiment, each of the at least one local characteristic is a local maximum.

The present teaching further discloses systems for target or object tracking, based on time reversal and massive MIMO, in an outdoor environment. Outdoor environment is known to be lacking or scarce in multipath, which is difficult for time reversal to be applied. The present teaching discloses an alternative way to realize time reversal (TR) in an outdoor environment - using massive MIMO antenna arrays to contribute additional multipaths. Although multipaths may not be rich for each antenna in outdoor environment, combined multipaths can be rich due to the massive amount of antennas in massive MIMO. Similar to the spatial "focusing ball" achieved by time reversal in indoor environment, a spatial "focusing beam" can be achieved by combining time reversal with massive MIMO. Based on this spatial focusing bean, the present teaching discloses a highly accurate target localization method suitable for outdoor environment. Extensive simulation results show that the disclosed system can obtain centimeter accuracy for outdoor localization and tracking.

One main advantage of the disclosed system over GPS based tracking and navigation systems is NLOS operation. GPS requires direct LOS with multiple GPS satellites in space and thus would not work in partially covered/blocked areas such as football stadium (e.g. Super Bowl stadium with top opened), parking structures, down-town areas (e.g. New York city Manhattan area) with dense, tall buildings/ skyscrapers, forest with mildly dense trees/vegetations, maze, canyons/valleys, etc. The disclosed system can work equally well in both LOS and NLOS conditions. Thus, unlike GPS, the disclosed system can operate perfectly well in these problematic areas of GPS.

In a primary set up: a wireless transmitter (Type 1 device) is stationary (e.g. an installed router or access point, AP) with massive amount of antennas (e.g. more than 20) while a wireless receiver (Type 2 device) is a mobile device (e.g. smart phone, car, AGV) with at least one antenna. The mobile device (Type 2 device) is the device of which the location is to be tracked.

In an alternative set up in another embodiment: a wireless transmitter (Type 1 device) is stationary (e.g. an installed router or access point, AP) with at least one antenna while a wireless receiver (Type 2 device) is a mobile device (e.g. smart phone, car, AGV) with massive amount of antennas (e.g. more than 20). The mobile device (Type 2 device) is the device to be tracked. In an alternative set up in another embodiment: a wireless receiver (Type 2 device) is stationary (e.g. an installed router or access point, AP) with massive amount of antennas (e.g. more than 20) while a wireless transmitter (Type 1 device) is a mobile device (e.g. smart phone, car, AGV) with at least one antenna. The mobile device (Type 1 device) is the device to be tracked. In an alternative set up in another embodiment: a wireless receiver (Type 2 device) is stationary (e.g. an installed router or access point, AP) with at least one antenna while a wireless transmitter (Type 1 device) is a mobile device (e.g. smart phone, car, AGV) with massive amount of antennas (e.g. more than 20). The mobile device (Type 1 device) is the device to be tracked.

In another embodiment, instead of "a distance", "a speed" or "an acceleration" of the current movement of the moving device is computed. In yet another embodiment, instead of "a distance", a function of "a distance", "a speed" and/or "an acceleration" is computed. In one embodiment, the "distance" may be based on an autocorrelation focusing strength (ACFS).

The "first spatial-temporal information" or first STI may comprise at least one of: a distance, a height, a depth, a displacement, a location, a speed, an acceleration, an angle, an angular speed, an angular acceleration, a derivative, a high order derivative, an integration, a direction, a time, a time period, a timing, a time trend, an incremental change of a spatial-temporal information (STI), an aggregate change of STI, a timed change of STI, a set of STI, and/or another spatial-temporal quantity. Similarly, the second STI may comprise at least one of: a distance, a height, a depth, a displacement, a location, a speed, an acceleration, an angle, an angular speed, an angular acceleration, a derivative, a high order derivative, an integration, a direction, a time, a time period, a timing, a time trend, an incremental change of a spatial-temporal information (STI), an aggregate change of STI, a timed change of STI, a set of STI, and/or another spatial-temporal quantity. The second STI may be computed based on the first STI. For example, the first STI may be a distance, a directional distance, or a projection of a distance in a certain direction. In one embodiment, the first STI may be a distance in an orthogonal direction of an "axis". The axis connects a center of the MIMO antenna of the stationary device (e.g. base station) and a center of the antenna of the moving device (or object). In another example, the moving device may have the MIMO antennas (the large number of antennas, e.g. 50, or 100 or 200 or 1000 or 10000, etc.) while the stationary device may have only a few antennas (e.g. 1, 2, 3, or 4).

The first STI may be computed based on a "focusing beam principle". That is, a CSI-based feature such as time reversal resonating strength or auto-correlation focusing strength may be a sinc function of the distance of the orthogonal direction. The CSI-based feature may form a beam around the axis, with the beam strength being a sinc function of the radial distance. The CSI-based feature (e.g. ACFS) may be first computed and then the first STI (e.g. distance) may be computed based on the CSI-based feature and the sinc function (e.g. by using table look-up, or by computing/estimating the inverse sinc function).

The ACFS may be computed between the current CSI at time t, and a past CSI at time t-k (where k is an integer). The k may be allowed to change from 1 to 2, 2 to 3, and so on, in search of a k such that the ACFS between the CSI at time t and the CSI at time t-k is a characteristic point of ACFS (e.g. a local minimum, a local maximum, the first local minimum, the first local maximum, etc.)

The first STI may be computed based on a trigonometric function (e.g. it may comprise at least one of: sine, cosine, tangent, arc-sine, arc-cosine, arc-tangent, secant, cosecant, co-tangent, or another trigonometric function) of an angle between the axis and another axis of the MIMO antenna of the stationary device (e.g. base station). For example, the MIMO antenna may be a 1-dimensional array of equally spaced antenna, and the another axis may be the straight line formed by the antenna array, and the axis may be connecting the center of the antenna array of the stationary device to the moving device. The first STI may be the distance travelled by the moving device in the orthogonal direction (e.g. a projection of the distance travelled by the moving device onto the orthogonal direction) between time t-k and time t. The first STI may be computed based on an aperture of the MIMO antenna. If there are L antennas in a straight line equally spaced at d, the aperture may be (L-1)d. The antennas may not be equally spaced. The MIMO antennas may not be in a 1-dimensional configuration. For example, the MIMO antennas may comprise several groups of antennas, each group forming a straight line. The straight lines may or may not be co-planar. In another example, the MIMO antennas may be in a 2-dimensional lattice, or a 3-dimensional lattice. In another example, the MIMO antennas may be at pseudo-random locations in a 2-D or 3-D area. The first STI may be computed based on a distance between the center of the antenna array and the moving device (e.g. at time t-k). The second STI may be a location. The location (second STI) of the moving device (or location of the object) may be computed based on the first STI. The second STI may be computed as a point on a line which is parallel to the axis and at a distance of the first STI from the axis. The first STI and second STI may be computed at different rates: e.g. 1 Hz, 2Hz, 3Hz, 5Hz, 10Hz, 20Hz, 30Hz, 50Hz, 100Hz, 200Hz, 300Hz, 500Hz, 1000Hz, 1500Hz, 2000Hz, 3000Hz, 5000Hz, 10000Hz, etc. For example, the first STI may be computed at 100Hz while the second STI may be computed at 1Hz.

The "object" may be a person, an animal, another device, a vehicle, a machine, a movable structure, a structure/material subject to a force (e.g. wind, earthquake, vibration, impact, stress, tension, buoyance, fluid flow, etc.), a robotic device, an AGV. In another embodiment, the "distance" may be a first STI computed and used to compute a second STI.

TR has been proved to be effective in localizing the target accurately even in environment with serious multipath signals. By further exploring the resonance phenomenon in spatial domain, a high accuracy indoor speed estimation system has been developed. Inspired by this promising property, the present teaching discloses that: in far field scenario, massive MIMO can harvest a similar spatial focusing beam as TR does. As such, a high accuracy target localization method is developed.

The present teaching further discloses a target speed estimation using the focusing beam of multiple distributed base stations equipped with future 5G massive MIMO antennas. Furthermore, a new target localization method is disclosed based on the speed estimation results. Extensive numerical simulations show that this new method can achieve centimeter accuracy in some ideal case while at least sub-meter accuracy in some extreme environments.

In one embodiment, the disclosed method enjoys a low complexity because the computation of ACFS distribution is linear proportional to the received data size. Based on the speed estimation, a method is disclosed to estimate the moving direction by further using the BS deployment information. Most of the existing works such as DOA estimation methods can only estimate the local direction of the target, while the disclosed method can obtain the absolute direction. In addition, the disclosed method can achieve less than 2 degrees accuracy in the presence of NLOS components, which outperforms the benchmark methods.

In another embodiment, some lower-layer details (e.g. a mode, an operation, a transmission, a measurement, a capability, an information, a feedback) of a method/system/device may enable a higher layer application (e.g. software) to perform wireless sensing. Wireless sensing enablement (WSE) is for enabling of wireless sensing. Suppose an application (e.g. a software application, a mobile app, an embedded software, a firmware, etc.) wants to perform wireless sensing based on some wireless data (e.g. WSE measurement) obtained at some lower layers. In the present teaching, WSE refers to anything (e.g. a mode, a capability, a wireless transmission, a measurement, an operation, an information, a feedback) in the Physical (PHY) layer, or Median-Access (MAC) layer, or other layers below the application layer, of any Type 1 heterogeneous wireless device (transmitter or TX) or any Type 2 heterogeneous wireless device (receiver or Rx) or another device (e.g. a local server, a cloud server) that enable the applications to perform wireless sensing.

Navigation systems have been widely used in modern applications in which the moving speed and direction estimations are two key steps. Instead of using traditional device-based orientation sensor such as accelerometer and magnetometer to estimate the moving direction, the present teaching discloses a novel radio frequency (RF) signal-based moving direction sensing scheme by using 5G massive multiple input and output (MIMO) system. The energy distribution of the received signal in massive MIMO in both near- and far-field scenarios is developed herein. The energy distribution in near-field is here proved to be highly related to the geometric shape of the antenna deployment. In contrast, the energy distribution turns out to be a stationary sinc-like focusing beam in far-field scenario. Inspired by such an observation, a novel method is disclosed to estimate the speed of a moving target with respect to a single based station. The moving direction can be further determined by jointly considering the speed estimation results and the geometric property of the locations between the target and nearby base stations. Finally, numerical simulations show that the disclosed RF-based method can achieve a high accuracy in which the moving speed estimation error is less than 1.5m/s while the moving direction estimation error is within 2 degrees.

The present teaching utilizes the time-reversal resonating strength (TRRS), which is proved to be a stationary and location-independent focusing-ball shaped distribution around the receiver. By leveraging such an observation, a target tracking method with centimeter-level accuracy has been disclosed and verified by extensive experiments.

In the present teaching, a massive MIMO system utilizes multiple antennas to physically generate a large number of signal components which play similar roles as multipaths in a rich-scattering environment. In addition, the incident signals generated by massive MIMO can only get to the receiver from the transmitter side. One can prove that in far-field scenario, the autocorrelation function strength (ACFS) distribution of the received signal around the receiver exhibits a sinc-like beam in spatial domain, which can provide direction information. By further using the dense deployment of 5G massive MIMO base stations (BSs), a new radio frequency (RF) signal based moving speed and direction estimation method is disclosed. In addition, it leverages the natural superposition property of the received signal, which reduces the computational load greatly.

In one embodiment, the ACFS distribution of the received signal of a massive MIMO communication system in both near- and far-field scenarios is derived. In near-field situation, the ACFS distribution is closely related to the geometric parameters of the antenna array while it shows a stationary sinc-like beam in far-field scenario.

In one embodiment, considering the practical far-field scenario, a moving speed estimation algorithm is developed by using the aforementioned ACFS distribution, which achieves high accuracy with the speed estimation error less than 1.5m/s. Because the ACFS distribution of the received signal is stable, meaning the computation complexity for calculating the ACFS is linearly proportional to the received data size, the disclosed speed estimation algorithm also enjoys a low complexity.

In one embodiment, based on the speed estimation, an approach is disclosed to estimate the moving direction by further using the BS deployment information. Numerical simulations show that the method is environment independent and the moving direction estimation error is less than 2 degrees, which outperforms the benchmark methods.

In a signal model of one embodiment, a base station (BS) equipped with a massive MIMO array of *M* antennas communicates with a receiver fixed on a mobile object. In a typical downlink system, BS transmits probing signals which are recorded by the receiver. FIG. 17 illustrates the set-up of the communication system in which 'B' and 'R' represents the center of the base station and receiver respectively. $H_B$ and $L_{BR}$ denote the altitude of the BS and the horizontal distance between the BS and the receiver respectively. $A_e$ is the aperture of the antenna A.

Assume the inner element space is *d,* the aperture can be expressed as $A_e = (M - 1)d$ where $d = \lambda$ equals to the wavelength of the transmitting signal. Then, in a far-field scenario, it is reasonable that $L_{BR} \gg A_e$ holds. In addition, one can assume that all the transducers are deployed with omni-directional antennas. In a free space with no boundary, the receiving signal at baseband can be expressed as

$$y(t) = \sum_{m=1}^{M} \frac{exp(j(k|\mathbf{x}_m - \mathbf{r}| + \phi_m))}{4\pi|\mathbf{x}_m - \mathbf{r}|} + n(t), \tag{13}$$

where k=2π/λ is the wave number and |x_m-r| denotes the Euclidean spatial distance between the m-th antenna and the receiver. n(t) represents the additive Gaussian noise. ϕ_m (m=1,2,...,M) is the synthetic phase distortion of the m-th propagation path including initial phase, phase error caused by inner system interference, propagation attenuation, reflectors and so on. In general, this synthetic phase distortion can be assumed as i.i.d. uniform distributions over [-π,π) for all m=1,2,...,M.

TR is a signal processing technique which tries to utilize the channel state information (CSI) embedded in multipath signals. Consider a rich-scattering environment such as indoor or urban area, usually there are many obstacles between the transmitter and receiver, which causes the unavoidable non-line-of-sight (NLOS) multipath propagation. Given a sufficiently large bandwidth, these multipath components (MPCs) can be decomposed into different taps in discrete-time. Here, one can denote the channel impulse response (CIR) from the transmitter *T* to receiver R at the *k*th tap as $h(k; T \to R)$. Typically, in wireless communication system, the receiver *R* first transmits a pilot impulse which is then captured by transmitter *T*. One can then easily estimate the CIR $h(k; R \to T)$ by analyzing the relationship between the signal collected by the transmitter and original pilot impulse. Then, the transmitter T sends back a reversed and conjugate counterpart of the CIR, i.e., $h^*(-k; R \to T)$ where * is complex conjugation operation. When the channel reciprocity holds, which has been verified by experiments, the receiving signal at position $R_s$ can be given by

$$r_s(k; R) = \sum_{l=0}^{L-1} h(l; R_s)h^*(l - k; R), \tag{14}$$

where *L* is the length of the CIR, i.e., $h(l - k;R) = 0$ when $(l - k) \notin \{0,1,..., L - 1\}$ and $k \in \{-(L - 1), ..., (L - 1)\}$. Here and after, one can simplify $h(k; T \to R_s)$ as $h(k;R_s)$ because the transmitter *T* is fixed usually. In addition, the CIR length *L* is related with the channel state and also the bandwidth of transmitting signal. From (14), only when $R_s = R$ and $k = 0$, one can get the maximum value $\sum_{l=0}^{L-1} |h(l, R)|^2$. This means that all the MPCs add up coherently at the exact position *R* and a specific time instance. Energy of the receiving signal at other positions or time stamps, or both different time and location decays differently. This is the so called spatial-temporal focusing in TR system.

In one embodiment, one can fix *k* = 0 and mainly study the TR focusing effect in spatial domain, in which one can estimate the target speed and location. To further quantify the TR focusing strength (TRFS), one can define the normalized energy of the received signal at location $R_s$ as

$$\rho(R, R_s) = \left| \frac{r_s(0;R)}{\sqrt{\sum_{l=0}^{L-1}|h(l;R)|^2}\sqrt{\sum_{l=0}^{L-1}h(l;R_s)|^2}} \right|^2 . \tag{15}$$

From the previous description about TRFS, one can easily conclude that rich multipath signals is one necessary condition to obtain the TRFS effect. In signal processing terminology, TR scheme takes large bandwidth to resolve multipaths naturally existing in a rich-scattering environment and control each of them. However, in an open-air environment such as a street, plaza or an outdoor parking lot, there is usually not enough multipath signals. Moreover, bandwidth is limited in real communication system. Thanks for the booming massive MIMO technique, one can explore a good alternative which can achieve similar performance to TR system for outdoor localization problem. Intuitively, a massive MIMO system utilizes multiple antennas to physically generate a large number of signal components which play the similar roles to multipaths in a rich-scattering environment. Instead of computing the TRFS, one can make a tiny change by compute the autocorrelation function (ACF) of the receiving signal. The reason is that ACF is more tolerable with noise in practical system by self-averaging process automatically. Different from the TRFS which is a ball, the ACF distribution in massive MIMO system turns out to a focusing beam. To distinguish it from the previous TRFS, one can name it as autocorrelation focusing strength (ACFS) whose derivation is as follows.

Recall (13), one can denote the positions of the moving object in two different time stamps $t_0$ and $t_s$ as $\mathbf{r}_0$ and $\mathbf{r}_s$, respectively. Then, the autocorrelation function (ACF) of the received energy between $\mathbf{r}_0$ and $\mathbf{r}_s$ is given by

$$\begin{aligned}
\eta_y(\mathbf{r}_0, \mathbf{r}_s) &= \eta_y(t_0, t_s) = \mathbb{E}[y(t_0)y^*(t_s)] \\
&= \sum_{i=1}^M \sum_{m=1}^M \mathbb{E}_\phi \left\{ \frac{\exp(j(k|\mathbf{x}_i - \mathbf{r}_0| + \phi_i))}{4\pi|\mathbf{x}_i - \mathbf{r}_0|} \right. \\
&\left. \cdot \frac{\exp(-j(k|\mathbf{x}_m - \mathbf{r}_s| + \phi_m))}{4\pi|\mathbf{x}_m - \mathbf{r}_s|} \right\},
\end{aligned} \tag{16}$$

where $\mathbb{E}$ represents expectation operator. Note that the Gaussian noise $n(t)$ does not impact the ACF distribution of the receiving signal when SNR is high because it is independent with the signal. However, when SNR decreases, $n(t)$ influences the ACF distribution in some extent, which is interpreted in the simulation part. Moreover, by the far-field approximation where $L_{BR} \gg A_e$, $|\mathbf{x}_i - \mathbf{r}_0|$ and $|\mathbf{x}_m - \mathbf{r}_s|$ in the denominator of (16) can be approximated as the same for all elements, i.e., $|\mathbf{x}_i - \mathbf{r}_0| \approx |\mathbf{x}_0 - \mathbf{r}_0|$ and $|\mathbf{x}_m - \mathbf{r}_s| \approx |\mathbf{x}_0 - \mathbf{r}_s|$. This approximation is reasonable because $|\mathbf{x}_i - \mathbf{r}_0|$ and $|\mathbf{x}_m - \mathbf{r}_s|$ in the denominator of (16) only scale the amplitude of $\eta_y(t_0, t_s)$. However, similar approximation cannot be applied in the numerator of (16) because there is an extra wave number factor $k = 2\pi/\lambda$. In other words, the phase term in (16) will change $2\pi$ rad whenever $|\mathbf{x}_i - \mathbf{r}_0|$ and $|\mathbf{x}_m - \mathbf{r}_s|$ in the numerator change by $\lambda = 1/f_c$, which is very small for 5G communication system because the carrier frequency $f_c$ (could be 28GHz) is usually very high. In the next derivation process, one can omit the denominator part of (16) for simplicity because they can be regarded as a constant in far field scenario.

Next, one can decompose (16) into two different cases, i.e., a) i = m and b) $i \neq m$. For notation simplicity, one can define the following two phase terms

$$\psi_{i,0} = k|\mathbf{x}_i - \mathbf{r}_0|, \psi_{m,s} = k|\mathbf{x}_m - \mathbf{r}_s|, \tag{17}$$

Subsequently, $\eta_y(\mathbf{r}_0, \mathbf{r}_s)$ is derived for each of the two cases. Consider $i = m$, one can have

$$\eta_y^{1st}(\mathbf{r}_0, \mathbf{r}_s) = \sum_{m=1}^M \exp(jk(\psi_{m,0} - \psi_{m,s})) \tag{18}$$

From FIG. 17, the coordinates of the mth transmitting antenna element is $(md/2, 0, H_B)$, then one can get

$$|\mathbf{x}_m - \mathbf{r}_0| = \sqrt{x_m^2 + L_{BR}^2 + H_B^2} = \sqrt{x_m^2 + L^2} \tag{19}$$

where $x_m = md/2$ and $L = \sqrt{L_{BR}^2 + H_B^2}$ is the Euclidean spatial distance between the antenna center and the receiver. Next, one can define $\mathbf{r}_s$ and compute corresponding $|\mathbf{x}_m - \mathbf{r}_s|$.

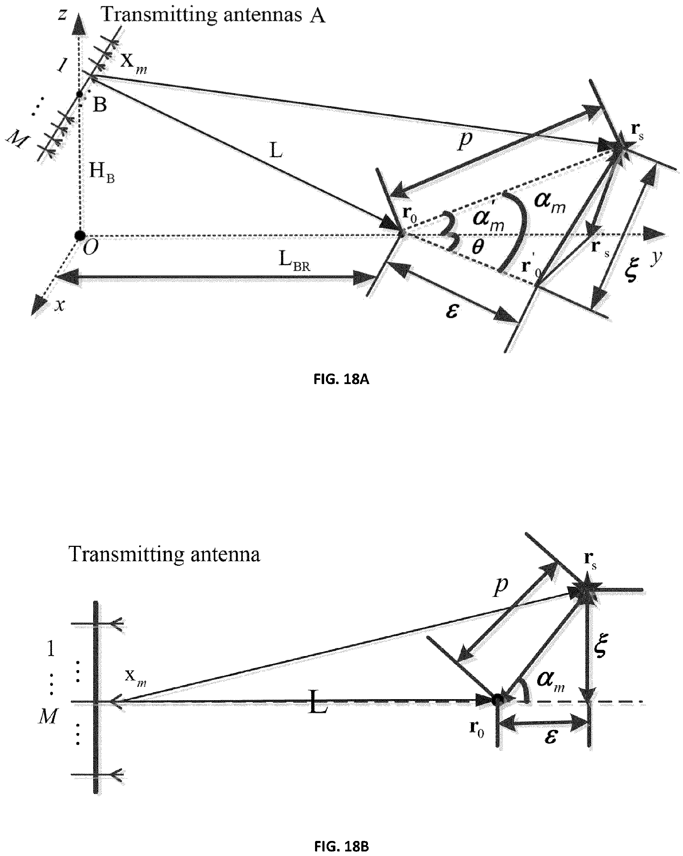

Assume that $\mathbf{r}_0$ and $\mathbf{r}_s$ are in the $xOy$ plane as shown in FIG. 18A, and $p$, $\varepsilon$ and $\xi$ represents the Euclidean

spatial distance, range and cross-range between $\mathbf{r}_0$ and $\mathbf{r}_s$. From FIG. 18A, one can get the following equations by using the law of cosines

$$\cos\alpha_m = \cos\alpha_m' \cdot \cos\theta \, \text{and} \, \cos\theta = L_{BR}/\sqrt{H_B^2 + L_{BR}^2}. \tag{20}$$

In a far field scenario, $L_{BR}$ is usually ten times or more bigger than $H_B$. Hence, the approximation $\cos\theta \approx 1$ holds. Then, it is easy to obtain $\cos\alpha_m = \cos\alpha_m'$ by substituting $\cos\theta \approx 1$ into (20). Consequently, the 3-dimension signal propagation geometry shown in FIG. 18A can be simplified as FIG. 18B. Then, similar to (19), for $\mathbf{r}_s$ close to $\mathbf{r}_0$, $|\mathbf{x}_m - \mathbf{r}_s|$ can be expressed as

$$|\mathbf{x}_m - \mathbf{r}_s| = \sqrt{(L + \varepsilon)^2 + (x_m - \xi)^2}. \tag{21}$$

As shown in FIG. 18B, the Euclidean spatial distance between $\mathbf{r}_0$ and $\mathbf{r}_s$ is $p$. However, the electromagnetic wave is a vector which contains both modulus and direction rather than just a scaler. From antenna propagation theory, the propagation path difference which finally influences the phase of the receiving signal at $\mathbf{r}_s$ ($\mathbf{r}_0$ working as the reference point) is the projection of $p$ along $\overleftarrow{x_m r_0}$ direction, i.e., $\overleftarrow{Oy}$ shown in FIG. 18B. Based on the above analyses, one can have

$$p\cos\alpha_m = \frac{|\mathbf{x}_m - \mathbf{r}_0|^2 + p^2 - |\mathbf{x}_m - \mathbf{r}_s|^2}{2|\mathbf{x}_m - \mathbf{r}_0|} = \frac{-L\varepsilon + x_m\xi}{\sqrt{L^2 + x_m^2}}. \tag{22}$$

In far-field scenario, $L \gg x_m$ holds. By using parabolic approximation, one can get

$$\sqrt{L^2 + x_m^2} \approx L + \frac{x_m^2}{2L} \approx L \tag{23}$$

Inserting (23) into (22), $p\cos\alpha_m$ is re-written as

$$p\cos\alpha_m = -\varepsilon + x_m\xi/L \tag{24}$$

Referring to (17) and (18), one can compute the phase difference ($\psi_{m,0} - \psi_{m,s}$) by

$$\psi_{m,0} - \psi_{m,s} = -kp\cos\alpha_m = k(\varepsilon - x_m\xi/L). \tag{25}$$

Therefore, $\eta_y^{1st}(\mathbf{r}_0, \mathbf{r}_s)$ in (18) can be re-formulated as

$$\begin{aligned}\eta_y^{1st}(\mathbf{r}_0, \mathbf{r}_s) &= \sum_{m=1}^{M} exp(jk(\varepsilon - x_m\xi/L)) \\ &= exp(jk\varepsilon) \sum_{m=1}^{M} exp(-jkx_m\xi/L)\end{aligned} \tag{26}$$

From the definition in (19), $x_m = md/2$ and $d$ is very small (compared with aperture $A_e = Md/2$ and $L$). As a result, one can approximate the summation in (26) by an integration expressed as

$$\begin{aligned}\eta_y^{1st}(\mathbf{r}_0, \mathbf{r}_s) &= exp(jk\varepsilon) \sum_{m=1}^{M} exp(-jkmd\xi/(2L)) \\ &= \frac{exp(jk\varepsilon)}{d} \int_{-\frac{A_e}{2}}^{\frac{A_e}{2}} exp(-jkx\xi/L)dx \\ &= \frac{2L exp(jk\varepsilon)}{dk\xi} \sin(\frac{k\xi A_e}{2L}) \\ &= \frac{A_e exp(jk\varepsilon)}{d} \mathrm{sinc}(\frac{k\xi A_e}{2L})\end{aligned} \tag{27}$$

where sinc($t$) = sin($t$)/$t$. Take the square of $\eta_y^{1st}(\mathbf{r}_0, \mathbf{r}_s)$ in (27) and then normalize it, one can then obtain

$$\left|\eta_y^{1st}(\mathbf{r}_0, \mathbf{r}_s)\right|^2 = \left|\text{sinc}\left(\frac{k\xi A_e}{2L}\right)\right|^2. \tag{28}$$

From (28), when $\xi = 0$, $\left|\eta_y^{1st}(\mathbf{r}_0, \mathbf{r}_s)\right|^2$ can get the maximum value 1. Otherwise, when $\xi \neq 0$, $\left|\eta_y^{1st}(\mathbf{r}_0, \mathbf{r}_s)\right|^2$ will be smaller than 1. Next, one can derive the case where $i \neq m$ in (16), i.e.,

$$\begin{aligned}
&\eta_y^{2nd}(\mathbf{r}_0, \mathbf{r}_s) \\
&= \sum_{i=1}^{M} \sum_{m=1, m \neq i}^{M} \mathbb{E}_\phi \left\{ \frac{exp(j(k|\mathbf{x}_i - \mathbf{r}_0| + \phi_i))}{4\pi |\mathbf{x}_i - \mathbf{r}_0|} \right. \\
&\left. \cdot \frac{exp(j(k|\mathbf{x}_m - \mathbf{r}_s| + \phi_m))}{4\pi |\mathbf{x}_m - \mathbf{r}_s|} \right\},
\end{aligned} \tag{29}$$

Similar to the analyses of (16), one can omit the denominator and also corresponding constant terms of (29). Then, the ($i,m$) pair of (29) can be given by

$$\begin{aligned}
&\eta_y^{2nd}(\mathbf{r}_0, \mathbf{r}_s)_{i,m} \\
&= \mathbb{E}_\phi \left\{ \exp[j(k(\psi_{i,0} - \psi_{m,s}) + \phi_i - \phi_m)] \right\}.
\end{aligned} \tag{30}$$

Exchanging the subscript $i$ and $m$, one can get the symmetrical ($m,i$) counterpart of (30), i.e.,

$$\begin{aligned}
&\eta_y^{2nd}(\mathbf{r}_0, \mathbf{r}_s)_{m,i} \\
&= \mathbb{E}_\phi \left\{ \exp[j(k(\psi_{m,0} - \psi_{i,s}) + \phi_m - \phi_i)] \right\} \\
&= \mathbb{E}_\phi \left\{ \exp[-j(k(\psi_{i,0} - \psi_{m,s}) + \phi_i - \phi_m)] \right\}
\end{aligned} \tag{31}$$

The summation of (30) and (31) is simplified as

$$\begin{aligned}
&\eta_y^{2nd}(\mathbf{r}_0, \mathbf{r}_s)_{i,\overline{m}} \\
&= \eta_y^{2nd}(\mathbf{r}_0, \mathbf{r}_s)_{i,m} + \eta_y^{2nd}(\mathbf{r}_0, \mathbf{r}_s)_{m,i} \\
&= \mathbb{E}_\phi \{ \cos[\underbrace{k(\psi_{i,0} - \psi_{m,s})}_{\Psi_{im}} + \underbrace{(\phi_i - \phi_m)}_{\Phi}] \}
\end{aligned} \tag{32}$$

Since $\phi_i$ and $\phi_m$ are uniformly distributed over $[-\pi, \pi)$, the probability function of $\Phi = (\phi_i - \phi_m)$ is

$$f_\Phi(\phi) = \begin{cases} 2\pi + \phi, -2\pi \leq \phi \leq 0 \\ 2\pi - \phi, 0 \leq \phi \leq 2\pi \\ 0, \text{others} \end{cases} \tag{33}$$

Then, the expectation in (32) can be reformulated as

$$\begin{aligned}
\eta_y^{2nd}(\mathbf{r}_0, \mathbf{r}_s)_{i,\overline{m}} &= \int_{-2\pi}^{2\pi} f_\Phi(\phi) \cos(\Psi_{im} + \phi) d\phi \\
&= \int_{-2\pi}^{2\pi} f_\Phi(\phi) \cos\Psi_{im} \cos\phi d\phi - \int_{-2\pi}^{2\pi} f_\Phi(\phi) \sin\Psi_{im} \sin\phi d\phi
\end{aligned} \tag{34}$$

Substituting $f_\Phi(\phi)$ into (34), one can get the following equations when $2\pi \leq \phi \leq 0$,

$$\int_{-2\pi}^{0} (2\pi + \phi) \cos\Psi_{im} \cos\phi d\phi = 2\pi \cos\Psi_{im} \sin\phi \Big|_{-2\pi}^{0} + \cos\Psi_{im} (\phi\sin\phi + \cos\phi) \Big|_{-2\pi}^{0} = 0, \tag{35}$$

$$\int_{-2\pi}^{0} (2\pi + \phi)\sin\Psi_{im}\sin\phi\, d\phi = -2\pi\sin\Psi_{im}\cos\phi\big|_{-2\pi}^{0} + \sin\Psi_{im}(\sin\phi - \phi\cos\phi)\big|_{-2\pi}^{0} = 0. \quad (36)$$

Similar to (35) and (36), the integration value of (34) when $0 \leq \phi \leq 2\pi$ is also 0. Therefore, it is easy to conclude that $\eta_y^{2nd}(\mathbf{r}_0, \mathbf{r}_s)_{\overline{i,m}} = 0$ holds. In addition, $\eta_y^{2nd}(\mathbf{r}_0, \mathbf{r}_s)_{\overline{i,m}} = 0$ is independent with the subscript $i$ and $m$, which can be verified by the derivation process in (35) and (36). Recall (29), one can directly get

$$\eta_y^{2nd}(\mathbf{r}_0, \mathbf{r}_s) = \sum_{i=1}^{M} \sum_{\substack{m=1, \\ m \neq i}}^{M} \eta_y^{2nd}(\mathbf{r}_0, \mathbf{r}_s)_{\overline{i,m}} = 0. \quad (37)$$

Combining equations (16), (28) and (37), take the square of $\eta_y(\mathbf{r}_0, \mathbf{r}_s)$ and then normalize it as

$$\begin{aligned}
&\left|\eta_y(\mathbf{r}_0, \mathbf{r}_s)\right|^2 \\
&= \left|\left|\eta_y^{1st}(\mathbf{r}_0, \mathbf{r}_s)\right| + \sum_{i=1}^{M} \sum_{m=1, m \neq i}^{M} \eta_y^{2nd}(\mathbf{r}_0, \mathbf{r}_s)_{\overline{i,m}}\right|^2 \quad (38) \\
&= \left|\eta_y^{1st}(\mathbf{r}_0, \mathbf{r}_s)\right|^2 = \left|\text{sinc}(\frac{k\xi A_e}{2L})\right|^2.
\end{aligned}$$

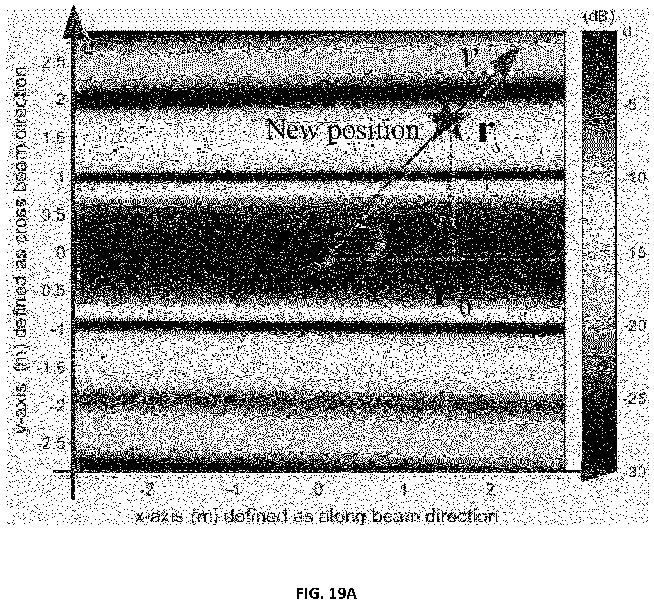

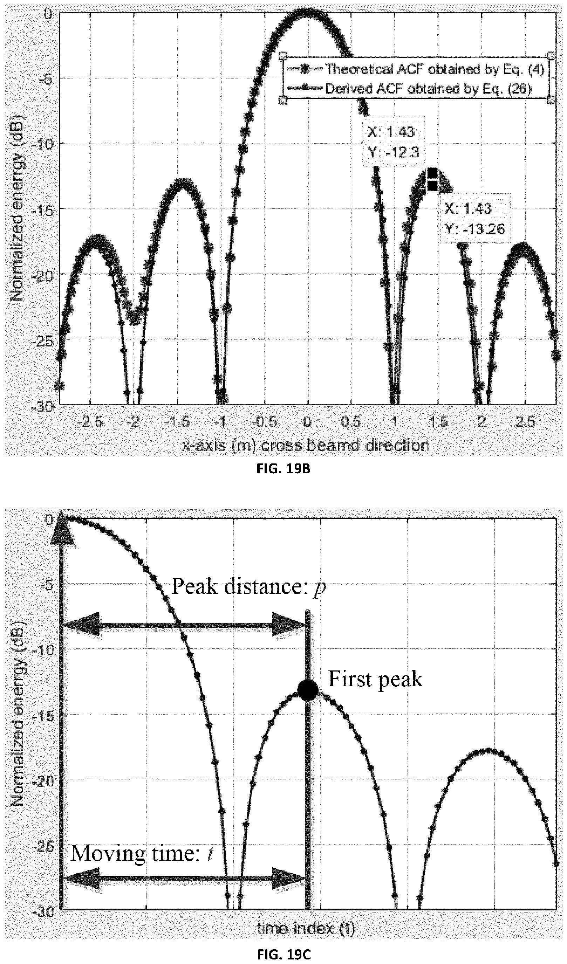

By computing the ACF of the received signal between position $r_0$ and $r_s$, one can get a similar autocorrelation focusing strength (ACFS) as the TRFS in time reversal scheme. To verify this conclusion, one can build a numerical simulation system using a massive MIMO antenna arrays with 50 elements. Also, one can consider a 5G communication system with carrier frequency $f_0$ = 28GHz. In FIG. 19, $r_0$ is set as the center of square. The ACFS distribution around $r_0$ in the spatial domain is shown in FIG. 19A, while FIG. 19B specifically demonstrates the ACFS distribution along the cross-beam direction. In FIG. 19B, numerical simulation ACF obtained by (16) is represented by red solid line while the derived ACF obtained by (38) is plotted as blue solid line. FIG. 19 intuitively indicates that the derivation ACFS matches with the theoretical ACFS well in the positions of peaks and valleys.

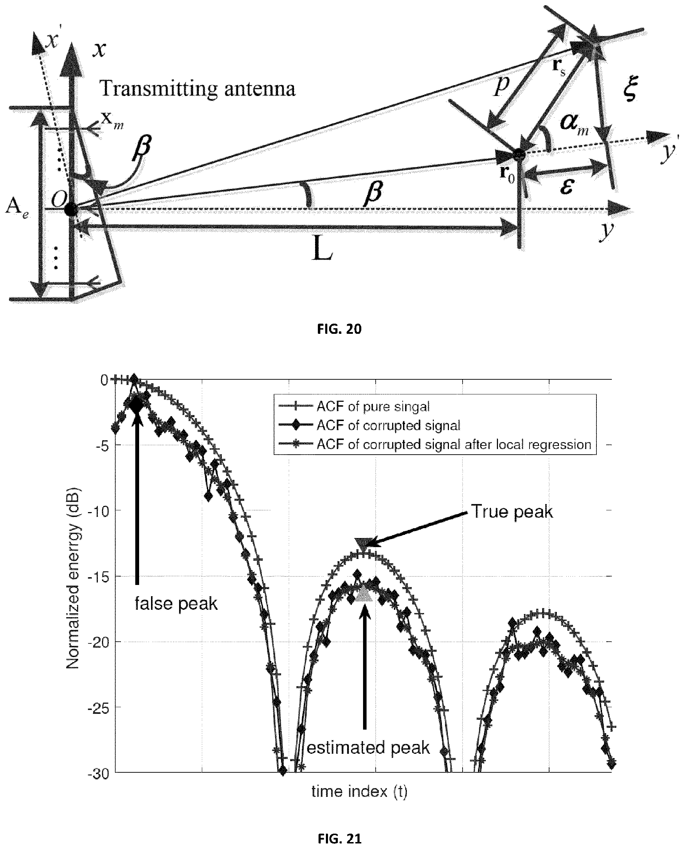

When the target keeps moving, the line between the antenna center and $r_0$ (i.e., $\overleftarrow{Or_0}$) may not be perpendicular to the direction line (i.e., $\overleftarrow{Ox}$) of the antenna deployment. As shown in FIG. 20, $\overleftarrow{Or_0} \perp \overleftarrow{Ox}$ does not hold always. In this case, the effective aperture $A_e$ in (38) should be changed into $A_e\cos\beta$. Correspondingly, distance L should be replaced by $L/\cos\beta$. This variable transformation is also consistent with the effective aperture definition used in antenna propagation and electromagnetic. Also, interested readers can refer equation (27) for better understanding about this variable transformation process. Next, one can introduce a speed estimation and target localization method based on the ACFS obtained.

The present teaching discloses a novel target speed estimation method by using the ACFS derived for the massive MIMO system. For description clarity, one can start by defining two directions, i.e., along-beam direction and cross-beam direction as shown in FIG. 19A. Moreover, the peak distance $p$ and corresponding moving time t during which the target moves from the reference position ($t$ = 0) to the first peak (time index $t$) are illustrated in FIG. 19C.

Referring back to the equation (38), the shape of ACFS distribution (shown in FIG. 19A) is determined by four parameters in total including wave number $k$, distance $L$, aperture $A_e$ and cross range $\xi$. Since $k$ and $A_e$ are independent of specific locations, the retaining parameters which affect the ACFS distribution are L and $\xi$ only. Moreover, in the far field scenario, $\xi \ll L$ holds in general. In other words, once an initial point is chosen, $L$ can be assumed as a constant when computing the ACFS in the neighbor area around the selected initial point. Therefore, the only parameter which determines the ACFS distribution with respect to an initial point is the cross-range $\xi$. Consider a special case where the target moves along the cross-beam direction with a constant speed $v$ and the receiver fixed on the target keeps recording signals transmitted by the massive MIMO array with a regular sampling rate. Then, the ACFS measured at the receiver is just a sampled version of function $\left|\text{sinc}(\frac{k\xi A_e}{2L})\right|^2$ as depicted in FIG. 19B. In this case, the peak distance (denoted by $p$ in FIG. 19C) between the initial point and the first peak of $\left|\text{sinc}(\frac{k\xi A_e}{2L})\right|^2$ can be computed mathematically, i.e., $p$ = $2.86L/kA_e \approx 1.432$m. It is very close to the result $p$ = 1.43m obtained from a simulation experiment.

Next, to estimate the speed $v$, one can know the moving time t during which the receiver moves from the initial point to the first peak. This is feasible by searching for the time stamp $\hat{t}$ corresponding to the first peak of the ACFS distribution. Accordingly, one can get the speed estimation by $\hat{v} = p/\hat{t}$. How to accurately localize the first peak so as to estimate the moving time $\hat{t}$ is a crucial step in this speed estimation method.

To get a more robust result, one can take a local regression method to firstly fit the ACFS distribution curve from which one can find the statistical peak then. This pre-processing is necessary because in practice the true peak may be corrupted by the noise or other distortions. Hence, searching for a peak directly may cause unexpected time estimation error, thus, degrading the speed estimation performance seriously. Numerical simulation in FIG. 21 shows that when the signal is corrupted, there are many glitches in the ACF of the corrupted signal, thus, causing difficulties for us to find the true peak accurately. However, after local regression process, the estimated peak is very close to the actual peak, which shows the effectiveness of the local regression process. On the other hand, the true peak should not be too close to the reference point ($t = 0$) because of the ACF distribution given by equation (38). In practice, the moving velocity is naturally limited (cannot be too fast) which implies that distance between the base station and receiver centers does not change so much during two adjacent measurements. As a result, one can use the last position to get a rough estimation about peak distance $p$ which works as a new constraint to eliminate the obvious false peak shown in FIG. 21.

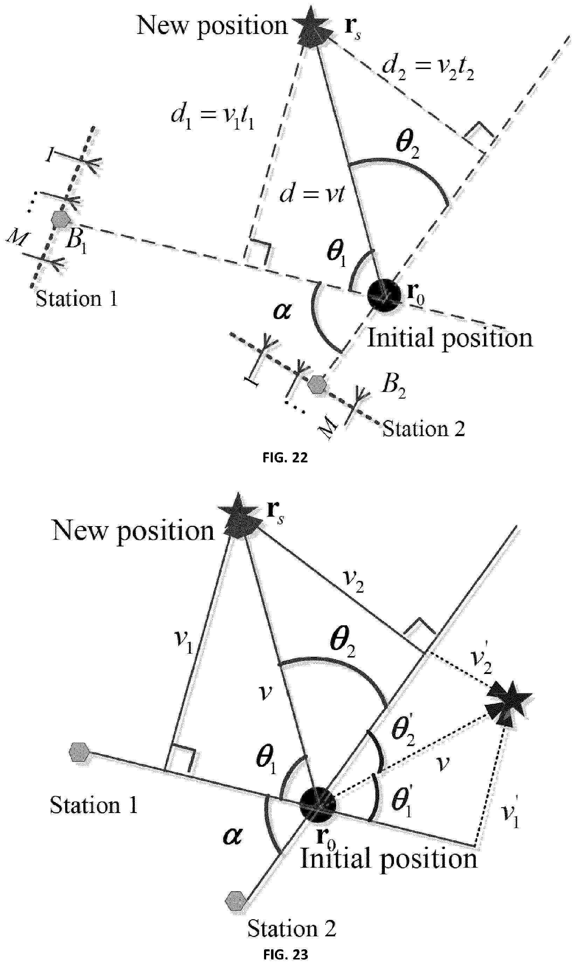

In the former special case, one can easily obtain the speed estimation result under the assumption that the target moves along the cross-beam direction. However, this assumption usually does not hold in practice. Take the moving direction $\overleftarrow{r_0 r_s}$ (different from the cross-beam direction) in FIG. 19A as an example. If one can estimate the speed by taking the same method as introduced in the previous special case 1), what one can obtain is actually the estimation of $v'$ rather than the real speed $v$. The reason is that the peak distance $p$ defined in FIG. 19C is along the cross-beam direction (the same as $\overleftarrow{r_0' r_s}$ ). Hence, one can only get the estimation of $v'$ in this case. To solve this problem, one can introduce one more station as depicted in FIG. 22. Combining the speed estimation results from these two stations, one can get the equation as follows

$$v = \frac{v_1}{\sin\theta_1} = \frac{v_2}{\sin\theta_2}. \tag{39}$$

However, one cannot obtain the value of $\theta_1$ and $\theta_2$ from equation (39) only. Further analysis about the positions of base center $B_1$, $B_2$ and the initial position $r_0$ gives us a promising way to solve $\theta_1$ and $\theta_2$. Specifically, one can get a new equation (40) from the triangular relationship among $B_1$, $B_2$ and $r_0$.

$$180° - \alpha = \theta_1 + \theta_2. \tag{40}$$

It seems that the problem has been solved perfectly because one can have already obtain an equation group composed by (39) and (40). On the contrary, the angle ambiguity becomes a new problem as shown in FIG. 23. In detail, for every pair of $(\theta_1, \theta_2)$, there exists a coupling pair $(\theta_1', \theta_2')$ because one can only know the ratio $\frac{\sin\theta_1}{\sin\theta_2} = \frac{v_1}{v_2}$ from (39) while the summation $\theta_1 + \theta_2$ cannot be determined. In other words, without further priori information, one cannot determine whether $\alpha = \theta_1 + \theta_2$ or $180° - \alpha = \theta_1 + \theta_2$. For example, if one can assume $(\theta_1, \theta_2) = (50°, 70°)$, one may get the following coupling equation groups

$$\begin{cases} \frac{\sin\theta_1}{\sin\theta_2} = \frac{v_1}{v_2} = 0.8152 \\ \theta_1 + \theta_2 = 120° \end{cases} \Rightarrow \begin{cases} \theta_1 = 50° \\ \theta_2 = 70° \end{cases} \tag{41}$$

$$\begin{cases} \frac{\sin\theta_1'}{\sin\theta_2'} = \frac{v_1}{v_2} = 0.8152 \\ \theta_1' + \theta_2' = 60° \end{cases} \Rightarrow \begin{cases} \theta_1' = 26.63° \\ \theta_2' = 33.37° \end{cases} \tag{42}$$

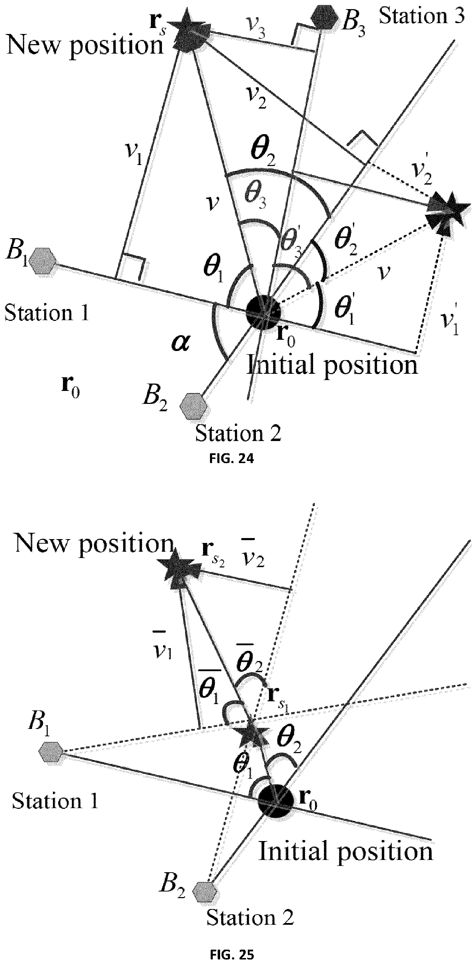

Next, one can firstly solve the angle ambiguity problem by introducing an extra station as shown in FIG. 24. To better understand the function of the third base station, one can take one particular case as an example. In FIG. 24, one can assume $(\theta_1, \theta_2, \theta_3) = (50°, 70°, 30°)$ is the true value. According to the aforementioned process, by only using base 1 and base 2, one can get the following results

$$\begin{cases} \theta_1 = 50° \\ \theta_2 = 70° \end{cases} \Rightarrow \begin{cases} \theta'_1 = 26.63° \\ \theta'_2 = 33.37°' \end{cases} \tag{43}$$

Similarly, by using base 1 and base 3, one can get another pair of value

$$\begin{cases} \theta_1 = 50° \\ \theta_3 = 30° \end{cases} \Rightarrow \begin{cases} \theta'_1 = 64.05° \\ \theta'_3 = 35.95°' \end{cases} \tag{44}$$

The true value of $\theta_1$ is unique. In other words, the $\theta_1$ value obtained from (43) and (44) should be the same. Hence, the value of $\theta_1$ can be accurately selected by comparing these two pair of results. In sequence, $\theta_2$ and $\theta_3$ can be further determined.

Once one gets the initial value of $(\theta_1, \theta_2, \theta_3)$, the third station can be omitted in the following process because one can get a new constraint. Specifically, due to the natural limitations about the moving velocity and the high sampling rates of the devices (embedded in the receiver), the direction of one particular moving object cannot be changed too rapidly between two adjacent locations. FIG. 25 intuitively demonstrates the new constraint. If the value $(\theta_1, \theta_2)$ = (50°, 70°) is already known, then in next position $\mathbf{r}_{s_1}$, one may get two pair of values $(\overline{\theta}_1, \overline{\theta}_2)$ = (51°, 29°) and $(\overline{\theta}'_1, \overline{\theta}'_2) = (65.43°, 34.57°)$. With the new constraint, $(\overline{\theta}_1, \overline{\theta}_2)$ holds a high probability to be $(\overline{\theta}_1, \overline{\theta}_2)$ = (51°,29°) because they are more close to the previous value $(\theta_1, \theta_2)$ = (50°, 70°). From this point of view, one can effectively eliminate the angle ambiguity by using just two stations as long as one can get the initial value of $(\overline{\theta}_1, \overline{\theta}_2)$ In consequently, real speed $v$ can be estimated.

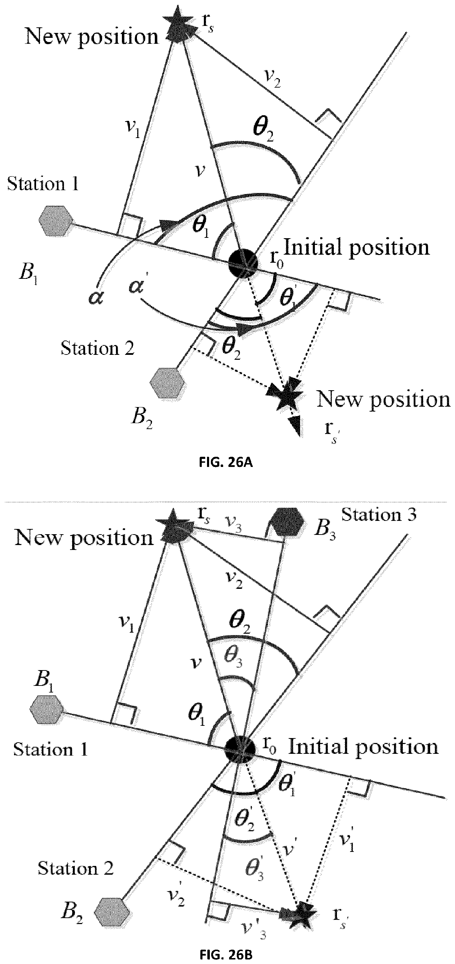

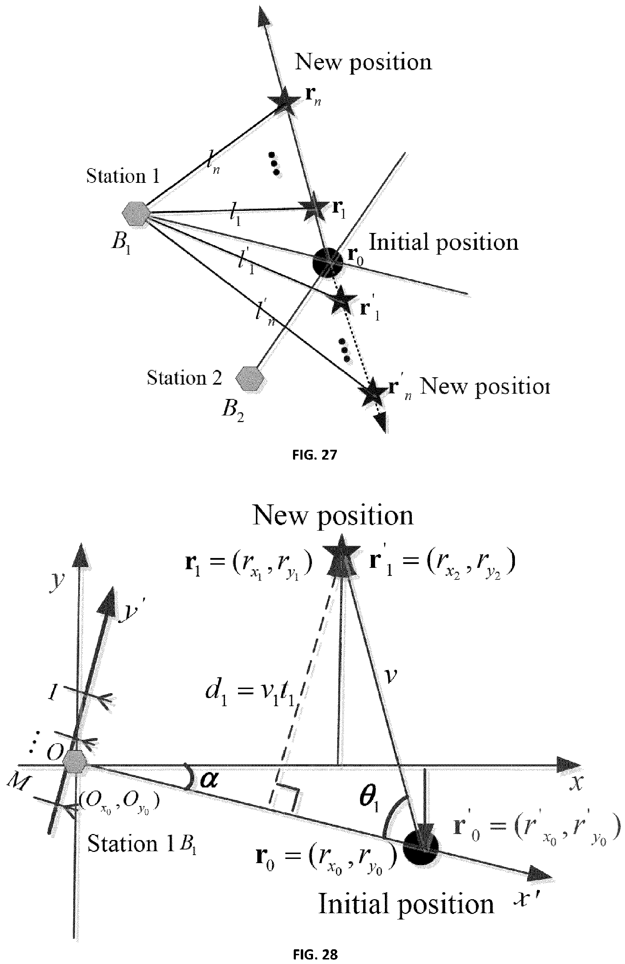

As illustrated in FIG. 26A, even one knows the initial angle of $(\theta_1, \theta_2)$, one cannot distinguish it from its opposite vertical angle pair $(\theta'_1, \theta'_2)$ painted in blue. In this case, simply adding an extra station $B_3$ does not help. FIG. 26B intuitively depicts the reason that there is also another opposite vertical angle $\theta'_3$ with respect to $\theta_3$. Clearly, it is a geometrical ambiguity problem which is independent with the station number. This problem is solved by considering a consecutive movement process shown in FIG. 27. Assuming sequence $\mathbf{r}_0, \mathbf{r}_1, \cdots, \mathbf{r}_n$ is the true position of the target moving, one can denote the peak distance between two adjacent positions $\mathbf{r}_{i-1}, \mathbf{r}_i$ as $p_{(i-1,i)}, i \in [1,2,\cdots,n]$. As a result, $p_{(i-1,i)}$ will either increase of decrease with the target moving toward to or far away from the reference base station $B_1$. The intrinsic reason comes from the ACFS distribution expressed in (38) which shows that the peak distance $p$ is proportional to the distance $L$ between the base center and the receiver. From this point of view, then variation trend (increasing or decreasing) of $p_{(i-1,i)}$ works as a sign to judge the moving direction. Note that Doppler frequency of the receiving signal may be another way to judge the moving direction since it increases when the target moves close to the base station and decreases with the target moves away. Then, one can estimate the target localization by the following methods.

Once one can get the accurate value of $\theta_1$, peak distance $p_1$ and the sign (close to or far away from) the base station (take station 1 as an example shown in FIG. 28), one can compute the coordinates of the new position $\mathbf{r}_1$ in the *x'Oy'* Cartesian coordinates as follows

$$\begin{cases} r_{x_1} = r_{x_0} - p_1 \cos\theta_1 \\ r_{y_1} = r_{y_0} + p_1 \sin\theta_1 \end{cases} \tag{45}$$

Then, by a simple coordinate system transformation, one can transform the $(r_{x_1}, r_{y_1})$ into the absolute coordinate system *xOy* by the following

$$\begin{bmatrix} r_{x'_1} \\ r_{y'_1} \end{bmatrix} = \begin{bmatrix} r_{x_1} & r_{y_1} \\ r_{y_1} & -r_{x_1} \end{bmatrix} \begin{bmatrix} \cos\alpha \\ \sin\alpha \end{bmatrix}. \tag{46}$$

Consequently, one can get the new position of $\mathbf{r}_1$ with respect to the *xOy* coordinate system which takes the base station center $B_1$ as the original point. Since the base station position information in 5G systems is a-priori, one can easily compute the exact position of $\mathbf{r}_1$ as well. By updating $\mathbf{r}_1$ as the new initial point and repeating the aforementioned localization process, one can then get the new position sequence $r_2, r_3, \cdots, r_n$, which tracks the target timely.

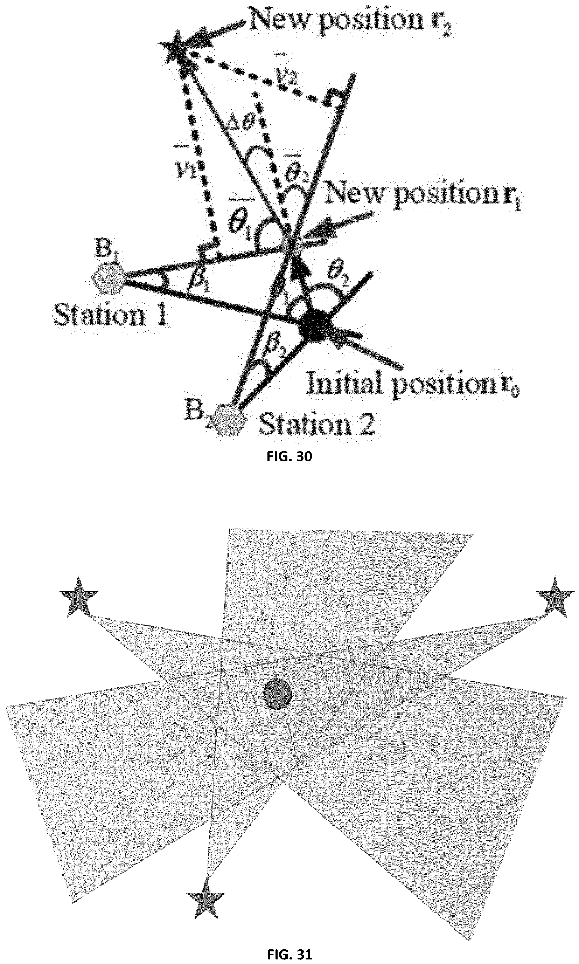

FIG. 30 shows three consecutive adjacent points $r_0, r_1$ and $r_2$ along the moving trace. $r_0$ is the initial point and $\Delta\theta$ is the turning angle. From equations (39) and (40), one can obtain the estimation of $\theta_1$, $\theta_2$ and the true speed $v$. Thus, one can have $|r_0 r_1| = vt$ and the distance between the target and BS

$$|B_1 r_1| = \sqrt{|B_1 r_0|^2 + |r_0 r_1|^2 - 2|B_1 r_0||r_0 r_1|\overline{\cos\theta_1}}. \qquad (47)$$

Once the new $|B_1 r_1|$ is obtained, one can update the ACFS distribution to estimate a new pair of $(\overline{v_1}, \overline{v_2})$. By using triangular relationships, one can also easily get $\beta_1$, $\beta_2$ and

$$\begin{cases} \overline{\theta_1} + \Delta\theta = \theta_1 + \beta_1 \\ \overline{\theta_2} = \theta_2 - \beta_2 \end{cases} \Rightarrow \overline{\theta_1} + \overline{\theta_2} + \Delta\theta \text{ is obtained.} \qquad (48)$$

Then, one can update equations (39) and (40). Finally, one may use $(\overline{v_1}, \overline{v_2})$ and $\overline{\theta_1} + \overline{\theta_2} + \Delta\theta$ to estimate $\overline{\theta_1}$ and $\overline{\theta_2}$ respectively. In one embodiment, assuming that $r_0$ is the initial location and target moves from $r_0$ to $r_1$, the main steps of a massive MIMO algorithm according to a disclosed method can be summarized as the following steps.

Step 1: get the initial location information about the base station $B_1$, $B_2$ and $r_0$; and compute the initial distance $L_1 = |B_1 r_0|$, $L_2 = |B_2 r_0|$.

Step 2: compute $\theta_1 + \theta_2$ according to the location information of $B_1$, $B_2$, $r_0$. In the initial iteration, a third base station is needed to determine whether $\theta_1 + \theta_2$ is an acute angle or obtuse angle because they own same after "sin(·)". However, after the initial iteration, the third BS is not needed any more, because the previous $\theta_1 + \theta_2$ can be an auxiliary constraint to help to determine whether $\theta_1 + \theta_2$ is an acute angle or obtuse angle.

Step 3: based on the received signal, compute the ACFS to get the peak width $d_1$ and $d_2$.

Step 4: when a target moves from $r_0$ to $r_1$, use the following equations (A1) and (A2) to compute $\theta_1$, $\theta_2$ and absolute moving speed $v$.

$$v = \frac{v_1}{\sin\theta_1} = \frac{v_2}{\sin\theta_2}. \qquad (A1)$$

$$180° - \alpha = \theta_1 + \theta_2. \qquad (A2)$$

Step 5: compute the moving distance $p = v \cdot T_{win}$. $T_{win}$ is a pre-set parameter.

Step 6: once $p$, $\theta_1$ and $\theta_2$ are obtained, the location of $r_0$ can be estimated by

$$\begin{cases} r_{x_1} = r_{x_0} - p_1\cos\theta_1 \\ r_{y_1} = r_{y_0} + p_1\sin\theta_1 \end{cases}. \qquad (A3)$$

Step 7: transform the $(r_{x_1}, r_{y_1})$ into the absolute coordinate system $xOy$ by the following (A4) to get the location of $r_1$.

$$\begin{bmatrix} r_{x_1'} \\ r_{y_1'} \end{bmatrix} = \begin{bmatrix} r_{x_1} & r_{y_1} \\ r_{y_1} & -r_{x_1} \end{bmatrix} \begin{bmatrix} \cos\alpha \\ \sin\alpha \end{bmatrix}. \qquad (A4)$$

Step 8: go back to Step 1 and Step 2 and update $L_1 = |B_1 r_0|$, $L_2 = |B_2 r_0|$ and $\theta_1 + \theta_2$, and continue to track the target.

Simulations are conducted to verify the speed and location estimation performance of the disclosed method by using future 5G communication system. The carrier frequency is set as $f_c$ = 28GHz. The range between the base station and receiver is assumed to within 200m. The inner element distance is set as $\lambda_0$, wavelength of the signal.

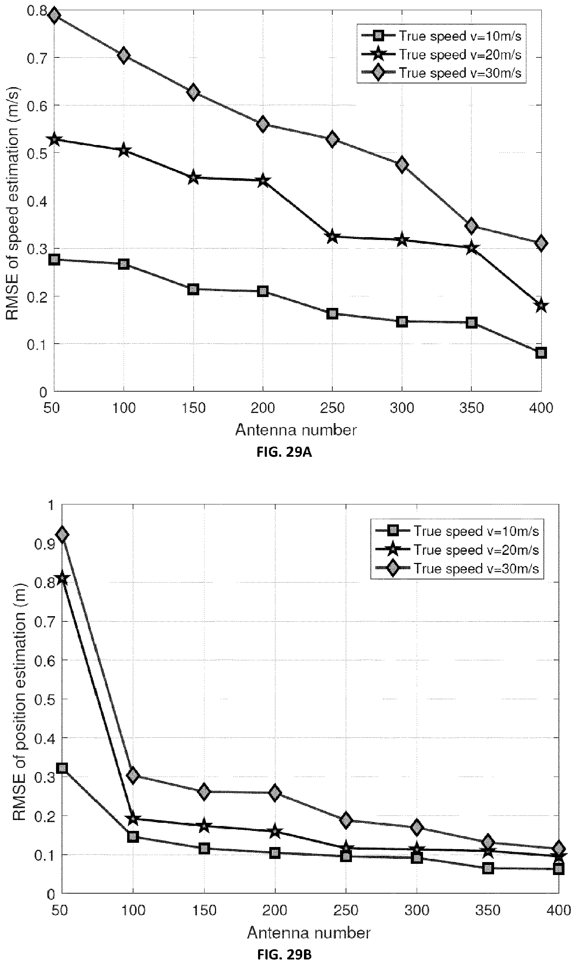

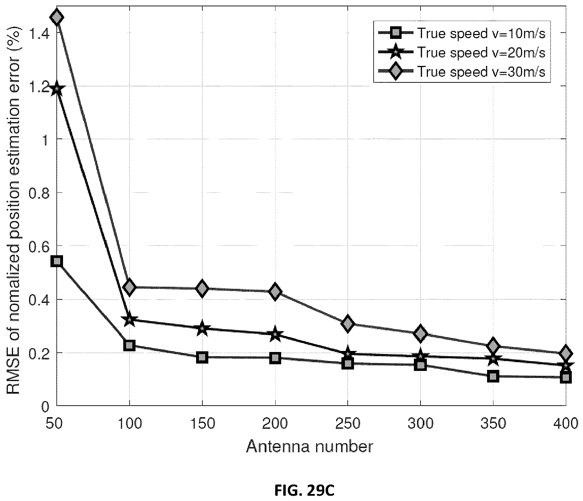

To explore how antenna number impacts the speed and position estimation performance, one can conduct extensive Monte Carlo simulations with SNR fixed as 10dB to get the Root Mean Square Error (RMSE) of corresponding estimation results as shown in FIGs. 29A-29C. Both speed and position estimation accuracy are improved with the increment of antenna number. Specifically, when antenna number is less than 100, it may not work well when the moving velocity is too fast (e.g., $v$ =30m/s in FIGs. 29A-29C). However, the disclosed system can localize the target within 0.3m accuracy for different speed scenarios when the antenna number is no less than 100. However, when $M$ is greater than a specific threshold, i.e., $M$ = 200, this approximation does not improve obviously anymore. Note that when antenna number $M$ approaches to 400, the position estimation error can be as low as 8cm, which indicates centimeter accuracy in another aspect.

The disclosed method works especially well when antenna number is greater than 50. Intuitively, one cannot harvest enough signal components to observe the so called TRFS phenomenon. In mathematical terminology, approximation in equation (27) does not hold when $M$ is too small. The disclosed outdoor tracking is a new candidate for outdoor target localization when the 5G based massive MIMO deployment is applicable and the when GPS satellite signal is blocked by buildings in the urban area. The disclosed system can guide pedestrians to their destination with a centimeter accuracy in a typical metropolitan area where skyscrapers stand along the street densely which will block the GPS from functioning reliably. The disclosed system is also capable of estimating the walking speed of user, which can be used for health monitoring. The gathered walking information of pedestrians in a specific area can also be used to perform geographical based behavior and activity analysis.

In one embodiment, some lower-layer details (e.g. a mode, an operation, a transmission, a measurement, a capability, an information, a feedback) of a method/system/device may enable a higher layer application (e.g. software) to perform wireless sensing. WSE means an enabling of wireless sensing. Suppose an application (e.g. a software application, a mobile app, an embedded software, a firmware, etc.) wants to perform wireless sensing based on some wireless data (e.g. WSE measurement) obtained at some lower layers. In the present teaching, WSE refers to anything (e.g. a mode, a capability, a wireless transmission, a measurement, an operation, an information, a feedback) in the Physical (PHY) layer, or Median-Access (MAC) layer, or other layers below the application layer, of any Type 1 heterogeneous wireless device (transmitter or TX) or any Type 2 heterogeneous wireless device (receiver or Rx) or another device (e.g. a local server, a cloud server) that enable the applications to perform wireless sensing.

WSE mode is a mode that enables wireless sensing. It may be a standard-compliant mode in PHY layer or MAC layer or other layer below the application layer. In the WSE mode, standard-compliant devices (e.g. the Type 1 device(s), and/or the Type 2 device(s)) may perform a WSE operation, request/set up WSE transmissions, make WSE measurements (e.g. compute CSI) based on the WSE transmission, make the WSE measurement, information, and feedback available to the application for the sake of wireless sensing. WSE capability is a capability of a device that enables wireless sensing. The Type 1 device and/or Type 2 device and/or another device may have WSE capability because they are capable of performing WSE operations, request/set up/perform/handle WSE transmission, make WSE measurements, compute/generate/handle WSE information, etc. A device compliant to a standard with a WSE mode will automatically be WSE capable. WSE transmission is a (wireless) transmission that enables wireless sensing. The wireless signal (e.g. probe signal, beacon signal, null data packet, etc.) sent from the Type 1 device to the Type 2 device is a WSE transmission that allows the Type 2 device to make WSE measurements (e.g. CSI), especially in a WSE mode. WSE measurement is a measurement that enables wireless sensing. For example a Type 2 device may receive the wireless signal from the Type 1 device and compute WSE measurements (e.g. CSI) based on the received wireless signal. WSE operation is an operation that enables wireless sensing. The operation may comprise request/set up WSE transmissions, make WSE measurements (e.g. compute CSI) based on the WSE transmission, make the WSE measurement, information, and feedback available to the application for the sake of wireless sensing. WSE information is information related to the WSE measurements. WSE feedback is a feedback related to the WSE measurements, the WSE transmission, the WSE operations.

In one embodiment, the application to perform wireless sensing may use an interface above MAC layer to request, control (e.g. set up, set alert) and obtain WSE transmission and WSE measurements in the MAC and/or PHY layers. The request and the control may cause the Type 1 device and/or the Type 2 device (both devices with WSE capability) to operate in a standard-compliant WSE mode. The Type 1 device and the Type 2 device may exchange (e.g. handshake) WSE capabilities. In the standard-compliant WSE mode, the Type 1 device transmits a wireless signal to the Type 2 device, wherein the wireless signal is a WSE transmission. The wireless signal may include an indication (e.g. a bit or a flag) that it is a WSE transmission from which any compatible Type 2 device can make WSE measurements. The WSE transmission by the Type 1 device may be requested by the Type 2 device or another device. The WSE transmission may also be unsolicited (i.e. transmitted by the Type 1 device without any request from the Type 2 device or another device). The WSE transmission may be partly requested and/or partly unsolicited. WSE measurements, WSE information and/or WSE feedback may be exchanged by the Type 2 device with another device (e.g. the Type 1 device, a local server, a cloud server), the application and/or another application. The exchange may be secured. The WSE measurement may be in a secure format. A transmission of the WSE measurement may be secured.

In one embodiment, considering 5G networks, a receiver measures the received signal strength (RSS) and beam direction information (a.k.a., Angle of Arrival, AoA) from multiple 5G base stations. Denote the RSS measurements as $R = \{r_1, r_2, \cdots, r_N\}$ and the AoA as $\Theta = \{\theta_1, \theta_2, \cdots, \theta_N\}$, where $r_i$ and $\theta_i$ are the RSS and AoA of the ith hearable base station $b_i$ and N is the total number of nearby base stations. The locations of the N base stations are known as $X = \{x_1, x_2, \cdots, x_N\}$. The positioning task is to obtain the location estimate of the receiver, denoted as x. FIG. 31 illustrates a convex hull formed by AoA measurements.

The AoA measurements can determine a feasible zone of x as convex hull. Considering an error term of the AoA estimate, arising from both the imperfect beam patterns and the measurement noises, the feasible direction to a base station $b_i$ becomes $[\theta_i - \Delta\theta, \theta_i + \Delta\theta]$, forming a cone area for candidate locations of x. Such zones of multiple base stations, assuming the AoA estimates are accurate, will intersect a convex hull, denoted as ConvexHull($\hat{\Theta}$), in which the true location of the receiver falls. Here $\hat{\Theta} = \{\Theta + \Delta\theta, \Theta - \Delta\theta\}$ denotes the directional beams given the AoA measurements $\Theta$, and $\Delta\theta$ is the known beam width of the base stations plus a minor error term to account for measurement noises.

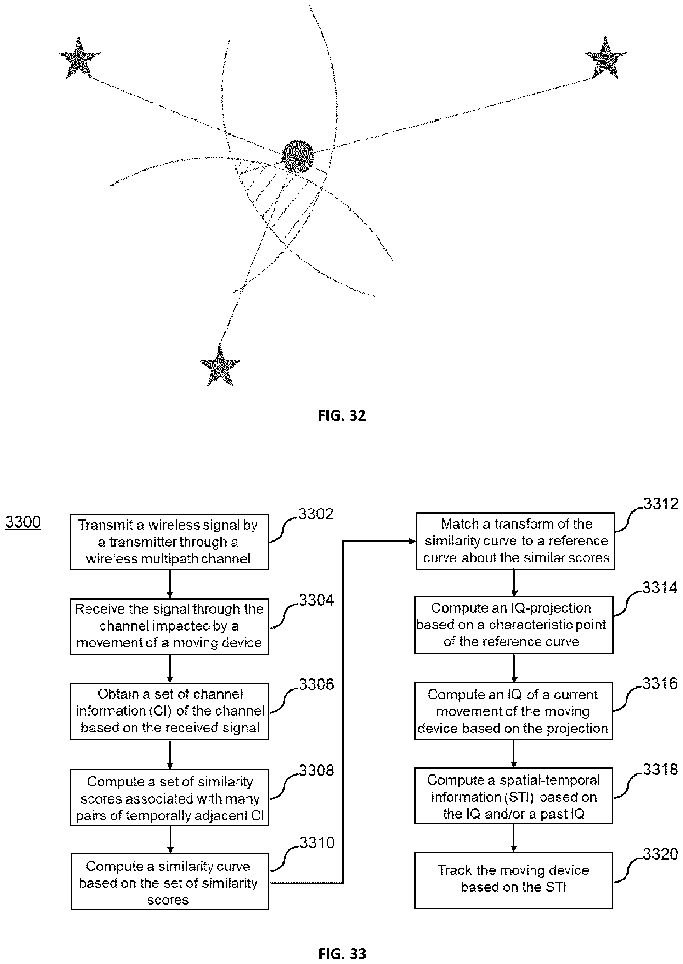

The RSS measurements R could be converted into distance estimates via certain signal propagation models. Thanks to the high carrier frequency of 5G networks, typically at millimeter wave frequency band, the signals mainly undergo Line-Of-Sight propagation with few multipath effects, underpinning high-precision distance estimation from RSS. And like AoA, such distance constrains, if sufficiently accurate, will intersect an area as the feasible zone for x. FIG. 32 illustrates a method of target positioning by RSS-based ranging. While the location of the receiver can be obtained by either AoA or RSS measurements separately, the accuracy may be limited due to potential measurement errors. Therefore, an algorithm is disclosed to jointly leverage the AoA and RSS information for precise positioning of the receiver.

The distance estimation based on RSS will be derived from the log-normal shadowing path loss model with reasonable accuracy in 5G networks:

$$r_i = PL(d_0) + 10\eta \log_{10} \frac{||x - x_i||}{d_0} + n,$$

where $PL(d_0)$ is a known reference power value in dBm at a reference distance $d_0$ from the base station, $\eta$ is the path loss exponent, and n denotes a noise term as a zero-mean Gaussian distributed random variable with standard deviation $\sigma$, i.e.,

$$n \sim \mathcal{N}(0, \sigma^2).$$

The task is formulated as an optimization problem. Given observations R, $\Theta$, X, the location with maximum likelihood is found by

$$x^* = \underset{x}{\arg\max} \, P(x|R, \Theta)$$

$$s.\, t.\, x \text{ in ConvexHull}(\hat{\Theta}).$$

Assuming the RSS and AoA measurements from different base stations are independent from each other, P(x|R,$\Theta$) can be expressed as

$$P(x|R, \Theta) = \prod_{i=1}^{N} P(x|r_i, \theta_i)$$

$$= \frac{1}{(2\pi\sigma^2)^{N/2}} \prod_{i=1}^{N} \exp\left(-\frac{1}{2\sigma^2}\left(PL(d_0) + 10\eta \log_{10}\frac{||x - x_i||}{d_0} + G_i(\theta_i) - r_i\right)^2\right).$$

Here $G_i(\theta_i)$ indicates the beamforming gain under the specific beam pattern associated with $\theta_i$ of base station $b_i$. The above problem can be solved by many solvers, such as Expectation-Maximization (EM) algorithms, Alternating Direction Method of Multipliers, Genetic Algorithms, or Simulated Annealing algorithms. The probability $P(x|r_i, \theta_i)$ is expressed with one theoretical path loss model. Other models could also be used here, especially accounting the channel propagation properties of 5G networks. The path loss model can also be derived from experimental data measurements rather than a theoretical one. Using a different model does not change the above problem formulation and so the solver.

The disclosed algorithm is not only limited to AoA and RSS but is extensible to account for other dimensions of channel parameters such as time of flight (ToF), provided they are available. In one embodiment, the disclosed method includes: first, the target/object/device listens to wireless signals from N base stations (BSs) (N>=3); second, a computing unit on the target (or some server) calculates feasible region based on the wireless signals features from the N base stations (based on e.g., AoA, ToF, RSS, etc.); third, the computing unit calculates the target position by solving an optimization problem, wherein the objective function is the expected likelihood probability, such that the position is inside the feasible region. The optimization problem is solved by utilizing RSS and AoA, which is novel, e.g. considering the gain as a function of direction $G\_i(\theta\_i)$.

FIG. 33 illustrates a flow chart of an exemplary method 3300 for wireless object tracking, according to some embodiments of the present teaching. At operation 3302, a wireless signal is transmitted by a transmitter through a wireless multipath channel. At operation 3304, the wireless signal is received through the wireless multipath channel by a receiver, where the wireless multipath channel is impacted by a moving device, which is one of the transmitter and the receiver. At operation 3306, a set of channel information (CI) of the channel is obtained based on the received wireless signal. At operation 3308, a set of similarity scores associated with many pairs of temporally adjacent CI is computed. At operation 3310, a similarity curve is computed based on the set of similarity scores.

At operation 3312, a transform of the similarity curve is matched to a reference curve about the similar scores. At operation 3314, an IQ-projection is computed based on a characteristic point of the reference curve matched to the similarity curve. At operation 3316, an IQ of a current movement of the moving device is computed based on the IQ-projection. At operation 3318, a spatial-temporal information (STI) of the moving device is computed based on the IQ and/or a past IQ. At operation 3320, the moving device is tracked based on the STI. According to various embodiments, the order of the operations in FIG. 33 may be changed.

The following numbered clauses provide additional implementation examples.

Clause 1: A tracking system, comprising: a transmitter configured for transmitting a first wireless signal through a wireless multipath channel; a receiver configured for receiving a second wireless signal through the wireless multipath channel between the transmitter and the receiver, wherein: one of the transmitter and the receiver is a located device at a known location, the other of the transmitter and the receiver is a moving device, the second wireless signal differs from the first wireless signal due to the wireless multipath channel which is impacted by a movement of the moving device, at least one of the transmitter and the receiver comprises a number of antennas, the number is larger than a threshold; and a processor configured for: obtaining a plurality of time series of channel information (CI) of the wireless multipath channel based on the second wireless signal, computing a spatial-temporal information (STI) of the moving device based on at least one of: the plurality of time series of CI (TSCI) and a past STI, and tracking the moving device based on the STI.

Clause 2: The tracking system of clause 1, wherein: the threshold is one of: 10, 16, 20; each of the plurality of TSCI is associated with a pair of a transmit antenna on the transmitter and a receive antenna on the receiver; and the located device is stationary at a fixed location or moving at locations known to the processor.

Clause 3: The tracking system of clause 1, wherein the STI comprises at least one of: a location, coordinate, horizontal location, map coordinate, vertical location, height, map location, locationing, relative location, navigation, guidance, a direction, angle, bearing, base distance, distance, directional distance, radial distance, range, length, area, region, volume, capacity, displacement, movement, speed, velocity, acceleration, rotational speed, angular speed, rotational acceleration, angular acceleration, motion cycle, motion appearance, motion re-appearance, gait cycle, presence, period, frequency, count, quantity, rhythm, breathing rate, heart rate, motion type, motion classification, motion characteristics, motion signature, motion state, fall, danger, intrusion, gesture, expression, target motion, body expression, activity, motion sequence, event, timed event, sudden motion, transient motion, impulsive motion, periodic motion, period of the periodic motion, frequency of the periodic motion, occurrence rate, occurrence timing, a timing, timestamp, starting time, ending time, time period, time window, sliding time window, time trend, daily trend, history, frequency trend, spatial-temporal trend, change, spatial-temporal change, and another analytics.

Clause 4: The tracking system of clause 1, wherein: the moving device is moving with an object; the processor is further configured for tracking an object based on tracking the moving device; and tracking at least one of the object and the moving device comprises at least one of: determining a map location, tracking the location, tracking another analytics, navigating, guiding movement along a trajectory, guiding the movement to avoid an obstacle, tracking motion, tracking behavior, identifying behavior, detecting presence, detecting motion, recognizing motion, detecting vital sign, detecting periodic motion, detecting breathing, detecting heartbeat, monitoring breathing, monitoring heartbeat, detecting event, detecting fall-down, detecting intrusion, counting, recognizing gesture, recognizing activity, recognizing state, detecting deviation of regular activity, detecting anomaly, monitoring timing, monitoring trends, generating a presentation of at least one of: the IQ, the STI, the map location, the location, the analytics, the navigation, the trajectory, the guidance, the obstacle, the movement, the behavior, the identification, the presence, the motion, the vital sign, the periodic motion, the breathing, the heartbeat, the event, the fall-down, the intrusion, the counting, the gesture, the activity, the state, the deviation, the anomaly, the timing, the trends, generating a presentation of the location, displaying graphically on a user device, and generating another presentation.

Clause 5: The tracking system of clause 1, wherein the processor is further configured for: computing an intermediate quantity (IQ) of a current movement of the moving device based on the plurality of TSCI; and computing the STI based on at least one of: the IQ and a past IQ, wherein the IQ comprises at least one of: a time stamp, starting time, ending time, time code, timing, time period, time duration, frequency, period, cycle, rhythm, pace, count, indicator, occurrence, state, set, a location, distance, displacement, direction, speed, velocity, acceleration, angular distance, angular speed, angular acceleration, change of location, change of direction, change of speed, change of acceleration, proximity, presence, absence, appearance, disappearance, location, statistics, motion statistics, breathing statistics, distance statistics, speed statistics, acceleration statistics, metric, $1\_k$ distance metric, $1\_0$ distance metric, $1\_1$ distance metric, absolute distance metric, $1\_2$ distance metric, Euclidean distance metric, $1\_infinity$ distance metric, path, volume, mass, surface area, shape, posture, energy, trend, time sequence, label, tag, class, category, time profile, time quantity, frequency quantity, transient quantity, incremental quantity, instantaneous quantity, averaged quantity, locally averaged quantity, filtered quantity, quantity change, repeating quantity, an event, recognized event, recognized motion sequence, gesture, hand gesture, finger gesture, wrist gesture, elbow gesture, arm gesture, shoulder gesture, head gesture, facial gesture, neck gesture, waist gesture, leg gesture, foot gesture, a maximum, minimum, constrained maximum, constrained minimum, local maximum, local minimum, first local maximum, first local minimum, k-th local maximum, k-th local minimum, average, weighted average, percentile, mean, median, mode, trimmed mean, conditional mean, conditional statistics, ordered statistics, variance, skewness, kurtosis, moment, high order moment, cumulant, correlation, covariance, co-skewness, co-kurtosis, first order statistics, second order statistics, third order statistics, high order statistics, robust quantity, argument associated with another quantity, a feature of CI, complex component of CI, magnitude of the complex component, phase of the complex component, function of the complex component of the CI, polynomial of the magnitude of the complex component, square of the magnitude of the complex component, time series of the feature of CI, autocorrelation function of the feature of CI, and a function of another quantity.

Clause 6: The tracking system of clause 5, wherein the processor is further configured for: obtaining a supplementary quantity (SQ), wherein the SQ comprises at least one of: an additional IQ of the current movement of the moving device computed based on an additional plurality of TSCI extracted from an additional wireless signal transmitted between an additional transmitter and an additional receiver, an additional quantity (AQ) of the current movement of the moving device needed for STI computation but not in IQ, wherein the AQ comprises at least one of: a direction, distance, speed, acceleration, map, movement constraint, boundary, a sensor quantity from at least one of: a sensor, inertia sensor, accelerometer, gyroscope, magnetometer, GPS device, infrared sensor, radar, proximity sensor, ambient light sensor, microphone, camera, touchscreen sensor, fingerprint sensor, pedometer, barcode sensor, QR code sensor, barometer, heart-rate sensor, thermometer, humidity sensor, and Geiger counter, a past quantity associated with at least one of: the STI, the IQ, the SQ, the additional IQ, the AQ, and the sensor quantity, and an initial quantity associated with at least one of: the STI, the IQ, the SQ, the additional IQ, the AQ, and the sensor quantity; and computing the STI based on both the IQ and the SQ.

Clause 7: The tracking system of clause 5, wherein the processor is further configured for: computing an IQ-projection based on the plurality of TSCI; and computing the IQ base on the IQ-projection, wherein the IQ-projection is a projection of the IQ onto at least one of: an along-beam direction as a baseline, a cross-beam direction orthogonal to the baseline, a direction relative to the baseline, a direction of at least one antenna of the located device, a direction of at least one antenna of the moving device, a direction of the number of antennas, wherein the baseline is determined based on the at least one antenna of the moving device and the at least one antenna of the located device and is time-varying as the moving device moves, wherein the baseline comprises at least one of: a line between a region associated with the at least one antenna of the moving device and a region associated with the at least one antenna of the located device, the line being at least one of: a curve, a path, a transmission path, a piecewise straight and linear line, a locally straight line, a locally linear line, a straight line, a surface connecting a region associated with the at least one antenna of the moving device and a region associated with the at least one antenna of the located device, the surface being at least one of: a nonlinear surface, a manifold, a linear surface, a locally linear surface, a hyperplane, a 1-dimensional plane, a 2-dimensional plane, and a higher-dimensional plane, a line connecting an antenna of the moving device and an antenna of the located device, a line connecting an antenna of the moving device and a characteristic location of the at least one antenna of the located device, a line connecting an antenna of the moving device and a center of the at least one antenna of the located device, a line connecting a characteristic location of the at least one antenna of the moving device and an antenna of the located device, a line connecting a characteristic location of the at least one antenna of the moving device and a characteristic location of the at least one antenna of the located device, a line connecting a characteristic location of the at least one antenna of the moving device and a center of the at least one antenna of the located device, a line connecting a center of the at least one antenna of the moving device and an antenna of the located device, a line connecting a center of the at least one antenna of the moving device and a characteristic location of the at least one antenna of the located device, and a line connecting a center of the at least one antenna of the moving device and a center of the at least one antenna of the located device.

Clause 8: The tracking system of clause 5, wherein the processor is further configured for: computing a set of similarity scores associated with many pairs of temporally adjacent CI of the plurality of TSCI, each pair comprising two temporally adjacent CI of one of the plurality of TSCI; and computing the IQ based on the similarity scores, wherein each of the similarity scores comprises at least one of: a time reversal resonating strength (TRRS), a correlation, a cross-correlation, an auto-correlation, an auto-correlation function (ACF), an auto-correlation focusing strength (ACFS), a covariance, a cross-covariance, an auto-covariance, an inner product of two vectors, a distance score, norm, metric, statistical characteristics, a discrimination score, a metric, a neural network output, a deep learning network output, and another measure of similarity, wherein computing the set of similarity scores comprises at least one of: a similarity computation, machine learning, training, discrimination, weighted averaging, preprocessing, denoising, signal conditioning, filtering, time correction, timing compensation, phase offset compensation, transformation, component-wise operation, feature extraction, finite state machine, and another operation.

Clause 9: The tracking system of clause 8, wherein the processor is further configured for: computing a similarity curve based on the set of similarity scores; and computing the IQ based on at least one of: the similarity curve and the set of similarity scores.

Clause 10: The tracking system of clause 9, wherein the processor is further configured for: computing an IQ-projection based on at least one of: the similarity curve and the set of similarity scores, wherein the IQ-projection is a projection of the IQ onto a direction; and computing the IQ based on the IQ-projection.

Clause 11: The tracking system of clause 10, wherein: computing the IQ-projection comprises matching a transform of the similarity curve to a reference curve associated with the set of similar scores, the transform is associated with at least one of: a scalar function, a 1-dimensional function, a vector function, a multi-variate function, a linear function, a nonlinear function, a magnitude, a phase, a polynomial, a function of the magnitude, a function of the phase, an exponential function, a logarithmic function, a trigonometric function, an inverse function, an absolute value, a thresholding function, a step function, an indicator function, and another transform, and the reference curve comprises at least one of: a trigonometric function, a tangent function, a sine function, a cosine function, an inverse function, an inverse trigonomic function, an arc-tangent function, an arc-sine function, an arc-cosine function, a hyperbolic tangent function, a sinc function, a logarithmic function, an exponential function, a parabolic function, an elliptic function, a hyperbola function, a polynomial, a Taylor series, an asympototic series, another series, a special function, Bessel function, sigmoid function, logistic function, generalized logistic function, error function, complementary error function, Gauss error function, Gundermannian function, smoothstep function, Gaussian function, generalized Gaussian function, hyperbolic function, hypergeometric function, Blasius function, de Brujin function, Buchstab function, Dawson function, Hankel function, Heine function, zeta function, Kelvin function, Riccati-Bessel function, Riemann zeta-function, Struve function, Wangerein function, Weber function, Weierstrass function, Whittaker function, a first function of a second function, and another function.

Clause 12: The tracking system of clause 11, wherein the processor is further configured for: determining that the transform of the similarity curve is matched to a characteristic point of the reference curve, wherein the characteristic point comprises at least one of: a local maximum, local minimum, local extremum, local extremum with positive argument, first local extremum with positive argument, $n$^th local extremum with positive argument, local extremum with negative argument, first local extremum with negative argument, $n$^th local extremum with negative argument, constrained maximum, constrained minimum, constrained extremum, maximum slope, minimum slope, local maximum slope, local minimum slope, local maximum slope with positive argument, local minimum slope with positive argument, constrained maximum slope, constrained minimum slope, maximum higher order derivative, minimum higher order derivative, constrained higher order derivative, zero-crossing, a distance between two zero-crossing, zero crossing with positive argument, $n$^th zero crossing with positive argument, zero crossing with negative argument, $n$^th zero crossing with negative argument, constrained zero-crossing, zero-crossing of slope, zero-crossing of higher order derivative, and another characteristics, wherein the IQ-projection is computed based on at least one of: the characteristic point of the reference curve, a property of the characteristic point of the reference curve, wherein the property comprises at least one of: a time, a time offset, a timing, a time duration, and a time range, an argument of the reference curve to achieve the characteristic point of the reference curve, a time offset associated with the characteristic point of the reference curve, a length of a line between a region with the at least one antenna of the located device and a region with the at least one antenna of the moving device, a length of a line on a surface connecting a region associated with the located device and a region associated with the moving device, a distance between the located device and the moving device, a distance between the located device and the moving device along the baseline, a length of a segment of the baseline, an angle between the baseline and the number of antennas, an angle between the baseline and a direction of the at least one antenna of the moving device, an angle between the baseline and a direction of the at least one antenna of the located device, an angle between a direction of the at least one antenna of the moving device and a direction of the at least one antenna of the located device, an angle between a direction of the current movement of the moving device and the baseline, an angle between a direction of the current movement of the moving device and a direction of the at least one antenna of the moving device, an angle between a direction of the current movement of the moving device and a direction of the at least one antenna of the located device, an effective aperture of the number of antennas, a scaling factor based on the angle between the baseline and the number of antennas, a scaling factor comprising a trigonometric function of the angle between the baseline and the number of antennas, a scaling factor comprising a cosine of the angle between the baseline and the number of antennas, the effective aperture of the number of antennas scaled by a scaling factor, the distance between the located device and the moving device scaled by a scaling factor, and a carrier frequency of the second wireless signal.

Clause 13: The tracking system of clause 12, wherein: the IQ-projection is computed based on a time quantity associated with the similarity curve, the transform of the similarity curve, the reference curve, and the characteristic point of the reference curve; and the time quantity comprises at least one of: a timing, a time stamp, a current time, a previous time, a sliding time, a time associated with the matching of the characteristic point of the reference curve to the transform of the similarity curve, a time associated with a matching of another characteristic point of the reference curve to the transform of the similarity curve, a time associated with a matching of at least one of: the characteristic point, the another characteristic point, and yet another characteristic point of the reference curve to a transform of a past similarity curve, a time associated with at least one of: a condition, a matching, a state, a status, a situation, and an event, a timing associated with one of: a first condition, a first matching, a first state, a first status, a first situation, and a first event, and one of: a second condition, a second matching, a second state, a second status, a second situation, and a second event, a time offset, a time difference, a time duration, a time period, an incremental time, and a timed quantity.

Clause 14: The tracking system of clause 5, wherein the processor is further configured for: computing a speed based on at least one of: a distance included in the IQ, a derivative of the distance, the distance divided by a duration taken to traverse the distance, the distance divided by an incremental time, and a fraction of an incremental distance associated with the distance over an associated increment time.

Clause 15: The tracking system of clause 14, wherein the processor is further configured for: computing an acceleration based on at least one of: the distance, a derivative of the derivative of the distance, a second derivative of the distance, a derivative of the speed, the speed divided by the duration taken to traverse the distance, a change in speed divided by a duration taken to achieve the speed change, a change in speed divided by a duration associated with the speed change, a change in speed in a time interval divided by a duration of the time interval, a fraction of: (a) a difference between the speed and a previous speed, over (b) the duration taken to traverse the distance, a fraction of: (c) a difference between the speed and a previous speed, over (d) a duration associated with the speed and the previous speed, and a fraction of: (e) a difference between the speed and a previous speed, over (f) a difference between a time associated with the speed and another time associated with the previous speed.

Clause 16: The tracking system of clause 14, wherein the processor is further configured for: computing a distance-projection which is a projection of the distance onto a direction; computing the distance based on the distance-projection; computing a speed-projection which is a projection of the speed onto the direction; computing the speed based on the speed-projection, wherein the speed-projection is computed based on at least one of: the distance-projection, a derivative of the distance-projection, a projection of a derivative of the distance onto the direction, and a projection of the speed onto the direction.

Clause 17: The tracking system of clause 7, wherein the processor is further configured for: resolving an ambiguity of at least one of: the IQ, the IQ-projection, and the STI, of the moving device based on at least one of: an additional plurality of TSCI extracted from an additional wireless signal transmitted between the transmitter and an additional receiver, an additional plurality of TSCI extracted from an additional wireless signal transmitted between an additional transmitter and the receiver, an additional plurality of TSCI extracted from an additional wireless signal transmitted from an additional located device to the moving device, an additional plurality of TSCI extracted from an additional wireless signal transmitted from the moving device to an additional located device, a plurality of sets of TSCI, wherein each set of TSCI is extracted from a respective wireless signal transmitted between the transmitter and a respective one of a plurality of heterogeneous receivers, a plurality of sets of TSCI, wherein each set of TSCI is extracted from a respective wireless signal transmitted from a respective one of a plurality of heterogeneous transmitters to the receiver, a plurality of sets of TSCI, wherein each set of TSCI is extracted from a respective wireless signal transmitted between a respective one of a plurality of located devices and the moving device, the IQ, the IQ-projection, the STI, another IQ computed based on at least one of the above TSCI, another IQ-projection based on at least one of the above TSCI, another STI based on at least one of the above TSCI, and at least one supplementary quantity (SQ).

Clause 18: The tracking system of clause 1, wherein the processor is further configured for, for each of the plurality of TSCI: determining a reference CI of the respective TSCI associated with a time t associated with a current movement of the moving device; computing a series of similarity scores associated with the time t, each similarity score based on the reference CI, and one of a series of temporally adjacent CI of the respective TSCI within a time window around the time t; and determining a respective similarity curve associated with the series of similarity scores, wherein the STI of the moving device is computed based on at least one of: a first combined characteristic point of a combined curve obtained by combining a large number of similarity curves, each similarity curve associated with a respective TSCI and its respective series of similarity scores, a second combined characteristic point obtained by combining a large number of characteristic points each of a respective similarity curve associated with a respective TSCI and its respective series of similarity scores, wherein at least one of a characteristic point and the combined characteristic point comprises at least one of: a local maximum, local minimum, local extremum, local extremum with positive argument, first local extremum with positive argument, n^th local extremum with positive argument, local extremum with negative argument, first local extremum with negative argument, n^th local extremum with negative argument, constrained maximum, constrained minimum, constrained extremum, slope, derivative, higher order derivative, maximum slope, minimum slope, local maximum slope, local minimum slope, local maximum slope with positive argument, local minimum slope with positive argument, constrained maximum slope, constrained minimum slope, maximum higher order derivative, minimum higher order derivative, constrained higher order derivative, zero-crossing, a distance between two zero-crossing, zero crossing with positive argument, n^th zero crossing with positive argument, zero crossing with negative argument, n^th zero crossing with negative argument, constrained zero-crossing, zero-crossing of slope, zero-crossing of higher order derivative, and/or another characteristics; wherein the large number of similarity curves and/or the large number of characteristic points are combined based on: an average, weighted average, mean, median, mode, arithmetic mean, geometric mean, harmonic mean, weighted mean, trimmed mean, weighted median, percentile, maximal-ratio combining (MRC), principal component analysis (PCA), PCA with different kernels, independent component analysis (ICA), eigen-decomposition, Fisher linear discriminant, vector quantization, machine learning, supervised learning, unsupervised learning, clustering, self-organizing maps, auto-encoder, neural network, deep neural network, and another method.

Clause 19: The tracking system of clause 1, wherein the processor is further configured for: combining at least two of the plurality of TSCI to form a time series of compound CI, each compound CI comprising a CI from each of the at least two TSCI; determining a reference compound CI associated with a time t associated with a current movement of the moving device; computing a series of compound similarity scores associated with the time t, wherein each compound similarity score is computed based on the reference compound CI and one of a series of temporally adjacent compound CI within a time window around the time t; and determining a similarity curve associated with the series of compound similarity scores, wherein the STI of the moving device is computed based on a characteristic point of the similarity curve.

Clause 20: The tracking system of clause 1, wherein the processor is further configured for: computing at least one heterogeneous time series of compound CI (CCI), wherein: each heterogeneous time series of CCI (TSCCI) is associated with at least one respective TSCI, each CCI of the TSCCI comprises a weighted CI from each of the at least one respective TSCI, and the weighted CI is weighted by a respective weight associated with the respective TSCI; and for each heterogeneous TSCCI: determining a reference CCI of the TSCCI associated with a time t associated with a current movement of the moving device, computing a series of compound similarity scores associated with the time t, wherein each compound similarity score is computed based on the reference CCI and a temporally adjacent CCI of the TSCCI within a time window around the time t, and determining a similarity curve associated with the series of compound similarity scores, wherein the STI of the moving device is computed based on at least one of: a first combined characteristic point of a combined curve obtained by combining at least one similarity curve, each of which being associated with a respective TSCCI and its respective series of compound similarity scores, a second combined characteristic point obtained by combining at least one characteristic point, each of which being associated with a respective TSCCI and its respective series of compound similarity scores, wherein at least one of a characteristic point and the combined characteristic point comprises at least one of: a local maximum, local minimum, local extremum, local extremum with positive argument, first local extremum with positive argument, n^th local extremum with positive argument, local extremum with negative argument, first local extremum with negative argument, n^th local extremum with negative argument, constrained maximum, constrained minimum, constrained extremum, slope, derivative, higher order derivative, maximum slope, minimum slope, local maximum slope, local minimum slope, local maximum slope with positive argument, local minimum slope with positive argument, constrained maximum slope, constrained minimum slope, maximum higher order derivative, minimum higher order derivative, constrained higher order derivative, zero-crossing, a distance between two zero-crossing, zero crossing with positive argument, n^th zero crossing with positive argument, zero crossing with negative argument, n^th zero crossing with negative argument, constrained zero-crossing, zero-crossing of slope, zero-crossing of higher order derivative, and/or another characteristics, wherein the at least one similarity curve and/or the at least one characteristic point are combined based on: an average, weighted average, mean, median, mode, arithmetic mean, geometric mean, harmonic mean, weighted mean, trimmed mean, weighted median, percentile, maximal-ratio combining (MRC), principal component analysis (PCA), PCA with different kernels, independent component analysis (ICA), eigen-decomposition, Fisher linear discriminant, vector quantization, machine learning, supervised learning, unsupervised learning, clustering, self-organizing maps, auto-encoder, neural network, deep neural network, and/or another method.

Clause 21: The tracking system of clause 1, wherein the processor is further configured for: computing a similarity score associated with a current movement of the moving device based on the plurality of TSCI; and determining the moving device to be stationary and the current movement to be a null movement when the similarity score is greater than a threshold.

Clause 22: The tracking system of clause 1, wherein the processor is further configured for: preprocessing the plurality of TSCI, wherein the preprocessing comprises at least one of: doing nothing, de-noising, smoothing, conditioning, enhancement, restoration, feature extraction, weighted averaging, low-pass filtering, bandpass filtering, high-pass filtering, median filtering, ranked filtering, quartile filtering, percentile filtering, mode filtering, linear filtering, nonlinear filtering, finite impulse response (FIR) filtering, infinite impulse response (IIR) filtering, moving average (MA) filtering, auto-regressive (AR) filtering, auto-regressive moving average (ARMA) filtering, thresholding, soft thresholding, hard thresholding, soft clipping, local maximization, local minimization, optimization of a cost function, neural network, machine learning, supervised learning, unsupervised learning, semi-supervised learning, transform, Fourier transform, Laplace, Hadamard transform, transformation, decomposition, selective filtering, adaptive filtering, derivative, first order derivative, second order derivative, higher order derivative, integration, zero crossing, indicator function, absolute conversion, convolution, multiplication, division, another transform, another processing, another filter, a third function, and another preprocessing; and computing a similarity score based on the preprocessed plurality of TSCI, wherein the STI is computed based on at least one of: the similarity score and the preprocessed TSCI, wherein the similarity score comprises at least one of: a time reversal resonating strength (TRRS), a correlation, a cross-correlation, an auto-correlation, a covariance, a cross-covariance, an auto-covariance, an inner product of two vectors, a distance score, a discriminating score, a metric, a neural network output, a deep learning network output, and another score, wherein each CI of the wireless multipath channel comprises at least one of: a signal strength, signal amplitude, signal phase, an attenuation of the wireless signal through the wireless multipath channel, a received signal strength indicator (RSSI), a channel state information (CSI), channel impulse response (CIR), channel frequency response (CFR), an equalizer information, a time domain transfer function, a frequency domain transfer function, information associated with at least one of: a frequency band, a frequency signature, a frequency phase, a frequency amplitude, a frequency trend, a frequency characteristics, a frequency-like characteristics, an orthogonal decomposition characteristics, and a non-orthogonal decomposition characteristics, information associated with at least one of: a time period, a time signature, a time amplitude, a time phase, a time trend, and a time characteristics, information associated with at least one of: a time-frequency partition, a time-frequency signature, a time-frequency amplitude, a time-frequency phase, a time-frequency trend, and a time-frequency characteristics, information associated with a direction, an angle of arrival, an angle of a directional antenna, and a phase, and another CI.

Clause 23: The tracking system of clause 1, wherein the processor is further configured for: determining an initial location of the moving device at an initial time based on an additional wireless signal transmitted from an additional transmitter to an additional receiver through an additional wireless multipath channel at the initial time, wherein the additional receiver is close to the moving device and moves with the moving device; and computing a location of the moving device based on the initial location at the initial time prior to a current movement of the moving device, wherein the moving device is tracked based on the location of the moving device, wherein the initial location is determined to be a known location associated with the additional wireless signal based on at least one of: obtaining an identification of at least one of: the additional transmitter and the additional wireless signal received at the initial time, associating the identification with the known location based on a location database, and sending a query to a query device, wherein the query device contains a location database and an associated query service based on the location database.

Clause 24: The tracking system of clause 23, wherein: the additional transmitter is stationary and comprises a directional antenna such that the additional wireless signal is steered towards a target area; the additional receiver receives the additional wireless signal when the additional receiver is in the target area; and the known location is at least one of: an installation location of the additional transmitter, a location near the installation location, the target area associated with the additional wireless signal, a location near the target area, and a location that is near the target area and adaptively determined based on at least one of: a height of the moving device and a likelihood of the height.

Clause 25: The tracking system of clause 23, wherein: the additional transmitter is moving; a previous location of the additional transmitter is known; and the location database comprises an updated location of the additional transmitter.

Clause 26: A method for object tracking, comprising: obtaining a plurality of time series of channel information (CI) of a wireless multipath channel, using a processor, a memory communicatively coupled with the processor, and a set of instructions stored in the memory to be executed by the processor, wherein: the plurality of time series of CI (TSCI) are extracted from a wireless signal transmitted between a transmitter and a receiver through the wireless multipath channel, each of the plurality of TSCI is associated with a pair of a transmit antenna on the transmitter and a receive antenna on the receiver, one of the transmitter and the receiver is a located device at a known location, the other of the transmitter and the receiver is a moving device moving with an object, the wireless multipath channel is impacted by a movement of the object, at least one of the transmitter and the receiver comprises more than 16 antennas; computing a spatial-temporal information (STI) of the moving device based on at least one of: the plurality of TSCI and a past STI, and tracking the object based on the STI.

Clause 27: The method of clause 26, further comprising: causing the transmitter to switch wireless coupling from the receiver to an additional receiver; causing an additional wireless signal to be transmitted between the transmitter and the additional receiver through the wireless multipath channel that is impacted by a current movement of the object; extracting an additional plurality of TSCI of the wireless multipath channel from the additional wireless signal; computing an intermediate quantity (IQ) of the current movement of the moving device based on the additional plurality of TSCI; and computing the STI of the moving device based on the additional plurality of TSCI and the IQ.

Clause 28: The method of clause 26, further comprising: causing the receiver to switch wireless coupling from the transmitter to an additional transmitter; causing an additional wireless signal to be transmitted between the additional transmitter and the receiver through the wireless multipath channel that is impacted by a current movement of the object; extracting an additional plurality of TSCI of the wireless multipath channel from the additional wireless signal; computing an intermediate quantity (IQ) of the current movement of the moving device based on the additional plurality of TSCI; and computing the STI of the moving device based on the additional plurality of TSCI and the IQ.

Clause 29: The method of clause 26, further comprising: computing at least one time series of power information (PI) based on the plurality of TSCI, wherein each PI is associated with a CI and has a real part computed based on at least one of: a magnitude, square of magnitude, phase, real part, imaginary part and another operation on the CI; computing a function of the at least one time series of PI (TSPI), wherein the function comprises an operation on at least one of: an autocorrelation function (ACF), a square of autocorrelation function, an auto-covariance function, a square of auto-covariance function, an inner product, an auto-correlation-like function, and a covariance-like function, wherein the operation comprises at least one of: a polynomial function, a linear function, a nonlinear function, a filtering, a de-noising, a smoothing, a conditioning, an enhancement, a restoration, a feature extraction, a weighted averaging, a high-pass filtering, a low-pass filtering, a bandpass filtering, a median filtering, a quartile filtering, a percentile filtering, a mode filtering, a linear filtering, a nonlinear filtering, a finite impulse response (FIR) filtering, an infinite impulse response (IIR) filtering, a moving average (MA) filtering, an auto-regressive (AR) filtering, an auto-regressive moving average (ARMA) filtering, a selective filtering, an adaptive filtering, a thresholding, a soft thresholding, a hard thresholding, a soft clipping, a first derivative, a second derivative, a higher order derivative, a local maximization, a local minimization, an optimization of a cost function, a neural network, a machine learning, a supervised learning, an unsupervised learning, a semi-supervised learning, a zero crossing, an absolute function, an indicator function, a Fourier transform, a Laplace transform, a Hadamard transform, another transform, a transformation, a decomposition, a derivative, a first order derivative, a second order derivative, a higher order derivative, a convolution, a multiplication, a division, zero crossing, an indicator function, an absolute conversion, a preprocessing, a post-processing, and another operation; determining at least one characteristics of the function, wherein the at least one characteristics comprises: a local maximum, a local minimum, a local extremum, a constrained maximum, a constrained minimum, a constrained extremum, a maximum slope, a minimum slope, a constrained maximum slope, a constrained minimum slope, a maximum higher order derivative, a minimum higher order derivative, a constrained higher order derivative, a zero-crossing, a constrained zero-crossing, a zero-crossing of slope, a zero-crossing of higher order derivative, and another characteristics; and identifying at least one argument of the function associated with the at least one characteristics of the function, wherein: the STI of the moving device is computed based on at least one of: the at least one argument, the plurality of TSCI, the at least one TSPI, the function of the TSPI, a workload for computing the STI is shared among the processor, the transmitter and the receiver, an STI of the object moving with the moving device is determined based on the at least one argument of the function.

Clause 30: An apparatus for object tracking, comprising: at least one of a transmitter and a receiver, wherein: the transmitter is configured for transmitting a first wireless signal through a wireless multipath channel, and the receiver is configured for receiving a second wireless signal through the wireless multipath channel, one of the transmitter and the receiver is a located device, the other of the transmitter and the receiver is a moving device moving with an object, the second wireless signal differs from the first wireless signal due to the wireless multipath channel which is impacted by a movement of the object, at least one of the transmitter and the receiver comprises at least 16 antennas; and a processor configured for: obtaining a plurality of time series of channel information (CI) of the wireless multipath channel based on the second wireless signal, computing an intermediate quantity (IQ) of a current movement of the moving device based on a set of similarity scores associated with many pairs of CI of the plurality of time series of CI (TSCI), each pair comprising two temporally adjacent CI of a TSCI of the plurality of TSCI, computing a spatial-temporal information (STI) of the current movement of the moving device based on at least one of: the IQ, the plurality of TSCI, a time quantity associated with the current movement, a past IQ, and a past STI, and tracking, based on the STI, at least one of: the moving device and the object.

A wireless mesh network (WMN) is a communications network made up of radio nodes organized in a mesh topology. It is also a form of wireless ad hoc network. A mesh refers to rich interconnection among devices or nodes. Wireless mesh networks often include mesh clients, mesh routers and gateways. Mobility of nodes is less frequent. If nodes constantly or frequently move, the mesh spends more time updating routes than delivering data. In a wireless mesh network, topology tends to be more static, so that routes computation can converge and delivery of data to their destinations can occur. Hence, this is a low-mobility centralized form of wireless ad hoc network. In addition, because it sometimes relies on static nodes to act as gateways, it is not a truly all-wireless ad hoc network. Mesh clients are often laptops, cell phones, and other wireless devices. Mesh routers forward traffic to and from the gateways, which may, but need not, be connected to the Internet. The coverage area of all radio nodes working as a single network is sometimes called a mesh cloud. Access to this mesh cloud depends on the radio nodes working together to create a radio network. A mesh network is reliable and offers redundancy. When one node can no longer operate, the rest of the nodes can still communicate with each other, directly or through one or more intermediate nodes. Wireless mesh networks can self-form and self-heal. Wireless mesh networks work with different wireless technologies including 802.11, 802.15, 802.16, cellular technologies and need not be restricted to any one technology or protocol.

With the significant growth of the semiconductor industry, creating small devices with powerful processing ability and network capabilities are no longer a dream for engineers. Internet of Things (IoT) has become one of the hottest topics in both industry and academia of wireless communication field. Today, most of the research of the IoT-enabled devices is mainly of the data collecting and processing units namely creating new sensors. However, the network that integrating the IoT devices to the Internet is usually left untouched by simply using existing computer network solutions such as WLAN or Bluetooth. These computer networks are not designed for low-powered devices such as remote sensors even these IoT devices are considered to be mini computers. The single point of failure nature of these networks makes the entire system extremely vulnerable when it comes to disasters or even difficult environment as the sensors may need to be deployed into some hardly reachable locations. In addition, the capacity of the central hub/router of the network can also limit the coverage of the service provided by IoT devices, and the range is also constrained by the same factors. As most of these remote IoT devices are small, and the devices are usually battery powered, so the power-hungry network options such as using cellular network or satellite are also not ideal for most of the remote scenarios in the IoT networks. The distributed network nature of the wireless mesh network with its simple configuration is ideal for implementing the IoT networks to take advantage of its expanded range as well as keep the hardware design minimal using smaller network module. Such networks are also more robust in the harsh environment as the networks are distributed with no single central point of failure.

The present teaching further discloses how to improve topology and architecture of a wireless sensing system, e.g. based on wireless channel information. How to distribute wireless sensing capability of IoT in a WMN will be disclosed in details. In the present disclosure, a Type B device may be a transceiver that may perform as both Origin (a Type 2 device, a Rx device) and Bot (a Type 1 device, a Tx device), i.e., a Type B device may be both Type 1 (Tx) and Type 2 (Rx) devices (e.g. simultaneously or alternately), for example, mesh devices, a mesh router, etc. In the present disclosure, a Type A device may be a transceiver that may only function as Bot (a Tx device), i.e., Type 1 device only or Tx only, e.g., simple IoT devices. It may have the capability of Origin (Type 2 device, Rx device), but somehow it is functioning only as Bot in the present disclosure. All the Type A and Type B devices form a tree structure. The root may be a Type B device with network (e.g. internet) access. For example, it may be connected to broadband service through a wired connection (e.g. Ethernet, cable modem, ADSL/HDSL modem) connection or a wireless connection (e.g. LTE, 3G/4G/5G, WiFi, Bluetooth, microwave link, satellite link, etc.). All the Type A devices are leaf node. Each Type B device may be the root node, non-leaf node, or leaf node.

Pairwise wireless links may be established between many pairs of devices, forming the tree structure. In each pair (and the associated link), a device (second device) may be a non-leaf (Type B). The other device (first device) may be a leaf (Type A or Type B) or non-leaf (Type B). In the link, the first device functions as a bot (Type 1 device or a Tx device) to send a wireless signal (e.g. probe signal) through the wireless multipath channel to the second device. The second device may function as an Origin (Type 2 device or Rx device) to receive the wireless signal, obtain the TSCI and compute a "linkwise analytics" based on the TSCI.

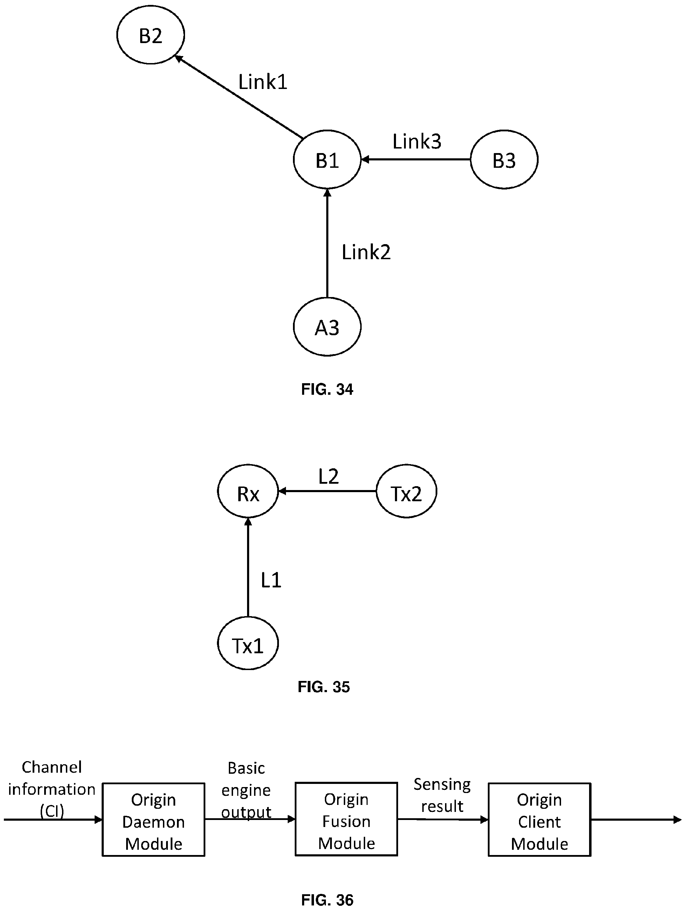

When a pairwise link Link1 comprises of two Type B devices (i.e. when both the first device and the second device are Type B devices), something special happened, as shown in FIG. 34. Let B1 be the first device (Tx, transmit probe signal) and B2 be the second device (Rx, obtain TSCI, compute linkwise analytics) in the current link. In addition to the current link, B1 may be in addition links (e.g. a Link2 connecting B1 to a Type A device called A3, a Link3 connecting B1 to another Type B device called B3). In Link2, A3 is Tx and transmits probe signal while B1 is Rx and computes linkwise analytics. This linkwise analytics will be transmitted from B1 to B2 in Link1. In Link3, B3 is Tx and transmits probe signal while B1 is Rx and computes linkwise analytics. This linkwise analytics will be transmitted from B1 to B2 in Link1. Furthermore, B3 may have obtained some linkwise analytics and may transmit/pass along to B1 in Link3. These linkwise analytics may be sent from B1 to B2 in Link1.

Another exemplary system (mesh network) topology is shown in FIG. 35. Two Type 1 devices (transmitter, or Tx, or Bot) are linked to one Type 2 device (receiver, or Rx, or Origin) via some radio interface links L1 and L2. The routing format may be like "TxID:ifname:hwaddr", where "TxId" is the identifier (that may be unique) of the device, "ifname" is the interface name, and "hwaddr" is the MAC address used for communication. Then, the routing table for the topology shown in FIG. 34 may be routing = {Tx1:L1:Tx1_mac, Tx2:L2:Tx2_mac}.

Additional functionalities may be associated with the Rx in order to pass the sensing outputs to the cloud. In this case, the Rx may be a "Master Origin". For example, in FIG. 36, the Master Origin (MO) may be composed of a daemon module, a fusion module, and a client module. The daemon module may receive channel state information (CSI)/ channel information (CI) sent from other devices, e.g., Bots, for sensing. The MOs may consist of basic engines that support different applications, such as motion engine, breathing engine, etc., which may output motion statistics, breathing rates, etc. These outputs may also be called "radio analytics", because they are analytics calculated based on the CSI extracted from radio signals. These outputs are then fed into the fusion module for the different applications, such as home security, sleep monitoring, wellbeing monitoring, child presence detection, etc. Final results may be determined in the fusion module, and fed into the client module to be sent to the cloud via Ethernet connection through a hub. Another cloud fusion module may be run in the cloud.

FIG. 35 shows an example of only one daemon module existing in the mesh network. In certain cases, more than one daemon module may be required to boost calculation capability. The reason for multiple origin daemon is to perform basic engine sensing on the links that do not have direct link to the master origin (MO). One needs to have Origin daemon module to receive the channel state information (CSI) from the local wireless LAN (WLAN) driver and calculate the basic engine output that consumes CSI. One may do the fusion of the basic engine results in the Origin fusion module in a MO.

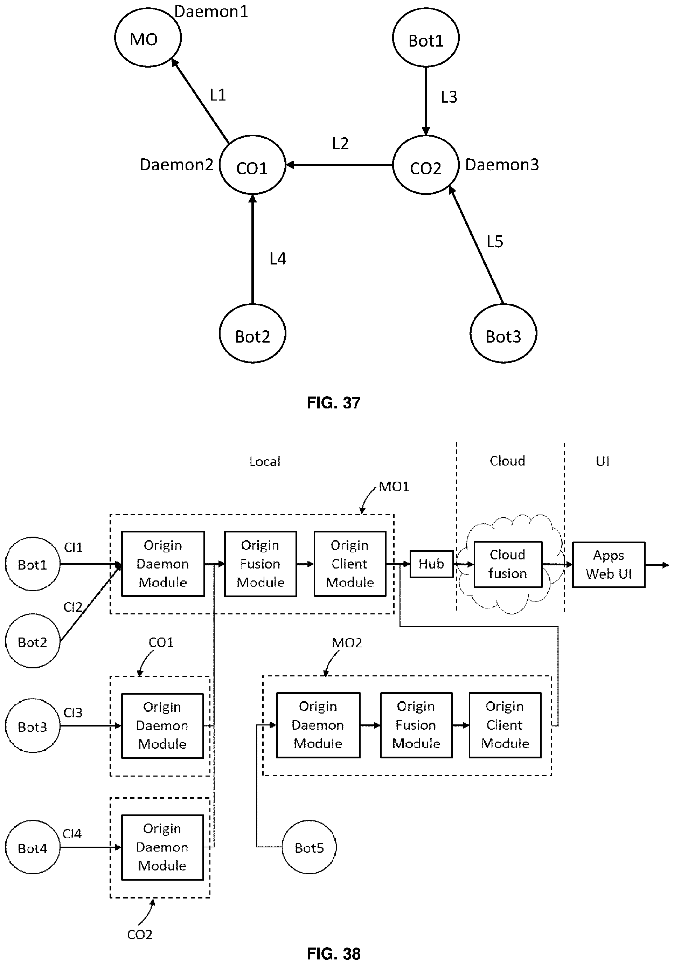

Another example with more than one daemon module is shown in FIG. 37. There is one Master Origin (MO), two Child Origins (CO), and three Bots. Different from the MO, the CO may not have the fusion and client modules, and include only the daemon module. The MO connects every local device to cloud through a client module. Here, L1, L2, L3, L4, L5 are radio interface links connecting two devices. Routing format may be like "TxID:origindaemon_IP:if-name:hwaddr", where "TxId" is the identifier (that should be unique) of the transmitting device, "origindaemon_IP" is the local area network (LAN) IP address of the receiving node running the local origin daemon, "ifname" is the interface name of the radio link to the TxID device, and "hwaddr" is the MAC address used to identify the device in the wireless LAN (WLAN) driver. The routing table for this topology becomes {CO1:MO_IP:L1:CO1_mac, CO2:CO1_IP:L2:CO2_mac, B1:CO2_IP:L3:B1_mac, B2:CO1_IP:L4:B2_mac, B3:CO2_IP:L5:B3_mac}. The IP addresses may be used to open UDP socket connections between the MO and all daemon modules on the local LAN.

Another exemplary topology is shown in FIG. 38, where there are two MOs {MO1, MO2}, two COs {CO1, CO2}, and five Bots B1-B5. Both MO1 and MO2 may send final results to the cloud through the hub. The output from the cloud may be sent to some user interface (UI) in user apps or web UI.

The following numbered clauses provide additional implementation examples.

Clause A1: A method of a tree-structured wireless sensing system, comprising: determining that there are a plurality of wireless devices in a venue, each device being at least one of: a Type A heterogeneous wireless device and a Type B heterogeneous wireless device, wherein each Type A device has at least one wireless transmitter capable of transmitting RF signals, wherein each Type B device has at least one wireless transceiver capable of both transmitting and receiving RF signals; determining a tree interconnection structure of the plurality of wireless devices comprising of: all wireless devices being nodes of the tree with each Type A device being a leaf of the tree, and each Type B device being either a leaf or a non-leaf of the tree, a Type B device being the root of the tree, and at least one pairwise wireless link each being a branch of the tree, wherein each wireless link connects a pair of the wireless devices at least one of which being a Type B device; storing an information of the plurality of wireless devices and the tree interconnection structure in a database; in each pairwise wireless link connecting a first device and a second device, wherein the first device is at least one of: a Type A device and a non-root Type B device, and the second device is a Type B device: transmitting a wireless signal through a wireless multipath channel of the venue to the second device by the first device using a processor, a memory and a set of instructions of the first device, wherein the wireless multipath channel is impacted by an object motion of an object in the venue, receiving the wireless signal by the second device using a processor, a memory and a set of instruction of the second device, obtaining a time series of channel information (CI) of the wireless multipath channel from the wireless signal, monitoring the object motion based on the time series of CI (TSCI), and computing a linkwise analytics related to the object motion based on the TSCI.

Clause A2: The method of the tree-structured wireless sensing system of Clause A1: wherein the root Type B device is a gateway device with network access through at least one of: a wired network connection and a wireless network connection; wherein the network access comprises access to at least one of: internet, PAN, LAN, WLAN, CAN, MAN, WAN, SAN, and another network; wherein the wired network connection comprises at least one of: an Ethernet, a token ring, an FDDI, an optical fiber, a cable modem, a DSL modem, an ADSL modem, a HDSL modem, and another wired network connection; wherein the wireless network connection comprises at least one of: WiFi, LTE, 3G, 4G, 5G, 6G, Bluetooth, BLE, RFID, microwave link, satellite link and another wireless network connection.

Clause A3: The method of the tree-structured wireless sensing system of Clause A1, further comprising: storing the information of the plurality of wireless devices and the tree interconnection structure in the database in a server; and updating the database in the server, wherein the server is at least one of: one of the plurality of wireless devices, a local device, a cloud device, a dedicated device, and another device.

Clause A4: The method of the tree-structured wireless sensing system of Clause A1: wherein the information of the plurality of wireless devices and the tree interconnection structure in the database to comprise at least one of: an identifier (ID) of the tree, an address of the tree, a setting of the tree, an attribute of the tree, a structure of the tree, an identifier (ID) of the venue associated with the tree, a physical location of the venue associated with the tree, an address of the venue associated with the tree, a cost associated with the tree, another association of the tree, an identifier (ID) of each of the plurality of wireless devices, an address of each wireless device comprising at least one of: an IP address, a MAC address and another address, a setting of each wireless device, an attribute of each wireless device, a physical location of the venue associated with each wireless device, a region of the venue associated with each wireless device, a cost associated with each wireless device, another association of each wireless device, an ID of a root device, a description of each pairwise wireless link comprising at least one of: an identifier (ID) of the link, a setting of the link, an attribute of the link, a physical location of the venue associated with the link, a physical region of the venue associated with the link, an event associated with the link, a cost associated with the link, another association of the link, a Type 1 heterogeneous wireless device, a transmitting wireless device, a Tx device, an ID of the transmitting wireless device, and a Type 2 heterogeneous wireless device, a receiving wireless device, an Rx device, an ID of the receiving wireless device, a MAC address of the receiving wireless device, and a routing table comprising at least one of: the description of the pairwise wireless links, a routing of data from a wireless device towards another wireless device through the pairwise wireless links, and a routing of linkwise analytics towards at least one of: a destination device, a root device, a local server and a cloud server, a distance of each wireless device to the root device, and a count of pairwise wireless links between each wireless device and a destination device.

Clause A5: The method of the tree-structured wireless sensing system of Clause A1, further comprises: updating at least one of: the information and a routing table.

Clause A6: The method of the tree-structured wireless sensing system of Clause A1, further comprises: finding a path to send data through the nodes of the tree towards a destination device based on at least one of: the information, a routing table, a requirement and a criterion.

Clause A7: The method of the tree-structured wireless sensing system of Clause A6, further comprises: changing the path based on at least one of: updated destination, updated information, updated routing table, updated requirement and updated criterion.

Clause A8: The method of the tree-structured wireless sensing system of Clause A1, further comprises: routing a linkwise analytics towards the root device of the tree based on at least one of: a routing table, and the information of the plurality of wireless devices and the tree interconnection structure.

Clause A9: The method of the tree-structured wireless sensing system of Clause A8, further comprises: updating the routing based on at least one of: updated destination, updated information, updated routing table, updated requirement and updated criterion.

Clause A10: The method of the tree-structured wireless sensing system of Clause A1, further comprises: transmitting a linkwise analytics through a number of nodes of the tree to the root device of the tree based on at least one of: a routing table, and the information of the plurality of wireless devices and the tree interconnection structure.

Clause A11: The method of the tree-structured wireless sensing system of Clause A1, further comprises: identifying at least one pairwise wireless link of the tree is associated with a target region of the venue based on at least one of: the information and a routing table.

Clause A12: The method of the tree-structured wireless sensing system of Clause A1, further comprises: identifying at least one pairwise wireless link of the tree is associated with the object motion of the object in the venue based on at least one of: the information and a routing table.

Clause A13: The method of the tree-structured wireless sensing system of Clause A1, further comprises: identifying at least one pairwise wireless link of the tree is associated with a trajectory of the object moving through the venue based on at least one of: the information and a routing table.

Clause A14: The method of the tree-structured wireless sensing system of Clause A1, further comprising: in each pairwise wireless link connecting a first wireless device and a second wireless device, wherein both the first device and second device are Type B devices: transmitting at least one linkwise analytics related to the object motion of the object in the venue by the first device to the second device, receiving the at least one linkwise analytics by the second device, and monitoring the object motion based on the at least one linkwise analytics.

Clause A15: The method of the tree-structured wireless sensing system of Clause A1: wherein a wireless signal is transmitted by the first device to the second device.

Clause A16: The method of the tree-structured wireless sensing system of Clause A1: wherein a wireless signal is transmitted by the second device to the first device.

Clause A17: The method of the tree-structured wireless sensing system of Clause A1, further comprising: obtaining all the linkwise analytics by the root device, monitoring the object motion by the root device based on all the linkwise analytics, and computing at least one overall analytics by the root device based on all the linkwise analytics.

Clause A18: The method of the tree-structured wireless sensing system of Clause A17, further comprising: receiving at least one wireless signal by the root device from at least one of the plurality of wireless devices, obtaining at least one TSCI by the root device from the at least one wireless signal, and computing at least one linkwise analytics by the root device based on the at least one TSCI.

Clause A19: The method of the tree-structured wireless sensing system of Clause A17, further comprising: making at least one of: an overall analytics, an linkwise analytics, and a portion of the information of the plurality of wireless devices and the tree interconnection structure in the database available through a network by the root device.

Clause A20: The method of the tree-structured wireless sensing system of Clause A1, further comprising: transmitting a message by the root device to a user device through a network based on the overall analytics, the linkwise analytics, and the information of the plurality of wireless devices and the tree interconnection structure in the database.

Clause A21: The method of the tree-structured wireless sensing system of Clause A1: wherein a Type A device has at least one wireless receiver.

Clause A22: The method of the tree-structured wireless sensing system of Clause A1: wherein a Type B device receives a first wireless signal from a first wireless device and transmits a second wireless signal to a second wireless device in at least one of: simultaneous manner, concurrent manner, alternative manner, contemporaneous manner, and asynchronous manner.

Clause A23: The method of the tree-structured wireless sensing system of Clause A1: wherein a Type B device has at least two radios such that it uses a first radio to receive a first wireless signal from a first wireless device and uses a second radio to transmit a second wireless signal to a second wireless device simultaneously and asynchronously.

Clause A24: The method of the tree-structured wireless sensing system of Clause A1: wherein one of the plurality of wireless devices is in more than one pairwise wireless links; wherein the device sends a first wireless signal to a first Type B device in a first link; wherein the device sends a second wireless signal to a second Type B device in a second link; wherein each of the first and second Type B devices receives the respective wireless signal, obtains a respective TSCI from the respective wireless signal, and computes a respective linkwise analytics based on the respective TSCI.

Clause A25: The method of the tree-structured wireless sensing system of Clause A24: wherein the device sends at least one pairwise analytics to at least one of: the first Type B device and the second Type B device.

Clause A26: The method of the tree-structured wireless sensing system of Clause A1: wherein one of the plurality of wireless devices is in more than one pairwise wireless links; wherein the device sends a wireless signal in a broadcasting manner to the more than one respective Type B devices associated with the more than one pairwise wireless links; wherein each of the more than one Type B devices receives the wireless signal, obtains a respective TSCI from the wireless signal, and computes a respective linkwise analytics based on the respective TSCI.

Clause A27: The method of the tree-structured wireless sensing system of Clause A26: wherein the device sends at least one pairwise analytics to at least one of the more than one Type B devices.

Clause A28: The method of the tree-structured wireless sensing system of Clause A1, further comprising: modifying the tree by modifying the pairwise wireless links without adding any new wireless device or removing any existing wireless device; updating the information of the plurality of the wireless device and the tree interconnection structure in the database.

Clause A29: The method of the tree-structured wireless sensing system of Clause A1, further comprising: modifying the tree by rearranging the pairwise wireless links among the set of the plurality of wireless devices, while keeping the composition of the set unchanged; updating the information of the plurality of the wireless device and the tree interconnection structure in the database.

Clause A30: The method of the tree-structured wireless sensing system of Clause A1, further comprising: modifying the tree by at least one of: adding a new wireless device, and removing an existing wireless device, wherein the new wireless device is at least one of: a Type A device and a Type B device, a leaf node, a non-leaf node and a root node, wherein the existing wireless device is at least one of: a Type A device and a Type B device, a leaf node, a non-leaf node and a root node; updating the information of the plurality of the wireless device and the tree interconnection structure in the database.

Clause A31: The method of the tree-structured wireless sensing system of Clause A1, further comprising: modifying the tree by changing the root device, and replacing it by another of the plurality of wireless devices; updating the information of the plurality of the wireless device and the tree interconnection structure in the database.

Clause A32: The method of the tree-structured wireless sensing system of Clause A1, further comprising: modifying the tree by choosing a new root device from among the plurality of wireless devices and changing at least one pairwise wireless links; updating the information of the plurality of the wireless device and the tree interconnection structure in the database.

Clause A33: The method of the tree-structured wireless sensing system of Clause A1, further comprising: modifying the tree by at least one of: adding a new pairwise wireless link, and removing an existing pairwise wireless link; updating the information of the plurality of the wireless device and the tree interconnection structure in the database.

Clause A34: The method of the tree-structured wireless sensing system of Clause A33, further comprising: modifying the tree by removing an existing pairwise wireless link of the tree without removing any wireless device of the tree; and updating the information of the plurality of the wireless device and the tree interconnection structure in the database.

Clause A35: The method of the tree-structured wireless sensing system of Clause A33, further comprising: modifying the tree by removing an existing pairwise wireless link of the tree by removing a wireless device of the tree; and updating the information of the plurality of the wireless device and the tree interconnection structure in the database.

Clause A36: The method of the tree-structured wireless sensing system of Clause A33, further comprising: modifying the tree by adding a new pairwise wireless link of the tree without adding any new wireless device to the tree; and updating the information of the plurality of the wireless device and the tree interconnection structure in the database.

Clause A37: The method of the tree-structured wireless sensing system of Clause A33, further comprising: modifying the tree by adding a new pairwise wireless link of the tree by adding a new wireless device to the tree; and updating the information of the plurality of the wireless device and the tree interconnection structure in the database.

Clause A38: The method of the tree-structured wireless sensing system of Clause A3, further comprising: sending a request to the server to add a new wireless device to the plurality of wireless devices as a node of tree; sending control signals to the new wireless device and at least one existing device of the tree such that at least one pairwise wireless link is established between the new wireless device and the at least one existing devices; updating at least one of: a routing table and the information of the plurality of wireless devices and the tree interconnection structure in the database.

Clause A39: The method of the tree-structured wireless sensing system of Clause A38, further comprising: transmitting a wireless signal by the new wireless device to one of the at least one existing device in one of the at least one pairwise wireless link, wherein the existing device is a Type B device.

Clause A40: The method of the tree-structured wireless sensing system of Clause A39, further comprising: transmitting at least one linkwise analytics related to the object motion by the new wireless device to the existing device in the pairwise wireless link, wherein the new wireless device is a Type B device.

Clause A41: The method of the tree-structured wireless sensing system of Clause A39, further comprising: transmitting another wireless signal by the new wireless device to another one of the at least one existing device in another one of the at least one pairwise wireless link, wherein the another existing device is a Type B device.

Clause A42: The method of the tree-structured wireless sensing system of Clause A38, further comprising: wherein the new wireless device is a Type B device; receiving by the new wireless device a wireless signal from one of the at least one existing device in one of the at least one pairwise wireless link.

Clause A43: The method of the tree-structured wireless sensing system of Clause A42, further comprising: receiving at least one linkwise analytics related to the object motion from the existing device in the pairwise wireless link, wherein the existing device is a Type B device.

Clause A44: The method of the tree-structured wireless sensing system of Clause A3, further comprising: sending a request to the server to remove an existing wireless device from the tree; sending control signals to the existing wireless device and at least one other device of the tree such that at least one pairwise wireless link associated with the existing wireless device is removed.

Clause A45: The method of the tree-structured wireless sensing system of Clause A3, further comprising: sending a request to the server to remove an existing wireless devices from the tree; sending control signals to the existing wireless device and a first device of the tree such that a first pairwise wireless link between them is removed; sending control signals to the wireless device and a second device of the tree such that a second pairwise wireless link between them is removed; sending control signals to the first device and the second device such that a new pairwise wireless link is established between the them.

Clause A46: The method of the tree-structured wireless sensing system of Clause A45: wherein the first device sends a first wireless signal to the wireless device in the first pairwise wireless link; wherein the wireless device sends a second wireless signal to the second device in the second pairwise wireless link; wherein the first device sends a third wireless signal to the second device in the new pairwise wireless link.

Clause A47: The method of the tree-structured wireless sensing system of Clause A1, further comprising: adding a new wireless device to the plurality of wireless devices as a leaf node of the tree, wherein the new wireless device is at least one of: a Type A device and a Type B device.

Clause A48: The method of the tree-structured wireless sensing system of Clause A1, further comprising: adding a new wireless device to the plurality of wireless devices as a node of the tree, adding a new pairwise wireless link between the new device and an existing wireless device of the tree, updating the information of the plurality of the wireless device and the tree interconnection structure in the database, transmitting a wireless signal by the new device to the existing device, receiving the wireless signal by the existing device, obtaining a TSCI by the existing device from the wireless signal, monitoring the object motion by the existing device based on the TSCI, and computing a linkwise analytics by the existing device based on the TSCI.

Clause A49: The method of the tree-structured wireless sensing system of Clause A48, further comprising: wherein the existing device is a Type A device, converting the existing device to a Type B device.

Clause A50: The method of the tree-structured wireless sensing system of Clause A48, further comprising: wherein the new device is a Type B device, adding another new pairwise wireless link between the new device and another existing wireless device of the tree, receiving a wireless signal by the new device from the another existing device, obtaining a TSCI by the new device from the wireless signal, monitoring the object motion by the new device based on the TSCI, and computing a linkwise analytics by the new device based on the TSCI.

Clause A51: The method of the tree-structured wireless sensing system of Clause A48, further comprising: wherein the new device is a Type B device, receiving at least one linkwise analytics by the new device from another device.

Clause A52: The method of the tree-structured wireless sensing system of Clause A1, further comprising: adding a new wireless device to the plurality of wireless devices as a non-leaf node to the tree, wherein the new device is a Type B device.

Clause A53: The method of the tree-structured wireless sensing system of Clause A1, further comprising: adding a new wireless device to the plurality of wireless devices as a non-leaf node to the tree, wherein the new device is a Type B device, adding a new pairwise wireless link between the new device and an existing wireless device of the tree, updating the information of the plurality of the wireless device and the tree interconnection structure in the database, receiving a wireless signal by the new device from the existing device, obtaining a TSCI by the new device from the wireless signal, monitoring the object motion by the new device based on the TSCI, and computing a linkwise analytics by the new device based on the TSCI.

Clause A54: The method of the tree-structured wireless sensing system of Clause A53, further comprising: sending the linkwise analytics by the new device to another existing device.

Clause A55: The method of the tree-structured wireless sensing system of Clause A53, further comprising: wherein the existing device is a Type B device, receiving at least one linkwise analytics by the new device from the existing device.

Clause A56: The method of the tree-structured wireless sensing system of Clause A55, further comprising: monitoring the object motion by the new device based jointly on all the linkwise analytics.

Clause A57: The method of the tree-structured wireless sensing system of Clause A55, further comprising: forwarding the at least one linkwise analytics by the new device to another existing device.

Clause A58: The method of the tree-structured wireless sensing system of Clause A53: wherein the existing device is the existing root of the existing tree, wherein the new device replaces the existing device as the new root of the tree.

Clause A59: The method of the tree-structured wireless sensing system of Clause A1, further comprising: removing an existing wireless device from the tree, removing a pairwise wireless link in which the existing wireless device transmits a wireless signal to another existing device, and updating the information of the plurality of the wireless device and the tree interconnection structure in the database.

Clause A60: The method of the tree-structured wireless sensing system of Clause A1, further comprising: removing an existing wireless device from the tree, wherein the existing device is a Type B device, removing a pairwise wireless link in which the existing wireless device receives a wireless signal from another existing device, and updating the information of the plurality of the wireless device and the tree interconnection structure in the database.

Clause A61: The method of the tree-structured wireless sensing system of Clause A1, further comprising: removing an existing wireless device from the tree, wherein the existing device is a Type B device, removing a pairwise wireless link in which the existing wireless device receives a first wireless signal from a first existing device, removing a pairwise wireless link in which the existing wireless device transmits a second wireless signal to a second existing device, adding a pairwise wireless link in which the first existing device transmits a third wireless signal to the second existing device, and updating the information of the plurality of the wireless device and the tree interconnection structure in the database.

Clause A62: The method of the tree-structured wireless sensing system of Clause A1, further comprising: adding a pairwise wireless link between a first existing device and a second existing device of the tree, updating the information of the plurality of the wireless device and the tree interconnection structure in the database.

Clause A63: The method of the tree-structured wireless sensing system of Clause A1, further comprising: adding a pairwise wireless link between a first existing device and a second existing device of the tree, updating the information of the plurality of the wireless device and the tree interconnection structure in the database, transmitting a wireless signal by the first device to the second device, receiving the wireless signal by the second device, obtaining a TSCI from the wireless signal by the second device, monitoring the object motion based on the TSCI by the second device, and computing a linkwise analytics based on the TSCI by the second device.

Clause A64: The method of the tree-structured wireless sensing system of Clause A1, further comprising: removing an existing pairwise wireless link between a first existing device and a second existing device of the tree, updating the information of the plurality of the wireless device and the tree interconnection structure in the database.

Clause A65: The method of the tree-structured wireless sensing system of Clause A1, further comprising: removing an existing pairwise wireless link between a first existing device and a second existing device of the tree, updating the information of the plurality of the wireless device and the tree interconnection structure in the database, stopping to transmit a wireless signal by the first device to the second device, stopping to receive the wireless signal by the second device, stopping to obtain a TSCI from the wireless signal by the second device, stopping to monitor the object motion based on the TSCI by the second device, and stopping to compute a linkwise analytics based on the TSCI by the second device.

Clause A66: The method of the tree-structured wireless sensing system of Clause A1, further comprising: updating at least one of: a routing table, and the information of the plurality of wireless devices and the tree interconnection structure in the database, and propagating the update to the plurality of wireless devices.

Clause A67: The method of the tree-structured wireless sensing system of Clause A1: wherein the tree interconnection structure has more than one roots, wherein each of the more than one roots of the tree is a Type B device.

Clause A68: The method of the tree-structured wireless sensing system of Clause A1: wherein there are more than one routes from a first node of the tree to a second node of the tree. System, device and software clauses from: the angle of a Type A device, the angle of a Type B device as a leaf node, the angle of a Type B device as a non-leaf node, the angle of a Type B device as root node, the angle of server.

The present teaching also discloses how to qualify a wireless system for achieving wireless sensing and how to distribute wireless sensing capability of IoT in a WMN. A 802.11 sensing, or WiFi sensing, is the use of 802.11 signals to sense (e.g. detect) events/changes in the environment. It can be utilized with signal processing and machine learning.

In one embodiment, a wireless transmitter (Tx) transmits a 802.11 signal to a wireless receiver (Rx) in a multipath-rich venue. The 802.11 signal bounces back and forth in the venue generating lots of multipaths. Although undesirable to communications, the bouncing of the 802.11 signal effectively "scan" or "sense" the venue. By monitoring the multipaths (e.g. through CSI), the disclosed system can detect target events and changes in the venue. This disclosed motion detection method does not require a line-of-sight (LOS) between the transmitter and the receiver, and can work in both LOS and Non-LOS (NLOS) situations. In most cases, the transmitter and the receiver are not required to be a wearable device. This provides new functionalities for 802.11-enabled devices (e.g. TV, speaker, router, IoT devices) and facilities (stadiums, halls, rooms, warehouse, factory); and provides new industry-wide business opportunities for all 802.11 related companies (components/ devices/services). The disclosed system does not need any dedicated hardware.

A passive infra-red (PIR) motion sensor may only work in an LOS scenario, and does not support machine learning. It needs many (e.g. 6) PIR sensors to cover an entire house. A video camera based on motion sensing may only work in an LOS scenario, and is intensive in terms of memory and computation. The video monitoring or recording intrudes human privacy. In contrast, a 802.11 sensing can work in both LOS and NLOS scenarios; only need 1 pair of transmitter and receiver to cover an entire house; can support machine learning; and has a much lower storage requirement and computation requirement compared to video camera based on motion sensing. In addition, there is no video to intrude privacy in the 802.11 sensing.

The 802.11 sensing can be applied in many scenarios: e.g. motion detection for intruder detection, security, smart IoT; breathing monitoring for sleep monitoring, well-being, caregiving; locationing/tracking for smart factory, indoor GPS companion, traffic planning; fall detection for older adults, accident detection, caregiving; child detection in hot cars for smart cars, accident prevention; presence/proximity detection for conference room, convenience, smart IoT; human identification for user identification, personalization, smart IoT; gesture recognition for smart office; and activity recognition for user interface. Many of these applications do not need a wearable device and can work in a contactless mode, and does not need video or a LOS requirement.

The 802.11 sensing can give business opportunities to service providers, device manufacturers (e.g. manufacturers of smart home/IoT devices, consumer electronics, computing devices, home appliance, lighting, accessories), and component providers. For example, the disclosed method can provide a new wave of "802.11 sensing" services, firmware, software and/or devices regarding daily living, safety, lifestyle, convenience, personalization, caregiving, digital health, etc., in a traditional mode (e.g. broadband, mobile, streaming, cable) or a new mode (e.g. smart device makers).

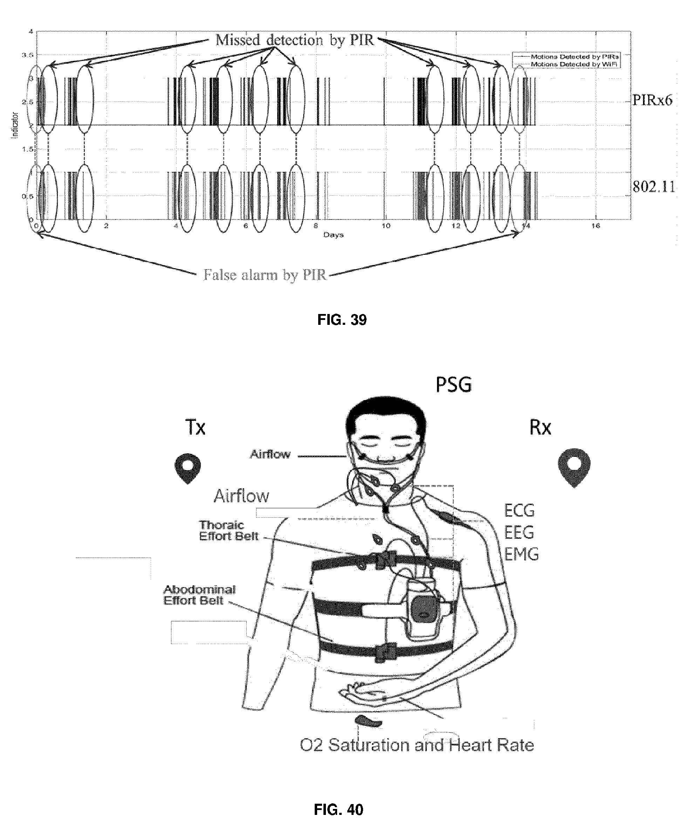

FIG. 39 shows exemplary performances of motion detections based on passive infrared (PIR) sensing and WiFi sensing, according to some embodiments of the present teaching. In a long-term side-by-side comparison with PIR, in a test house covered by 6 professionally installed PIRs and a pair of 802.11 Tx/Rx), 802.11 motion sensing performs better than the PIRs. As shown in FIG. 39, the PIR sensing and the 802.11 sensing have similar false alarm rates, while the 802.11 sensing has a better detection rate than the PIR sensing.

FIG. 40 shows an exemplary setup for breathing monitoring, according to some embodiments of the present teaching. In a multi-night side-by-side comparison with other breathing sensors (pressure sensor, radar sensor, with PSG being ground truth), 802.11 breathing monitoring (contactless) outperforms pressure sensor and radar sensor, in terms of: stage (awake/REM/NREM) detection rate, and median (abs) error. In walking experiment along paths in buildings, 802.11 tracking (tracking based on 802.11 signal) shows high tracking accuracy, with an average (abs) tracking error being less than 20-30 cm, in a NLOS operation.

The bouncing of 802.11 signals creates multipaths which effectively scan or sense the environment, including any object motions, events and changes. The multipaths can be captured in channel state information (CSI). In 802.11 sensing, various signal processing/machine learning algorithms and systems may be applied to obtain and analyze CSI to achieve various tasks, regarding motions/events/changes, e.g. a motion detection by detecting change in CSI, a breathing detection by detecting cyclic behavior of CSI, a locationing by CSI recognition. Standardization may be needed for the systems regarding CSI generation, timing, accuracy, consistency, protocol, etc. Interface may also be standardized. Some 802.11 sensing demos use periodic probing/CSI generation at 1/10/100/1000Hz, with sounding overhead less than 0.1% of available data bandwidth at 10Hz. A protocol can be determined to control the generation of CSI, repetition rate, accuracy of timing, antenna selection and probing for 802.11 sensing, with CSI consistency, precision and format.

The following numbered clauses provide additional implementation examples.

Clause B1: A method of a qualified wireless system, comprising: transmitting a wireless signal from a Type 1 heterogeneous wireless device to a Type 2 heterogeneous wireless device through a wireless multipath channel of a venue; receiving the wireless signal by a Type 2 heterogeneous wireless device; obtaining a time series of channel information (CI) of the wireless multipath channel based on the wireless signal using a processor of the Type 2 device, a memory communicatively coupled with the process and a set of instructions stored in the memory; and making the TSCI available for a task, wherein at least one of: the Type 1 device, a module of the Type 1 device, an integrated circuit (IC) of the Type 1 device, the Type 2 device, a module of the Type 2 device, and an IC of the Type 2 device, is qualified if a respective qualification criterion is satisfied.

Clause B2: The method of the qualified wireless system of clause B1: wherein the Type 1 device and the Type 2 device are placed at two different locations in the venue.

Clause B3: The method of the qualified wireless system of clause B1: wherein the Type 1 device and the Type 2 device are collocated, being placed at the similar locations in the venue.

Clause B4: The method of the qualified wireless system of clause B3: wherein the Type 1 device and the Type 2 device are the same device.

Clause B5: The method of the qualified wireless system of clause B1: wherein there are more than one pairs of Type 1 device and Type 2 device in the venue, each respective Type 2 device of a pair receiving a respective wireless signal asynchronously from the respective Type 1 device of the pair and obtaining asynchronously a respective TSCI; wherein the Type 1 device and the Type 2 device of a first pair are collocated; wherein the Type 1 device and the Type 2 device of a second pair are placed at two different locations in the venue.

Clause B6: The method of the qualified wireless system of clause B1: associating at least one of: the Type 1 device and the Type 2 device, with an identifier (ID).

Clause B7: The method of the qualified wireless system of clause B6: wherein the ID comprises at least one of: a name, a number, an alphanumeric ID, a string of text, numbers and symbols, a file, a database, an item of the database, a pointer to the item, a link to a webpage, a link to a storage, a MAC address, an IP address, a network address, a network ID, a domain ID, a web ID, an internet ID, a mobile network ID, a LAN ID, a platform ID, a software ID, a software application ID, an administration ID, a supervision ID a hardware ID, a device ID, a device profile, a hardware component ID, a computer ID, a processor ID, a storage ID, a process ID, a serial number, a task ID, a class, a class information, a category, a category information, a performance information, a capability information, a policy information, a pair ID of the Type 1 device and the Type 2 device, a pair profile, a link ID, a link profile, an antenna ID, an antenna profile, a system ID, a user, a customer, a supervisor, super-user, an administrator, a guardian, a service, an account, a password, a service account, a user account, a user profile, a user name, a user password, a user information, a user ID, a service provider, a service profile, a manufacturer, a sales channel, a vendor, a retailer, a distribution channel, a content channel, an Apple ID, a Amazon ID, a Samsung ID, a Google ID, a Facebook ID, a Microsoft ID, a company ID, a service ID, a service provider ID, a service ID, an access ID, a hash of another ID, a user association, a user grouping, an account privilege, a user history, a task, a task ID, a task information, a task requirement, a user associated with the task, a physical address, a physical location, a home, a household, an office, a company, a school, a warehouse, a store, a factory, a station, a stadium, a hall, an enclosure, a venue, a site, a district, a zone, a region, an area, a proximity, a neighborhood, a map, a map location, a location-based information, a street, a city, a county, a state, a province, a precinct, a prefecture, a country, a continent, a zip code, a postal code, a GPS coordinate, another code, a phone number, a payment card information, a grouping, a classification, a category, and another ID.

Clause B8: The method of the qualified wireless system of clause B6, further comprising: sharing the ID between any two of: the Type 1 device, the Type 2 device, another wireless heterogeneous device and a user device.

Clause B9: The method of the qualified wireless system of clause B8, further comprising: sharing the ID based on at last one of: a standard, a WiFi standard, a WLAN standard, a mesh network standard, an IEEE standard, an IEEE 802 standard, an IEEE 802.11 standard, an IEEE 802.15 standard, an IEEE 802.16 standard, a WiFi Alliance specification, a 3GPP standard, a mobile communication standard, a cellular communication standard, 3G/4G/LTE/5G/6G/7G/8G, an international standard, a national standard, an industry standard, a de facto standard, a protocol, a handshake, an enquiry, a response, an acknowledge, a database, another format, another channel, another exchange and another mechanism.

Clause B10: The method of the qualified wireless system of clause B6: wherein the ID is time-varying.

Clause B11: The method of the qualified wireless system of clause B10: wherein the time-varying ID varies over time based on at least one of: a standard, a protocol, an established protocol, an interoperable protocol, a specification, a requirement, a qualification requirement, the qualification criterion, a protocol agreed by the Type 1 device and the Type 2 device, a timing agreed by the Type 1 device and the Type 2 device, a timing based on at least one of: the standard and the protocol, a strategy, a time table, a user setting, a user request, a control by a server, the Type 1 device, the Type 2 device, another device, the server, a control signal communicated between two of: the Type 1 device, the Type 2 device, the another device, the server, another Type 1 device, another Type 2 device, a situation, a condition of the wireless multipath channel, another criterion, and another mechanism.

Clause B12: The method of the qualified wireless system of clause B1, further comprising: transmitting a first part of the wireless signal by the Type 1 device using at least one of: a first carrier frequency, a first channel, a first antenna, and a first group of antennas, of the Type 1 device; and transmitting a second part of the wireless signal by the Type 1 device using at least one of: a second carrier frequency, a second channel, a second antenna, and a second group of antennas, of the Type 1 device.

Clause B13: The method of the qualified wireless system of clause B12, further comprising: wherein the first part and the second part of the wireless signal are transmitted by the Type 1 device in at least one of the following manner: coordinated, un-coordinated, independent, together, un-separated, separated, simultaneous, contemporaneous, non-simultaneous, at the same time, at different time, synchronized, and non-synchronized.

Clause B14: The method of the qualified wireless system of clause B12: wherein the first carrier frequency and the second carrier frequency are at least one of: the same and different.

Clause B15: The method of the qualified wireless system of clause B12: wherein the first channel and the second channel are at least one of: the same channel, overlapping channels, and different channels.

Clause B16: The method of the qualified wireless system of clause B12: wherein the first antenna and the second antenna are at least one of: the same antenna, and different antennas.

Clause B17: wherein the first group of antennas and the second group of antennas are at least one of: the same group of antennas, overlapping groups of antennas, and disjoint groups of antennas.

Clause B18: The method of the qualified wireless system of clause B12: wherein a first characteristics of the first part of the wireless signal and a second characteristics of the second part of the wireless signal are at least one of: determined, worked out, exchanged, hand-shaked, shared, negotiated, arranged, and coordinated, among at least two of: the Type 1 device, the Type 2 device and another device.

Clause B19: The method of the qualified wireless system of clause B18: wherein the first characteristics of the first part of the wireless signal and the second characteristics of the second part of the wireless signal are at least one of: determined, worked out, exchanged, hand-shaked, shared, negotiated, arranged, and coordinated, based on at least one of: a standard, a protocol, an established protocol, an interoperable protocol, a specification, a requirement, a qualification requirement, the qualification criterion, a protocol agreed by the Type 1 device and the Type 2 device, a timing agreed by the Type 1 device and the Type 2 device, a timing based on at least one of: the standard and the protocol, a strategy, a time table, a user setting, a user request, a control by a server, the Type 1 device, the Type 2 device, another device, the server, a control signal communicated between two of: the Type 1 device, the Type 2 device, the another device, the server, another Type 1 device, another Type 2 device, a situation, a condition of the wireless multipath channel, another criterion, and another mechanism.

Clause B20: The method of the qualified wireless system of clause B1, further comprising: receiving a first part of the wireless signal by the Type 2 device using at least one of: the first carrier frequency, the first channel, a third antenna, and a third group of antennas, of the Type 2 device; and receiving a second part of the wireless signal by the Type 2 device using at least one of: the second carrier frequency, the second channel, a fourth antenna, and a fourth group of antennas, of the Type 2 device.

Clause B21: The method of the qualified wireless system of clause B20: wherein the first part and the second part of the wireless signal are received by the Type 2 device in at least one of the following manner: coordinated, un-coordinated, independent, together, un-separated, separated, simultaneous, contemporaneous, non-simultaneous, at the same time, at different time, synchronized, and non-synchronized.

Clause B22: The method of the qualified wireless system of clause B20: wherein the third antenna and the fourth antenna of the Type 2 device are at least one of: the same antenna, and different antennas.

Clause B23: The method of the qualified wireless system of clause B20: wherein the third group of antennas and the fourth group of antennas of the Type 2 device are at least one of: the same group of antennas, overlapping groups of antennas, and disjoint groups of antennas.

Clause B24: The method of the qualified wireless system of clause B1, further comprising: transmitting the wireless signal from the Type 1 device to a second Type 2 device, wherein a first part of the wireless signal is intended for the Type 2 device, wherein a second part of the wireless signal is intended for the second Type 2 device; transmitting the first part of the wireless signal by the Type 1 device using at least one of: a first carrier frequency, a first channel, a first antenna, and a first group of antennas, of the Type 1 device; and transmitting the second part of the wireless signal by the Type 1 device using at least one of: a second carrier frequency, a second channel, a second antenna, and a second group of antennas, of the Type 1 device.

Clause B25: The method of the qualified wireless system of clause B24: wherein the first part and the second part of the wireless signal are transmitted by the Type 1 device in at least one of the following manner: coordinated, un-coordinated, independent, together, un-separated, separated, simultaneous, contemporaneous, non-simultaneous, at the same time, at different time, synchronized, and non-synchronized.

Clause B26: The method of the qualified wireless system of clause B24: wherein the first carrier frequency and the second carrier frequency are at least one of: the same and different.

Clause B27: The method of the qualified wireless system of clause B24: wherein the first channel and the second channel are at least one of: the same channel, overlapping channels, and different channels.

Clause B28: The method of the qualified wireless system of clause B24: wherein the first antenna and the second antenna of the Type 1 device are at least one of: the same antenna, and different antennas.

Clause B29: The method of the qualified wireless system of clause B24: wherein the first group of antennas and the second group of antennas of the Type 1 device are at least one of: the same group of antennas, overlapping groups of antennas, and disjoint groups of antennas.

Clause B30: The method of the qualified wireless system of clause B24: wherein a first characteristics of the first part of the wireless signal and a second characteristics of the second part of the wireless signal are at least one of: determined, worked out, exchanged, hand-shaked, shared, negotiated, arranged, and coordinated, among at least two of: the Type 1 device, the Type 2 device, the second Type 2 device, and another device.

Clause B31: The method of the qualified wireless system of clause B30: wherein the first characteristics of the first part of the wireless signal and the second characteristics of the second part of the wireless signal are at least one of: determined, worked out, exchanged, hand-shaked, shared, negotiated, arranged, and coordinated, based on at least one of: a standard, a protocol, an established protocol, an interoperable protocol, a specification, a requirement, a qualification requirement, the qualification criterion, a protocol agreed by the Type 1 device and the Type 2 device, a timing agreed by the Type 1 device and the Type 2 device, a timing based on at least one of: the standard and the protocol, a strategy, a time table, a user setting, a user request, a control by a server, the Type 1 device, the Type 2 device, another device, the server, a control signal communicated between two of: the Type 1 device, the Type 2 device, the another device, the server, another Type 1 device, another Type 2 device, a situation, a condition of the wireless multipath channel, another criterion, and another mechanism.

Clause B32: The method of the qualified wireless system of clause B24: wherein the first part of the wireless signal is received by the Type 2 device using at least one of: the first carrier frequency, the first channel, a third antenna, and a third group of antennas, of the Type 2 device, wherein the second part of the wireless signal is received by the second Type 2 device using at least one of: the second carrier frequency, the second channel, a fourth antenna, and a fourth group of antennas, of the second Type 2 device.

Clause B33: The method of the qualified wireless system of clause B1, further comprising: transmitting a second wireless signal from a second Type 1 device to the Type 2 device; receiving the wireless signal by the Type 2 device using at least one of: a first carrier frequency, a first channel, a first antenna, and a first group of antennas, of the Type 2 device; and receiving the second wireless signal by the Type 2 device using at least one of: a second carrier frequency, a second channel, a second antenna, and a second group of antennas, of the Type 2 device.

Clause B34: The method of the qualified wireless system of clause B33: wherein the first wireless signal and the second wireless signal are transmitted by the Type 1 device and the second Type 1 device respectively in at least one of the following manner: coordinated, un-coordinated, independent, together, un-separated, separated, simultaneous, contemporaneous, non-simultaneous, at the same time, at different time, synchronized, and non-synchronized.

Clause B35: The method of the qualified wireless system of clause B31: wherein the first carrier frequency and the second carrier frequency are at least one of: the same and different.

Clause B36: The method of the qualified wireless system of clause B31: wherein the first channel and the second channel are at least one of: the same channel, overlapping channels, and different channels.

Clause B37: The method of the qualified wireless system of clause B31: wherein the first antenna and the second antenna of the Type 2 device are at least one of: the same antenna, and different antennas.

Clause B38: The method of the qualified wireless system of clause B31: wherein the first group of antennas and the second group of antennas of the Type 2 device are at least one of: the same group of antennas, overlapping groups of antennas, and disjoint groups of antennas.

Clause B39: The method of the qualified wireless system of clause B31: wherein at least one of: a first characteristics of the wireless signal and a second characteristics of the second wireless signal is at least one of: determined, worked out, exchanged, hand-shaked, shared, negotiated, arranged, and coordinated, among at least two of: the Type 1 device, the second Type 1 device, the Type 2 device, and another device.

Clause B40: The method of the qualified wireless system of clause B39: wherein at least one of: the first characteristics of the wireless signal and the second characteristics of the second wireless signal is at least one of: determined, worked out, exchanged, hand-shaked, shared, negotiated, arranged, and coordinated, based on at least one of: a standard, a protocol, an established protocol, an interoperable protocol, a specification, a requirement, a qualification requirement, the qualification criterion, a protocol agreed by the Type 1 device and the Type 2 device, a timing agreed by the Type 1 device and the Type 2 device, a timing based on at least one of: the standard and the protocol, a strategy, a time table, a user setting, a user request, a control by a server, the Type 1 device, the Type 2 device, another device, the server, a control signal communicated between two of: the Type 1 device, the Type 2 device, the another device, the server, another Type 1 device, another Type 2 device, a situation, a condition of the wireless multipath channel, another criterion, and another mechanism.

Clause B41: The method of the qualified wireless system of clause B1: wherein the wireless signal comprises at least one of: an electromagnetic (EM) wave, a radio frequency (RF) signal, a RF transmission, a RF signal transmitted by one or more transmitting antennas, a RF signal received by one or more receiving antennas, a RF signal repeated by a RF repeater, a RF signal retransmitted by a RF repeater, a 800/900MHz signal, a 1.8/1.9 GHz signal, a 2.4GHz signal, a 5GHz signal, a 6GHz signal, a 24GHz signal, a 76-81GHz signal, a 28GHz signal, a 60GHz signal, a 122GHz signal, a 244GHz signal, a microwave signal, an infrared signal, a light signal, an ultraviolet signal, an audio signal, an OFDM signal, a CDMA signal, a FDMA signal, a TDMA signal, an OFDMA signal, a MIMO signal, a MU-MIMO signal, a QAM signal, a 4-QAM, a 8-QAM, a 16-QAM, a 32-QAM, a 64-QAM, a 128-QAM, a 256-QAM, a 512-QAM, a 1024-QAM, a 2048-QAM, a 4096-QAM, a 8192-QAM, a 16384-QAM, a 32768-QAM, a 65536-QAM, a WiFi signal, an IEEE 802 compliant signal, an IEEE 802.11 signal, an 802.15 signal, an IEEE 802.16 signal, a standard compliant signal, a wireless local area network (WLAN) signal, a Zigbee signal, a Bluetooth signal, an RFID signal, a cellular network signal, a 3GPP compliant signal, a 3G/4G/LTE/5G/6G/7G/8G signal, a cellular communication signal, an RF signal that uses an ISM band, an RF signal that uses an unlicensed band, an RF signal that uses a licensed band, a GPS signal, a baseband signal, a bandlimited signal, an ultra-wide band (UWB) signal, a wireless standard-compliant signal, a frequency-hopping signal, a burst signal, a train of signals, a steady stream of signals, a stream of signals with irregular timings, a stream of signals with regular timings, a null signal, a protocol signal, a protocol compliant signal, a data signal, a control signal, a beacon signal, a pilot signal, a probe signal, an excitation signal, an illumination signal, a reference signal, a training signal, a synchronization signal, a request signal, a enquiry signal, a response signal, an acknowledgement signal, a downlink signal, an uplink signal, a unicast signal, a multicast signal, a broadcast signal, a pulsed signal, a signal burst, a motion probe signal, a motion detection signal, a motion sensing signal, a line-of-sight (LOS) signal, a non-line-of-sight (NLOS) signal, a combination of signals, a mixture of signals, a succession of signals, a series of signals, and another wireless signal.

Clause B42: The method of the qualified wireless system of clause B41: wherein the wireless signal comprises at least one of: a response to a second wireless signal transmitted from at least one of: the Type 2 device, another Type 1 device, another Type 2 device, and another wireless device, to the Type 1 device, a response to at least one of: a request signal, a query signal, a probe-request signal, a command signal, a control signal, a data signal, a non-wireless signal, an electronic signal, and another signal, received by the Type 1 device, an acknowledgement to the another wireless signal, a protocol signal, a handshake signal, an enquiry signal, a request signal, a query signal, a probe-request signal, a command signal, a control signal, a data signal, a response signal, a probe response signal, an acknowledgement signal, a reply signal, a beacon signal, a pilot signal, a probe signal, a sounding signal, a broadcast signal, a train of signals, and a part of a handshake between the Type 1 device and the Type 2 device.

Clause B43: The method of the qualified wireless system of clause B42, further comprising: wherein the wireless signal comprises a series of sounding signals, controlling at least one of: a timing, an appearance, a generation, a repetition, a repeated occurrence, a regular occurrence, a cyclic occurrence, a periodic occurrence, an occurrence with a regular interval, a sounding interval, a sounding period, a sounding rate, a sounding timing, and another aspect, of the sounding signals of the wireless signal based on the second wireless signal.

Clause B44: The method of the qualified wireless system of clause B43, further comprising: wherein the second wireless signal comprises a series of probe-request signals, wherein each sounding signal is a probe-response signal to a respective probe-request signal of the second wireless signal, controlling at least one of: the timing, the appearance, the generation, the repetition, the repeated occurrence, the regular occurrence, the cyclic occurrence, the periodic occurrence, the occurrence with a regular interval, the sounding interval, the sounding period, the sounding rate, the sounding timing, and the another aspect, of the sounding signals of the wireless signal by controlling at least one of: a timing, an appearance, a generation, a repetition, a repeated occurrence, a regular occurrence, a cyclic occurrence, a periodic occurrence, an occurrence with a regular interval, a sounding interval, a sounding period, a sounding rate, a sounding timing, and another aspect, of the probe-request signals of the second wireless signal.

Clause B45: The method of the qualified wireless system of clause B1, further comprising: wherein the wireless signal is at least one of: a response, a reply, an acknowledgement, and a handshake, to a second wireless signal, controlling a characteristics of the wireless signal by controlling corresponding characteristics of the second wireless signal.

Clause B46: The method of the qualified wireless system of clause B42, further comprising: controlling at least one timing of the wireless signal by controlling at least one timing of the second wireless signal.

Clause B47: The method of the qualified wireless system of clause B42, further comprising: controlling at least one rate of the wireless signal by controlling at least one corresponding rate of the second wireless signal.

Clause B48: The method of the qualified wireless system of clause B1: wherein the wireless signal comprises a series of probe signals (or sounding signal or beacon signal or pilot signal), wherein each CI of the TSCI is obtained based on a corresponding probe signal.

Clause B49: The method of the qualified wireless system of clause B43: wherein the series of probe signals comprise at least one of: a steady stream of probe signals, a locally-steady stream of probe signals, a locally-absence of probe signals, a momentarily-steady stream of probe signals, a momentary absence of probe signals, a burst of probe signals, a pause of probe signals, a first steady stream (or wave, or train, or succession) of probe signals associated with a first rate, a second steady stream (or wave, or train, or succession) of probe signals associated with a second rate, a probe signal associated with a target transmission time, a probe signal transmitted by the Type 1 device at the target transmission time, a late probe signal, a probe signal transmitted after the target transmission time, a late probe signal that is late due to congestion of the wireless multipath channel, a late probe signal that is late because the wireless multipath channel is used by another device, a lost probe signal, a probe signal not received successfully by the Type 2 device, a protocol signal, a data signal, a control signal, a beacon signal, a pilot signal, an excitation signal, an illumination signal, a reference signal, a training signal, a synchronization signal, a handshake signal, a request signal, an enquiry signal, a response signal, a probe request signal, a probe enquiry signal, a probe response signal, an acknowledgement signal, a synchronization signal, a training signal, a reference signal, a unicast signal, a multi-cast signal, a broadcast signal, an uplink signal, an downlink signal, a pulsed signal, a signal burst, a data frame, a control frame, a beacon frame, a pilot frame, a beacon frame in a primary channel, a probe request frame, a probe response frame, a unicast frame, a multicast frame, a broadcast frame, a frame with a header, a frame with a preamble, a frame with a data payload, an IEEE 802.11 frame, a FHSS frame, a DSSS frame, an OFDM frame, a HR-DSSS frame, an ERP-OFDM frame, a DSSS-OFDM frame, an ERP-PBCC frame, an HT-OFDM frame, a VHT-OFDM frame, a HE-OFDM frame, a DMG frame, an EDMG frame, a TVHT frame, an SIG frame, a WUR frame, an IR frame, an IEEE 802.11 MAC protocol data unit (MPDU), an IEEE 802.11 PHY protocol data unit (PPDU), an IEEE 802.11 PLCP Service Data Unit (PSDU), and another frame.

Clause B50: The method of the qualified wireless system of clause B43: wherein a timing is associated with at least one of: the series of probe signals, the TSCI, the transmission of the series of probe signals by the Type 1 device, the reception of the series of probe signals by the Type 2 device, at least one of: a channel condition, a channel state, a channel usage information of the wireless multipath channel, and at least one of: a condition and a state, of the TSCI; wherein the timing comprises at least one of: a timing associated with the transmission of a probe signal from the Type 1 device, a timing associated with the reception of the probe signal by the Type 2 device, a timing associated with the transmission of the series of probe signals from the Type 1 device, a timing associated with the reception of the series of probe signals by the Type 2 device, a timing associated with the task, a timing associated with a requirement of the task, a timing associated with a user requirement of the task, a timing required for the task to achieve a performance level, a timing associated with a requirement of the task regarding at least one of: a frequency, a period, a starting time, an ending time, a pause, a stand-by, a power-down, a sleep, a power-up, a wake-up, an alarm, an alert, a notification, a schedule, a plan, a time table, a control, a signaling, a request, an enquiry, an acknowledgement, a response, an event, a triggering event, a triggering condition, a triggering situation, and another timing feature, a timing associated with at least one of: a request, an enquiry, a response, and an acknowledgement, based on the task, a timing associated with a control signal sent between at least two of: the Type 1 device, the Type 2 device and another device, a change time, a state transition time, a frequency, a period, a starting time, a stopping time, a pause time, a stand-by time, a power-down time, a sleep time, a power-up time, a wake-up time, an alarm time, an alert time, a notification time, a scheduled time, a planned time, a time table, a control time, a signaling time, a request time, an enquiry time, an acknowledgement time, a response time, an event time, a triggering time, a condition/situation to at least one of: change, remain unchanged, enter a state, change state, remain in a state, start, stop, pause, stand by, detect a event, respond to an event, trigger, power down, sleep, power up, wake up, alert, alarm, notify, schedule, set up a timed event, and perform another action, a time period, a duration, a period based on the task, a regular period, a high alert period, a low alert period, more than one related timings, and a timing associated with another timing associated with at least one of: another wireless signal, another time series of probe signal, and another TSCI.

Clause B51: The method of the qualified wireless system of clause B43, further comprising: signaling between at least two of: the Type 1 device, the Type 2 device, and another device, a timing of at least one of: the series of probe signals, the TSCI, the task, and a user requirement of the task.

Clause B52: The method of the qualified wireless system of clause B43, further comprising: signaling an information of at least one of: the series of probe signals, the TSCI, the task, and a user requirement of the task, between at least two of: the Type 1 device, the Type 2 device, and another device.

Clause B53: The method of the qualified wireless system of clause B47, further comprising: signaling an information of at least one of: the series of probe signals, the TSCI, the task, and a user requirement of the task, between the Type 1 device and the Type 2 device using the wireless signal.

Clause B54: The method of the qualified wireless system of clause B43, further comprising: signaling a requirement of at least one of: the series of probe signals, the TSCI, the task, and a user requirement of the task, between at least two of: the Type 1 device, the Type 2 device, and another device.

Clause B55: The method of the qualified wireless system of clause B49, further comprising: signaling a capability in relation to the requirement of at least one of: the series of probe signals, the TSCI, the task, and a user requirement of the task, between at least two of: the Type 1 device, the Type 2 device, and another device.

Clause B56: The method of the qualified wireless system of clause B43, further comprising: negotiating a timing of at least one of: the series of probe signals, the TSCI, the task, and a user requirement of the task, between at least two of: the Type 1 device, the Type 2 device, and another device.

Clause B57: The method of the qualified wireless system of clause B43, further comprising: coordinating a timing of at least one of: the series of probe signals, the TSCI, the task, and a user requirement of the task, between at least two of: the Type 1 device, the Type 2 device, and another device.

Clause B58: The method of the qualified wireless system of clause B43, further comprising: determining a timing of at least one of: the series of probe signals, the TSCI, the task, and a user requirement of the task, between at least two of: the Type 1 device, the Type 2 device, and another device.

Clause B59: The method of the qualified wireless system of clause B43, further comprising: signaling an information regarding a timing of at least one of: the series of probe signals, the TSCI, the task, and a user requirement of the task, between at least two of: the Type 1 device, the Type 2 device, and another device.

Clause B60: The method of the qualified wireless system of clause B43, further comprising: signaling a capability regarding a timing of at least one of: the series of probe signals, the TSCI, the task, and a user requirement of the task, between at least two of: the Type 1 device, the Type 2 device, and another device.

Clause B61: The method of the qualified wireless system of clause B43, further comprising: negotiating an information regarding a timing of at least one of: the series of probe signals, the TSCI, the task, and a user requirement of the task, between at least two of: the Type 1 device, the Type 2 device, and another device.

Clause B62: The method of the qualified wireless system of clause B43, further comprising: coordinating an information of a timing of at least one of: the series of probe signals, the TSCI, the task, and a user requirement of the task, between at least two of: the Type 1 device, the Type 2 device, and another device.

Clause B63: The method of the qualified wireless system of clause B43, further comprising: determining an information of a timing of at least one of: the series of probe signals, the TSCI, the task, and a user requirement of the task, between at least two of: the Type 1 device, the Type 2 device, and another device.

Clause B64: The method of the qualified wireless system of clause B43, further comprising: computing a statistics associated with at least one of: the series of probe signals, the TSCI obtained based on the series of probe signals, the task performed based on the TSCI, and a user requirement of the task; wherein the statistics comprises at least one of: a frequency, a rate, a sounding rate, a sounding rate associated with the task, a period, a period associated with the task, an accuracy, a regularity, a consistency, an irregularity, a deviation, an error, a hit rate, a miss rate, a detection, a false alarm, a false positive, a mean, a variance, a high order statistics, a timing accuracy, a timing regularity, a frequency accuracy, a frequency regularity, a timing irregularity, a frequency irregularity, a deviation, a frequency deviation, a timing deviation, a performance, a performance measure, a performance statistics, a time window, a current statistics associated with a current time window, a current frequency, a current rate, and a current period.

Clause B65: The method of the qualified wireless system of clause B59, further comprising: signaling the statistics between at least two of: the Type 1 device, the Type 2 device, and another device.

Clause B66: The method of the qualified wireless system of clause B59, further comprising: negotiating the statistics between at least two of: the Type 1 device, the Type 2 device, and another device.

Clause B67: The method of the qualified wireless system of clause B59, further comprising: negotiating settings of at least one of: the Type 1 device, the Type 2 device, and another device, to achieve a requirement related to at least one of: the statistics, the task and a user requirement of the task.

Clause B68: The method of the qualified wireless system of clause B59, further comprising: coordinating at least two of: the Type 1 device, the Type 2 device, and another device, to achieve a performance related to at least one of: the statistics, the task and a user requirement of the task.

Clause B69: The method of the qualified wireless system of clause B59, further comprising: determining the statistics between at least two of: the Type 1 device, the Type 2 device and another device.

Clause B70: The method of the qualified wireless system of clause B1: checking whether the TSCI is suitable for the task.

Clause B71: The method of the qualified wireless system of clause B65, further comprising: wherein the wireless signal comprises a series of probe signals, obtaining an information of at least one of: the series of probe signals, and the TSCI.

Clause B72: The method of the qualified wireless system of clause B1: wherein the information to comprise at least one of: at least one of: an attribute, a size, a dimension, a cardinality, an amount of components, and a count of components of a CI, associated with at least one of: the TSCI, and a CI of the TSCI, at least one of: a characteristics, a property, a feature, a function, a data type, a precision level, a data structure, an amplitude, a phase, and a power, associated with at least one of: the TSCI, a CI of the TSCI, and a component of the CI, at least one of: a time stamp, a timing, a time duration, a sounding rate, a sounding period, a sounding timing, a regularity, an accuracy, a consistency, an irregularity, a timing jitter, a variation, a deviation, an error, a channel condition, a noise power, an interference, and another channel information associated with at least one of: the TSCI, a CI of the TSCI, a component of the CI, the wireless signal, a probe signal of the wireless signal, the wireless multipath channel, the Type 1 device and the Type 2 device, at least one of: an amount of transmit antenna, a type of transmit antenna, an information of transmit antenna, an antenna gain, a group of at least one transmit antenna, an information of transmit radio, a hardware, a processor, a hardware accelerator, a memory, a sensor, a module, a software, an operating system, an application software, a firmware, a version of a set of instructions, an update history of the set of instructions, a connectivity, a network neighborhood, a capability, an available computing power, a memory bandwidth, a data transfer capability, a storage capacity, a wake-up timing, an operating condition, a setting, a standard compliance, a battery level, a power setting, a system setting, and a location of the Type 1 device, at least one of: an amount of receive antenna, a type of receive antenna, an information of receive antenna, an antenna gain, a group of at least one receive antenna, an information of receive radio, a hardware, a processor, a hardware accelerator, a memory, a sensor, a module, a software, an operating system, an application software, a firmware, a version of a set of instructions, an update history of the set of instructions, a connectivity, a network neighborhood, a capability, an available computing power, a memory bandwidth, a data transfer capability, a storage capacity, a wake-up timing, an operating condition, a setting, a battery level, a power setting, a system setting, and a location of the Type 2 device, at least one of: an amount of transmit antenna, an amount of receive antenna, a type of transmit antenna, a type of receive antenna, a group of at least one transmit antenna, a group of at least one receiver antenna, a processor, a memory, a software, a firmware, a version of a set of instructions, a update history of the set of instructions, a connectivity, a network neighborhood, a capability, and a location, of a wireless relaying device associated with at least one of: the TSCI, a CI of the TSCI, a component of the CI, the wireless signal, the wireless multipath channel, and the Type 1 device, wherein the wireless signal is relayed by the wireless relaying device during the transmission from the Type 1 device to the Type 2 device, a noise condition associated with at least one of: the TSCI, a CI of the TSCI, a component of the CI, the wireless multipath channel, the Type 1 device and the Type 2 device, a signal-to-noise condition associated with at least one of: the TSCI, a CI of the TSCI, a component of the CI, the wireless signal, the wireless multipath channel, the Type 1 device and the Type 2 device, a bandwidth associated with at least one of: the TSCI, a CI of the TSCI, a component of the CI, the wireless signal, the wireless multipath channel, the Type 1 device and the Type 2 device, an effective bandwidth associated with at least one of: the TSCI, a CI of the TSCI, a component of the CI, the wireless signal, the wireless multipath channel, the Type 1 device and the Type 2 device, a quantity associated with at least one of: the TSCI, and a CI of the TSCI, a type associated with at least one of: the TSCI, and a CI of the TSCI, and another information.

Clause B73: The method of the qualified wireless system of clause B1: obtaining the TSCI or an information of the TSCI based on at least one of: a primitive, a service primitive, a PHY layer primitive, a MAC layer primitive, an instructive command, a set of configurations, a set of instructions, a firmware, a firmware driver, a firmware call, a software, a software driver, a software call, an operating system (OS), an OS driver, an OS call, a system call, a firmware interface, a function call, a software interface, at least one of: a parameter, a data field, an input data and an output data of one of the above, a collection of at least one of: primitives, instructive commands, configurations, instructions, firmware, software, firmware drivers, software drivers, and OS drivers, any of the above that provide/perform/achieve low-level control of at least one of: a device, a hardware of the device, and an IC of the device, any of the above of at least one of: the Type 1 device, the IC of the Type 1 device, the Type 2 device, the IC of the Type 2 device, another device and an IC of the another device, a format of the TSCI or the information of the TSCI, a representation of the TSCI or the information of the TSCI, a compression scheme for the TSCI or the information of the TSCI, an encryption scheme for the TSCI or the information of the TSCI, a file format or storage scheme of the TSCI or the information of the TSCI, a transmission format/scheme, or streaming format/scheme of the TSCI or the information of the TSCI, a requirement based on the task comprising: a CI precision requirement, a CI time stamp requirement, a timing requirement of the TSCI or the wireless signal, a timing accuracy requirement of the TSCI or the wireless signal, a timing regularity of the TSCI or the wireless signal, a sounding rate of the TSCI or the wireless signal, an transmit antenna and receive antenna requirement, a bandwidth associated with the TSCI or the wireless signal, an effective bandwidth associated with the TSCI or the wireless signal, a quantity of TSCI, the qualification criterion, a qualification of at least one of: the TSCI, the Type 1 device, the IC of the Type 1 device, the Type 2 device, the IC of the Type 2 device, and another requirement.

Clause B74: The method of the qualified wireless system of clause B1: comparing a characteristics of the TSCI with a requirement of the task.

Clause B75: The method of the qualified wireless system of clause B1: making a request for the TSCI based on the task.

Clause B76: The method of the qualified wireless system of clause B1: making a request for the TSCI for the task.

Clause B77: The method of the qualified wireless system of clause B7: making a request for the TSCI with a requirement. (precision, timing, sounding rate)

Clause B78: The method of the qualified wireless system of clause B12: making a request for the particular TSCI in a particular representation.

Clause B79: The method of the qualified wireless system of clause B12: making a request for the particular TSCI with a particular precision.

Clause B80: The method of the qualified wireless system of clause B1: wherein there are a plurality of pairs of Type 1 device and Type 2 device in the venue, each respective Type 2 device receiving a respective wireless signal asynchronously from the respective Type 1 device and obtaining asynchronously a respective TSCI; wherein more than one pairs share a common Type 2 device.

Clause B81: The method of the qualified wireless system of clause B6: wherein the common Type 2 device is active alternately in the more than one pairs.

Clause B82: The method of the qualified wireless system of clause B7: wherein the common Type 2 device is active alternately in the same wireless channel in the more than one pairs.

Clause B83: The method of the qualified wireless system of clause B6: wherein the common Type 2 device is active simultaneously or contemporaneously in the more than one pairs.

Clause B84: The method of the qualified wireless system of clause B9: wherein the common Type 2 device is active simultaneously or contemporaneously in the same wireless channel in the more than one pairs.

Clause B85: The method of the qualified wireless system of clause B6, further comprising: making a plurality of TSCI individually available, wherein each respective TSCI is obtained based on a respective wireless signal transmitted from a respective Type 1 device to the common Type 2 device.

Clause B86: The method of the qualified wireless system of clause B6: requesting a particular TSCI, wherein the particular TSCI is obtained based on a particular wireless signal transmitted from a particular Type 1 device to the common Type 2 device.

Clause B87: The method of the qualified wireless system of clause B12: making a request for the particular TSCI.

Clause B88: The method of the qualified wireless system of clause B12: making a request for the particular TSCI with a particular requirement. (precision, timing, sounding rate)

Clause B89: The method of the qualified wireless system of clause B12: making a request for the particular TSCI in a particular representation.

Clause B90: The method of the qualified wireless system of clause B12: making a request for the particular TSCI with a particular precision.

Clause B91: The method of the qualified wireless system of clause B12: making the particular TSCI available based on the request.

Clause B92: The method of the qualified wireless system of clause B12: making a software request for the particular TSCI.

Clause B93: The method of the qualified wireless system of clause B12: buffering the particular TSCI.

Clause B94: The method of the qualified wireless system of clause B6: obtaining the particular TSCI.

Clause B95: The method of the qualified wireless system of clause B6: obtaining the particular TSCI .

Clause B96: The method of the qualified wireless system of clause B6, further comprising: performing a first task based on a first TSCI obtained based on a first wireless signal transmitted from a first Type 1 device to the common Type 2 device; and performing a second task based on a second TSCI obtained based on a second wireless signal transmitted from a second Type 1 device to the common Type 2 device.

Clause B97: The method of the qualified wireless system of clause B6, further comprising: making a plurality of TSCI available, wherein each respective TSCI is obtained based on a respective wireless signal transmitted from a respective Type 1 device to the common Type 2 device.

Clause B98: The method of the qualified wireless system of clause B11, further comprising: making the plurality of TSCI available for a joint task.

Clause B99: The method of the qualified wireless system of clause B11, further comprising: requesting the plurality of TSCI available for a joint task.

Clause B100: The method of the qualified wireless system of clause B11, further comprising: performing the joint task jointly based on the plurality of TSCI.

Clause B101: The method of the qualified wireless system of clause B11, further comprising: performing the joint task jointly based on the plurality of TSCI.

Clause B102: The method of the qualified wireless system of clause B6, further comprising: monitoring a motion of an object in the venue jointly based on all TSCI obtained based on the plurality of pairs, each TSCI obtained separately from a respective pair.

Clause B103: The method of the qualified wireless system of clause B6, further comprising: monitoring a motion of an object in the venue individually each based on a TSCI obtained in one of the more than one pairs, monitoring the motion jointly by combining the individual monitoring.

Clause B104: The method of the qualified wireless system of clause B1: wherein there are a plurality of pairs of Type 1 device and Type 2 device in the venue, each respective Type 2 device receiving a respective wireless signal asynchronously from the respective Type 1 device and obtaining asynchronously a respective TSCI; wherein more than one pairs share a common Type 1 device.

Clause B105: The method of the qualified wireless system of clause B7: wherein the common Type 1 device is active alternately in the more than one pairs.

Clause B106: The method of the qualified wireless system of clause B8: wherein the common Type 1 device is active alternately in the same wireless channel in the more than one pairs.

Clause B107: The method of the qualified wireless system of clause B7: wherein the common Type 1 device is active simultaneously or contemporaneously in the more than one pairs.

Clause B108: The method of the qualified wireless system of clause B10: wherein the common Type 1 device is active simultaneously or contemporaneously in the same wireless channel in the more than one pairs.

Clause B109: The method of the qualified wireless system of clause B7: wherein the common Type 1 device broadcasts to more than one Type 2 devices in the more than one pairs, transmitting a common wireless signal to all the more than one Type 2 devices.

Clause B110: The method of the qualified wireless system of clause B12: wherein an information of the broadcast is announced.

Clause B111: The method of the qualified wireless system of clause B12: wherein an information of the broadcast is announced in at least one of: a data frame, a control frame, a beacon frame, a beacon frame in a primary channel, a probe request frame, a probe response frame, an acknowledgement frame, a multicast frame, a broadcast frame, a frame with a header, a FHSS frame, a DSSS frame, an OFDM frame, a HR-DSSS frame, an ERP-OFDM frame, a DSSS-OFDM frame, an ERP-PBCC frame, an HT-OFDM frame, a VHT-OFDM frame, a HE-OFDM frame, a DMG frame, an EDMG frame, a TVHT frame, an SIG frame, a WUR frame, an IR frame, another frame, a server, a cloud server, a local server, a web server, a database, a publicly accessible site, a published site, an announcement channel, a handshake channel, an enquiry channel, a signaling channel, a primary channel, a secondary channel, a side channel, an announcement, a handshake, an enquiry, a signaling, the wireless signal, a past wireless signal, and another channel.

Clause B112: The method of the qualified wireless system of clause B12: wherein the common Type 1 device broadcasts to more than one Type 2 devices in the more than one pairs; wherein the common Type 1 device transmits the common wireless signal to a common destination address; wherein each of the more than one Type 2 devices sets its address as the common destination address to receive the common wireless signal.

Clause B113: The method of the qualified wireless system of clause B15: wherein at least one of: the common destination address, and an information of broadcasting channel associated with the common wireless signal is announced.

Clause B114: The method of the qualified wireless system of clause B12: wherein the common Type 1 device broadcasts to more than one Type 2 devices in the more than one pairs; wherein the common Type 1 device transmits the common wireless signal to a universal destination address; wherein each of the more than one Type 2 devices is configured to respond to the universal destination address and receive the common wireless signal.

Clause B115: The method of the qualified wireless system of clause B12: wherein the common Type 1 device broadcasts to more than one Type 2 devices in the more than one pairs; wherein the common Type 1 device transmits a common wireless signal; wherein each of the more than one Type 2 devices is configured to respond and receive the common wireless signal.

Clause B116: The method of the qualified wireless system of clause B1: wherein an information of the transmitting of the wireless signal from the Type 1 device through the wireless channel is announced, at least in part, in at least one of: a data frame, a control frame, a beacon frame, a beacon frame in a primary channel, a probe request frame, a probe response frame, a multicast frame, a broadcast frame, a frame with a header, a FHSS frame, a DSSS frame, an OFDM frame, a HR-DSSS frame, an ERP-OFDM frame, a DSSS-OFDM frame, an ERP-PBCC frame, an HT-OFDM frame, a VHT-OFDM frame, a HE-OFDM frame, a DMG frame, an EDMG frame, a TVHT frame, an SIG frame, a WUR frame, an IR frame, another frame, a server, a cloud server, a local server, a web server, a database, a publicly accessible site, a published site, an announcement channel, a handshake channel, an enquiry channel, a signaling channel, a primary channel, a secondary channel, a side channel, an announcement, a handshake, an enquiry, a signaling, the wireless signal, a past wireless signal, and another channel.

Clause B117: The method of the qualified wireless system of clause B1, further comprising: exchanging an information between the Type 1 device and the Type 2 device.

Clause B118: The method of the qualified wireless system of clause B20, further comprising: exchanging the information between the Type 1 device and the Type 2 device using the wireless multipath channel.

Clause B119: The method of the qualified wireless system of clause B20, further comprising: exchanging the information based on a standardized protocol.

Clause B120: The method of the qualified wireless system of clause B20, further comprising: determining an agreed set of settings between the Type 1 device and the Type 2 device.

Clause B121: The method of the qualified wireless system of clause B23, further comprising: determining the agreed set of settings between the Type 1 device and the Type 2 device for a task.

Clause B122: The method of the qualified wireless system of clause B24, further comprising: determining that the agreed set of settings satisfy a requirement of the task.

Clause B123: The method of the qualified wireless system of clause B24: wherein the task is associated with the venue.

Clause B124: The method of the qualified wireless system of clause B23, further comprising: determining the agreed set of settings between the Type 1 device and the Type 2 device for monitoring a motion in the venue.

Clause B125: The method of the qualified wireless system of clause B27, further comprising: determining that the agreed set of settings satisfy a requirement for monitoring the motion in the venue.

Clause B126: The method of the qualified wireless system of clause B27: wherein the motion is a motion of an object in the venue.

Clause B127: The method of the qualified wireless system of clause B23: wherein the agreed set of settings is a set of settings for the transmission of the wireless signal from the Type 1 device to the Type 2 device.

Clause B128: The method of the qualified wireless system of clause B23: determining the agreed set of settings between the Type 1 device and the Type 2 device based on a standardized protocol.

Clause B129: The method of the qualified wireless system of clause B23, further comprising: transmitting the wireless signal based on the agreed set of settings.

Clause B130: The method of the qualified wireless system of clause B23, further comprising: broadcasting the wireless signal based on the agreed set of settings.

Clause B131: The method of the qualified wireless system of clause B20, further comprising: determining that no settings of the Type 1 device and the Type 2 device can satisfy a requirement of a task.

Clause B132: The method of the qualified wireless system of clause B34: determining based on a standardized protocol.

Clause B133: The method of the qualified wireless system of clause B34: wherein the task is a monitoring task related to the venue.

Clause B134: The method of the qualified wireless system of clause B34, further comprising: wherein the task is to monitor a motion of an object in the venue.

Clause B135: The method of the qualified wireless system of clause B20, further comprising: exchanging the information between the Type 1 device and the Type 2 device before the transmitting of the wireless signal.

Clause B136: The method of the qualified wireless system of clause B20, further comprising: exchanging the information between the Type 1 device and the Type 2 device during the transmitting of the wireless signal.

Clause B137: The method of the qualified wireless system of clause B20, further comprising: exchanging the information between the Type 1 device and the Type 2 device after the transmitting of the wireless signal.

Clause B138: The method of the qualified wireless system of clause B20: wherein the information comprises at least one of: a meta-data, a device information, a manufacturing information, a model information, a version information, a registration information, an identification information, a classification, a category, a grouping, a restriction, a usage information, a service provider information, a service information, a sales information, a logistical information, a system information, a companion system information, a capability information, a power information, a computation information, a processor information, a storage information, a supported system information, a supported task information, a performance requirement, a carrier frequency, a frequency information, a timing information, an antenna information, a location-based information, a software or firmware information, an updating information, a hardware information, a component information, a network information, a wireless network information, an address information, an access information, a security protection information, an encryption information, an internet information, and another information, of the Type 1 device, the Type 2 device or a device communicatively coupled with the Type 1 device or the Type 2 device.

Clause B139: The method of the qualified wireless system of clause B20, further comprising: exchanging a portion of the information using at least one of: a data stream, a point-to-point stream, a multicast stream, a broadcast stream, a file, a header of a file, a field of a file, a name of a file, a meta-data of a file, a directory of a file, a file based on a standardized format, a database, a database of a storage network, a data network, a database of a server, a database of a cloud server, a database of a local server, a data packet, a data record, a data structure, a data structure based on a standard, a field of a database, a field of a data record, a field of a data structure, a field of a data packet, a server, a cloud server, a local server, a web server, a database, a publicly accessible site, a published site, a frame, a frame of a data stream, a frame of a file, a preamble of a frame, a header of a frame, a payload of a frame, a flag of a frame, a field of a frame, a field of a preamble, a field of a header, a control field, a signaling field, a protocol field, a data field, a payload field, a signal field, a service field, a timing field, a duration field, an extension field, a trailer field, a data frame, a control frame, a beacon frame, a beacon frame in a primary channel, a beacon frame in a secondary channel, a probe request frame, a probe response frame, a multicast frame, a broadcast frame, a frame with a header, an enquiry frame, a response frame, an acknowledgement frame, a FHSS frame, a DSSS frame, an OFDM frame, a HR-DSSS frame, an ERP-OFDM frame, a DSSS-OFDM frame, an ERP-PBCC frame, an HT-OFDM frame, a VHT-OFDM frame, a HE-OFDM frame, a DMG frame, an EDMG frame, a TVHT frame, an SIG frame, a WUR frame, an IR frame, another frame, one or more radio frequency (RF) band with a bandwidth of B MHz where B is between 0.01 and 100000, a data rate less than or equal to the rate at which the wireless signal is being transmitted from the Type 1 device to the Type 2 device, a short preamble of a frame, a long preamble of a frame, a preamble sent at a data rate of A Mbps where A is between 0.01 and 100000, a preamble sent at a lowest supported rate in a physical layer of the wireless multipath channel, a frame or protocol compliant to at least one of: a wireless protocol, a wireless network standard, a wireless mess network standard, a wireless communication standard, WiFi, WiMax, WiGiG, WLAN, WMAN, an IEEE 802 standard, an IEEE 802.11 standard, an IEEE 802.15 standard, an 802.16 standard, a mobile communication standard, a cellular communication standard, a mobile network standard, a 3GPP standard, GSM, EDGE, WCDMA, LTE, a 2G/3G/4G/5G/6G/7G/8G related standard/system/protocol, a standard or a protocol comprising at least one of: time-division multiple access (TDMA), frequency-division multiple access (FDMA), code-division multiple access (CDMA), orthogonal frequency-division multiplexing (OFDM), multiple-input-multiple-output (MIMO), carrier aggregation (CA), orthogonal frequency-division multiple access (OFDMA), multiuser MIMO (MU-MIMO), beamforming (BF), transmit beamforming (TxBF), receiver beamforming (RxBF), target wake time (TWT), spatial streams (SS), BSS coloring, quadrature amplitude modulation (QAM), fast Fourier transform (FFT), the channel information (CI), channel state information (CSI), CSI associated with a transmitting antenna and a receiving antenna, channel impulse response (CIR), channel frequency response (CFR), compressed CSI, and uncompressed CSI, a wireless communication protocol that computes CSI of the wireless multipath channel, a defacto standard, an industry standard, a national standard, an international standard, a Bluetooth standard, BLE, a UWB standard, NFC, ZigBee, and another standard, an announcement, an announcement channel, a handshake, a handshake channel, an enquiry, an enquiry channel, a signaling, a signaling channel, a primary channel, a secondary channel, a side channel, the wireless signal, a signal exchange before the wireless signal is transmitted, a signal exchange between the Type 1 device and the Type 2 device, a signal exchange between the Type 1 device and a server, a signal exchange between the Type 2 device and a server, and another avenue.

Clause B140: The method of the qualified wireless system of clause B20, further comprising: transmitting the wireless signal based on the exchanged information.

Clause B141: The method of the qualified wireless system of clause B20, further comprising: broadcasting the wireless signal based on the exchanged information.

Clause B 142: The method of the qualified wireless system of clause B 1: wherein there are a plurality of pairs of Type 1 device and Type 2 device in the venue, each respective Type 2 device receivindg a respective wireless signal asynchronously from the respective Type 1 device and obtaining asynchronously a respective TSCI; wherein a device functions as a Type 1 device in a first pair and as a Type 2 device in a second pair.

Clause B143: The method of the qualified wireless system of clause B1, further comprising: receiving the wireless signal based on the IC of the Type 2 device, computing the TSCI based on the IC, obtaining the TSCI from the IC, wherein the qualification criterion for the qualification of the IC of the Type 2 device comprises at least one of: an estimation error requirement of each CI, an estimation error requirement of the TSCI, a precision requirement of each CI, a precision requirement of the TSCI, a representation requirement of the TSCI, an encoding requirement of the TSCI, a real-time requirement of the TSCI, a buffering requirement of the TSCI, a memory requirement of the TSCI, a stability requirement of the TSCI, a temporal consistency requirement of the TSCI, a correlation requirement of the TSCI, an outlier requirement of the TSCI, a deviation requirement of the TSCI, a tail requirement of the TSCI, a percentile requirement of the TSCI, a quantile requirement of the TSCI, a scalability requirement of the TSCI, a indicator to communicate the availability of at least one of: a CI, a group of recent CI, and the whole TSCI, an accuracy requirement of the computation of more than one consecutive CI, making the TSCI available to another set of instructions stored in another memory; wherein the another set of instructions when executed causes another processor to: monitoring a motion of an object in the venue.

Clause B144: The method of the qualified wireless system of clause B1, further comprising: wherein the wireless signal comprises a time series of probe signal (TSPS), wherein a field of a control signal indicates at least one of: a parameter, a destination address, a frequency, a timing, an allowable frequency and an allowable period, of the TSPS.

Clause B145: The method of the qualified wireless system of clause B18, further comprising: wherein the control signal is transmitted by at least one of: the Type 1 device and another device.

Clause B 146: The method of the qualified wireless system of clause B 19, further comprising: wherein the control signal is transmitted in at least one of: a regular manner, a repeated manner, an occasional manner, an on-demand manner, an acknowledgement-to-query manner, and a broadcasting manner.

Clause B 147: The method of the qualified wireless system of clause B1, further comprising: coordinating the Type 1 device and the Type 2 device.

Clause B 148: The method of the qualified wireless system of clause B1, further comprising: signaling between the Type 1 device and the Type 2 device.

Clause B 149: The method of the qualified wireless system of clause B1, further comprising: wherein the wireless signal comprises at least one probe signal, wherein the probe signal comprises a frame with at least one of: a preamble field, a header field, a data field, a data frame, a MAC frame, a synchronization field, a training field, a delimiter field, a header field, a signal field, a service field, a length field, an error-control field, and another field, sending the at least one probe signal from the Type 1 device to the Type 2 device.

Clause B 150: The method of the qualified wireless system of clause B1, further comprising: sending a physical layer packet from the Type 1 device to the Type 2 device, wherein the physical layer frame to comprise at least one of: a preamble transmitted in a first modulation at a first data rate, a header transmitted in a second modulation at a second data rate, and a physical layer payload in a third modulation at a third data rate.

Clause B 151: The method of the qualified wireless system of clause B6: wherein at least one of: the preamble, the header and the physical layer payload, contains a field related to wireless sensing.

Clause B 152: The method of the qualified wireless system of clause B6: wherein at least one of: the preamble, the header and the physical layer payload, contains a field related to sensing of the venue based on the TSCI.

Clause B 153: The method of the qualified wireless system of clause B6: wherein at least one of: the preamble, the header and the physical layer payload, contains a field related to the monitoring of an object motion in the venue based on the TSCI.

Clause B 154: The method of the qualified wireless system of clause B5, further comprising: causing the Type 2 device to at least one of: make the TSCI available, signal that the TSCI is available, processed the TSCI, make the processed TSCI available, signal that the processed TSCI is available, compute an analytics based on the TSCI, make the analytics available, signal that the analytics is available, perform a task based on the analytics, perform a task jointly with at least one of: another Type 1 device and another Type 2 device, and another operation.

Clause B 155: The method of the qualified wireless system of clause B1, further comprising: signaling at least one of: the Type 1 device and the Type 2 device.

Clause B 156: The method of the qualified wireless system of clause B1, further comprising: communicating at least one of: a request, a command, a response, a reply and a signal to at least one of: the Type 1 device and the Type 2 device.

Clause B 157: The method of the qualified wireless system of clause B1, further comprising: causing the Type 1 device to transmit the wireless signal to the Type 2 device.

Clause B 158: The method of the qualified wireless system of clause B4, further comprising: causing the Type 1 device to transmit the wireless signal to the Type 2 device in such a way that the TSCI satisfies the respective qualification criterion.

Clause B 159: The method of the qualified wireless system of clause B1, further comprising: causing the Type 2 device to receive the wireless signal from the Type 1 device.

Clause B 160: The method of the qualified wireless system of clause B6, further comprising: causing the Type 2 device to obtain the TSCI based on the wireless signal.

Clause B 161: The method of the qualified wireless system of clause B7, further comprising: causing the Type 2 device to monitor a motion of an object in the venue based on the TSCI, wherein the wireless multipath channel is impacted by the motion of the object in the venue.

Clause B 162: The method of the qualified wireless system of clause B7, further comprising: requesting the Type 2 device to compute a characteristics of the motion of the object based on the TSCI.

Clause B 163: The method of the qualified wireless system of clause B9, further comprising: requesting the Type 2 device to perform a task based on the characteristics of the motion of the object computed based on the TSCI.

Clause B164: The method of the qualified wireless system of clause B1, further comprising: conducting a negotiation between the Type 1 device and the Type 2 device.

Clause B165: The method of the qualified wireless system of clause B1: wherein the qualification criterion is applied at a time when the venue has no change and no object motion.

Clause B166: The method of the qualified wireless system of clause B1: wherein the respective qualification criterion comprises at least one of: a requirement of the venue, a requirement of the wireless multipath channel, a requirement of the wireless signal, a requirement of signaling between the Type 1 device and the Type 2 device, a requirement of the Type 1 device, a requirement of the Type 2 device, a requirement of the wireless transmitter of the Type 1 device, a requirement of the wireless receiver of the Type 2 device, a requirement of the antennas of the Type 1 device, a requirement of the antennas of the Type 2 device, a requirement of the number of antennas of the Type 1 device, a requirement of the number of antennas of the Type 2 device, a requirement of the placement of the Type 1 device, a requirement of the placement of the Type 2 device, a requirement of the module of the Type 1 device, a requirement of the module of the Type 2 device, a requirement of the IC of the Type 1 device, a requirement of the IC of the Type 2 device, and a type of qualification.

Clause B167: The method of the qualified wireless system of clause B3: wherein the requirement of the venue comprises at least one of: a type-of-venue requirement comprising at least one of: indoor, outdoor, semi-outdoor, underground, house, office, building, warehouse, lab, special testing facility, and another venue type, a type-of-partition requirement comprising at least one of: wall plaster, dry wall, fiberboard, paneling, gypsum, wood, metal, vinyl, stucco, shingle, asphalt, brick, stone, masonry, concrete, cement, tile, ceramic tile, glass, and another partition type, a size-of-venue requirement comprising at least one of: volume, area, width, length, height, depth, thickness, layering, and another venue size, a structure requirement comprising at least one of: furniture, supporting structures, columns, beams, tables, chairs, shelves, cabinets, vehicles, and another structure, a multipath-richness requirement of the venue, a state requirement of the venue, a motion requirement of the venue, a composition requirement of the venue, the venue is not changing at least temporarily, the venue is stationary at least temporarily, the venue is motionless at least temporarily, there is no object moving in the venue at least temporarily, and another venue-related requirement.

Clause B168: The method of the qualified wireless system of clause B3: wherein the requirement of the wireless multipath channel comprises at least one of: a bandwidth requirement comprising of at least one of: 10MHz, 20MHz, 30MHz, 40MHz, 50MHz, 60MHz, 70MHz, 80MHz, 100MHz, 160MHz, 320MHz, and another bandwidth, a carrier frequency requirement based on at least one of: an ISM band centered near at least one of: 6.78MHz, 13.56MHz, 27.12MHz, 40.68MHz, 4.5GHz, 33.93MHz, 915MHz, 2.45GHz, 5GHz, 5.8GHz, 24.125GHz, 61.25GHz, 122.5GHz, and 245GHz, a mobile communication band, a mobile communication channel, 3G, 4G, LTE, 5G, 6G, 7G, a WiFi band, a WiFi channel, and another carrier frequency, a standard compliance requirement comprising at least one of: WLAN, WiFi, a 802.11 standard, a 802.15 standard, a 802.20 standard, a mobile communication standard, a 3GPP standard, 3G, 4G, LTE, 5G, 6G, 7G, 8G, a Bluetooth standard, a standard using OFDM, a standard comprising computation of the CI, and another standard, a protocol requirement, a network requirement, a signaling requirement, a signal handshaking requirement, a multiple access requirement, a channel traffic requirement, a channel availability requirement, a frequency hopping requirement, a data transmission requirement, and another channel-related requirement.

Clause B169: The method of the qualified wireless system of clause B3: wherein the requirement of the wireless signal comprises at least one of: the wireless signal comprising a time series of probe signals (TSPS), a protocol requirement, a network requirement, a signaling requirement, a signal handshaking requirement, a multiple access requirement, a channel traffic requirement, a modulation requirement, a frequency hopping requirement, a data transmission requirement, a transmission power requirement of the TSPS, a probing frequency requirement of the TSPS, a probing timing requirement of the TSPS, a sounding frequency requirement of the TSPS, a sounding timing requirement of the TSPS, a timing requirement of the TSPS, a rapid-firing timing requirement of the TSPS, a pulsating timing requirement of the TSPS, a progressive timing requirement of the TSPS, a time-varying timing requirement of the TSPS, a timing jitter requirement of the TSPS, a broadcasting requirement associated with each probe signal, a signaling requirement associated with each probe signal, a protocol requirement associated with each probe signal,a handshake requirement associated with each probe signal, a requirement of a predecessor signal that triggers the Type 1 device to send a probe signal, a requirement of a predecessor signal to which a probe signal is an acknowledgement in a handshake, a requirement of a predecessor signal to which a probe signal is a reply in a handshake, a data field requirement of a probe signal that causes the obtaining of CI by the Type 2 device, a header field requirement of a probe signal that causes the obtaining of CI by the Type 2 device, a data field requirement of a packet of a probe signal that causes the obtaining of CI by the Type 2 device, a control data field requirement of a probe signal that causes the obtaining of CI by the Type 2 device, and another signal-related requirement.

Clause B170: The method of the qualified wireless system of clause B3: wherein the requirement of the Type 1 device comprises at least one of: a placement requirement, an installation requirement, a processor requirement, a memory requirement, a software requirement, a system requirement, a power requirement, an interface requirement, a transmission requirement, a housing requirement, a signaling requirement, an environment requirement, an antenna-type requirement, an antenna-count requirement, an antenna-gain requirement, an antenna-placement requirement, an antenna-radiation requirement, an antenna-material requirement, an antenna-structure requirement, and another Type 1 device requirement.

Clause B171: The method of the qualified wireless system of clause B3: wherein the requirement of the Type 2 device comprises at least one of: a placement requirement, an installation requirement, a processor requirement, a memory requirement, a software requirement, a system requirement, a power requirement, an interface requirement, a transmission requirement, a housing requirement, a signaling requirement, an environment requirement, an antenna-type requirement, an antenna-count requirement, an antenna-gain requirement, an antenna-placement requirement, an antenna-radiation requirement, an antenna-material requirement, an antenna-structure requirement, and another Type 2 device requirement.

Clause B172: The method of the qualified wireless system of clause B1: wherein a first CI of the TSCI is obtained by the Type 2 device at a first time; wherein a second CI of the TSCI is obtained by the Type 2 device at a second time; wherein the qualification criterion comprises at least one of: the first CI and the second CI are similar, there is little variation between the first CI and the second CI, the first CI is in a neighborhood of the second CI, a similarity score between the first CI and the second CI is greater than a threshold, a variation score between the first CI and the second CI is less than a threshold, a distance score between the first CI and the second CI is less than a threshold, the first CI and a predictor of the first CI based on the second CI are similar, there is little variation between the first CI and the predictor, the first CI is in a neighborhood of the predictor, a similarity score between the first CI and the predictor is greater than a threshold, a variation score between the first CI and the predictor is less than a threshold, a distance score between the first CI and the predictor is less than a threshold, a feature of the first CI and the feature of the second CI are similar, the feature of the first CI is in a neighborhood of the feature of the second CI, a similarity score between the feature of the first CI and the feature of the second CI is greater than a threshold, a variation score between the feature of the first CI and the feature of the second CI is less than a threshold, a distance score between the feature of the first CI and the feature of the second CI is less than a threshold, the feature of the first CI and the feature of the predictor of the first CI based on the second CI are similar, the feature of the first CI is in a neighborhood of the feature of the predictor, a similarity score between the feature of the first CI and the feature of the predictor is greater than a threshold, a variation score between the feature of the first CI and the feature of the predictor is less than a threshold, a distance score between the feature of the first CI and the feature of the predictor is less than a threshold, the first time and the second time are similar, the first time is in a neighborhood of the second time, the first CI and the second CI are neighboring CI in the TSCI, the first time and the second time are neighboring sampling time of the Type 2 device, a difference between the first time and the second time is less than a threshold, and another qualification criterion.

Clause B173: The method of the qualified wireless system of clause B1: wherein the wireless signal comprises a time series of probe signals (TSPS); wherein a first probe signal transmitted by the Type 1 device at a first transmitting time is associated with a first CI of the TSCI obtained by the Type 2 device at a first time; wherein a second probe signal transmitted by the Type 1 device at a second transmitting time is associated with a second CI of the TSCI obtained by the Type 2 device at a second time; wherein the qualification criterion comprises at least one of: the first transmitting time and the second transmitting time are similar, there is little variation between the first transmitting time and the second transmitting time, the first transmitting time is in a neighborhood of the second transmitting time, a first probe signal and the second probe signal are neighboring probe signals in the time series of probe signal, the first transmitting time and the second transmitting time are neighboring transmitting time of the Type 1 device, a difference between the first transmitting time and the second transmitting time is less than a threshold, two of the first transmitting time, the first time, the second transmitting time and the second time are similar, there is little variation between the two times, the two times are in a neighborhood of each other, a difference between the two times is less than a threshold, and another qualification criterion.

Clause B174: The method of the qualified wireless system of clause B1: wherein there are a plurality of CI of the TSCI in a period of time; wherein the qualification criterion comprises at least one of: the plurality of CI are similar, the plurality of CI are consistent, there is little variation among the plurality of CI, there is little outlier among the plurality of CI, all of the plurality of CI are in a neighborhood, all of the plurality of CI are in a dense cluster, a similarity score of the plurality of CI is greater than a threshold, a variation score of the plurality of CI is less than a threshold, a distance score of the plurality of CI is less than a threshold, a similarity score of the plurality of CI is greater than a threshold for at least a percentage of the period of time, a variation score of the plurality of CI is less than a threshold for at least a percentage of the period of time, a distance score of the plurality of CI is less than a threshold for at least a percentage of the period of time, a similarity score of the plurality of CI is less than a threshold for at most a percentage of the period of time, a variation score of the plurality of CI is greater than a threshold for at most a percentage of the period of time, a distance score of the plurality of CI is greater than than a threshold for at most a percentage of the period of time, a mean of a pairwise similarity score of the plurality of CI is greater than a threshold, a median of the pairwise similarity score of the plurality of CI is greater than a threshold, a mode of the pairwise similarity score of the plurality of CI is greater than a threshold, a percentile of the pairwise similarity score of the plurality of CI is greater than a threshold, a smallest pairwise similarity score of the plurality of CI is greater than a threshold, a weighted mean of the pairwise similarity score of the plurality of CI is greater than a threshold, a variance of the pairwise similarity score of the plurality of CI is less than a threshold, a mean of a pairwise similarity score of the plurality of CI from a centroid of the plurality of CI is greater than a threshold, a median of the pairwise similarity score of the plurality of CI from a centroid is greater than a threshold, a mode of the pairwise similarity score of the plurality of CI from a centroid is greater than a threshold, a percentile of the pairwise similarity score of the plurality of CI from a centroid is greater than a threshold, a smallest pairwise similarity score of the plurality of CI from a centroid is greater than a threshold, a weighted mean of the pairwise similarity score of the plurality of CI from a centroid is greater than a threshold, a variance of the pairwise similarity score of the plurality of CI from a centroid is less than a threshold, a variance of the plurality of CI is less than a threshold, a mean distance of the plurality of CI from a centroid is less than a threshold, a central moment of the plurality of CI is less than a threshold, a kurtosis of the plurality of CI is less than a threshold, a tailedness measure of the plurality of CI is less than a threshold, an outlier measure of the plurality of CI is less than a threshold, and another qualification criterion.

Clause B175: The method of the qualified wireless system of clause B1: wherein there are a plurality of CI of the TSCI in a period of time; wherein the qualification criterion comprises at least one of: a feature of each of the plurality of CI is similar, the feature of each of the plurality of CI is consistent, there is little variation of the feature among the plurality of CI, there is little outlier of the feature among the plurality of CI, the features of the plurality of CI are in a neighborhood, the features of the plurality of CI are in a dense cluster, a similarity score of the features of the plurality of CI is greater than a threshold, a variation score of the features of the plurality of CI is less than a threshold, a distance score of the features of the plurality of CI is less than a threshold, a similarity score of the features of the plurality of CI is greater than a threshold for at least a percentage of the period of time, a variation score of the features of the plurality of CI is less than a threshold for at least a percentage of the period of time, a distance score of the features of the plurality of CI is less than a threshold for at least a percentage of the period of time, a similarity score of the features of the plurality of CI is less than a threshold for at most a percentage of the period of time, a variation score of the features of the plurality of CI is greater than a threshold for at most a percentage of the period of time, a distance score of the features of the plurality of CI is greater than than a threshold for at most a percentage of the period of time, a mean of a pairwise similarity score of the features of the plurality of CI is greater than a threshold, a median of the pairwise similarity score of the features of the plurality of CI is greater than a threshold, a mode of the pairwise similarity score of the features of the plurality of CI is greater than a threshold, a percentile of the pairwise similarity score of the features of the plurality of CI is greater than a threshold, a smallest pairwise similarity score of the features of the plurality of CI is greater than a threshold, a weighted mean of the pairwise similarity score of the features of the plurality of CI is greater than a threshold, a variance of the pairwise similarity score of the features of the plurality of CI is less than a threshold, a mean of a pairwise similarity score of the features of the plurality of CI from a centroid of the plurality of CI is greater than a threshold, a median of the pairwise similarity score of the features of the plurality of CI from a centroid is greater than a threshold, a mode of the pairwise similarity score of the features of the plurality of CI from a centroid is greater than a threshold, a percentile of the pairwise similarity score of the features of the plurality of CI from a centroid is greater than a threshold, a smallest pairwise similarity score of the features of the plurality of CI from a centroid is greater than a threshold, a weighted mean of the pairwise similarity score of the features of the plurality of CI from a centroid is greater than a threshold, a variance of the pairwise similarity score of the features of the plurality of CI from a centroid is less than a threshold, a variance of the features of the plurality of CI is less than a threshold, a mean distance of the features of the plurality of CI from a centroid is less than a threshold, a central moment of the features of the plurality of CI is less than a threshold, a kurtosis of the features of the plurality of CI is less than a threshold, a tailedness measure of the features of the plurality of CI is less than a threshold, an outlier measure of the features of the plurality of CI is less than a threshold, and another qualification criterion.

Clause B176: The method of the qualified wireless system of clause B1: wherein there are a first set of CI in the TSCI in a first period of time; wherein there are a second set of CI in the TSCI in a second period of time; wherein the qualification criterion comprises at least one of: the first set of CI are self-similar, the first set of CI are self-consistent, the second set of CI are self-similar, the second set of CI are self-consistent, the first set of CI and the second set of CI are similar, there is little variation within the first set of CI, there is little variation within the second set of CI, there is little variation between the first set of CI and the second set of CI, there is little outlier within the first set of CI, there is little outlier within the second set of CI, there is little outlier within the first set of CI and the second set of CI combined, all of the first set of CI are in a neighborhood, all of the second set of CI are in a neighborhood, all of the first set of CI and the second set of CI are in a neighborhood, all of the first set of CI are in a dense cluster, all of the second set of CI are in a dense cluster, all of the first set of CI and second set of CI are in a dense cluster, a similarity score of the first set of CI is greater than a threshold, a similarity score of the second set of CI is greater than a threshold, a similarity score of the first set of CI and the second set of CI is greater than a threshold, a variation score of the first set of CI is less than a threshold, a variation score of the second set of CI is less than a threshold, a variation score of the first set of CI and the second set of CI is less than a threshold, a distance score of the first set of CI is less than a threshold, a distance score of the second set of CI is less than a threshold, a distance score of the first set of CI and the second set of CI is less than a threshold, a similarity score of the first set of CI is greater than a threshold for at least a percentage of the period of time, a similarity score of the second set of CI is greater than a threshold for at least a percentage of the period of time, a similarity score of the first set of CI and the second set of CI is greater than a threshold for at least a percentage of the period of time, a variation score of the first set of CI is less than a threshold for at least a percentage of the period of time, a variation score of the second set of CI is less than a threshold for at least a percentage of the period of time, a variation score of the first set of CI and the second set of CI is less than a threshold for at least a percentage of the period of time, a distance score of the first set of CI is less than a threshold for at least a percentage of the period of time, a distance score of the second set of CI is less than a threshold for at least a percentage of the period of time, a distance score of the first set of CI and the second set of CI is less than a threshold for at least a percentage of the period of time, a similarity score of the first set of CI is less than a threshold for at most a percentage of the period of time, a similarity score of the second set of CI is less than a threshold for at most a percentage of the period of time, a similarity score of the first set of CI and the second set of CI is less than a threshold for at most a percentage of the period of time, a variation score of the first set of CI is greater than a threshold for at most a percentage of the period of time, a variation score of the second set of CI is greater than a threshold for at most a percentage of the period of time, a variation score of the first set of CI and the second set of CI is greater than a threshold for at most a percentage of the period of time, a distance score of the first set of CI is greater than a threshold for at most a percentage of the period of time, a distance score of the second set of CI is greater than a threshold for at most a percentage of the period of time, a distance score of the first set of CI and the second set of CI is greater than a threshold for at most a percentage of the period of time, a mean of a pairwise similarity score of the first set of CI is greater than a threshold, a mean of a pairwise similarity score of the second set of CI is greater than a threshold, a mean of a pairwise similarity score of the first set of CI and the second set of CI is greater than a threshold, a median of the pairwise similarity score of the first set of CI is greater than a threshold, a median of the pairwise similarity score of the second set of CI is greater than a threshold, a median of the pairwise similarity score of the first set of CI and the second set of CI is greater than a threshold, a mode of the pairwise similarity score of the first set of CI is greater than a threshold, a mode of the pairwise similarity score of the second set of CI is greater than a threshold, a mode of the pairwise similarity score of the first set of CI and the second set of CI is greater than a threshold, a percentile of the pairwise similarity score of the first set of CI is greater than a threshold, a percentile of the pairwise similarity score of the second set of CI is greater than a threshold, a percentile of the pairwise similarity score of the first set of CI and the second set of CI is greater than a threshold, a smallest pairwise similarity score of the first set of CI is greater than a threshold, a smallest pairwise similarity score of the second set of CI is greater than a threshold, a smallest pairwise similarity score of the first set of CI and the second set of CI is greater than a threshold, a weighted mean of the pairwise similarity score of the first set of CI is greater than a threshold, a weighted mean of the pairwise similarity score of the second set of CI is greater than a threshold, a weighted mean of the pairwise similarity score of the first set of CI and the second set of CI is greater than a threshold, a variance of the pairwise similarity score of the first set of CI is less than a threshold, a variance of the pairwise similarity score of the second set of CI is less than a threshold, a variance of the pairwise similarity score of the first set of CI and the second set of CI is less than a threshold, a mean of a pairwise similarity score of the first set of CI from a centroid of the plurality of CI is greater than a threshold, a mean of a pairwise similarity score of the second set of CI from a centroid of the plurality of CI is greater than a threshold, a mean of a pairwise similarity score of the first set of CI and the second set of CI from a centroid of the plurality of CI is greater than a threshold, a median of the pairwise similarity score of the first set of CI from a centroid is greater than a threshold, a median of the pairwise similarity score of the second set of CI from a centroid is greater than a threshold, a median of the pairwise similarity score of the first set of CI and the second set of CI from a centroid is greater than a threshold, a mode of the pairwise similarity score of the first set of CI from a centroid is greater than a threshold, a mode of the pairwise similarity score of the second set of CI from a centroid is greater than a threshold, a mode of the pairwise similarity score of the first set of CI and the second set of CI from a centroid is greater than a threshold, a percentile of the pairwise similarity score of the first set of CI from a centroid is greater than a threshold, a percentile of the pairwise similarity score of the second set of CI from a centroid is greater than a threshold, a percentile of the pairwise similarity score of the first set of CI and the second set of CI from a centroid is greater than a threshold, a smallest pairwise similarity score of the first set of CI from a centroid is greater than a threshold, a smallest pairwise similarity score of the second set of CI from a centroid is greater than a threshold, a smallest pairwise similarity score of the first set of CI and the second set of CI from a centroid is greater than a threshold, a weighted mean of the pairwise similarity score of the first set of CI from a centroid is greater than a threshold, a weighted mean of the pairwise similarity score of the second set of CI from a centroid is greater than a threshold, a weighted mean of the pairwise similarity score of the first set of CI and the second set of CI from a centroid is greater than a threshold, a variance of the pairwise similarity score of the first set of CI from a centroid is less than a threshold, a variance of the pairwise similarity score of the second set of CI from a centroid is less than a threshold, a variance of the pairwise similarity score of the first set of CI and the second set of CI from a centroid is less than a threshold, a variance of the first set of CI is less than a threshold, a variance of the second set of CI is less than a threshold, a variance of the first set of CI and the second set of CI is less than a threshold, a mean distance of the first set of CI from a centroid is less than a threshold, a mean distance of the second set of CI from a centroid is less than a threshold, a mean distance of the first set of CI and the second set of CI from a centroid is less than a threshold, a central moment of the first set of CI is less than a threshold, a central moment of the second set of CI is less than a threshold, a central moment of the first set of CI and the second set of CI is less than a threshold, a kurtosis of the first set of CI is less than a threshold, a kurtosis of the second set of CI is less than a threshold, a kurtosis of the first set of CI and the second set of CI is less than a threshold, a tailedness measure of the first set of CI is less than a threshold, a tailedness measure of the second set of CI is less than a threshold, a tailedness measure of the first set of CI and the second set of CI is less than a threshold, an outlier measure of the first set of CI is less than a threshold, an outlier measure of the second set of CI is less than a threshold, an outlier measure of the first set of CI and the second set of CI is less than a threshold, and another qualification criterion.

Clause B177: The method of the qualified wireless system of clause B1, further comprising: performing a task based on the TSCI.

Clause B178: The method of the qualified wireless system of clause B1, further comprising: monitoring a motion of an object in the venue based on the TSCI, wherein the wireless multipath channel is impacted by the motion of the object in the venue.

Clause B179: The method of the qualified wireless system of clause B16, further comprising: computing a characteristics of the motion of the object based on the TSCI.

Clause B180: The method of the qualified wireless system of clause B17, further comprising: computing a current characteristics of the motion of the object based on at least one of: the TSCI and a past characteristics of the motion of the object.

Clause B181: The method of the qualified wireless system of clause B18, further comprising: computing the current characteristics of the motion of the object based on at least one of: a current window of the TSCI and the past characteristics, and computing the past characteristics of the motion of the object based on at least one of: a past window of the TSCI.

Clause B182: The method of the qualified wireless system of clause B17: wherein the characteristics comprises at least one of: a frequency of a repeating motion, a frequency characteristics, a vital characteristics, a breathing rate, a heart rate, a frequency spectrum, a period of the repeating motion, a temporal characteristics, a temporal profile, a time, a timing, a starting time, an ending time, a duration, a history, a trend, a prediction, a motion type, a motion characteristics, a motion intensity, a motion measure, a motion classification, an identity, a presence, a proximity, a proximity, a count, a people count, a location, a geometry, a speed, a velocity, a displacement, a distance, a range, a direction, an angle, an acceleration, a rotational speed, a rotational characteristics, a gait cycle of the object, gesture, a transient behavior of the object, a transient motion, a change, a change in the motion, a change in frequency, a change of period, a change of gait cycle, an event, a sudden motion, a fall-down event and another characteristics.

Clause B183: The method of the qualified wireless system of clause B17, further comprises: performing a task based on the characteristics of the motion of the object.

Clause B184: The method of the qualified wireless system of clause B21, further comprises: generating a presentation associated with the task.

Clause B185: The method of the qualified wireless system of clause B22, further comprises: generating the presentation in a user-interface (UI) of a user device.

Clause B186: The method of the qualified wireless system of clause B21: wherein the task to comprise at least one of: object detection, presence detection, proximity detection, object recognition, activity recognition, object verification, object counting, daily activity monitoring, well-being monitoring, vital sign monitoring, health condition monitoring, baby monitoring, elderly monitoring, sleep monitoring, sleep stage monitoring, walking monitoring, exercise monitoring, tool detection, tool recognition, tool verification, patient detection, patient monitoring, patient verification, machine detection, machine recognition, machine verification, human detection, human recognition, human verification, baby detection, baby recognition, baby verification, human breathing detection, human breathing recognition, human breathing estimation, human breathing verification, human heart beat detection, human heart beat recognition, human heart beat estimation, human heart beat verification, fall-down detection, fall-down recognition, fall-down estimation, fall-down verification, emotion detection, emotion recognition, emotion estimation, emotion verification, motion detection, motion degree estimation, motion recognition, motion estimation, motion verification, periodic motion detection, periodic motion recognition, periodic motion estimation, periodic motion verification, repeated motion detection, repeated motion recognition, repeated motion estimation, repeated motion verification, stationary motion detection, stationary motion recognition, stationary motion estimation, stationary motion verification, cyclo-stationary motion detection, cyclo-stationary motion recognition, cyclo-stationary motion estimation, cyclo-stationary motion verification, transient motion detection, transient motion recognition, transient motion estimation, transient motion verification, trend detection, trend recognition, trend estimation, trend verification, breathing detection, breathing recognition, breathing estimation, breathing verification, human biometrics detection, human biometrics recognition, human biometrics estimation, human biometrics verification, environment informatics detection, environment informatics recognition, environment informatics estimation, environment informatics verification, gait detection, gait recognition, gait estimation, gait verification, gesture detection, gesture recognition, gesture estimation, gesture verification, machine learning, supervised learning, unsupervised learning, semi-supervised learning, clustering, feature extraction, featuring training, principal component analysis, eigen-decomposition, frequency decomposition, time decomposition, time-frequency decomposition, functional decomposition, other decomposition, training, discriminative training, supervised training, unsupervised training, semi-supervised training, neural network, sudden motion detection, fall-down detection, danger detection, life-threat detection, regular motion detection, stationary motion detection, cyclo-stationary motion detection, intrusion detection, suspicious motion detection, security, safety monitoring, navigation, guidance, map-based processing, map-based correction, irregularity detection, locationing, room sensing, tracking, multiple object tracking, indoor tracking, indoor position, indoor navigation, energy management, power transfer, wireless power transfer, object counting, car tracking in parking garage, activating/deactivating/waking/sleeping/ controlling a device/system (e.g. security system, access system, alarm, siren, speaker, television, entertainment system, camera, heater/air-conditioning (HVAC) system, ventilation system, lighting system, gaming system, coffee machine, cooking device, cleaning device, housekeeping device, etc), geometry estimation, augmented reality, wireless communication, data communication, signal broadcasting, networking, coordination, administration, encryption, protection, cloud computing, other processing, and another task.

Clause B187: A method of a qualified wireless system, comprising: receiving a wireless signal by a Type 2 heterogeneous wireless device, wherein the wireless signal is transmitted to the Type 2 device by a Type 1 heterogeneous wireless device through a wireless multipath channel of a venue, and obtaining a time series of channel information (CI) of the wireless multipath channel based on the wireless signal using a processor of the Type 2 device, a memory communicatively coupled with the process and a set of instructions stored in the memory, wherein at least one of: the Type 1 device and the Type 2 device is qualified if the time series of CI (TSCI) satisfy a respective qualification criterion.

Clause B188: The method of the qualified wireless system of clause B1: wherein the wireless signal is compatible with a standard.

Clause B189: The method of the qualified wireless system of clause B1: wherein the wireless signal comprises at least one of: a pilot signal, a token signal, a control signal, a physical layer signal, a MAC layer signal, a handshake signal, a probing signal, a pinging signal, an acknowledgement signal, an enquiry signal, a response signal, a data signal, a hybrid signal, a standard compliant signal, a signal with a header, a signal with a header comprising a probing pattern, a signal with a header data field being a probing pattern, a signal with a payload, a signal with an identifier of the Type 1 device, a signal with an identifier of the Type 2 device, a signal with a time stamp, a signal with a destination address, a signal with a destination MAC address, a signal compatible with an IEEE 802 standard, a signal compatible with a mobile communication standard, a signal compatible with a wireless standard, a signal compliant with a protocol, a point-to-point signal, a broadcasting signal, and another signal.

Clause B190: The method of the qualified wireless system of clause B3: wherein the probe signal is compatible with a standard.

The present teaching further discloses detecting and monitoring motions of an object based on wireless channel information in a rich-scattering environment, e.g. an indoor environment or urban metropolitan area, enclosed environment, underground environment, open-air venue with barriers such as parking lot, storage, yard, square, forest, cavern, valley, etc. In one embodiment, a Type 1 device (transmitter, or Tx) and a Type 2 device (receiver, or Rx) may be on a same device (e.g. RF chip/IC) or simply the same device. The devices may operate at high frequency band, such as 28GHz, 60GHz, 77GHz, etc. The RF chip may have dedicated Tx antennas (e.g. 32 antennas) and dedicated Rx antennas (e.g. another 32 antennas).

In one embodiment, one Tx antenna may transmit a wireless signal (e.g. a series of probe signal, perhaps at 100Hz). Alternatively, all Tx antennas may be used to transmit the wireless signal with beamforming (in Tx), such that the wireless signal is focused in certain direction (e.g. for energy efficiency or boosting the signal to noise ratio in that direction, or low power operation when "scanning" that direction, or low power operation if the object is known to be in that direction). The wireless signal can hit an object (e.g. a living human lying on a bed 4 feet away from the Tx/Rx antennas, with breathing and heart beat) in a venue (e.g. a room). The object motion (e.g. lung movement according to breathing rate, or blood-vessel movement according to heart beat) may impact/modulate the wireless signal. All Rx antennas may be used to receive the wireless signal.

In one embodiment, beamforming (in Rx and/or Tx) may be applied (digitally) to "scan" different directions. Many directions can be scanned or monitored simultaneously. With beamforming, "sectors" (e.g. directions, orientations, bearings, zones, regions, segments) may be defined related to the Type 2 device (e.g. relative to center location of antenna array). For each probe signal (e.g. a pulse, an ACK, a control packet, etc.), a channel information or CI (e.g. channel impulse response/CIR, CSI, CFR) is obtained/computed for each sector (e.g. from the RF chip). In breathing detection, one may collect CIR in a sliding window (e.g. 30 sec, and with 100Hz sounding/probing rate, one may have 3000 CIR over 30 sec).

The CIR may have many taps (e.g. N1 components/taps). Each tap may be associated with a time lag, or a time-of-flight (tof, e.g. time to hit the human 4 feet away and back). When a person is breathing in a certain direction at a certain distance (e.g. 4ft), one may search for the CIR in the "certain direction". Then one may search for the tap corresponding to the "certain distance". Then one may compute the breathing rate and heart rate from that tap of that CIR. One may consider each tap in the sliding window (e.g. 30 second window of "component time series") as a time function (e.g. a "tap function", the "component time series"). One may examine each tap function in search of a strong periodic behavior (e.g. corresponds to breathing, perhaps in the range of 10bpm to 40bpm).

Alternatively, the Type 1 device and/or Type 2 device (together with the wireless signal, TSCI, processor, memory, set of instructions) can be used to monitor other kinds of motion, such as transient motion (e.g. presence/security, human daily activity, breathing, heartbeat, sleep, fall down, human biometrics collection, gait, gesture, writing, body motion, people movement, vehicle motion, in-vehicle activities).

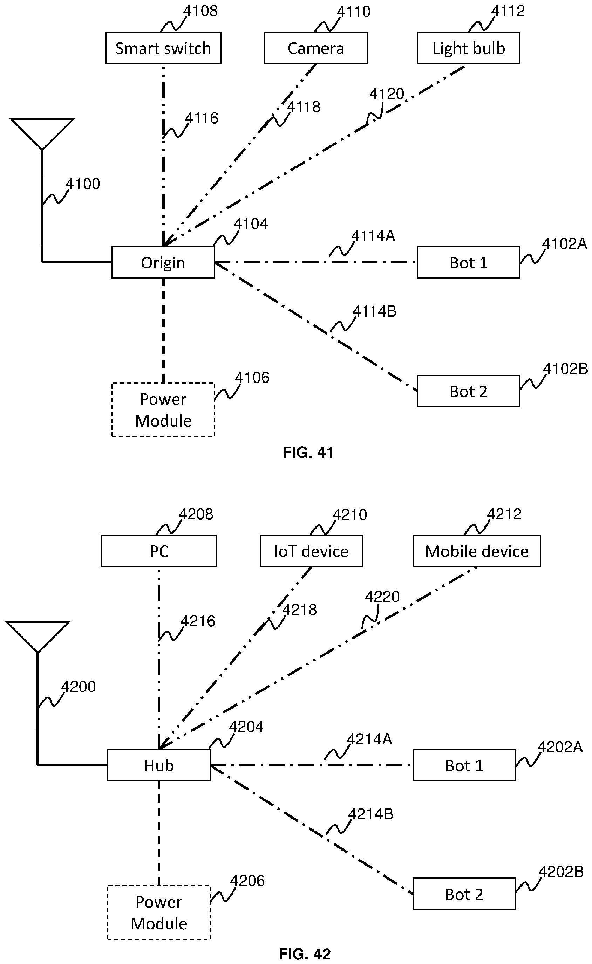

As shown in FIG. 41, the Type 1 device (Origin 4104) and/or the Type 2 device (Bot 1 4102A, Bot 2 4102B) may be a standalone device, or an embedded device. It may be connected to another system (for power 4116, signaling, network access, etc.) using a connector/port such as USB (e.g. Type A/B/C/D/E, micro-USB, mini-USB, etc.), Thunderbolt, Firewire, Lightning (e.g. in Apple devices such as iPhone, iPad, AirPods, etc.), OBD (on-board-diagnostic port), cigarette lighter port (e.g. 12V), PCI (e.g. PCI, PCI Express, etc.), VGA, DVI, HDMI, parallel port, serial port, ADAT, BNC, D-SUB, F, MIDI, UHF, MCX, N, PS/2, RCA, SATA, S-video, SATA, mSATA, m.2, SMA, SMB, SMC, S/PDIF, RJ-11, RJ-45, SCSI, TNC, TS, TRS, UHF, mini-UHF, XLR, coaxial, optical, Ethernet, display port, etc. For example in FIG. 41, the Origin 4104 may be connected to mobile devices, personal computers, home appliance, a smart switch 4108, cameras 4110, light bulbs 4112, smoke detector, and etc. The Origin 4104 may do automation trigger (e.g., turn on/off a light bulb, camera, electric appliance, AC, save a image/video clip on a camera, etc.) upon detecting motion, breathing, presence, security alert, body motion, people movement, vehicle motion, in-vehicle activities, etc., through direction connection, open/public IFTTT, closed/private API integration, etc. Storage may be communicatively associated with the Type 1 and/or Type device, such as SD card, hard disk, etc. For example, motion is detected, then the camera is triggered to capture a picture or video clip which is saved on the storage. Alert may also be sent to the end user.

The Type 1 device and/or the Type 2 device may have external connections/links and/or internal connections/links. The external connections (e.g. connection 4110) may be associated with 2G/2.5G/3G/3.5G/4G/LTE/ 5G/6G/7G/NBIoT, UWB, WiMax, Zigbee, 802.16 etc. The internal connections (e.g., 4114A and 4114B, 4116, 4118, 4120) may be associated with WiFi, an IEEE 802.11 standard, 802.11a/b/g/n/ac/ad/af/ag/ah/ai/aj/aq/ax/ay, Bluetooth, Bluetooth 1.0/1.1/1.2/2.0/2.1/3.0/4.0/4.1/ 4.2/5, BLE, mesh network, an IEEE 802.16/1/1a/1b/2/2a/a/b/c/d/e/f/g/h/i/j/k/l/m/n/o/p/ standard. The Type 1 device and/or Type 2 device may be powered by battery (e.g. AA battery, AAA battery, coin cell battery, button cell battery, miniature battery, bank of batteries, power bank, car battery, hybrid battery, vehicle battery, container battery, non-rechargeable battery, rechargeable battery, NiCd battery, NiMH battery, Lithium ion battery, Zinc carbon battery, Zinc chloride battery, lead acid battery, alkaline battery, battery with wireless charger, smart battery, solar battery, boat battery, plane battery, other battery, temporary energy storage device, capacitor, fly wheel).

Any device (Tx and/or Rx) may be powered by direct current (DC), e.g. from battery as described above, power generator, power convertor, solar panel, rectifier, DC-DC converter, with various voltages such as 1.2V, 1.5V, 3V, 5V, 6V, 9V, 12V, 24V, 40V, 42V, 48V, 110V, 220V, 380V, etc., and may thus have a DC connector or a connector with at least one pin for DC power. Any device (Tx and/or Rx) may be powered by alternating current (AC), e.g. wall socket in a home, transformer, invertor, shorepower, with various voltages such as 100V, 110V, 120V, 100-127V, 200V, 220V, 230V, 240V, 220-240V, 100-240V, 250V, 380V, 50Hz, 60Hz, etc., and thus may have an AC connector or a connector with at least one pin for AC power. The Type 1 device and/or the Type 2 device may be positioned (e.g. installed, placed, moved to) in the venue or outside the venue.

In one embodiment, in a vehicle (e.g. a car, truck, lorry, bus, special vehicle, tractor, digger, excavator, teleporter, bulldozer, crane, forklift, electric trolley, AGV, emergency vehicle, freight, wagon, trailer, container, boat, ferry, ship, submersible, airplane, air-ship, lift, mono-rail, train, tram, rail-vehicle, railcar, etc.), the Type 1 device and/or Type 2 device may be an embedded device embedded in the vehicle, or an add-on device (e.g. aftermarket device) plugged into a port in the vehicle (e.g. OBD port/socket, USB port/socket, accessory port/socket, 12V auxiliary power outlet, and/or 12V cigarette lighter port/socket). For example, one device (e.g. Type 2 device) may be plugged into 12V cigarette lighter/accessory port or OBD port or the USB port (e.g. of a car/truck/vehicle), while the other device (e.g. Type 1 device) may be plugged into 12V cigarette lighter/accessory port or the OBD port or the USB port. The OBD port and/or USB port can provide power, signaling and/or network (of the car/truck/vehicle). The two devices may jointly monitor the passengers including children/babies in the car. They may be used to count the passengers, recognize the driver, detect presence of passenger in a particular seat/position in the vehicle. In another example, one device may be plugged into 12V cigarette lighter/accessory port or OBD port or the USB port of a car/truck/vehicle, while the other device may be plugged into 12V cigarette lighter/accessory port or OBD port or the USB port of another car/truck/vehicle. In another example, there may be many devices of the same type A (e.g. Type 1 or Type 2) in many heterogeneous vehicles/portable devices/smart gadgets (e.g. automated guided vehicle/AGV, shopping/luggage/moving cart, parking ticket, golf cart, bicycle, smart phone, tablet, camera, recording device, smart watch, roller skate, shoes, jackets, goggle, hat, eye-wear, wearable, Segway, scooter, luggage tag, cleaning machine, vacuum cleaner, pet tag/collar/wearable/implant), each device either plugged into 12V accessory port/OBD port/USB port of a vehicle or embedded in a vehicle. There may be one or more device of the other type B (e.g. B is Type 1 if A is Type 2, or B is Type 2 if A is Type 1) installed at locations such as gas stations, street lamp post, street corners, tunnels, multi-storey parking facility, scattered locations to cover a big area such as factory/ stadium/ train station/ shopping mall/construction site. The Type A device may be located, tracked or monitored based on the TSCI.

The area/venue may have no local connectivity, e.g., broadband services, WiFi, etc. The Type 1 and/or Type 2 device may be portable. The Type 1 and/or Type 2 device may support plug and play. Any device (e.g., Origin 4104, Bot 1 4102A, Bot 2 4102B) may provide location information, based on GPS, MAC layer, PHY layer, Wi-Fi, an IEEE 802.11 standard, 802.11a/b/g/n/ac/ad/af/ag/ah/ai/aj/aq/ax/ay, Bluetooth, Bluetooth 1.0/1.1/1.2/2.0/2.1/3.0/4.0/4.1/ 4.2/5, BLE, mesh network, an IEEE 802.16/1/1a/1b/2/2a/a/b/c/d/e/f/g/h/i/j/k/l/m/n/o/p/, standard, 802.16, Zigbee, WiMax, UWB, mobile channel, 1G/2G/3G/3.5G/4G/LTE/5G/6G/7G/NBIoT, etc.

The present teaching also discloses an automatic and optimized device-to-cloud connection for WiFi sensing. In one embodiment, the Type 1 device and/or Type 2 device may be communicatively coupled with: the Internet, another device with access to internet (e.g. smart phone), a cloud server, an edge server, a local server, and/or a storage, a base station operating on 1G/2G/3G/3.5G/4G/LTE/5G/6G/7G/NBIoT etc, and the link/communication between the at least one of them and the Type 1 device and/or Type 2 device may be established with a simplified set up process. This may improve the user experience, and reduce the operational effort/cost of Internet service provider (ISP), dealers or technicians. In one aspect, the Type 1 device and/or Type 2 device can initiate/start the connection within x time after it is turned on without a user action, where x can be several milliseconds, several seconds or several minutes. In another aspect, the Type 1 and/or Type 2 device can automatically choose the best connection means, without a user action, to ensure the bet device-to-cloud connectivity. The best connection may be determined based on the stream of traffic, class of service (CoS), latency, user experience from end to end, packet loss, bandwidth, data rate supported, types of applications or data during the connection, performance and service level agreements (SLAs), availability, provisioning time, security, price per magabit, initial setup costs, etc. In another example, in FIG. 42, the Origin may be a hub 4204. It may connect to PC 4208, IoT devices 4210, mobile devices 4212, etc. for sending/receiving data. The data rate may or may not be low. It may also connect to Bot(s) 4202A, 4202B for wireless sensing.

The following numbered clauses provide additional implementation examples.

Clause C1: A method/system/device/software of a wireless sensing system having at least a processor and a memory with a set of instructions stored therein for sensing motion in a venue, comprising: a Type 1 heterogeneous wireless device configured for transmitting a wireless signal through a wireless multipath channel impacted by a motion of an object in the venue; a Type 2 heterogeneous wireless device configured for: receiving the wireless signal through the wireless multipath channel impacted by the motion of the object in the venue, and obtaining a time series of channel information (CI) of the wireless multipath channel based on the wireless signal; and a motion sensor configured for monitoring the motion of the object in the venue based on motion information related to the motion of the object, wherein the motion information associated with the Type 1 and Type 2 wireless devices is computed based on the time series of CI by at least one of: the motion detector and the Type 2 wireless device.

Clause C2: The method/system/device/software of the wireless sensing system of Clause C1, further comprising: another Type 1 heterogeneous wireless device configured for transmitting another wireless signal through another wireless multipath channel impacted by another motion of another object in another venue; the Type 2 heterogeneous wireless device is further configured for: receiving the another wireless signal through the another wireless multipath channel impacted by the another motion of the another object in the venue, and obtaining another time series of another channel information (CI) of the another wireless multipath channel based on the another wireless signal; and another motion sensor configured for monitoring the another motion of the another object in the another venue based on another motion information related to the another motion of the another object, wherein the another motion information associated with the another Type 1 device and the Type 2 wireless devices is computed based on the another time series of another CI by at least one of: the another motion detector and the second wireless device.

Clause C3: The method/system/device/software of the wireless sensing system of Clause C1, further comprising: another Type 1 heterogeneous wireless device configured for transmitting another wireless signal through another wireless multipath channel impacted by the motion of the object in the venue; wherein the Type 2 heterogeneous wireless device is further configured for: receiving the another wireless signal through the another wireless multipath channel impacted by the motion of the object in the venue, and obtaining another time series of another channel information (CI) of the another wireless multipath channel based on the another wireless signal; and wherein another motion sensor is further configured for monitoring the motion of the object in the venue based on another motion information related to the motion of the object, wherein the another motion information associated with the another Type 1 device and the Type 2 wireless devices is computed based on the another time series of another CI by at least one of: the another motion detector and the second wireless device.

Clause C4: The method/system/device/software of the wireless sensing system of Clause C1, further comprising: another Type 1 heterogeneous wireless device configured for transmitting another wireless signal through another wireless multipath channel impacted by the motion of the object in the venue; wherein the Type 2 heterogeneous wireless device is further configured for: receiving the another wireless signal through the another wireless multipath channel impacted by the motion of the object in the venue, and obtaining another time series of another channel information (CI) of the another wireless multipath channel based on the another wireless signal; and wherein the motion sensor is further configured for monitoring the motion of the object in the venue jointly based on the motion information and another motion information related to the motion of the object, wherein the another motion information associated with the another Type 1 device and the Type 2 wireless devices is computed based on the another time series of another CI by at least one of: the motion detector and the second wireless device.

Clause C5: The method/system/device/software of the wireless sensing system of Clause C1, further comprising: another Type 1 heterogeneous wireless device configured for transmitting another wireless signal through the wireless multipath channel impacted by the motion of the object in the venue; wherein the Type 2 heterogeneous wireless device is further configured for: receiving the another wireless signal through the wireless multipath channel impacted by the motion of the object in the venue, and obtaining another time series of channel information (CI) of the wireless multipath channel based on the another wireless signal; and wherein the motion sensor is further configured for monitoring the motion of the object in the venue jointly based on the motion information and another motion information related to the motion of the object, wherein the another motion information associated with the another Type 1 device and the Type 2 wireless devices is computed based on the another time series of CI by at least one of: the motion detector and the second wireless device.

Clause C6: The method/system/device/software of the wireless sensing system of Clause C1, further comprising: another Type 2 heterogeneous wireless device, wherein the Type 1 heterogeneous wireless device is further configured for transmitting another wireless signal through another wireless multipath channel impacted by another motion of another object in another venue, wherein the another Type 2 heterogeneous wireless device is configured for: receiving the another wireless signal through the another wireless multipath channel impacted by the another motion of the another object in the another venue, and obtaining another time series of another channel information (CI) of the another wireless multipath channel based on the another wireless signal; another motion sensor configured for monitoring the another motion of the another object in the another venue based on another motion information related to the another motion of the another object, wherein the another motion information associated with the Type 1 and the another Type 2 wireless devices is computed based on the another time series of another CI by at least one of: the another motion detector and the another Type 2 wireless device.

Clause C7: The method/system/device/software of the wireless sensing system of Clause C1, further comprising: another Type 2 heterogeneous wireless device, wherein the Type 1 heterogeneous wireless device is further configured for transmitting another wireless signal through another wireless multipath channel impacted by the motion of the object in the venue, wherein the another Type 2 heterogeneous wireless device is configured for: receiving the another wireless signal through the another wireless multipath channel impacted by the motion of the object in the venue, and obtaining another time series of another channel information (CI) of the another wireless multipath channel based on the another wireless signal; another motion sensor configured for monitoring the motion of the object in the venue based on another motion information related to the motion of the object, wherein the another motion information associated with the Type 1 and the another Type 2 wireless devices is computed based on the another time series of another CI by at least one of: the another motion detector and the another Type 2 wireless device.

Clause C8: The method/system/device/software of the wireless sensing system of Clause C1, further comprising: another Type 2 heterogeneous wireless device, wherein the Type 1 heterogeneous wireless device is further configured for transmitting another wireless signal through another wireless multipath channel impacted by the motion of the object in the venue, wherein the another Type 2 heterogeneous wireless device is configured for: receiving the another wireless signal through the another wireless multipath channel impacted by the motion of the object in the venue, and obtaining another time series of another channel information (CI) of the another wireless multipath channel based on the another wireless signal; wherein the motion sensor is further configured for monitoring the motion of the object in the venue jointly based on the motion information and another motion information related to the motion of the object, wherein the another motion information associated with the Type 1 and the another Type 2 wireless devices is computed based on the another time series of another CI by at least one of: the another motion detector and the another Type 2 wireless device.

Clause C9: The method/system/device/software of the wireless sensing system of Clause C1, further comprising: another Type 2 heterogeneous wireless device, wherein the Type 1 heterogeneous wireless device is further configured for transmitting another wireless signal through the wireless multipath channel impacted by the motion of the object in the venue, wherein the another Type 2 heterogeneous wireless device is configured for: receiving the another wireless signal through the wireless multipath channel impacted by the motion of the object in the venue, and obtaining another time series of channel information (CI) of the wireless multipath channel based on the another wireless signal; wherein the motion sensor is further configured for monitoring the motion of the object in the venue jointly based on the motion information and another motion information related to the motion of the object, wherein the another motion information associated with the Type 1 and the another Type 2 wireless devices is computed based on the another time series of CI by at least one of: the motion detector and the another Type 2 wireless device.

Clause C10: The method/system/device/software of the wireless sensing system of Clause C1, further comprising: wherein the Type 2 heterogeneous wireless device is further configured for transmitting another wireless signal through another wireless multipath channel impacted by another motion of another object in another venue, another Type 2 heterogeneous wireless device configured for: receiving the another wireless signal through the another wireless multipath channel impacted by the another motion of the another object in the another venue, and obtaining another time series of another channel information (CI) of the another wireless multipath channel based on the another wireless signal; and another motion sensor configured for monitoring the another motion of the another object in the another venue based on another motion information related to the another motion of the another object, wherein the another motion information associated with the Type 2 and the another Type 2 wireless devices is computed based on the another time series of another CI by at least one of: the another motion detector and the another Type 2 wireless device.

Clause C11: The method/system/device/software of the wireless sensing system of Clause C1, further comprising: wherein the Type 2 heterogeneous wireless device is further configured for transmitting another wireless signal through another wireless multipath channel impacted by the motion of the object in the venue, another Type 2 heterogeneous wireless device configured for: receiving the another wireless signal through the another wireless multipath channel impacted by the motion of the object in the venue, and obtaining another time series of another channel information (CI) of the another wireless multipath channel based on the another wireless signal; and another motion sensor configured for monitoring the motion of the object in the venue based on another motion information related to the motion of the object, wherein the another motion information associated with the Type 2 and the another Type 2 wireless devices is computed based on the another time series of another CI by at least one of: the another motion detector and the another Type 2 wireless device.

Clause C12: The method/system/device/software of the wireless sensing system of Clause C1, further comprising: wherein the Type 2 heterogeneous wireless device is further configured for transmitting another wireless signal through the wireless multipath channel impacted by the motion of the object in the venue, another Type 2 heterogeneous wireless device configured for: receiving the another wireless signal through the wireless multipath channel impacted by the motion of the object in the venue, and obtaining another time series of channel information (CI) of the wireless multipath channel based on the another wireless signal; and wherein the motion sensor is further configured for monitoring the motion of the object in the venue jointly based on the motion information and another motion information related to the motion of the object, wherein the another motion information associated with the Type 2 and the another Type 2 wireless devices is computed based on the another time series of CI by at least one of: the motion detector and the another Type 2 wireless device.

Clause C13: The method/system/device/software of the wireless sensing system of Clause C1, further comprising: another Type 1 heterogeneous wireless device configured for transmitting another wireless signal through another wireless multipath channel impacted by another motion of another object in another venue; wherein the Type 1 heterogeneous wireless device is further configured for: receiving the another wireless signal through the another wireless multipath channel impacted by the another motion of the another object in the another venue, and obtaining another time series of another channel information (CI) of the another wireless multipath channel based on the another wireless signal; and another motion sensor configured for monitoring the another motion of the another object in the another venue based on another motion information related to the another motion of the another object, wherein the another motion information associated with the another Type 1 and the Type 1 wireless devices is computed based on the another time series of another CI by at least one of: the another motion detector and the Type 1 wireless device.

Clause C14: The method/system/device/software of the wireless sensing system of Clause C1, further comprising: another Type 1 heterogeneous wireless device configured for transmitting another wireless signal through another wireless multipath channel impacted by the motion of the object in the venue; wherein the Type 1 heterogeneous wireless device is further configured for: receiving the another wireless signal through the another wireless multipath channel impacted by the motion of the object in the venue, and obtaining another time series of another channel information (CI) of the another wireless multipath channel based on the another wireless signal; and another motion sensor configured for monitoring the motion of the object in the venue based on another motion information related to the motion of the object, wherein the another motion information associated with the another Type 1 and the Type 1 wireless devices is computed based on the another time series of another CI by at least one of: the another motion detector and the Type 1 wireless device.

Clause C15: The method/system/device/software of the wireless sensing system of Clause C1, further comprising: another Type 1 heterogeneous wireless device configured for transmitting another wireless signal through the wireless multipath channel impacted by the motion of the object in the venue; wherein the Type 1 heterogeneous wireless device is further configured for: receiving the another wireless signal through the wireless multipath channel impacted by the motion of the object in the venue, and obtaining another time series of channel information (CI) of the wireless multipath channel based on the another wireless signal; and wherein the motion sensor is further configured for monitoring the motion of the object in the venue jointly based on the motion information and another motion information related to the motion of the object, wherein the another motion information associated with the another Type 1 and the Type 1 wireless devices is computed based on the another time series of CI by at least one of: the motion detector and the Type 1 wireless device.

Clause C16: The method/system/device/software of the wireless sensing system of Clause C1: wherein at least one of: the Type 1 wireless device, the Type 2 wireless device and the motion detector is powered by at least one of: external power source, internal power source, a wall power source, a fixed power source, a portable power source, a direct-current (DC) power source, an alternate-current (AC) power source, a power storage device, a power generating device, a power converting device, a power transforming device, a battery, a rechargeable battery, a fuel cell, a solar panel, a solar cell, a power supply, a USB port (e.g. Type A/B/C/D/E, micro USB, mini USB), a Thunderbolt port (in Apple devices such as iPhone, iPad, AirPods, etc., a Firewire port, a Lightning port, an OBD (on board diagnostic) port (e.g. in cars), a cigarette lighter port (12V), PoE (power over Ethernet), PCI (e.g. PCI, PCI Express, etc.), VGA, DVI, HDMI, parallel port, serial port, ADAT, BNC, D-SUB, F, MIDI, UHF, MCX, N, PS/2, RCA, SATA, S-video, SATA, mSATA, m.2, SMA, SMB, SMC, S/PDIF, RJ-11, RJ-45, SCSI, TNC, TS, TRS,UHF, mini-UHF, XLR, coaxial, optical, Ethernet, display port, and another power source.

Clause C17: The method/system/device/software of the wireless sensing system of Clause C1: wherein at least one of: the Type 1 wireless device, the Type 2 wireless device and the motion detector comprises at least one of: an external power source, an internal power source, a direct-current (DC) power source, a DC adapter, an alternate-current (AC) power source, an AC adapter, a power storage unit, a power generating unit, a power converting unit, a power transforming unit, a charging unit, a charger, a transformer, a magnetic coupling, an electro-magnetic coupling, a battery, a rechargeable battery, a fuel cell, a capacitor, an inductor, an alternator, a generator, a solar panel, a solar cell, a power supply, and another power management unit.

Clause C18: The method/system/device/software of the wireless sensing system of Clause C1: wherein at least one of: the Type 1 wireless device, the Type 2 wireless device and the motion detector establishes communication with a remote server in at least one of the following manners: (a) automatic, (b) fully automatic, (c) semi-automatic, (d) manual, (e) no user input, (f) preset, (g) upon power-up, (h) upon reboot, (i) upon reset or restart, (j) upon a key press, (k) upon a click, (l) upon set-up, (m) upon a user input, (n) direct, (o) indirect, and (p) another manner.

Clause C19: The method/system/device/software of the wireless sensing system of Clause C1: wherein at least one of: the Type 1 wireless device, the Type 2 wireless device and the motion detector associates with a remote server in at least one of the following manners: (a) automatic, (b) fully automatic, (c) semi-automatic, (d) manual, (e) no user input, (f) preset, (g) upon power-up, (h) upon reboot, (i) upon reset or restart, (j) upon a key press, (k) upon a click, (l) upon set-up, (m) upon a user input, (n) direct, (o) indirect, and (p) another manner.

Clause C20: The method/system/device/software of the wireless sensing system of Clause C1: wherein at least one of: the Type 1 wireless device, the Type 2 wireless device and the motion detector establishes communication with a remote server based on at least one of the following connectivity: wired connectivity, wired network, USB, Thunderbolt, Firewire, Lightning, OBD, cigarette lighter, PoE, PCI, VGA, DVI, HDMI, parallel port, serial port, ADAT, BNC, D-SUB, F, MIDI, UHF, MCX, N, PS/2, RCA, SATA, S-video, SATA, mSATA, m.2, SMA, SMB, SMC, S/PDIF, RJ-11, RJ-45, SCSI, TNC, TS, TRS,UHF, mini-UHF, XLR, coaxial, optical, Ethernet, display port, wireless connectivity, wireless network, WiFi, UWB, mesh network, Bluetooth, BLE, mmWave, Zigbee, microwave, light communication, connected lighting, 802.11, 802.15, 802.16, mobile connectivity, mobile network, 2G, 3G, 4G, LTE, 5G, 6G, 7G, 8G, WiMax, LTE, NBIoT, 3GPP, WLAN, WPAN, WMAN, and another connectivity.

Clause C21: The method/system/device/software of the wireless sensing system of Clause C1: wherein at least one of: the Type 1 wireless device, the Type 2 wireless device and the motion detector establishes communication with a remote server by selecting at least one of the following connectivity based on a selection criterion: wired connectivity, wired network, USB, Thunderbolt, Firewire, Lightning, OBD, cigarette lighter, PoE, PCI, VGA, DVI, HDMI, parallel port, serial port, ADAT, BNC, D-SUB, F, MIDI,UHF, MCX, N, PS/2, RCA, SATA, S-video, SATA, mSATA, m.2, SMA, SMB, SMC, S/PDIF, RJ-11, RJ-45, SCSI, TNC, TS, TRS,UHF, mini-UHF, XLR, coaxial, optical, Ethernet, display port, wireless connectivity, wireless network, WiFi, UWB, mesh network, Bluetooth, BLE, mmWave, Zigbee, microwave, light communication, connected lighting, 802.11, 802.15, 802.16, mobile connectivity, mobile network, 2G, 3G, 4G, LTE, 5G, 6G, 7G, 8G, WiMax, LTE, NBIoT, 3GPP, WLAN, WPAN, WMAN, and another connectivity.

Clause C22: The method/system/device/software of the wireless sensing system of Clause C1: wherein at least one of: the Type 1 wireless device, and the Type 2 wireless device comprises at least one of: an external storage unit, a removable storage, a harddisk, a flash drive, a flash memory, local storage, a memory card, a memory cartridge, an secure digital (SD) card, a microSD card, a CompactFlash (CF) card, a SmartMedia card, a miniature card, a multimedia card (MMC), a PCMCIA card, a memory stick, an XD card, a P2 card, a m.2 card, a SIM card, and another storage medium.

Clause C23: The method/system/device/software of the wireless sensing system of Clause C1: wherein the venue comprises at least one of: an indoor environment, an enclosed environment, an outdoor environment, an open-air environment, a facility, a warehouse, a factory, a manufacturing facility, an assembly line, a building, a multi-storey building, a house, a home, an office, a store, a supermarket, a casino, a hotel, a room, a box, a stadium, a hall, a station, a hospital, an airport, a port, a subway, a vehicle, a car, a boat, a ship, a cruise ship, a submersible, a plane, a drone, a cave, a tunnel, a pipe, a piping system, a crawl space, a maintenance system, a tube, an air conditioning/ventilation system, a fluid, and another venue.

Clause C24: The method/system/device/software of the wireless sensing system of Clause C1: wherein the venue comprises at least one of: people, pet, children, animals, plants, partitions, walls, dry walls, concrete walls, brick walls, glass walls, metal walls, doors, windows, glasses, floors, ceilings, attics, garage, fireplace, parking facilities, structure elements, columns, beams, movable objects, non-movable objects, furniture, wardrobe, fixtures, machines, devices, lighting, curtains, blinds, construction features, building features, water pipes, air ducts, crawl space, basement, elevator, stairs, stair wells, hall way, corridor, maintenance space, fan, ventilation system, air-condition system, heat, HVAC, electrical wiring, refrigerator, cooking devices, oven, microwave, stove, television, sound system, smart speaker, lights, carpets, restricted area, limited-access area, forbidden area, and another feature.

Clause C25: The method/system/device/software of the wireless sensing system and of Clause C1: wherein at least one of: the Type 1 device and the Type 2 device, to house at least one of: another device, an internet-of-thing (IoT) device, a smart-device, a smart appliance, a smart fixture, a smart clock, a smart stationary, a smart pen, a smart user-interface, a smart paper, a smart mat, a smart camera, a smart television (TV), a set-top-box, a smart microphone, a smart speaker, a smart refrigerator, a smart oven, a smart machine, a smart phone, a smart wallet, a smart furniture, a smart door, a smart window, a smart ceiling, a smart floor, a smart wall, a smart table, a smart chair, a smart bed, a smart night-stand, a smart air-conditioner, a smart heater, a smart pipe, a smart duct, a smart cable, a smart carpet, a smart decoration, a smart gadget, a smart USB device, a smart plug, a smart dongle, a smart lamp/light, a smart tile, a smart ornament, a smart bottle, a vehicle, a smart car, a smart AGV, a drone, a smart robot, a laptop, a tablet, a computer, a harddisk, a network card, a smart instrument, a smart racket, a smart ball, a smart shoe, a smart wearable, a smart clothing, a smart glasses, a smart hat, a smart necklace, a smart food, a smart pill, a small device that moves in the body of a creature, and another device.

Clause C26: The method/system/device/software of the wireless sensing system and of Clause C1: wherein at least one of: the Type 1 device and the Type 2 device, to trigger at least one of: another device, an internet-of-thing (IoT) device, a smart-device, a smart appliance, a smart fixture, a smart clock, a smart stationary, a smart pen, a smart user-interface, a smart paper, a smart mat, a smart camera, a smart television (TV), a set-top-box, a smart microphone, a smart speaker, a smart refrigerator, a smart oven, a smart machine, a smart phone, a smart wallet, a smart furniture, a smart door, a smart window, a smart ceiling, a smart floor, a smart wall, a smart table, a smart chair, a smart bed, a smart night-stand, a smart air-conditioner, a smart heater, a smart pipe, a smart duct, a smart cable, a smart carpet, a smart decoration, a smart gadget, a smart USB device, a smart plug, a smart dongle, a smart lamp/light, a smart tile, a smart ornament, a smart bottle, a vehicle, a smart car, a smart AGV, a drone, a smart robot, a laptop, a tablet, a computer, a harddisk, a network card, a smart instrument, a smart racket, a smart ball, a smart shoe, a smart wearable, a smart clothing, a smart glasses, a smart hat, a smart necklace, a smart food, a smart pill, a small device that moves in the body of a creature, and another device.

Clause C27: The method/system/device/software of the wireless sensing system and of Clause C1: wherein the motion sensor is configured to monitor at least one of: the motion of the object, a breathing motion of a person, a periodic motion of a person, a repeated motion of a person, a fall-down of a person, a transient motion of the person, a change of the object, a presence, an absence, a presence of motion, an absence of motion, a presence of the object, an absence of the object, a change of the object, a deformation of the object, an action of the object, a movement of the object, an entrance of the object, an exit of the object, a presence of a repetitive motion, a motion frequency, a motion period, a motion rhythm, a breathing motion frequency, a breathing motion period, a motion cycle, a motion count, a motion intensity, a breathing motion period, a motion classification, a gait information, a motion sequence, a presence of a transient motion, a motion change, a motion event information, an entrance event, an exit event, an object fall down motion, a resizing motion, a deformation motion, a turning motion, a translational motion, a rotational motion, a gesture, a handwriting motion, a head motion, a mouth motion, a heart motion, an internal organ motion, a presence of a statistical motion, a statistical motion quantity, a mean, a variance, an autocorrelation, an auto-covariance, a cross correlation, a cross covariance, a wind motion, a motion localization, a motion location, a motion intensity, a presence of motion, an absence of motion, a presence of the object, an absence of the object, a time history of the motion statistics, a change of the motion statistics, a behavior of the motion statistics, and/or, a trend of the motion statistics, an appearing, a disappearing, an increase, a decrease, a spending-up, a slowing-down, a direction change, a sudden motion, an impulsive motion, a size, a length, an area, a volume, a capacity, a shape, a form, a spatial quantity, a location, a distance, a spatial range, a spatial label, a spatial tag, a starting location, an ending location, a speed, an acceleration, a rotation, an angular motion, a differential, a direction, a spatial trend, a temporal quantity, a time stamp, a time label, a time tag, a starting quantity, an ending quantity, a starting time, an ending time, a duration, a count, a period, a time window, a rate, a timing, a time delay, a trend, a time trend, a motion profile, a time event, a time-space quantity, a time-frequency quantity, a time-frequency-space quantity, a periodic behavior, a transient behavior, a periodic motion, a pseudo-periodic motion, a transient motion, a steady state behavior, a planned motion, an unplanned motion, and/or a perturbation, a statistical behavior, a stationary behavior, a quasi-stationary behavior, a cyclo-stationary behavior, a chaotic behavior, a co-occurrence, a timed-occurrence, an interaction, a response, an activity, a sign of an activity, a gesture, a gait, a body motion, a body part motion, a hand motion, an arm motion, a foot motion, a leg motion, a head motion, a facial motion, a mouth motion, an eye motion, a gaze, a breathing motion, a heart motion, a writing motion, a drawing motion, a finger motion, a user-interface motion, an event, a fall-down event, a security event, an accident event, a home event, an office event, a factory event, a warehouse event, a manufacturing event, an assembly line event, a maintenance event, a car-related event, a navigation event, a tracking event, a door event, a door-open event, a door-close event, a window event, a window-open event, a window-close event, a repeatable event, a one-time event, another event, a frequency characteristics, a frequency, a spectrum, a starting frequency, an ending frequency, a changing frequency, a frequency shift, a frequency trend, a frequency event, a user, a household, an office, a factory, a warehouse, a facility, an identity (ID), a consumed quantity, an unconsumed quantity, a state, a physical state, a health state, a well-being state, an emotional state, a mental state, and another motion information.

Clause C28: The method/system/device/software of the wireless sensing system and of Clause C1: wherein the motion sensor is configured to compute an analytics based on the motion of the object.

Clause C29: The method/system/device/software of the wireless sensing system and of Clause C1: wherein the motion sensor is configured to process at least one of: an analytics computed based on the motion of the object, and a history of the analytics.

Clause C30: The method/system/device/software of the wireless sensing system and of Clause C1: wherein the motion sensor is configured to compute an analytics based on the motion of the object, wherein the motion sensor is further configured to at least one of: store at least one of: the analytics, and a history of the analytics, compute another analytics based on at least one of: the analytics, and a history of the analytics, communicate at least one of: the analytics, and the history of the analytics, to a remote device, process at least one of: the another analytics, and another history of the another analytics, communicate at least one of: the another analytics, and the another history of the another analytics, to a remote device.

Clause C31: The method/system/device/software of the wireless sensing system and of Clause C1: wherein the motion sensor is configured to compute an analytics based on the motion of the object, wherein at least one of: the analytics, and a history of the analytics, is communicated to a remote device, wherein at least one of: the analytics, and the history of the analytics, is displayed by the remote device.

Clause C32: The method/system/device/software of the wireless sensing system and of Clause C1: wherein the motion sensor is configured to compute an analytics based on the motion of the object, wherein at least one of: the analytics, and a history of the analytics, is communicated to a remote device, wherein at least one of: the analytics, and the history of the analytics, is analyzed by the remote device, wherein another analytics is computed and processed by the remote device based on at least one of: the analytics, and the history of the analytics.

Clause C33: The method/system/device/software of the wireless sensing system and of Clause C32: wherein at least one of: the another analytics, and another history of the another analytics, is communicated to and displayed by another remote device.

The present teaching also discloses processing, compilation, organization, grouping and presentation of time-stamped data for a visual display or other feedback or user-interface (UI) device. Various embodiments of the present teaching are regarding the processing, compilation, organization, grouping and presentation of time-stamped data for a visual display (e.g. computer monitor, smart phone display, tablet, TV, projector, animation, etc.) or other feedback or user-interface (UI) device (e.g. voice presentation via smart phones, computers, tablets, sound-enabled devices, a smart speaker such as Amazon Echo/Alexa, or vibration, or haptic device).

In one embodiment, each time-stamped data item may comprise a scalar, a duration, a timing, an ordered pair, an n-tuple, a coordinate, a location, a direction, an angle, an attribute, a description, a trend, a behavior, a motion, a movement, a gesture (e.g. handwriting, hand sign, keystroke, facial expression), a vital sign, a feature, an object, an identification, a characteristics, a phenomenon, an event (e.g. fall-down), a state, a transition, a status, a stage (e.g. sleep stage, awake, REM, NREM, etc.), a relationship, a vector, a matrix, a classification, a detection, a decision, a conclusion, a set, a group, a collection, an element, a subset, and/or any mixture of these. The time stamped data may be associated with one or more time series of channel information (CI). The time-stamped data may comprise one or more analytics obtained/determined/computed based on the one or more time series of CI (e.g. channel state information, or CSI) extracted from a wireless signal transmitted from a Type 1 heterogeneous wireless device to a Type 2 heterogeneous wireless device via a wireless multipath channel of a venue.

In one embodiment, a Type 1 device may transmit the wireless signal to multiple Type 2 devices. A Type 2 device may receive multiple wireless signals from multiple Type 1 devices, each wireless signal from a corresponding Type 1 device. A Type 1 device and a Type 2 device may be the same device (i.e. operating like a radar system). A Type 1 device and a Type 2 device may be attached to/in/on/of a machine. The wireless signal may be a series of probe signals. The probe signals may be sent at regular, basically regular, or irregular time intervals. The probe signal may or may not be transmitted with data. The probe signal may be a data packet. Each probe signal when received may be time stamped.

In an example, one (or more) Type 1 device and one (or more) Type 2 device may be placed around a bed (or anywhere) to monitor the motion and breathing of one (or more) person lying on the bed in the bedroom. For a bed with two people sleeping, a Type 1 device may be placed on one end (e.g. the left) and another Type 1 device on another end (e.g. the right) to monitor the motion and breathing of two people simultaneously. The Type 2 device may be placed near the bed (e.g. at the front, at the back, underneath the mattress/bed, above the bed), or at another location in the house.

In another example, the Type 1 device(s) and Type 2 device(s) may be placed at various places (e.g. at ceiling, on a wall, on the floor, on a table/furniture) of a house, an apartment, a warehouse, a parking lot, a building, a mall, a stadium, a station, an interchange, an office, a room, a meeting room, a warehouse, a facility, a public facility, a bathroom, a toilet, a staircase, a lift, etc. The Type 1 device(s) and/or Type 2 device(s) may be embedded in another device such as TV, remote control, set-top box, audio device, speaker, camera, router, access point, appliance, refrigerator, smoke detector, stove, furniture, chair, sofa, desk, table, vacuum cleaner, smoke detector, lock, tool, WiFi-enabled device, computer, printer, monitor, keyboard, mouse, mouse pad, computer accessory, tablet, phone, clock, alarm clock, thermometer, thermostat, light device, light switch, power socket, power plug, lamp, bedside lamp, bed, computer, phone, tablet, smart plug, charger, extension device, power meter, toy, child item, baby item, baby monitor, adult item, elderly person item, health care item, IoT device, another home device, an office device, a factory device, etc.

In one embodiment, there are N1 (e.g. N1=2 or 6 or 100 or 100000) time series of analytics (TSA), each item of each time series being associated with a time stamp. The analytic may be a motion intensity index, a presence/absence, an approaching, a receding, a motion sequence, a motion indicator, a motion direction, a location, a distance, a speed, an acceleration, a timing, a duration, a time period, a periodic motion analytic, a frequency, a period, a transform, a function/transformation of another analytic, a regularity, a transient measure, a manifestation, a revelation, a sign, a vital sign, an impact, a change, a deformation, a hand signal, a gesture signal, a health symptom, a duration, a count, a classification of motion, a breathing parameter/characteristics/statistics, a gait, a hand motion, gesture, a body language, a dancing move, a formation, an event, a state, status, a stage, an indicator of a motion/an event/a condition/a situation/a state, a digital representation (e.g. taking on value of 0 and 1), a continuous/analog representation (e.g. taking on any value between A and B, where A may be 0 or another value, B may be 1 or another value), etc. Some, if not all, analytics may be obtained/determined/computed based on the channel information (CI).

Any TSA may be sampled/computed/obtained at regular or irregular time interval. In a TSA, the analytics may be obtained (sampled) regularly for some time, irregularly for some time, intermittently for some time or spontaneously for some time. The analytics may be sampled at a low rate (e.g. 10 Hz) in a time period (e.g. 1 hour), and at a higher rate (e.g. 1000 Hz) in another time period (e.g. 5 minutes). A temporal change in the sampling may be in response to a change (e.g. in environment, detected motion/event/sign). For example, the sampling rate may be low in a standby mode and may be changed to high in an alarming mode or danger mode. For a particular time t, all or some or none of the N1 TSA may have an item associated with the time stamp (i.e. t).

There are two ways to present the N1 TSA. In the first way (same-graph presentation), there is one graph with N1 curves (basically N1 graphs superimposed on each other), with the y-axis (or y- and z-axes if an analytics is 2-dimensional, or M-dimensional if an analytics has M dimension) representing the N1 analytics and the x-axis representing time. To maximize the visualization of each analytics, the range of the y-axis may be mapped to different ranges of different analytics (e.g. from 0 to 1 for first analytics, from -10 to 10 for second analytics, from 0 to 100 for the third analytics, from 0 to 10 million for the fourth analytics and so on) while the x-axis is the time axis common to all the N1 curves. If a vertical line is drawn at a particular time t, it intersects with each curve at a point. Thus there are N1 intersection points on the vertical line, one for each curve.

In the second way of presenting the N1 TSA (separate-graph presentation), there are N1 separate graphs, one for each analytics. For ease of comparison, suppose the x-axes (time axes) of all graphs are the same/identical, and suppose that all graphs are time synchronized, with essentially same "width" so that they can be conveniently stacked. In each graph, there is only one curve of the analytics (e.g. it may be identical to the respective curve of the analytics drawn in the same-graph presentation with same x-axis and y-axis). The N1 graphs are stacked/placed vertically (e.g. positioned one on top of another), with the y-axis of the graphs co-linear (in a straight line) and the range of the y-axis of each graph mapped to the same respective range as in the same-graph presentation. If a vertical line is drawn at time t in a graph, there is only one intersection in each graph - because there is only one curve. If a vertical line is drawn at time t through the N1 graphs, there will be N1 intersection points - one in each graph.

In one embodiment of the present teaching, the N1 TSA may be presented in a novel hybrid way (hybrid presentation), with characteristics of both the same-graph and separate-graph presentation. In one way, the N1 TSA may be combined to be a single combined TSA and the combined TSA may be presented in a single combined graph with a single combined curve. Recall that in the separate-graph presentation, there may be N1 curves in N1 graphs (1 curve for each graph) stacked/placed vertically. All the graphs may have the same time axis or x-axis (synchronized). In the hybrid presentation, all the N1 graphs in the separate-group presentation may be merged into a "combined graph". The common time axis may be partitioned to form M partitions (e.g. partition 1 from time $t\_0$ to time $t\_1$, partition 2 from $t\_1$ to $t\_2$, ..., partition M from time $t\_{M-1}$ to $t\_M$). The M partitions may or may not span the whole time axis. In other words, there may or may not be gaps between adjacent partitions. The length (or duration) of different partitions may be different.

In the hybrid presentation, for each partition, only one of the N1 curves may be selected (to form part of the combined curve) and displayed, while the rest of the N1 curves (the remaining N1-1 curves) may not be displayed. Alternatively, a new combined TSA may be formed with the analytics in each partition chosen from the corresponding TSA associated with the selected curve. The hybrid presentation is equivalent to displaying the new combined TSA.

One may define an indicator function I(t) which takes on integer values in the range of 1 to N1. For each partition, the indicator function value is the value of the selected curve. For example, I(t)=k if the k^{th} curve is the selected curve at time t. One may define I_k(t), with k=1, .., N1, to be an indicator function of the selection of the k^{th} curve, taking on a value of 1 if the k^{th} curve is selected at time t, and a value of 0 otherwise. Then mathematically,

$$I(t)= \text{sum}\_{k=1}^{N1} k*I\_k(t)$$

Let f_k(t) be the function corresponding to the k^{th} curve. Then the displayed function f(t) in the hybrid presentation may effectively be:

$$f(t)= \text{sum}\_{k=1}^{N1} f\_k*I\_k(t)$$

Alternatively, the indicator function, I(t), may itself be displayed in the hybrid presentation to indicate which curve (TSA) is active, dominant, or selected, or highlighted, especially when the analytics are indicator of some mutually exclusive states (e.g. REM, NREM and AWAKE sleep states, or SLEEP and NO-SLEEP states, or MOTION and NO-MOTION states), or events (rest room visit).

Alternatively, all the N1 curves may be displayed in a first manner (e.g. in a non-dominant manner, in a subtle manner, in a background manner, and/or in less eye-catching manner, with light color, pastel color, grey color, unsaturated color, broken line, dotted line, lower intensity, smaller line thickness, broken/dotted line type, less intensity (e.g. zero intensity, i.e. not displayed), higher transparency (e.g. not visible, half-visible), and/or intermittent line type, etc.). For each partition, the selected curve may be displayed in a second manner (e.g. in a dominant manner, in a non-subtle manner, in a foreground manner, and/or in more eye-catching manner, with dark color, strong color, black, saturated color, solid line, higher intensity, larger line thickness, strong line type, more intensity, lower transparency, and/or continuous line type, etc.) instead of the first manner. This is similar to displaying the N1 TSA as N1 curves in the first manner and the new combined TSA as a curve in the second manner.

In the alternative way of hybrid display, the N1 curves may still be displayed similar to how they would be displayed in the separate-graph presentation. They may be stacked and placed vertically, similar to the separate-graph presentation. Each may or may not be displayed with its x-axis (or time axis), where the N1 x-axis may be replaced by a single line (with/without markings to show time scale) to indicate the location of axis.

Suppose the separation of adjacent graphs is P. Then, stacking the N1 graphs is similar to forming and displaying the function

$$f(t)= \text{sum\_}\{k=1\}^{\{N1\}} (f\_k+k*P)*I\_k(t),$$

such that adjacent "curves" are separated by a distance of P. If one chooses not to display the f_k and set P=1, the displayed function will be the indicator function I(t):

$$I(t)= \text{sum\_}\{k=1\}^{\{N1\}} k*I\_k(t).$$

More generally, the function displayed may be

$$f(t)= \text{sum\_}\{k=1\}^{\{N1\}} (f\_k+a\_k)*I\_k(t),$$

for some a_k values. In various embodiments, a_k can be k*P but can also be irregular.

Between the partitions, the curves may or may not be connected using a line (e.g. a straight line, a curve, etc.). If there is no gap between two adjacent partitions, the curves in the two adjacent partitions may be connected by a vertical line. In this way, there may be only one combined curve in the combined graph, with the line segment in any time partition being the line segment of one of the N1 curves. The combined curve may resemble a continuous function moving among the different graphs (or different curves). If a vertical line is drawn at time t (of a particular partition), it intersects with the combined curve at only one point (on the selected curve associated with the partition). At time t, the selected graph may be considered the "current" graph. The selected curve may be considered the "current" curve. The partition/segment may be considered the "current segment". The segments and the axis may be labeled. In an example, N1=6 TSA in a well-being monitoring application with two Type 1 devices and one Type 2 device in the home of a user living alone. A Type 1 device and the Type 2 device may be placed next to the user's bed. The other Type 1 device may be placed in a rest room. There are two wireless links: one between the first Type 1 device and the Type 2 device, and one between the second Type 1 device and the Type 2 device. One (or more, if more than one antenna/device) time series of channel information (TSCI) may be obtained for each wireless link based on a wireless signal being transmitted from the respective Type 1 device to the Type 2 device. For each wireless link, analytics (e.g. motion, breathing, etc.) may be computed based on the respective TSCI.

In this example, there may be N1=6 analytics, comprising 6 indicators of: (I1) REM sleep, (12) NREM sleep, (13) awake, (14) restroom, (15) in-house activity, and (16) no presence. In general, each indicator may take on values of 1 and 0. An indicator value of 1 may mean the description is active/happening/assertive, while a value of 0 may mean not active/not happening/not assertive. When (I1) is 1, REM (rapid eye movement) sleep may be detected (happening). When (I1) is zero, REM sleep may not be detected (not happening). The first three analytics, (I1) to (13), may represent different stages of sleep (REM, NREM or non-REM, AWAKE), active when the user is in SLEEP state (as opposed to NO-SLEEP state) typically taking turns to be asserted during bed time of a user. In a way, (I1) to (13) are three sub-states of the SLEEP state. That is, when the user has a good sleep, the AWAKE stage may be absent. These three analytics may be obtained from some sleep analysis procedure based on the TSCI. In some situations, these three analytics may be combined as one combined sleep analytics taking on four values: REM, NREM, AWAKE and NOT ASLEEP. Typically, a function such as the combined sleep analytics may be decomposed into, or represented as, a weighted sum of simple indicator functions. Analytics (14) may be asserted when activity (e.g. motion) is detected in the rest room. Analytics (15) may be asserted when activity is detected in the rest of the house. Analytics (16) may be asserted when no activity is detected. Note that typically, only one indicator is active while the others are not active.

In a separate-graph presentation, there may be 6 graphs, each with a function (a curve). Let $f\_1(t)$ be the function for analytics (I1), $f\_2(t)$ for (12), ..., and $f\_6(t)$ for (16). In the hybrid display, the combined function may be displayed with the original 6 graphs stacked and labeled. For example the curve of (I1) may be labeled as "REM"; (12) curve may be labeled as "NREM", (13) curve may be labeled as "Awake", (14) curve may be labeled as "Activities", (15) curve may be labeled as "Bathroom", and (16) curve may be labeled as "No activity". Different sections of the combined function may be labeled. For example, the user may go to bed at 9pm with the combined function taken on (14) immediately before 9pm and (I1) soon after 9pm. The transition from (14) to (I1) may be a vertical line labeled as "Go to bed". Periods of (15) may be labeled as "Bathroom". A similar process may go on.

Each indicator may be associated with a value, e.g. (I1) associated with a_1, (12) associated with a_2, (13) associated with a_3, (14) associated with a_4, (15) associated with a_5, and (16) associated with a_6. The combined function (or combined curve) may take on these values. The combined curve may be represented as (a_1+f_1(t))*I_1(t)+(a_2+f_2(t))*I_2(t)+(a_3+f_3(t))*I_3(t)+(a_4+f_4(t))*I_4(t)+(a_5+f_5(t))*I_5(t)+(a_6+f_6(t))*I_6(t). A possible combination is a_1=P, a_2=2P, a_3=3P, a 4=4P, a_5=5P, a_6=6P for some P so that the N1 graphs are spaced apart (e.g. with corresponding x-axis at a distance of P apart). Another possible combination is a_1=a_2=a_3=a_4=a_5=a_6, in which case the hybrid presentation may degenerate into same-graph presentation.

In another embodiment, the analytics may be 2-dimensional and the TSA may be presented as a curve in a 3-dimensional space (with x-axis being time, and y- and z-axes being the analytics) instead of a curve in 2-dimensional space (with x-axis being time and y-axis being the analytics). The N1 curves in 3-D space corresponding to the N1 TSA may be stacked/placed in parallel. The time axis may be divided in the M partitions. In each partition, only one of the N1 curves may be selected/displayed, while the other (N1-1) curves are not displayed. In yet another embodiment, the analytics may be K-dimensional and the TSA may be presented as a curve in K-dimensional space. The N1 curves corresponding to the N1 TSA may be spaced apart. The time axis may be divided in the M partitions. In each partition, only one of the N1 curves may be selected/displayed/animated/ highlighted, while the other (N1-1) curves may not be selected/displayed/animated/ highlighted.

The time-stamped data may be presented in a graphical user interface (GUI). The GUI may have a button which when clicked triggers a new page to show the combined curve, e.g. combined curve of (I1), (12), (13), (14), (15) and (16). The time scale (or time period) may be user selectable (e.g. 7-day, 24-hour period, 12-hour period, 8-hour period, 1-hour, 30-minute, 15-minute, 5-minute, 1-minute). The user may click on a section of the combined curve corresponding to a particular time partition with a particular selected curve. The click may cause a new page to appear showing the selected curve. In the above example, when the user clicks on the combined graph where (12) NREM sleep is selected, the new page may show the selected curve, (12) NREM sleep. Alternatively, the new page may show the selected curve, (12) NREM sleep, together with some related curves such as (I1) REM sleep, or (13) AWAKE, because together (I1), (12) and (13) are sub-states of the SLEEP state. In this case, the combined curve of (I1), (12) and (13) may be display. Alternatively, (I1), (12) and (13) may be displayed using the traditional same-graph presentation, or the traditional separate-graph presentation. While the combined curve (e.g. of (I1), (12), (13), (14), (I5) and/or (16)) is shown, the user may select a subset of the curves, for example, (I1), (12) and (13). This may cause a new page to appear showing a combined curve of (I1), (12) and (13). Alternatively, the new page may show (I1), (12) and (13) using the same-graph presentation, or the separate-graph presentation.

The GUI may show/display analytics such as (a) time for bed, (b) wake up time, (c) total time of sleep, (d) number of awakening during main sleep, (e) sleep score of main sleep, (f) number of times not at home or in room, (g) number of bathroom visits, (h) activity time duration, (i) no-activity time duration, (j) bathroom time duration. The analytics may be computed for a user-selectable time scale (e.g. 8 hours, 12 hours, 24 hours, 3 days, 7 days, 14 days, 1 month, 3 months, 6 months, 1 year). The instantaneous analytics may be displayed. An emoticon (or some graphics showing good versus bad) may be displayed when the analytics is average, above average or below average. A history of the analytics may be displayed for a time period (e.g. past week) at the user-selectable time scale. For example, the number of bath room visits may be displayed for a week, or a month, or a year to show long term trend and any anomaly.

In another view of GUI, sleeping states may be displayed for a time window (e.g. 7 days). For each day, the sleeping may be represented by a colored bar. Period of NO-SLEEP, SLEEP, REM, NREM and AWAKE may be represented by different colors. For example, NO-SLEEP may be transparent, AWAKE may have a light color, NREM may have a darker color, and REM may have a darkest color. The time window may be changed by the user (e.g. previous 7 day, previous 7 days, next 7 days, next 7 days, etc.). The GUI may have a button which when clicked causes a new page to appear showing a monthly view, or a weekly view, or a daily view, or an hourly view, or a timed view.

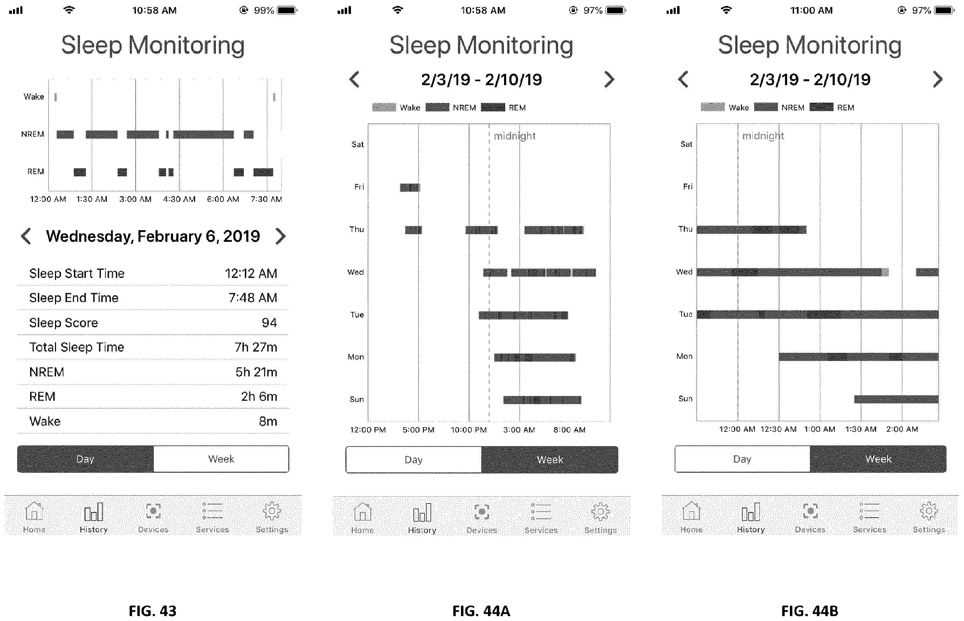

FIG. 43 illustrates an exemplary day view showing separate instances of sleep, according to some embodiments of the present teaching. A user can toggle back and forth using the arrows to view the previous or next days' sleep (this also includes naps - there can be more than one sleep in a day). On the top, a hypnogram is displayed. These are the different stages of sleep over the course of a night. On the bottom key sleep statistics are displayed. Sleep Score is calculated using some proposed custom equation that takes into account total sleep time and the time in each stage. It is to give users a holistic view of how they slept.

FIG. 44A and FIG. 44B illustrate exemplary weekly views showing a 24 hour scale, according to some embodiments of the present teaching. Sleep of each day(s) is displayed horizontally. The user can view sleep start time, sleep end time, and the different stages throughout the night, represented with different shades of green (see legend). The user can zoom-in on chart to get a better view of these sleep stages. As shown, the X-axis changes to a smaller scale.

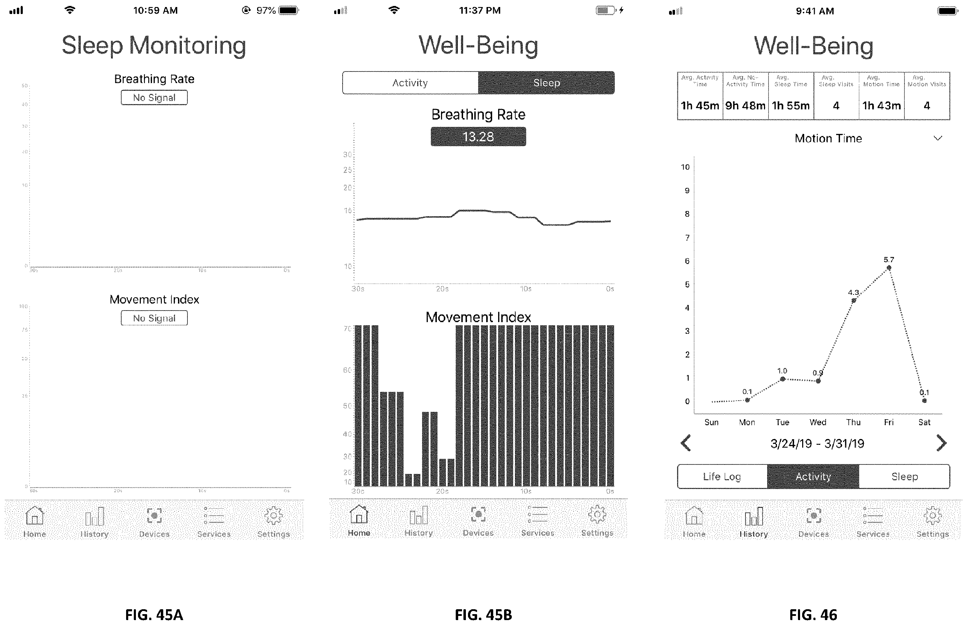

FIG. 45A and FIG. 45B illustrate exemplary home views showing real-time breathing rate and movement index, according to some embodiments of the present teaching. Breathing rate is in breaths per minute, which will appear as a line that slides horizontally and is constantly changing. Movement index shows a rough picture of a sleeping person's motion. The point is for it to show large movements (such as tossing and turning) that would cause the breathing rate to drop off. This can enable the user to know that the sleeping person has not actually stopped breathing. There may be just motion that disrupts the breathing signal. Movement index will appear as vertical bars.

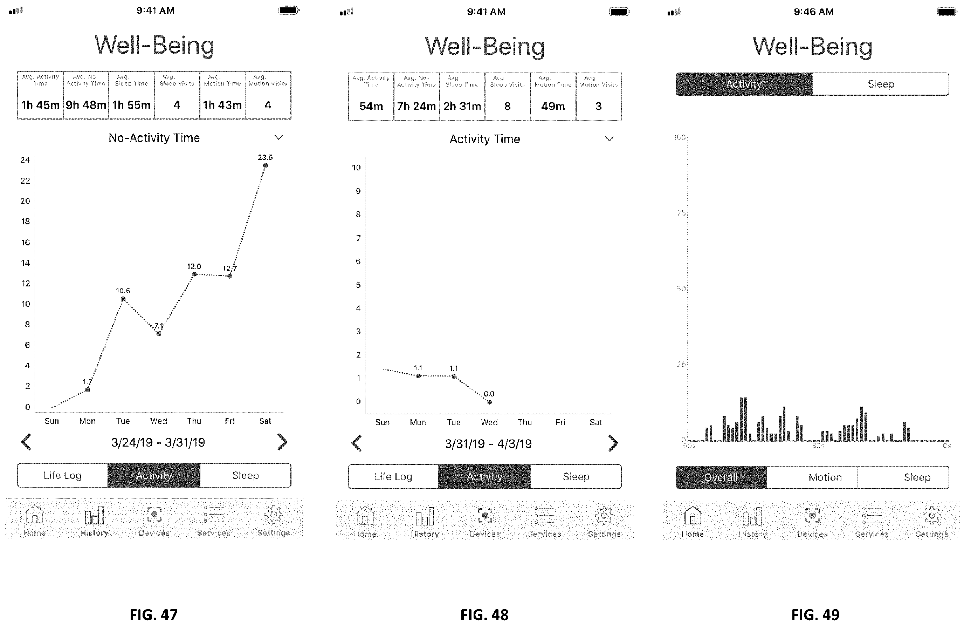

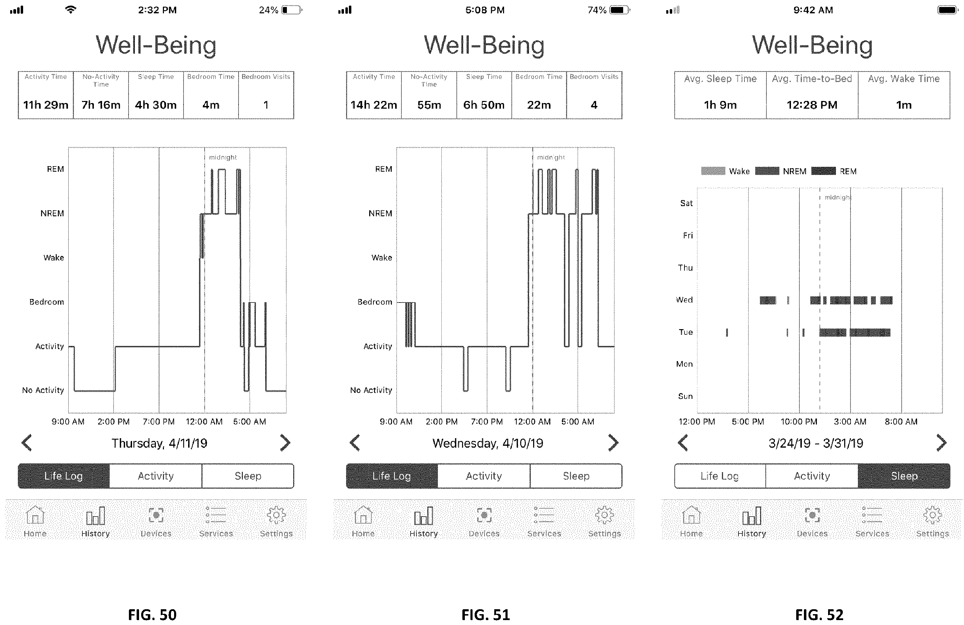

FIGs. 46-52 illustrate more exemplary views of lifelog display, according to some embodiments of the present teaching. In one embodiment, in each of FIGs. 43-52, the elements on each display may be associated with each other. For example, the hypnogram shown in FIG. 43 is for sleep monitoring data within a day. But once the user clicks on the "week" button at the bottom of the display in FIG. 43, the hypnogram will automatically be changed to show sleep monitoring data within a week. In one embodiment, after receiving an input from a user via a GUI displayed in any of FIGs. 43-52, the system performs data processing based on the input and generates updated data for display via the GUI. For example, after the user clicks the "Services" button at the bottom of FIG. 52, and selects one or more services, the system may associate the user's sleep data and/or other health or life related data to these services. As such, the system can present the user with these services (e.g. automatic 911 calling, reminder for sleeping, getting up, running, taking medicine, etc.) based on the user's sleep and/or life data logs. In addition, the system may collect, via the Type 1 and Type 2 devices described above, more life data of the user related to these services to improve user experience of these services. The data collection can be periodically based on a predetermined period, and/or dynamically in response to a user's input.

The following numbered clauses provide additional implementation examples.

Clause D1: A method/system/software/device of the presentation system, comprising: determining more than one time series of analytics (TSA) based on a processor, a memory communicatively coupled with the processor and a set of instructions stored in the memory; determining a common time axis for the more than one TSA; presenting the more than one TSA synchronously and jointly in a hybrid manner based on the common time axis.

Clause D2: The method/system/software/device of the presentation system of Clause D1: wherein a first TSA is at least one of: dependent, independent, synchronous, and asynchronous, with respect to a second TSA.

Clause D3: The method/system/software/device of the presentation system of Clause D1: wherein sampling of a first TSA is at least one of: dependent, independent, synchronous, and asynchronous, with respect to sampling of a second TSA.

Clause D4: The method/system/software/device of the presentation system of Clause D1: wherein a sampling attribute of a first TSA is at least one of: the same as, similar to, and different from, the sampling attribute of a second TSA; wherein the attribute comprises at least one of: time, frequency, period, interval, timing, time lag, time stamp, regularity, repetitiveness, variability, impulsiveness, pause, lapse, time-out, duration, source, type, sensor, memory, size, buffering, storage, mechanism, carrier frequency, carrier bandwidth, carrier band, modulation, precision, dynamic range, representation, fixed point, floating point, little endian, big endian, encapsulation, coding, encryption, scrambling, filtering, transformation, preprocessing, processing, postprocessing, noise floor, denoising, uncertainty, environment control, sensing condition, sensing setting, background attribute, dependence, interdependence, co-dependence, triggering, priority, instantaneous behavior, short-term behavior, long-term behavior, and another sensing attribute.

Clause D5: The method/system/software/device of the presentation system of Clause D1, further comprising: wherein an analytics comprises at least one of: scalar, vector, matrix, n-tuple, collection, set, subset, element, group, collection, mixture, Boolean, label, description, alphanumeric quantity, labeled quantity, time quantity, frequency quantity, statistical quantity, event quantity, sliding quantity, time-stamped quantity, processed quantity, attribute, motion intensity index, motion statistics, TRRS, presence/absence, approaching, receding, motion sequence, motion indicator, motion direction, security information, safety information, intrusion, alarm, alert, location, localization, distance, speed, acceleration, angle, angular speed, angular acceleration, timing, duration, time period, periodic motion analytic, frequency, period, transform, function/transformation of another analytic, distance measure of another two analytics, regularity, dependence, transient measure, manifestation, revelation, sign, vital sign, breathing rate, heart rate, impact, change, deformation, hand signal, gesture signal, health symptom, duration, count, classification of motion, health condition, biometric, sleep parameter, sleep score, sleep duration, sleep timing, sleep interruption, in-sleep, non-sleep, sleep stage, rapid-eye-movement (REM), non-REM (NREM), awake, detection/recognition/verification/tracking/monitoring/tracking/ counting/ locationing/ localization/navigation/guidance/occurrence/co-occurrence/relationship/filtering/processing/preprocessing/ postprocessing/correction/ activation/accessing/parameter/characteristics/feature/representation/statistics/state/ status/stage/condition/situation/indicator/ transition/change/timing/ classification/information of, or deduction/inference/observation/ summarization/decision/conclusion with respect to, at least one of: intruder, people, user, pet, human, child, older adult, patient, intruder, pet, animal, object, material, tool, machine, device, car, defect, fault, motion, movement, motion sequence, event, presence, proximity, activity, daily activity, behavior, movement, phenomenon, history, trend, variation, change, regularity, irregularity, repetitiveness, periodic motion, repeated motion, stationary motion, cyclo-stationary motion, regular motion, breathing, heartbeat, vital sign, gait, motion/feature/cycle/ characteristics of body parts/hand/elbow/arm/leg/foot/ limbs/head/waist/ wrist/eye during walking/running/exercise/locomotion/activity/man-machine interaction, transient motion, impulsive motion, sudden motion, fall-down, danger, life threat, user-interface, gesture, hand sign, handwriting, keystroke, facial expression, facial feature, emotion, body feature, body language, dancing movement, rhythmic movement, periodic motion, channel information (CI), channel state information (CSI), channel impulse response (CIR), channel frequency response (CFR), signal strength, angle-of-arrival (AoA), time-of-arrival (ToA), beamforming information, spectrum, derived analytics based on CI, and another analytics.

Clause D6: The method/system/software/device of the presentation system of Clause D1, further comprising: computing at least one hybrid, synchronous, and joint (HSJ) presentation based on the more than one TSA and the common time axis, wherein HSJ presentation comprises at least one of: a same-graph presentation, a separate-graph presentation and a hybrid presentation; presenting the at least one HSJ presentation on a device.

Clause D7: The method/system/software/device of the presentation system of Clause D6, further comprising: partitioning the common time axis into a number of time segments, wherein the HSJ presentation is at least one of: a graphical representation, a figure, and a plot, of the TSHA, wherein each time segment of the TSHA in the HSJ presentation is associated with at least one of: a line, line type, line color, line width, line attribute, area, region, shading color, shading type, shading texture, shading attribute, boundary, boundary type, boundary color, boundary width, boundary attribute, surface, surface color, surface type, surface texture, surface shading, surface attribute, animation, flashing, fade-in, fade-out, transition effect, label, symbol, graphical effect, voice, volume, user-interface setting, music, sound effect, and another presentation attribute.

Clause D8: The method/system/software/device of the presentation system of Clause D7, further comprising: wherein the number of time segments are consecutive and disjoint.

Clause D9: The method/system/software/device of the presentation system of Clause D6, further comprising: computing a time series of hybrid analytics (TSHA) based on at least one of: the more than one TSA, and the common time axis, wherein each hybrid analytics is associated with a time stamp; generating a HSJ presentation based on the TSHA.

Clause D10: The method/system/software/device of the presentation system of Clause D9, further comprising: wherein the HSJ presentation is at least one of: a graphical representation, an animation, a figure and a plot, of the TSHA.

Clause D11: The method/system/software/device of the presentation system of Clause D10, further comprising: storing at least one of: the more than one TSA, the TSHA, and the HSJ presentation, and the TSHA; communicating at least one of: the more than one TSA, the TSHA, and the HSJ presentation to the device. The TSHA may be indicator function of respective time segments (being constant in the respective time segments and taking on value (analytics ID) unique to the TSA.

Clause D12: The method/system/software/device of the presentation system of Clause D9, further comprising: partitioning the common time axis into a number of time segments; for each of the number of time segments: associating the time segment with one of the more than one TSA, and constructing the time segment of the TSHA based on the association.

Clause D13: The method/system/software/device of the presentation system of Clause D12: wherein the number of time segments are consecutive and disjoint.

Clause D14: The method/system/software/device of the presentation system of Clause D12, further comprising: associating each TSA with a unique analytics ID, each analytics ID being a real number; for each of the number of time segments: assigning at least one hybrid analytics of the TSHA in the time segment to be the analytics ID associated with the time segment.

Clause D15: The method/system/software/device of the presentation system of Clause D14: wherein there are N1 TSA; wherein the unique analytics ID is one of N1 consecutive integers.

Clause D16: The method/system/software/device of the presentation system of Clause D14: wherein there are N1 TSA; wherein the unique analytics ID is one of N1 equally spaced integers. The TSHA may be individual TSA restricted to respective time segments.

Clause D17: The method/system/software/device of the presentation system of Clause D9, further comprising: partitioning the common time axis into a number of time segments; for each of the number of time segments: associating each respective time segment with one of the more than one TSA, and constructing the respective time segment of the TSHA based on the respective time segment of the associated TSA.

Clause D18: The method/system/software/device of the presentation system of Clause D17, further comprising: for each of the number of time segments: constructing hybrid analytics in the respective time segment of the TSHA by copying analytics from the respective time segment of the associated TSA.

Clause D19: The method/system/software/device of the presentation system of Clause D6, further comprising: computing more than one graphs using the common time axis, each associated with a TSA; synchronizing the more than one graphs by restricting the graphs to a common time window and a common time scale such that they have a similar width; stacking the more than one synchronized graphs such that the time axes of the graphs are parallel and aligned; generating a HSJ by merging (or joining) the more than one stacked synchronized graphs.

Clause D20: The method/system/software/device of the presentation system of Clause D19, further comprising: scaling the TSA associated with each graph such that all stacked synchronized graphs have a similar height.

Clause D21: The method/system/software/device of the presentation system of Clause D20, further comprising: partitioning the common axis into a number of time segments; associating each time segment with one of the more than one TSA; constructing a highlighted graph by copying, for each respective time segment, respective stacked synchronized graph associated with the respective time segment; generating the HSJ by presenting the highlighted graph in a dominant way and the stacked synchronized graphs in a subservient way.

Clause D22: The method/system/software/device of the presentation system of Clause D21, further comprising: wherein each of: the highlighted graph and the stacked synchronized graphs, in a time segment is associated respectively with at least one of: a line, line type, line color, line width, line attribute, area, region, shading color, shading type, shading texture, shading attribute, boundary, boundary type, boundary color, boundary width, boundary attribute, surface, surface color, surface type, surface texture, surface shading, surface attribute, animation, flashing, fade-in, fade-out, transition effect, label, symbol, graphical effect, voice, volume, user-interface setting, music, sound effect, and another presentation attribute.

Clause D23: The method/system/software/device of the presentation system of Clause D22, further comprising: wherein a segment of the highlighted graph is at least one of: connected, and not connected, with a neighboring segment of the highlighted graph; wherein the connection, if any, is associated with a presentation attribute.

Clause D24: The method/system/software/device of the presentation system of Clause D21, further comprising: stacking the more than one synchronized graphs in a user-defined order.

Clause D25: The method/system/software/device of the presentation system of Clause D21, further comprising: stacking a subset of the more than one synchronized graphs such that the time axes of the graphs are parallel and aligned; generating another HSJ by merging (or joining) the subset of more than one stacked synchronized graphs.

Clause D26: The method/system/software/device of the presentation system of Clause D21, further comprising: generating another HSJ presentation based on at least one of: another common time window, another time scale, another width, another scaling, another height, a filtering of a TSA, a processing of a TSA, a resampling of a TSA, another TSA, another graph.

Clause D27: The method/system/software/device of the presentation system of Clause D6, further comprising: changing the HSJ presentation on the device to another HSJ presentation based on at least one of: a key-press, a user-selection, a device user-interface, a user command, a voice command, a user request, a plan, an animation sequence, a change, a warning, and a server command.

Clause D28: The method/system/software/device of the presentation system of Clause D1, comprising: wherein a TSA is computed based on a time series of channel information (TSCI) of a wireless multipath channel; wherein the TSCI is extracted from a wireless signal transmitted from a Type 1 heterogeneous wireless device to a Type 2 heterogeneous wireless device through the wireless multipath channel in a venue.

Clause D29: The method/system/software/device of the presentation system of Clause D28, comprising: wherein the wireless multipath channel is impacted by a motion of an object in the venue; wherein the TSA is associated with a monitoring task associated the motion of the object.

Clause D30: The method/system/software/device of the presentation system of Clause D29, comprising: monitoring the motion of the object in the venue; wherein the presentation of the more than one TSA synchronously and jointly in the hybrid manner is associated with the monitoring of the motion of the object in the venue.

Clause D31: The method/system/software/device of the presentation system of Clause D30, comprising: wherein the monitoring task comprises at least one of: detection/recognition/verification/tracking/monitoring/tracking/counting/locationing/ localization/ navigation/guidance/occurrence/co-occurrence/relationship/filtering/processing/pre-processing/postprocessing/correction/
activation/accessing/parameter/characteristics/feature/representation/statistics/state/status/stage/condition/situation/indicator/ transition/change/timing/ classification/information of, or deduction/inference/observation/summarization/decision/conclusion with respect to, at least one of: intruder, people, user, pet, human, child, older adult, patient, intruder, pet, animal, object, material, tool, machine, device, car, defect, fault, motion, movement, motion sequence, event, presence, proximity, activity, daily activity, behavior, movement, phenomenon, history, trend, variation, change, regularity, irregularity, repetitiveness, periodic motion, repeated motion, stationary motion, cyclo-stationary motion, regular motion, breathing, heartbeat, vital sign, gait, motion/feature/cycle/ characteristics of body parts/hand/elbow/arm/leg/foot/ limbs/head/waist/ wrist/eye during walking/running/exercise/locomotion/activity/man-machine interaction, transient motion, impulsive motion, sudden motion, fall-down, danger, life threat, user-interface, gesture, hand sign, handwriting, keystroke, facial expression, facial feature, emotion, body feature, body language, dancing movement, rhythmic movement, periodic motion, health, well-being, health condition, sleep, sleep stage, biometric, security, safety, intrusion, event, suspicious event, suspicious motion, alarm, alert, siren, location, distance, speed, acceleration, angle, angular speed, angular acceleration, locationing, and map, energy management, power transfer, wireless power transfer, geometry estimation, map learning, machine learning, machine learning, supervised learning, unsupervised learning, semi-supervised learning, clustering, feature extraction, featuring training, principal component analysis, eigen-decomposition, frequency decomposition, time decomposition, time-frequency decomposition, functional decomposition, other decomposition, training, discriminative training, supervised training, unsupervised training, semi-supervised training, neural network, augmented reality, wireless communication, data communication, signal broadcasting, networking, coordination, administration, encryption, protection, cloud computing, and another monitoring task.

The features described above may be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., C, Java), including compiled or interpreted languages, and it may be deployed in any form, including as a stand-alone program or as a module, component, subroutine, a browser-based web application, or other unit suitable for use in a computing environment.

Suitable processors for the execution of a program of instructions include, e.g., both general and special purpose microprocessors, digital signal processors, and the sole processor or one of multiple processors or cores, of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

While the present teaching contains many specific implementation details, these should not be construed as limitations on the scope of the present teaching or of what may be claimed, but rather as descriptions of features specific to particular embodiments of the present teaching. Certain features that are described in this specification in the context of separate embodiments may also be implemented in combination in a single embodiment. Conversely, various features that are described in the context of a single embodiment may also be implemented in multiple embodiments separately or in any suitable sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated together in a single software product or packaged into multiple software products.

Particular embodiments of the subject matter have been described. Any combination of the features and architectures described above is intended to be within the scope of the following claims. Other embodiments are also within the scope of the following claims. In some cases, the actions recited in the claims may be performed in a different order and still achieve desirable results. In addition, the processes depicted in the accompanying figures do not necessarily require the particular order shown, or sequential order, to achieve desirable results. In certain implementations, multitasking and parallel processing may be advantageous.

**Claims**

1. A system for rhythmic motion monitoring, comprising:

a transmitter configured for transmitting a first wireless signal towards an object in a venue through a wireless multipath channel of the venue;
a receiver configured for receiving a second wireless signal through the wireless multipath channel between the transmitter and the receiver, wherein the second wireless signal differs from the first wireless signal due to the wireless multipath channel which is impacted by a rhythmic motion of the object; and
a processor configured for:

obtaining a time series of channel information (CI) of the wireless multipath channel based on the second wireless signal,
monitoring the rhythmic motion of the object based on the time series of CI (TSCI), and
triggering a response action based on a result of the monitoring.

2. The system of claim 1, wherein the processor is further configured for:

computing a time series of intermediate quantity (IQ) based on the TSCI; and
monitoring the rhythmic motion of the object based on the time series of IQ,
wherein the rhythmic motion comprises at least one of:

a walking motion, a gait, a marching motion, a pacing motion, a running motion, a galloping action, a trotting action, a body motion, a leg motion, a hand motion, a finger motion, a trunk motion, a torso motion, a head motion,
a repeated motion, a complex repeated motion, a robotic motion, a mechanic motion, a wind-induced motion, a curtain motion, a current-induced motion, a fluid motion, a vibration, an earthquake, a tremor, a shaking motion, a quivering motion, a trembling motion,
a musical motion, a dancing motion, an oscillation, a regular motion, a periodic motion, a breathing motion, a heart beat, a palpitating motion, a relaxation oscillation, an increasing motion, a decreasing motion, an expanding motion, a contracting motion, a pulsating motion, a pumping motion, a pounding motion, a thudding motion, a throbbing motion, a hammering motion,
an alternating motion, a coordinated motion, a combination of multiple repeated motion, a modulated motion, a mixed motion, a composite motion with at least one underlying rhythm, a motion coupled to another rhythmic motion of another object, and a motion coupled to a rhythm,

wherein the IQ comprises at least one of:

a time stamp, a starting time, an ending time, a time code, a timing, a time period, a time duration, a frequency, a period, a cycle, a rhythm, a pace, a count, an indicator, an occurrence, a state, a set,
a distance, a displacement, a direction, a speed, a velocity, an acceleration, an angular distance, an angular speed, an angular acceleration, a change of location, a change of direction, a change of speed, a change of acceleration, a proximity, a presence, an absence, an appearance, a disappearance, a location, a statistics, a motion statistics, a breathing statistics, a distance statistics, a speed statistics, an acceleration statistics, a metric, an 1_k distance metric, an 1_0 distance metric, an 1_1 distance metric, an absolute distance metric, an 1_2 distance metric, a Euclidean distance metric, an 1_infinity distance metric, a path, a volume, a mass, a surface area, a shape, a posture, an energy,
a trend, a time sequence, a label, a tag, a class, a category, a time profile, a time quantity, a frequency quantity, a transient quantity, an incremental quantity, an instantaneous quantity, an averaged quantity, a locally averaged quantity, a filtered quantity, a quantity change, a repeating quantity,
an event, a recognized event, a recognized motion sequence, a gesture, a hand gesture, a finger gesture, a wrist gesture, an elbow gesture, an arm gesture, a shoulder gesture, a head gesture, a facial gesture, a neck gesture, a waist gesture, a leg gesture, a foot gesture,
a maximum, a minimum, a constrained maximum, a constrained minimum, a local maximum, a local minimum, a first local maximum, a first local minimum, a k-th local maximum, a k-th local minimum, an average, a weighted average, a percentile, a mean, a median, a mode, a trimmed mean, a conditional mean, a conditional statistics, an ordered statistics, a variance, a skewness, a kurtosis, a moment, a high order moment, a cumulant, a correlation, a covariance, a co-skewness, a co-kurtosis, a first order statistics, a second order statistics, a third order statistics, a high order statistics, a robust quantity, an argument associated with another quantity,

a feature of a CI, a complex component of a CI, a magnitude of the complex component, a phase of the complex component, a function of the complex component of the CI, a polynomial of the magnitude of the complex component, a square of the magnitude of the complex component, a time series of the feature of CI, an autocorrelation function of the feature of CI, and a function of another quantity.

3. The system of claim 2, wherein the processor is further configured for:

identifying a time window when the object has a stable rhythmic motion;
determining a time stamp associated with the stable rhythmic motion;
adding the time stamp to the time window when at least one of the following is satisfied:

a weighted average of the IQ in a sliding window associated with the time stamp is greater than a first threshold,
a feature of an autocorrelation function of the IQ around the time stamp is greater than a second threshold, and
another criterion associated with the time stamp;

computing at least one local characteristic of the IQ in the time window of the stable rhythmic motion, wherein the at least one local characteristic comprises at least one of: a local maximum, local minimum, zero crossing, local maximum of a derivation of the IQ, local minimum of the derivative, and zero crossing of the derivative;
segmenting the time window into at least one step segment based on time stamps associated with the at least one local characteristic of the IQ, each step segment spanning from a time associated with a local characteristic to another time associated with a next local characteristic;
identifying at least one motion cycle, each motion cycle comprising N consecutive step segments, wherein N is a positive integer; and
computing at least one motion feature based on at least one of: the IQ in the time window of the stable rhythmic motion, the at least one local characteristic of the IQ, the at least one step segment, and the at least one motion cycle.

4. The system of claim 3, wherein:

the at least one motion feature is associated with at least one of: a time stamp in the time window and the at least one motion cycle; and
the at least one motion feature comprises at least one of the following features:

a motion speed,
a mean speed being A-average of motion speed in a subwindow of the time window around the time stamp, wherein the subwindow is at least one of: the whole time window, a sliding window, at least one motion cycle, at least one step segment and another subwindow, and wherein A-average comprises at least one of: an average, a weighted average and a trimmed mean,
a mean-subtracted speed being a motion speed minus a mean speed, a maximum speed being maximum of motion speed within the subwindow, a minimum speed being minimum of motion speed within the subwindow, a speed variance being variance of motion speed within the subwindow, a speed deviation being X-percentile of sample distribution of mean-subtracted speed in the subwindow, wherein X is a number between 0 and 100,
a maximum positive speed deviation being maximum of mean-subtracted speed in the subwindow, a maximum negative speed deviation being minimum of mean-subtracted speed in the subwindow, a speed peak variance being variance of local peak (local maximum) of motion speed in the subwindow, a speed valley variance being variance of local valley (local minimum) of motion speed in the subwindow,
at least one k-th speed-ACF-peak (k-SAP) being a k-th SAP at the time stamp, wherien SAP (speed-ACF-peak) is a local peak of an autocorrelation function (ACF) of motion speed at the time stamp, wherein k is a non-negative integer, a mean k-SAP being A-average of k-SAP in the subwindow, a k-SAP variance being variance of k-SAP in the subwindow, a k-SAP-difference (k-SAPD) being difference of k-th SAP and (k+1)-th SAP at the time stamp, a mean k-SAPD being A-average of k-SAPD in the subwindow, a k-SAPD variance being variance of k-SAPD in the subwindow, a speed-ACF-peak-count (SAPC) being a count of significant SAP at the time stamp, a mean SAPC being A-average of SAPC in the subwindow, a SAPC variance being variance of SAPC in the subwindow, a SAPC-pdf being a sample probability of SAPC being k in the subwindow, a SAPC-difference (SAPCD) being a difference of the SAPC and another SAPC at an adjacent time stamp, a mean SAPCD being A-average of SAPCD in the subwindow, a SAPCD variance being variance of SAPCD in the subwindow,

a speed recurrent plot (SRP) being a 2-dimensional plot of R(i,j), wherein i and j are running time indices in the subwindow, wherein R(i,j) is a similarity score of a first vector at time i and a second vector at time j, wherein each of first and second vectors is a vector at a time t comprising at least one speed-related feature in a sliding subwindow of the time window around the time t,

a SRP feature comprising at least one of: recurrence rate, determinism, entropy, average diagonal line, and another feature, of the SRP,

a generalized recurrent plot (GRP) being a 2-dimensional plot of R(i,j), wherein i and j are running time indices in the subwindow, wherein R(i,j) is a similarity score of a first vector at time i and a second vector at time j, wherein each of first and second vectors is a vector at a time t comprising at least one motion feature in a sliding subwindow of the time window around the time t,

a GRP feature comprising at least one of: recurrence rate, determinism, entropy, average diagonal line, and another feature, of the GRP,

a TSA feature of a time-scaled ACF (TSA), the TSA being an ACF of a speed-related feature in a subwindow of the time stamp with the ACF time axis scaled such that its first peak occurs at a selected time lag,

a speed harmonic ratio associated with a motion cycle, each being a ratio of sum of amplitude of even harmonics of Fourier transform of motion speed in the motion cycle to sum of amplitude of odd harmonics of the Fourier transform, a speed harmonic feature associated with a motion cycle, being computed based on a function of even terms of a frequency transform of motion speed of the motion cycle and on the function of odd terms of the frequency transform, a generalized speed harmonic feature associated with the at least one motion cycle and computed based on a first function of even terms of a transform of speed-related features in the at least one motion cycle and a second function of odd terms of the transform, a generalized harmonic feature associated with the at least one motion cycle and computed based on a first function of even terms of a transform of motion features in the at least one motion cycle and a second function of odd terms of the transform,

a left-right-step speed symmetry feature, a function of at least one of the above features,

a motion acceleration being a derivative of a motion speed, a mean acceleration being A-average of motion acceleration in a subwindow of the time window around the time stamp, wherein the subwindow is at least one of: the whole time window, a sliding window, at least one motion cycle, at least one step segment and another subwindow, wherein A-average comprises at least one of: an average, a weighted average and a trimmed mean,

a mean-subtracted acceleration being a motion acceleration minus a mean acceleration, a maximum acceleration being maximum of motion acceleration within the subwindow, a minimum acceleration being minimum of motion acceleration within the subwindow, an acceleration variance being variance of motion acceleration within the subwindow, an acceleration deviation being X-percentile of sample distribution of mean-subtracted acceleration in the subwindow, wherein X is a number between 0 and 100,

a maximum positive acceleration deviation being maximum of mean-subtracted acceleration in the subwindow, and a maximum negative acceleration deviation being minimum of mean-subtracted acceleration in the subwindow, an acceleration peak variance being variance of local peak (local maximum) of motion acceleration in the subwindow, an acceleration valley variance being variance of local valley (local minimum) of motion acceleration in the subwindow,

at least one k-th acceleration-ACF-peak (k-AAP) being a k-th AAP at the time stamp, wherien AAP (acceleration-ACF-peak) is a local peak of an autocorrelation function (ACF) of motion acceleration at the time stamp, wherein k is a non-negative integer,

a mean k-AAP being A-average of k-AAP in the subwindow, a k-AAP variance being variance of k-AAP in the subwindow, a k-AAP-difference (k-AAPD) being difference of k-th AAP and (k+1)-th AAP at the time stamp, a mean k-AAPD being A-average of k-AAPD in the subwindow, a k-AAPD variance being variance of k-AAPD in the subwindow, an acceleration-ACF-peak-count (AAPC) being a count of significant AAP at the time stamp, a mean AAPC being A-average of AAPC in the subwindow, an AAPC variance being variance of AAPC in the subwindow, an AAPC-pdf being a sample probability of AAPC being k in the subwindow, an AAPC-difference (AAPCD) being a difference of the AAPC and another AAPC at an adjacent time stamp, a mean AAPCD being A-average of AAPCD in the subwindow, an AAPCD variance being variance of AAPCD in the subwindow,

an acceleration recurrent plot (RP) being a 2-dimensional plot of R(i,j), wherein i and j are running time indices in the subwindow, wherein R(i,j) is a similarity score of a first vector at time i and a second vector at time j, wherein each of first and second vectors is a vector at a time t comprising at least one gait-acceleration-related feature in a sliding subwindow of the time window around the time t,

an acceleration recurrent plot (RP) feature each comprising at least one of: recurrence rate, determinism, entropy, average diagonal line, and another feature, of the acceleration RP,

a TSA feature of a time-scaled ACF (TSA), the TSA being an ACF of a gait-acceleration-related feature in a subwindow of the time stamp with the ACF time axis scaled such that its first peak occurs at a selected time lag, an acceleration harmonic ratio associated with a motion cycle, being a ratio of sum of amplitude of even harmonics of Fourier transform of motion acceleration in the motion cycle to sum of amplitude of odd harmonics of the Fourier transform, an acceleration harmonic feature associated with a motion cycle, being computed based on a function of even terms of a frequency transform of motion acceleration of the motion cycle and on the function of odd terms of the frequency transform, a generalized acceleration harmonic feature associated with the at least one motion cycle and computed based on a first function of even terms of a transform of gait-acceleration-related features in the at least one motion cycle and a second function of odd terms of the transform,

a left-right-step acceleration symmetry feature, a function of at least one of the above features, and a function of another feature.

5. The system of claim 3 or claim 4, wherein:

the N consecutive step segments (N-step) have N phases;

an i-th phase segment (i-SSeg) is the i-th step segment in the N-step, i being a positive integer not greater than N;

the at least one motion feature is associated with at least one of: a time stamp in the time window, the at least one step segment (SSeg) and the at least one motion cycles; and

the at least one motion feature comprises at least one of the following features:

a motion speed, a motion acceleration being a derivative of motion speed, a step length being an integration of motion speed over a SSeg around the time stamp, a step period being duration of the SSeg, a step frequency being inversely proportion to the step period, a stepwise mean speed being A-average of motion speed in the SSeg, wherein A-average comprises at least one of: an average, a weighted average and a trimmed mean,

a stepwise max speed being maximum motion speed in the SSeg, a stepwise min speed being minimum motion speed in the SSeg, a stepwise speed variance being variance of motion speed in the SSeg, a stepwise speed deviation being X-percentile of sample distribution of mean-subtracted speed in the SSeg, wherein the mean-subtracted speed is motion speed minus a stepwise mean speed and X is a number between 0 and 100,

a stepwise mean acceleration being A-average of motion acceleration in the SSeg, a stepwise max acceleration being maximum motion acceleration in the SSeg, a stepwise min acceleration being minimum motion acceleration in the SSeg, a stepwise acceleration variance being variance of motion acceleration in the SSeg, a stepwise acceleration deviation being X-percentile of sample distribution of mean-subtracted acceleration in the SSeg, wherein the mean-subtracted acceleration is motion acceleration minus a stepwise mean acceleration,

an N-step length being an integration of motion speed over a N-step around the time stamp, N-step period being duration of the N-step, N-step frequency being inversely proportion to N-step period, N-step mean speed being A-average of motion speed in the N-step, N-step max speed being local maximum of motion speed in the N-step, N-step min speed being local minimum of motion speed in the N-step, N-step speed variance being variance of motion speed in the N-step, N-step speed deviation being X-percentile of sample distribution of mean-subtracted speed in the N-step, wherein the mean-subtracted speed is motion speed minus N-step mean speed and X is a number between 0 and 100,

an N-step mean acceleration being A-average of motion acceleration in the N-step, N-step max acceleration being local maximum of motion acceleration in the N-step, N-step min acceleration being local minimum of motion acceleration in the N-step, N-step acceleration variance being variance of motion acceleration in the N-step, N-step acceleration deviation being X-percentile of sample distribution of mean-subtracted acceleration in the N-step, wherein the mean-subtracted acceleration is motion acceleration minus N-step mean acceleration,

a step feature (SF) being at least one of: a step length, step period, step frequency, stepwise mean speed, stepwise max speed, stepwise min speed, stepwise speed variance, stepwise speed deviation, stepwise speed peak variance, stepwise speed valley variance, k-th stepwise speed-ACF-peak (k-SSAP), mean k-SSAP, k-SSAP variance, k-SSAP difference (k-SSAPD), mean k-SSAPD, k-SSAPD variance, stepwise speed-ACF-peak-count (SSAPC), mean SSAPC, SSAPC variance, SSAPC-pdf, SSAPC-difference (SSAPCD), mean SSAPCD, SSAPCD variance, stepwise speed recurrent plot (SSRP), SSRP feature, stepwise time-scaled speed ACF (STSSA) feature, stepwise speed harmonic ratio, stepwise speed harmonic feature, stepwise generalized speed harmonic feature, stepwise speed symmetry measure, stepwise mean acceleration, stepwise max acceleration, stepwise min acceleration, stepwise acceleration variance, stepwise acceleration deviation, stepwise speed peak variance, stepwise speed valley variance, k-th stepwise acceleration-ACF-peak (k-SAAP), mean k-SAAP, k-SAAP variance, k-SAAP difference (k-SAAPD), mean k-SAAPD, k-SAAPD variance, stepwise acceleration-ACF-peak-count (SAAPC), mean SAAPC, SAAPC variance, SAAPC-pdf, SAAPC-difference (SAAPCD), mean SAAPCD, SAAPCD variance, stepwise acceleration RP (SARP), SARP feature, stepwise time-scaled acceleration ACF (STSAA) feature, stepwise acceleration harmonic ratio, stepwise acceleration harmonic feature, stepwise generalized acceleration harmonic feature, stepwise symmetry measure, N-step length, N-step period, N-step frequency, N-step mean speed, N-step max speed, N-step min speed, N-step speed variance, N-step speed deviation, N-step speed peak variance, N-step speed valley variance, k-th N-step speed-ACF-peak (k-NSAP), mean k-NSAP, k-NSAP variance, k-NSAP difference (k-NSAPD), mean k-NSAPD, k-NSAPD variance, N-step speed-ACF-peak-count (NSAPC), mean NSAPC, NSAPC variance, NSAPC-pdf, NSAPC-difference (NSAPCD), mean NSAPCD, NSAPCD variance, N-step speed recurrent plot (NSRP), NSRP feature, N-step time-scaled speed ACF (NTSSA) feature, N-step speed harmonic ratio, N-step speed harmonic feature, N-step generalized speed harmonic feature, N-step speed symmetry measure, N-step mean acceleration, N-step max acceleration, N-step min acceleration, N-step acceleration variance, N-step acceleration deviation, N-step speed peak variance, N-step speed valley variance, k-th N-step acceleration-ACF-peak (k-NAAP), mean k-NAAP, k-NAAP variance, k-NAAP difference (k-NAAPD), mean k-NAAPD, k-NAAPD variance, N-step acceleration-ACF-peak-count (NAAPC), mean NAAPC, NAAPC variance, NAAPC-pdf, NAAPC-difference (NAAPCD), mean NAAPCD, NAAPCD variance, N-step acceleration RP (NARP), NARP feature, N-step time-scaled acceleration ACF (NTSAA) feature, N-step acceleration harmonic ratio, N-step acceleration harmonic feature, N-step generalized acceleration harmonic feature, N-step symmetry measure, a function of at least one of the above statistics, a function of another statistics, and a function of another SF, wherein an i-SF is a stepwise SF associated with an i-SSeg,

a mean SF being A-average of the SF in a subwindow of the time window around the time stamp, wherein the subwindow is at least one of: the whole time window, a sliding window, at least one motion cycle, at least one SSeg and another subwindow,

an odd mean SF being A-average of the SF of odd SSegs in the subwindow, an even mean SF being A-average of the SF of even SSegs in the subwindow, a max SF being maximum of the SF in the subwindow, an odd max SF being maximum of the SF of odd SSegs in the subwindow, an even max SF being maximum of the SF of even SSegs in the subwindow, a min SF being minimum of the SF in the subwindow, an odd min SF being minimum of the SF of odd SSegs in the subwindow, an even min SF being minimum of the SF of even SSegs in the subwindow, a SF variance being variance of SF in the subwindow, an odd SF variance being variance of SF of odd SSegs in the subwindow, an even SF variance being variance of SF of even SSegs in the subwindow, a SF deviation being X-percentile of sample distribution of mean-subtracted SF in the subwindow, wherein the mean-subtracted SF is SF minus mean SF, an odd SF deviation being X-percentile of sample distribution of mean-subtracted SF of odd SSegs in the subwindow, an even SF deviation being X-percentile of sample distribution of mean-subtracted SF of even SSegs in the subwindow, a mean i-SF being A-average of the SF of i-SSeg (i-SF) in the subwindow, a max SF being maximum of the SF in the subwindow, a max i-SF being maximum of the i-SF in the subwindow, a min SF being minimum of the SF in the subwindow, a min i-SF being minimum of the i-SF in the subwindow, an SF variance being variance of SF in the subwindow, an i-SF variance being variance of i-SF in the subwindow, an SF deviation being X-percentile of sample distribution of mean-subtracted SF in the subwindow, wherein the mean-subtracted SF is SF minus mean SF, an i-SF deviation being X-percentile of sample distribution of mean-subtracted i-SF in the subwindow, wherein the mean-subtracted i-SF is i-SF minus mean i-SF,

SF statistics being at least one of: mean SF, max SF, min SF, SF variance, SF deviation, and another statistics of SF,

odd statistics of SF being at least one of: odd mean SF, odd max SF, odd min SF, odd SF variance, odd SF deviation, and another odd statistics of SF,

even statistics of SF being at least one of: even mean SF, even max SF, even min SF, even SF variance, even SF deviation, and another even statistics of SF,

i-SF statistics being at least one of: mean i-SF, max i-SF, min i-SF, i-SF variance, i-SF deviation, and another statistics of i-SF,

a left SF, right SF, front left SF, front right SF, back left SF, back right SF, wavefront SF, odd wavefront SF, even wavefront SF, a ratio of even statistics of the SF and odd statistics of the SF, a difference of even statistics of the SF and odd statistics of the SF, a similarity measure of even statistics of the SF and odd statistics of the SF, a function of at least one of: statistics of SF, odd statistics of SF, and even statistics of SF, a ratio of a function of even statistics of the SF and a function of odd statistics of the SF, a first function of a second function of even statistics of the SF and a third function of odd statistics of the SF,

a ratio of a SF statistics and another SF statistics, a ratio of an i-SF statistics and another i-SF statistics with same i, a ratio of an i-SF statistics and another i-SF statistics with different i, a ratio of an i-SF statistics and the same i-SF statistics with different i, a ratio of a SF statistics and an i-SF statistics, a difference of a SF statistics and another SF statistics, a difference of an i-SF statistics and another i-SF statistics with same i, a difference of an i-SF statistics and another i-SF statistics with different i, a difference of an i-SF statistics and the same i-SF statistics with different i, a difference of a SF statistics and an i-SF statistics, a similarity measure of a SF statistics and another SF statistics, a similarity measure of an i-SF statistics and another i-SF statistics with same i, a similarity measure of an i-SF statistics and another i-SF statistics with different i, a similarity measure of an i-SF statistics and the same i-SF statistics with different i, a similarity measure of a SF statistics and an i-SF statistics, a function of at least one of: at least one SF statistics, and at least one i-SF statistics,

a ratio of a function of a i-SF statistics and another function of another i-SF statistics with same i, a ratio of a function of a i-SF statistics and another function of another i-SF statistics with different i, a ratio of a function of a i-SF statistics and another function of the same i-SF statistics with different i,

a composite function of: a function of a i-SF statistics and another function of another i-SF statistics with same i, a composite function of: a function of a i-SF statistics and another function of another i-SF statistics with different i, a composite function of: a function of a i-SF statistics and another function of the same i-SF statistics with different i, and another statistics of at least one of: at least one SF, and at least one i-SF.

6. The system according to any of claims 3 to 5, wherein:

the at least one motion feature is associated with at least one of: a time stamp in the time window and at least one motion cycle; and
the at least one motion feature comprises at least one of the following stride-related features:

a motion speed, a motion acceleration being a derivative of motion speed, a stride length being an integration of motion speed over a motion cycle around the time stamp, a stride period being duration of the motion cycle, a stride frequency being inversely proportion to the stride period, a stride-wise mean speed being A-average of motion speed in the motion cycle, wherein A-average comprises at least one of: an average, a weighted average and a trimmed mean,
a stride-wise max speed being maximum motion speed in the motion cycle, a stride-wise min speed being minimum motion speed in the motion cycle, a stride-wise speed variance being variance of motion speed in the motion cycle, a stride-wise speed deviation being X-percentile of sample distribution of mean-subtracted speed in the motion cycle, wherein the mean-subtracted speed is motion speed minus a stride-wise mean speed and X is a number between 0 and 100,
a stride-wise mean acceleration being A-average of motion acceleration in the motion cycle, a stride-wise max acceleration being maximum motion acceleration in the motion cycle, a stride-wise min acceleration being minimum motion acceleration in the motion cycle, a stride-wise acceleration variance being variance of motion acceleration in the motion cycle, a stride-wise acceleration deviation being X-percentile of sample distribution of mean-subtracted acceleration in the motion cycle, wherein the mean-subtracted acceleration is motion acceleration minus a stride-wise mean acceleration,
a stride feature (SF) being at least one of: a stride length, stride period, stride frequency, stride-wise mean speed, stride-wise max speed, stride-wise min speed, stride-wise speed variance, stride-wise speed deviation, stride-wise speed peak variance, stride-wise speed valley variance, k-th stride-wise speed-ACF-peak (k-SSAP), mean k-SSAP, k-SSAP variance, k-SSAP difference (k-SSAPD), mean k-SSAPD, k-SSAPD variance, stride-wise speed-ACF-peak-count (SSAPC), mean SSAPC, SSAPC variance, SSAPC-pdf, SSAPC-difference (SSAPCD), mean SSAPCD, SSAPCD variance, stride-wise speed recurrent plot (SSRP), SSRP feature, stride-wise time-scaled speed ACF (STSSA) feature, stride-wise speed harmonic ratio, stride-wise speed harmonic feature, stride-wise generalized speed harmonic feature, stride-wise speed symmetry measure, stride-wise mean acceleration, stride-wise max acceleration, stride-wise min acceleration, stride-wise acceleration variance, stride-wise acceleration deviation, stride-wise speed peak variance, stride-wise speed valley variance, k-th stride-wise acceleration-ACF-peak (k-SAAP), mean k-SAAP, k-SAAP variance, k-SAAP difference (k-SAAPD), mean k-SAAPD, k-SAAPD variance, stride-wise acceleration-ACF-peak-count (SAAPC), mean SAAPC, SAAPC variance, SAAPC-pdf, SAAPC-difference (SAAPCD), mean SAAPCD, SAAPCD variance, stride-wise acceleration RP (SARP), SARP feature, stride-wise time-scaled acceleration ACF (STSAA) feature, stride-wise acceleration harmonic ratio, stride-wise acceleration harmonic feature, stride-wise generalized acceleration harmonic feature, stride-wise symmetry measure, a function of at least one of the above statistics, a function of another statistics, and a function of another SF,

a mean SF being A-average of the SF in a subwindow of the time window around the time stamp, wherein the subwindow is at least one of: the whole time window, a sliding window, at least one motion cycle, at least one step segments and another subwindow,

an odd mean SF being A-average of the SF of odd motion cycles in the subwindow, an even mean SF being A-average of the SF of even motion cycles in the subwindow, a max SF being maximum of the SF in the subwindow, an odd max SF being maximum of the SF of odd motion cycles in the subwindow, an even max SF being maximum of the SF of even motion cycles in the subwindow, a min SF being minimum of the SF in the subwindow, an odd min SF being minimum of the SF of odd motion cycles in the subwindow, an even min SF being minimum of the SF of even motion cycles in the subwindow, a SF variance being variance of SF in the subwindow, an odd SF variance being variance of SF of odd motion cycles in the subwindow, an even SF variance being variance of SF of even motion cycles in the subwindow, a SF deviation being X-percentile of sample distribution of mean-subtracted SF in the subwindow, wherein the mean-subtracted SF is SF minus mean SF, an odd SF deviation being X-percentile of sample distribution of mean-subtracted SF of odd motion cycles in the subwindow, an even SF deviation being X-percentile of sample distribution of mean-subtracted SF of even motion cycles in the subwindow,

statistics of SF being at least one of: mean SF, max SF, min SF, SF variance, SF deviation, and another statistics of SF,

odd statistics of SF being at least one of: odd mean SF, odd max SF, odd min SF, odd SF variance, odd SF deviation, and another odd statistics of SF,

even statistics of SF being at least one of: even mean SF, even max SF, even min SF, even SF variance, even SF deviation, and another even statistics of SF,

a left SF, right SF, front left SF, front right SF, back left SF, back right SF, wavefront SF, odd wavefront SF, even wavefront SF, a ratio of even statistics of SF and odd statistics of SF, a difference of even statistics of SF and odd statistics of SF, a similarity measure of even statistics of SF and odd statistics of SF, a function of at least one of: statistics of SF, odd statistics of SF, and even statistics of SF, a ratio of a function of even statistics of SF and a function of odd statistics of SF, a first function of: a second function of even statistics of SF and a third function of odd statistics of the SF,

and another statistics of the SF.

7. The system according to any of claims 3 to 6, wherein the processor is further configured for:
   skipping the time window of the stable rhythmic motion when a quantity of local characteristics of the IQ is less than a threshold.

8. The system according to any of claims 3 to 7, wherein the processor is further configured for:

   communicating the at least one motion feature to a server or a user device; and
   providing a presentation on the user device based on the at least one motion feature.

9. The system according to any of claims 3 to 8, further comprising:

   an additional transmitter configured for transmitting a third wireless signal through an additional wireless multipath channel; and
   an additional receiver configured for receiving a fourth wireless signal through the additional wireless multipath channel, wherein the fourth wireless signal differs from the third wireless signal due to the additional wireless multipath channel which is impacted by the rhythmic motion of the object in the venue,
   wherein the processor is further configured for:

   obtaining an additional TSCI of the additional wireless multipath channel based on the fourth wireless signal;
   computing an additional time series of IQ based on the additional TSCI; and
   monitoring the rhythmic motion jointly based on the time series of IQ and the additional time series of IQ.

10. The system of claim 6, wherein the processor is further configured for:

applying a smoothing filter to the IQ to compute a time series of smoothed IQ in the time window of the stable rhythmic motion of the object; and
segmenting the time window into at least one step segment based on the smoothed IQ.

**11.** An apparatus for rhythmic motion monitoring in a venue where a transmitter and a receiver are located, comprising:

at least one of the transmitter and the receiver, wherein:

the transmitter is configured for transmitting a first wireless signal through a wireless multipath channel of the venue, and
the receiver is configured for receiving a second wireless signal through the wireless multipath channel, wherein the second wireless signal differs from the first wireless signal due to the wireless multipath channel which is impacted by a rhythmic motion of an object in the venue; and

a processor configured for:

obtaining a time series of channel information (CI) of the wireless multipath channel based on the second wireless signal,
monitoring the rhythmic motion of the object based on the time series of CI (TSCI), and
triggering a response action based on a result of the monitoring.

**12.** The apparatus of claim 11, wherein the processor is further configured for:

computing a time series of intermediate quantity (IQ) based on the TSCI;
identifying a time window when the object has a stable rhythmic motion;
determining a time stamp associated with the stable rhythmic motion; and
adding the time stamp to the time window when at least one of the following is satisfied:

a weighted average of the IQ in a sliding window associated with the time stamp is greater than a first threshold,
a feature of an autocorrelation function of the IQ around the time stamp is greater than a second threshold, and
another criterion associated with the time stamp.

**13.** A method, implemented by a processor, a memory communicatively coupled with the processor, and a set of instructions stored in the memory to be executed by the processor, comprising:

obtaining a time series of channel information (CI) of a wireless multipath channel of a venue, wherein:

a transmitter transmits a first wireless signal towards an object in a venue through the wireless multipath channel of the venue,
a receiver receives a second wireless signal through the wireless multipath channel and computes the time series of CI (TSCI) of the wireless multipath channel based on the second wireless signal, and
the second wireless signal differs from the first wireless signal due to the wireless multipath channel which is impacted by a rhythmic motion of the object in the venue;

monitoring the rhythmic motion of the object based on the TSCI, and
triggering a response action based on a result of the monitoring.

**14.** The method of claim 13, further comprising:

computing a time series of intermediate quantity (IQ) based on the TSCI;
identifying a time window when the object has a stable rhythmic motion;
determining a time stamp associated with the stable rhythmic motion; and
adding the time stamp to the time window when at least one of the following is satisfied:

a weighted average of the IQ in a sliding window associated with the time stamp is greater than a first threshold,

a feature of an autocorrelation function of the IQ around the time stamp is greater than a second threshold, and

another criterion associated with the time stamp.

**15.** The method of claim 14, further comprising:

computing at least one local characteristic of the IQ in the time window of the stable rhythmic motion, wherein the at least one local characteristic comprises at least one of: a local maximum, local minimum, zero crossing, local maximum of a derivation of the IQ, local minimum of the derivative, and zero crossing of the derivative;

segmenting the time window into at least one step segment based on time stamps associated with the at least one local characteristic of the IQ, each step segment spanning from a time associated with a local characteristic to another time associated with a next local characteristic;

identifying at least one motion cycle, each motion cycle comprising N consecutive step segments, wherein N is a positive integer; and

computing at least one motion feature based on at least one of: the IQ in the time window of the stable rhythmic motion, the at least one local characteristic of the IQ, the at least one step segment, and the at least one motion cycle, wherein:

the at least one motion feature is associated with at least one of: a time stamp in the time window, the at least one motion cycle, and the at least one step segment, and

the at least one motion feature comprises at least one feature related to: a speed of the rhythmic motion, an acceleration being a derivative of the speed of the rhythmic motion, a stride, the at least one motion cycle, and/or the at least one step segment.

**FIG. 1A**

**FIG. 1B**

FIG. 2B

FIG. 2D

FIG. 2A

FIG. 2C

FIG. 3

FIG. 4

FIG. 5

FIG. 6

EP 3 695 783 A1

FIG. 7

FIG. 8

116

FIG. 9

FIG. 10

FIG. 11A

FIG. 11B

FIG. 12

FIG. 13

FIG. 14

FIG. 15

**FIG. 16**

Transmitting antennas A

The $m$th antenna $\mathbf{x}_m = (md/2, 0, H_B)$

Receiver

R located at
$\mathbf{r} = (r_x, r_y, 0)$

**FIG. 17**

**FIG. 18A**

**FIG. 18B**

**FIG. 19A**

FIG. 19B

FIG. 19C

**FIG. 20**

**FIG. 21**

FIG. 22

FIG. 23

FIG. 24

FIG. 25

FIG. 26A

FIG. 26B

128

New position

**r**$_n$

Station 1

$l_n$

$l_1$

**r**$_1$

Initial position

$l'_1$

**r**$_0$

$l'_n$

**r'**$_1$

Station 2

$B_2$

**r'**$_n$ New position

**FIG. 27**

New position

$\mathbf{r}_1 = (r_{x_1}, r_{y_1})$  $\mathbf{r'}_1 = (r_{x_2}, r_{y_2})$

$y$

$y'$

$l$

$d_1 = v_1 t_1$

$v$

$O$

$\alpha$

$\theta_1$

$M$

$(O_{x_0}, O_{y_0})$

Station 1 $B_1$

$\mathbf{r}_0 = (r_{x_0}, r_{y_0})$

$\mathbf{r'}_0 = (r'_{x_0}, r'_{y_0})$

$x$

$x'$

Initial position

**FIG. 28**

FIG. 29A

FIG. 29B

**FIG. 29C**

FIG. 30

FIG. 31

**FIG. 32**

3300

| | |
|---|---|
| Transmit a wireless signal by a transmitter through a wireless multipath channel | 3302 |
| Receive the signal through the channel impacted by a movement of a moving device | 3304 |
| Obtain a set of channel information (CI) of the channel based on the received signal | 3306 |
| Compute a set of similarity scores associated with many pairs of temporally adjacent CI | 3308 |
| Compute a similarity curve based on the set of similarity scores | 3310 |

| | |
|---|---|
| Match a transform of the similarity curve to a reference curve about the similar scores | 3312 |
| Compute an IQ-projection based on a characteristic point of the reference curve | 3314 |
| Compute an IQ of a current movement of the moving device based on the projection | 3316 |
| Compute a spatial-temporal information (STI) based on the IQ and/or a past IQ | 3318 |
| Track the moving device based on the STI | 3320 |

**FIG. 33**

**FIG. 34**

**FIG. 35**

**FIG. 36**

**FIG. 37**

**FIG. 38**

Missed detection by PIR

Motions Detected by PIRs
Motions Detected by WiFi

PIRx6

802.11

Days

False alarm by PIR

**FIG. 39**

PSG

Tx

Rx

Airflow

Airflow

Thoraic
Effort Belt

Abodominal
Effort Belt

ECG
EEG
EMG

O2 Saturation and Heart Rate

**FIG. 40**

FIG. 41

FIG. 42

**FIG. 43**

**FIG. 44A**

**FIG. 44B**

**Sleep Monitoring**

Breathing Rate

| No Signal |

Movement Index

| No Signal |

Home · History · Devices · Services · Settings

**FIG. 45A**

**Well-Being**

| Activity | Sleep |

Breathing Rate

| 13.28 |

Movement Index

Home · History · Devices · Services · Settings

**FIG. 45B**

**Well-Being**

| Avg. Activity Time | Avg. No-Activity Time | Avg. Sleep Time | Avg. Sleep Visits | Avg. Motion Time | Avg. Motion Visits |
|---|---|---|---|---|---|
| 1h 45m | 9h 48m | 1h 55m | 4 | 1h 43m | 4 |

Motion Time ⌄

0.1 — 1.0 — 0.9 — 4.3 — 5.7 — 0.1

Sun  Mon  Tue  Wed  Thu  Fri  Sat

< 3/24/19 - 3/31/19 >

| Life Log | Activity | Sleep |

Home · History · Devices · Services · Settings

**FIG. 46**

EP 3 695 783 A1

139

EP 3 695 783 A1

## FIG. 47

9:41 AM

# Well-Being

| Avg. Activity Time | Avg. No-Activity Time | Avg. Sleep Time | Avg. Sleep Visits | Avg. Motion Time | Avg. Motion Visits |
|---|---|---|---|---|---|
| 1h 45m | 9h 48m | 1h 55m | 4 | 1h 43m | 4 |

No-Activity Time ∨

| Life Log | Activity | Sleep |

Home | History | Devices | Services | Settings

3/24/19 - 3/31/19

## FIG. 48

9:41 AM

# Well-Being

| Avg. Activity Time | Avg. No-Activity Time | Avg. Sleep Time | Avg. Sleep Visits | Avg. Motion Time | Avg. Motion Visits |
|---|---|---|---|---|---|
| 54m | 7h 24m | 2h 31m | 8 | 49m | 3 |

Activity Time ∨

3/31/19 - 4/3/19

| Life Log | Activity | Sleep |

Home | History | Devices | Services | Settings

## FIG. 49

9:46 AM

# Well-Being

| Activity | Sleep |

| Overall | Motion | Sleep |

Home | History | Devices | Services | Settings

## Well-Being

9:42 AM

| Avg. Sleep Time | Avg. Time-to-Bed | Avg. Wake Time |
|---|---|---|
| 1h 9m | 12:28 PM | 1m |

Wake  NREM  REM

midnight

Sat
Fri
Thu
Wed
Tue
Mon
Sun

12:00 PM   5:00 PM   10:00 PM   3:00 AM   8:00 AM

3/24/19 - 3/31/19

Life Log   Activity   Sleep

Home   History   Devices   Services   Settings

**FIG. 52**

## Well-Being

5:08 PM   74%

| Activity Time | No-Activity Time | Sleep Time | Bedroom Time | Bedroom Visits |
|---|---|---|---|---|
| 14h 22m | 55m | 6h 50m | 22m | 4 |

REM
NREM
Wake
Bedroom
Activity
No Activity

midnight

9:00 AM   2:00 PM   7:00 PM   12:00 AM   5:00 AM

Wednesday, 4/10/19

Life Log   Activity   Sleep

Home   History   Devices   Services   Settings

**FIG. 51**

## Well-Being

2:32 PM   24%

| Activity Time | No-Activity Time | Sleep Time | Bedroom Time | Bedroom Visits |
|---|---|---|---|---|
| 11h 29m | 7h 16m | 4h 30m | 4m | 1 |

REM
NREM
Wake
Bedroom
Activity
No Activity

midnight

9:00 AM   2:00 PM   7:00 PM   12:00 AM   5:00 AM

Thursday, 4/11/19

Life Log   Activity   Sleep

Home   History   Devices   Services   Settings

**FIG. 50**

141

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/351775 A1 (ZHANG FENG [US] ET AL) 6 December 2018 (2018-12-06) | 1,2, 11-14 | INV. A61B5/11 |
| A | * paragraphs [0095], [0119] - [0131], [0172], [0174], [0197], [0198], [0203], [0244], [0252]; figures 1,3,10-14 * | 3-10,15 | G01S7/41 G01S13/56 G06K9/00 H04L29/08 |
| | ----- | | ADD. G01S13/66 G01S13/88 G08B13/181 |

| TECHNICAL FIELDS SEARCHED (IPC) |
|---|
| A61B |
| G06K |
| H04L |
| G01S |
| H04W |
| G08B |
| H04B |

The present search report has been drawn up for all claims

| Place of search | Date of completion of the search | Examiner |
|---|---|---|
| Munich | 1 July 2020 | Reeck, Guido |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 ...............................................................................

& : member of the same patent family, corresponding
   document

EPO FORM 1503 03.82 (P04C01)

## EP 3 695 783 A1

### ANNEX TO THE EUROPEAN SEARCH REPORT
### ON EUROPEAN PATENT APPLICATION NO.

EP 20 15 7771

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2020

| Patent document cited in search report | Publication date | Patent family member(s) | Publication date |
|---|---|---|---|
| US 2018351775 A1 | 06-12-2018 | NONE | |

EPO FORM P0459

For more details about this annex : see Official Journal of the European Patent Office, No. 12/82

143

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62806688
- US 62806694
- US 62846688
- US 62873781
- US 62900565
- US 62902357
- US 62950093
- US 79061020
- US 79062720